# EUROPEAN PATENT SPECIFICATION

(11) **EP 3 196 304 B1**
(45) Date of publication and mention of the grant of the patent: **06.10.2021**
(21) Application number: 15826531.4
(22) Date of filing: 29.07.2015
(51) Int. Cl.: C12N 15/09, C12N 1/19, C12P 21/02

(54) **METHOD FOR IMPROVED HIGH-LEVEL SECRETORY PRODUCTION OF PROTEINS**
VERFAHREN ZUR VERBESSERTEN HOCHGRADIGEN SEKRETORISCHEN PRODUKTION VON PROTEINEN
PROCÉDÉ DESTINÉ À LA PRODUCTION SÉCRÉTOIRE AMÉLIORÉE À HAUT NIVEAU DE PROTÉINES

(30) Priority: 30.07.2014 JP 2014155272
(43) Date of publication of application: 26.07.2017
(73) Proprietor: Daiichi Sankyo Company, Limited, Tokyo 103-8426 (JP)
(72) Inventor: NONAKA, Koichi, Oura-gun Gunma 370-0503 (JP); SUZUKI, Takeshi, Oura-gun Gunma 370-0503 (JP); TSUDA, Masashi, Oura-gun Gunma 370-0503 (JP); BABA, Satoshi, Tokyo 140-8710 (JP); ICHIKAWA, Kimihisa, Oura-gun Gunma 370-0503 (JP); CHIBA, Yasunori, Tsukuba-shi Ibaraki 305-8566 (JP); YOKO-O, Takehiko, Tsukuba-shi Ibaraki 305-8566 (JP); ITO, Rie, Tsukuba-shi Ibaraki 305-8566 (JP)
(74) Representative: Hoffmann Eitle
(86) International application number: PCT/JP2015/071503
(87) International publication number: WO 2016/017693

(56) References cited:
- WO-A1-03/091431
- WO-A1-2009/057813
- JP-A- S62 104 585
- JP-A- 2000 078 978
- US-A- 5 612 198
- AHMAD M ET AL: "Protein expression in Pichia pastoris: recent achievements and perspectives for heterologous protein production", APPLIED MICROBIOLOGY AND BIOTECHNOLOGY, SPRINGER, BERLIN, DE, vol. 98, no. 12, 1 June 2014 (2014-06-01), pages 5301-5317, XP002745344, ISSN: 1432-0614, DOI: 10.1007/S00253-014-5732-5 [retrieved on 2014-04-18]
- CREGG,J.: 'The Pichia System' ARCHIVE, WAYBACK MACHINE, [Online] XP055394752 Retrieved from the Internet: <URL:HTTP://WWW.PICHIA.COM/SCIENCE-CENTER/J AMES-M-CREGG-PH-D/URL:HTTPS://WEB.ARCHIVE.O RG/WEB/20140408110740/HTTP://WWW.PICHIA.COM /SCIENCE-CENTER/JAMES-M-CREGG-PH-D/> [retrieved on 2015-09-29]
- CHIRUVOLU,V. ET AL.: 'Recombinant protein production in an alcohol oxidase-defective strain of Pichia pastoris in fedbatch fermentations' ENZYME AND MICROBIAL TECHNOLOGY vol. 21, no. 4, 01 September 1997, pages 277 - 283, XP055390303 DOI: 10.1016/S0141-0229(97)00042-2
- TSCHOPP,J.F. ET AL.: 'High-Level Secretion of Glycosylated Invertase in the Methylotrophic Yeast' BIOTECHNOLOGY vol. 5, no. 12, 05 December 1987, pages 1305 - 1308, XP001313021
- None

## Description

### Technical Field

The present invention relates to a method for high-level secretory production of a protein in yeast.

### Background Art

The market for protein pharmaceuticals such as therapeutic proteins and antibody drugs is rapidly expanding due to the development of genetic engineering techniques. Animal cells such as CHO or NSO, insects such as silkworms, insect cells such as SF9, and microorganisms such as *E. coli* or yeast have been used as hosts in which protein pharmaceuticals are to be produced. In particular, yeast systems are capable of high-density culture and thus they are extensively used as systems that are capable of secretory production of useful proteins in relatively inexpensive media.

Secretory proteins pass through the translocon and enter into the endoplasmic reticulum when the amino acid regions of signal sequences at their N terminuses are recognized by signal recognition particles (SRPs). When secretory proteins pass through the translocon, the higher-order structures thereof are loosened, and the proteins are folded in the endoplasmic reticulum. While secretory protein folding is able to spontaneously occur, various molecular chaperones assist such folding. A native conformation formed in the endoplasmic reticulum is critical for secretion, and misfolded proteins cannot enter the secretory pathway located downstream. Thus, proteins having abnormal higher-order structures are disadvantageously accumulated therein. Such disturbance in modification that takes place in the endoplasmic reticulum (i.e., addition of a sugar chain or a disulfide bond) and deteriorated transportation from the endoplasmic reticulum causes "endoplasmic reticulum stress." As a means for dealing with such endoplasmic reticulum stress, a stress response referred to as "unfolded protein response (UPR)" is induced in eukaryotic cells. Transcription induction and translation regulation of UPR are responses that restore accumulated abnormal proteins. There is also a mechanism referred to as "ER-associated degradation (ERAD)" that degrades and eliminates abnormal proteins so as to maintain homeostasis in the endoplasmic reticulum. Further, molecular chaperones that loosen the aggregated proteins for the purpose of folding are known, as are molecular chaperones that assist protein folding in the endoplasmic reticulum. For example, HSP104 can perform a reaction that cannot be performed with the aid of other chaperones that cooperate with HSP70 and solubilizes proteins from the aggregates (Non-Patent Document 1).

Meanwhile, a variety of interactions, such as hydrogen bonds, electrostatic interactions, and hydrophobic interactions, occur between amino acids inside a protein steric structure. In particular, covalent bonds between sulfur atoms that are formed upon two-electron oxidation of two cysteines (which are referred to as "disulfide bonds") play very important roles in stabilizing protein steric structure because of their strong properties. In fact, many secretory proteins that are secreted extracellularly have disulfide bonds. This is presumed to be the case because of the necessity of strengthening of protein structure, so that a protein can function outside a cell, where the environment is physically and chemically more severe than that in the environment inside the cell, which is enveloped by a membrane. In the case of eukaryotic cells such as yeast cells, introduction of a disulfide bond via protein oxidative folding is carried out by the oxidative protein disulfide isomerase (PDI) in the endoplasmic reticulum (Non-Patent Document 2). PDI that is reduced via oxidization of substrate proteins is reoxidized by oxidative ERO1 localized in the vicinity of the membrane (Non-Patent Documents 3 and 4). In yeast endoplasmic reticulum, there are 5 types of PDI families (i.e., PDI1, EUG1, MPD1, MPD2, and EPS1) (Non-Patent Document 5). Among such PDI families, those that are confirmed to form an intramolecular disulfide bond with ERO1 are limited to PDI1 and MPD2. It is also reported that the efficiency of protein oxidative folding is improved with BiP/Kar2, which functions in conjunction with PDI (Non-Patent Document 6). BiP/Kar2 is also associated with induction by active HAC1 of various genes associated with the aforementioned UPR. Active HAC1 is activated by the splicing of HAC1 via the IRE1 transmembrane kinase/nuclease. IRE1 to which BiP/Kar2 is bound is dissociated when BiP/Kar2 acts on a protein having an abnormal structure in the endoplasmic reticulum, it exhibits nuclease activity through the formation of a dimmer, and it produces active HAC1 through the splicing of HAC1 (Non-Patent Documents 7 and 8). Also, Bip/Kar2 is associated with protein folding in the endoplasmic reticulum in conjunction with SCJ1 located in the endoplasmic reticulum (Non-Patent Document 9).

Thus, it has been demonstrated that various molecular chaperones are associated with the correct folding of secretory proteins. It has been reported that one or more types of genes encoding molecular chaperone proteins, such as PDI1, ERO1, or Kar2, are co-expressed in the presence of a target protein to be expressed in yeast, so as to improve the secretory productivity of a target protein having a complicated steric structure (Patent Document 1).

Even if the productivity of target protein secretion into media is improved with coexpression of genes encoding chaperone proteins, such as PDI1, ERO1, or Kar2, some target proteins may occasionally rapidly degrade in media. In particular, a protease existing in a vacuole that is known as a protein-degrading organelle of yeast is reported to be associated with secretory protein degradation (Non-Patent Document 10). Many proteolytic enzymes are present in vacuoles, such as vacuolar trehalase, aminopeptidase I, vacuolar alkaline phosphatase, and vacuolar RNase, in addition to proteinase A, proteinase B, and carboxypeptidase Y, and activity thereof is regulated such that it is exerted in vacuoles. In particular, proteinase A and proteinase B function as key proteases that activate themselves or carboxypeptidase Y, and they play key roles in a proteolytic system (Non-Patent Documents 11 and 12). It has been reported that an acidic protease (i.e., proteinase A) exerts strong activity under acidic conditions, but such activity is attenuated as pH increases (Non-Patent Document 13). Thus, a culture method in which the pH of a culture medium is adjusted so as to inhibit protease activity has been studied, although such method may affect the proliferation of host cells.

In methhylotrophic yeast, methanol metabolism is initiated upon oxidation of methanol by alcohol oxidase (AOX), the generated formaldehyde is fixed to xylose 5-phosphate with the aid of a dihydroxyacetone synthase (DAS), it is used as a cell constituent in a glycolysis system, and it is also oxidized to CO₂ with the aid of glutathione-dependent formaldehyde dehydrogenase (FLD) and formate dehydrogenase (FDH) in the cytoplasm (Non-Patent Document 14). Many gene promoters encoding enzymes associated with methanol metabolism, such as pmp20- and pmp47- promoters, have been known as gene promoters the expression of which is regulated by methanol, and examples thereof include alcohol oxidase (aoxl, aox2) promoter, dihydroxyacetone synthase (dasl) promoter, formate dehydrogenase (fdh1) promoter, and methanol oxidase (mox) promoter (Non-Patent Document 15). Promoters that regulate the expression of enzymes associated with methanol metabolism are very strong. Thus, such promoters are generally used to achieve secretory production of various target proteins in methhylotrophic yeast. In particular, an aoxl promoter of *Pichia pastoris* is known as a very strong promoter induced by methanol.

When target proteins are to be secreted and produced under the control of a promoter that regulates the expression of enzymes associated with methanol metabolism, methanol induction is considered to be necessary. Methanol is a deleterious substance classified as a Class 2 Flammable Liquid, the use thereof in an amount exceeding the designated level is regulated under the Fire Defense Law, and explosion-proof factories and facilities are required. If secretory production of target proteins equivalent to that induced to express with the aid of a large quantity of methanol can be achieved with the use of methanol in as small an amount as possible, accordingly, industrial values thereof are significant. In addition, various positive transcription factors used as methanol-metabolizing enzyme promoters of methhylotrophic yeast are used to induce the expression of methanol-metabolizing enzymes inherent to yeast, as well as the expression of target proteins that have been newly introduced. In fact, expression of various oxidases, such as D-amino acid oxidase, fructosyl amino acid oxidase, and peroxisome/acetyl spermidine oxidase, in addition to the hepatitis B surface antigen gene, has been attempted under the control of the aoxl promoter with the use of a methhylotrophic yeast *(Candida boidinii)* in which the aoxl gene inherent thereto has been disrupted, and the target protein expression level is enhanced in a strain in which the aoxl gene inherent thereto has been disrupted, compared with the original parent strain (Patent Document 2 and Non-Patent Documents 16, 17, and 18).

As described above, the use of a wide variety of methods has been demonstrated regarding high-level secretory expression of proteins in yeast. When host cells are transformed via gene introduction, gene disruption, or other means, in general, cells receive some stress. Thus, synergistic or additive effects cannot always be attained merely by employing several conventional techniques in combination. In addition, there have been no reports concerning high-level secretory expression of target proteins with the use of the various chaperones in combination with the various techniques described above.

### Prior Art Documents

### Patent Documents

[Patent Document 1] WO 2009/057813
[Patent Document 2] JP S62-104585 A (1987)

### [Non-Patent Document]

[Non-Patent Document 1] Glover JR, Lindquist S, Hsp104, Hsp70, and Hsp40: A novel chaperone system that rescues previously aggregated proteins. Cell (1998) 94:73-82 [Non-Patent Document 2] Benjamin P. Tu and Jonathan S. Weissman, Oxidative protein folding in eukaryotes: mechanisms and consequences. J. Cell Biol. (2004) 164:341-346 [Non-Patent Document 3] Mezghrani, A., Fassio, A., Benham, A., Simmen, T., Braakman, I., and Sitia, R., Manipulationof oxidative proteinfolding and PDIredox state inmammalian cells. EMBO. J.(2001) 20: 6288-6296
[Non-Patent Document 4] Frand, A. R. and C. A. Kaiser, Erolp oxidizes protein disulfide isomerase in a pathway for disulfide bond formation in the endoplasmic reticulum. Mol. Cell (1999) 4:469-477
[Non-Patent Document 5] Per Norgaard, Vibeke Westphal, Christine Tachibana, Lene Alsoe, Bjorn Holst, Jakob R. Winther, Functional Differences in Yeast Protein Disulfide Isomerases. J. Cell Biology (2001) 152(3): 553-562,
[Non-Patent Document 6] Marcus Mayer, Ursula Kies, Robert Kammermeier, and Johannes Buchner, BiP and PDI Cooperate in the Oxidative Folding of Antibodies in Vitro, J. Biol. Chem. (2000) 275(38): 29421-29425.
[Non-Patent Document 7] Cox JS., Shamu CE., Walter P., Transcriptional induction of genes encoding endoplasmic reticulum resident proteins requires a transmembrane protein kinase. Cell (1993) 73:1197-1206
[Non-Patent Document 8] Sidrauski C. and Walter P., The transmembrane kinase Irelp is a site-specific endonuclease that initiates mRNA splicing in the unfolded protein response. Cell (1997) 90(6): 1031-1039
[Non-Patent Document 9] Susana Silberstein, Gabriel Schlenstedt, Pam A. Silver, and Reid Gilmore, A Role for the DnaJ Homologue Scj1p in Protein Folding in the Yeast Endoplasmic Reticulum. J. Cell Biol. (1998) 143(4): 921-933
[Non-Patent Document 10] Morozkina EV, Marchenko AN, Keruchenko JS, Keruchenko ID, Khotchenkov VP, Popov VO, and Benevolensky SV, Proteinase B disruption is required for high level production of human mechano-growth factor in Saccharomyces cerevisiae. J. Mol. Microbiol. Biotechnol. (2010) 18(3): 188-194
[Non-Patent Document 11] H. Bart van den HAZEL, Morten C. KIELLAND-BRANDT, and Jakob R. WINTHER, Autoactivation of proteinase A initiates activation of yeast vacuolar zymogens. Eur. J. Biochem. (1992) 207: 277-283
[Non-Patent Document 12] Vicki L. Nebes and Elizabeth W. Jones, Activation of the proteinase B precursor of the yeast Saccharomyces cerevisiae by autocatalysis and by an internal sequence. J. Biol. Chem. (1991) 266(34): 22851-22857
[Non-Patent Document 13] Susanne O. SORENSEN, H. Bart VAN DEN HAZEL, Morten C. KIELLAND-BRANDT, and Jakob R. WINTHER, pH-dependent processing of yeast procarboxypeptidase Y by proteinase A in vivo and in vitro. Eur. J. Biochem. (1994) 220: 19-27
[Non-Patent Document 14] Ida J. van der Kleia, Yurimotob H, Sakaib Y, Venhuisa M, The significance of peroxisomes in methanol metabolism in methylotrophic yeast. Biochim. Biophys. Acta. (2006) 1763: 1453-1462
[Non-Patent Document 15] Yurimoto H, Komeda T, Lim CR, Nakagawa T, Kato N, Sakai Y, Regulation and evaluation of five methanol-inducible promoters in methylotrophic yeast Candida boidinii. Biochim. Biophys. Acta. (2000) 1493(1-2): 56-63
[Non-Patent Document 16] Yurimoto H, Hasegawa T, Sakai Y, Kato N, Characterization and High-level production of D-amino acid oxidase in Candida boidinii. Biosci. Biotechnol. Biochem. (2001) 65(3), 627-633
[Non-Patent Document 17] Sakai Y, Yoshida H, Yurimoto H, Yoshida N, Fukuya H, Takabe K, Kato N, Production of fungal fructosyl amino acid oxidase useful for diabetic diagnosis in the peroxisome of Candida boidnii. FEBS Lett. (1999) 459, 233-237
[Non-Patent Document 18] Nishikawa M, Hagishita T, Yurimoto H, Kato N, Sakai Y, Hatanaka T, Primary structure and expression of peroxisomal acetylspermidine oxidase in the methylotrophic yeast Candida boidinii. FEBS Lett. (2000) 476, 150-154

### Summary of the Invention

### Problems to be Solved by the Invention

It is an object of the present invention to provide a production system that is capable of high-level secretory production of a protein (and in particular, a protein with a complicated structure, such as a structure with S-S bonds) in a host cell such as yeast, is suitable for industrial production with high safety, and does not require explosion-proof facilities.

### Means for Solving the Problem

The present inventors have conducted concentrated studies in order to attain the above objects. As a result, they discovered that a yeast strain into which a chaperone gene has been introduced and in which the aoxl gene encoding alcohol oxidase has been disrupted may be used, so that high-level secretory production of a target protein induced by low-concentration methanol would become possible under the control of the aoxl promoter. The present inventors also discovered that acidic proteases, such as proteinase B (PRB1) and proteinase A (PEP4), were significantly associated with degradation of the target protein expressed in yeast, and they confirmed that regulation of the pH level of a medium aimed at disruption of the prb1 gene encoding proteinase B and/or suppression of activity of acidic protease such as proteinase A in yeast would lead to significant improvement in the secretory production amount of the target protein.

The high-level protein secretory production system comprising the above described features in combination enables a significant reduction in the amount of methanol to be added. Thus, such system can be used as a highly safe production system that is suitable for industrial production. The present invention has been completed on the basis of such findings.

Specifically, the present invention includes the following.
[1] A transformed yeast into which a chaperone gene has been introduced and in which
   the aoxl gene has been disrupted, and
   a protease gene has been disrupted, wherein optionally the protease gene is a prb1 gene.
[2] The transformed yeast according to [1], wherein the chaperone gene is at least one gene selected from the group consisting of genes (a) to (d) below:
   (a) a gene encoding PDI1, ERO1, Kar2, MPD1, SCJ1, EUG1, or HSP104 derived from *Ogataea minuto (O. minuto*);
   (b) a gene encoding PDI1, MPD1, SCJ1, ERO1, FKB2, JEM1, LHS1, MPD2, ERJ5, or EUG1 derived from *Saccharomyces cerevisiae (S. cerevisia*);
   (c) a gene encoding PDI, ERO1-Lα, ERO1-Lβ, or GRP78 derived from a human; and
   (d) a gene exhibiting 95% or higher sequence homology to a base sequence of any of the genes (a) to (c).
[3] The transformed yeast according to [1], wherein the chaperone gene is at least one gene selected from the group consisting of genes (a) to (g) below:
   (a) a gene encoding PDI1 derived from *O*. *minuta;*
   (b) a gene encoding ERO1 derived from *O. minuta;*
   (c) a gene encoding Kar2 derived from *O*. *minuta;*
   (d) a gene encoding PDI1 derived from *S. cerevisiae;*
   (e) a gene encoding PDI derived from a human;
   (f) a gene encoding ERO1 derived from a human; and
   (g) a gene exhibiting 95% or higher sequence homology to a base sequence of any of the genes (a) to (f).
[4] The transformed yeast according to [1], wherein the chaperone gene is any of the chaperone genes (a) to (g) below:
   (a) a combination of a gene encoding PDI1, a gene encoding ERO1, and a gene encoding Kar2 derived from *O*. *minuta;*
   (b) a combination of a gene encoding PDI1 and a gene encoding Kar2 derived from *O*. *minuta;*
   (c) a combination of a gene encoding PDI derived from a human and a gene encoding ERO1 derived from *O. minuta;*
   (d) a combination of a gene encoding PDI1 and a gene encoding ERO1 derived from *O. minuta;*
   (e) a combination of a gene encoding PDI derived from a human, a gene encoding ERO1-Lβ derived from a human, and a gene encoding GRP78 derived from a human;
   (f) a combination of a gene encoding PDI derived from a human, a gene encoding ERO1 derived from *O. minuta,* and a gene encoding GRP78 derived from a human; and
   (g) a gene exhibiting 95% or higher sequence homology to a base sequence of any of the genes (a) to (f).
[5] The transformed yeast according to any of [1] to [4], wherein the yeast is a methylotrophic yeast.
[6] The transformed yeast according to any of [1] to [5], which comprises a gene encoding a target protein introduced thereinto.
[7] Use of the transformed yeast according to any of [1] to [6] for the production of a target protein.
[8] A method for producing a protein comprising culturing the transformed yeast according to [6] in a medium and sampling a target protein from the culture product.
[9] The method for producing a protein according to [8], wherein culture is conducted under conditions in which protease activity is inhibited.
[10] The method for producing a protein according to [8] or [9], wherein culture is conducted in a medium with a pH of 6.0 to 7.5.
[11] The method for producing a protein according to any of [8] to [10], wherein a nitrogen source is added to the medium.
[12] The method for producing a protein according to [8] to [11], wherein (a) methanol is not added to the medium or (b) the amount of methanol added to the medium is 2% (v/v) or less.
[13] A method for producing a transformed yeast comprising step (i) in addition to both step (ii) and (iii):
   (i) a step of introducing a chaperone gene into yeast; and
   (ii) a step of disrupting the aox1 gene in yeast; and
   (iii) a step of disrupting the prbl gene in yeast,
      optionally further comprising a step of introducing a gene encoding a target protein.

Further disclosed herein are:
(1) A transformed yeast into which a chaperone gene has been introduced and in which the aoxl gene has been disrupted.
(2) The transformed yeast according to (1), wherein the chaperone gene is at least one gene selected from the group consisting of genes (a) to (d) below:
   (a) a gene encoding PDI1, ERO1, Kar2, MPD1, SCJ1, EUG1, or HSP104 derived from *Ogataea minuta* (*O. minuta*);
   (b) a gene encoding PDI1, MPD1, SCJ1, ERO1, FKB2, JEM1, LHS1, MPD2, ERJ5, or EUG1 derived from *Saccharomyces cerevisiae (S. cerevisia*);
   (c) a gene encoding PDI, ERO1-Lα, ERO1-Lβ, or GRP78 derived from a human; and
   (d) a gene exhibiting 95% or higher sequence homology to a base sequence of any of the genes (a) to (c).
(3) The transformed yeast according to (1), wherein the chaperone gene is at least one gene selected from the group consisting of genes (a) to (g) below:
   (a) a gene encoding PDI1 derived from *O. minuta;*
   (b) a gene encoding ERO1 derived from *O. minuta;*
   (c) a gene encoding Kar2 derived from *O. minuta;*
   (d) a gene encoding PDI1 derived from *S. cerevisiae;*
   (e) a gene encoding PDI derived from a human;
   (f) a gene encoding ERO1 derived from a human; and
   (g) a gene exhibiting 95% or higher sequence homology to a base sequence of any of the genes (a) to (f).
(4) The transformed yeast according to (1), wherein the chaperone gene is any of the chaperone genes (a) to (g) below:
   (a) a combination of a gene encoding PDI1, a gene encoding ERO1, and a gene encoding Kar2 derived from *O. minuta;*
   (b) a combination of a gene encoding PDI1 and a gene encoding Kar2 derived from *O. minuta;*
   (c) a combination of a gene encoding PDI derived from a human and a gene encoding ERO1 derived from *O. minuta;*
   (d) a combination of a gene encoding PDI1 and a gene encoding ERO1 derived from *O. minuta;*
   (e) a combination of a gene encoding PDI derived from a human, a gene encoding ERO1-Lβ derived from a human, and a gene encoding GRP78 derived from a human;
   (f) a combination of a gene encoding PDI derived from a human, a gene encoding ERO1 derived from *O. minuta,* and a gene encoding GRP78 derived from a human; and
   (g) a gene exhibiting 95% or higher sequence homology to a base sequence of any of the genes (a) to (f).
(5) The transformed yeast according to any of (1) to (4), wherein the protease gene has been disrupted.
(6) The transformed yeast according to (5), wherein the protease is a prbl gene.
(7) A transformed yeast into which a chaperone gene has been introduced and in which a protease gene has been disrupted.
(8) The transformed yeast according to (7), wherein the protease is a prbl gene.
(9) The transformed yeast according to any of (1) to (8), wherein the yeast is a methhylotrophic yeast.
(10) The transformed yeast according to any of (1) to (9), which comprises a gene encoding a target protein introduced thereinto.
(11) Use of the transformed yeast according to any of (1) to (10) for the production of a target protein.
(12) A method for producing a protein comprising culturing the transformed yeast according to (10) in a medium and sampling a target protein from the culture product.
(13) The method for producing a protein according to (12), wherein culture is conducted under conditions in which protease activity is inhibited.
(14) The method for producing a protein according to (12) or (13), wherein culture is conducted in a medium with a pH of 6.0 to 7.5.
(15) The method for producing a protein according to any of (12) to (14), wherein a nitrogen source is added to the medium.
(16) The method for producing a protein according to any of (12) to (15), wherein the amount of methanol added to the medium is 2% (v/v) or less.
(17) A target protein produced by the method according to any of (12) to (16).
(18) A method for producing a transformed yeast comprising step (i) in addition to either or both step (ii) and/or (iii):
   (i) a step of introducing a chaperone gene into yeast; and
   (ii) a step of disrupting the aox1 gene in yeast; and/or
   (iii) a step of disrupting the prb1 gene in yeast.
(19) The method of production according to (18), which further comprises a step of introducing a gene encoding a target protein.

### Effects of the Invention

The present invention enables high-level secretory production of a protein having a complicated structure, such as a structure with S-S bonds. as well as a normal protein, in a correctly folded form in a transformed yeast as claimed resulting from the introduction of a chaperone gene, the disruption of the aox1 gene, and the disruption of a protease gene. In addition, long-term culture (mass production) can be performed by culturing such transformed yeast under conditions in which protease activity is inhibited. In the protein production system involving the use of the transformed yeast according to the present invention, the amount of methanol used can be reduced to a significant extent. Thus, such system can be used as a highly safe protein production system that is suitable for industrial production (mass production).

This patent application claims priority from Japanese Patent Application No. 2014-155272 filed on July 30, 2014, and it includes part or all of the contents as disclosed in the description thereof.

### Brief Description of the Drawings

[Fig. 1] Fig. 1 shows a method for producing a strain in which the ura3 gene has been disrupted.
[Fig. 2] Fig. 2 shows a method for producing a strain in which the aoxl gene has been disrupted.
[Fig. 3] Fig. 3 shows the structure of a chaperone gene expression vector (onaPl 1007: OmPDI1+OmERO1+OmKar2 expression vector).
[Fig. 4] Fig. 4 shows a method for producing a strain in which the prbl gene has been interrupted.
[Fig. 5] Fig. 5 shows a method for producing a kex2 expression plasmid (kex2 expression plasmid: pOMEA-Z1-KEX2, kex2 expression cassette).

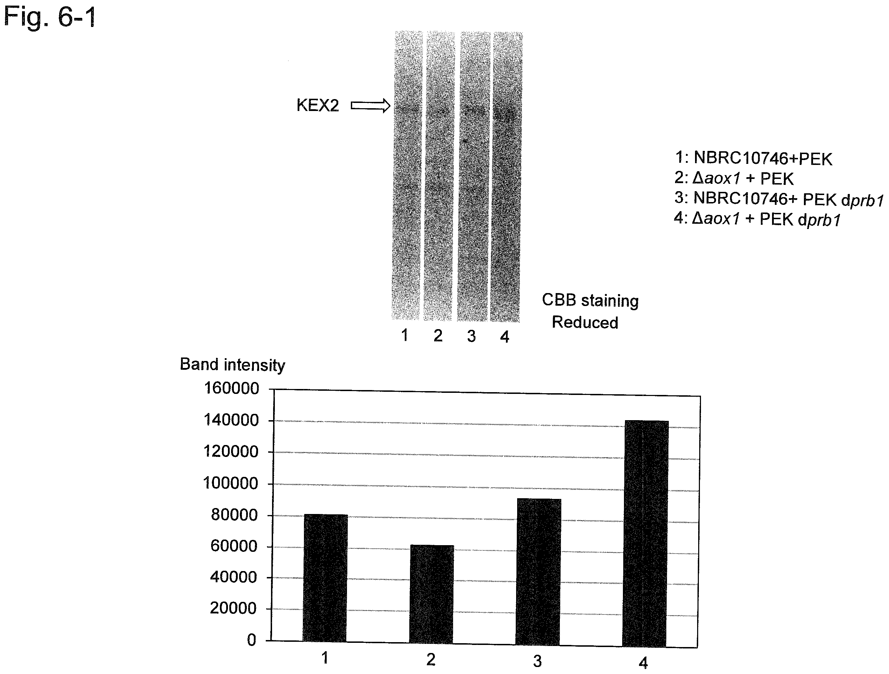

[Fig. 6-1] Fig. 6-1 shows a comparison of the secretory production amount of a KEX2 protein induced by methanol (1: the NBRC 10746 strain into which a chaperone (OmPDI1/OmERO1/OmKar2) has been introduced (a KEX2-producing strain derived from the NBRC10746+PEK strain); 2: a strain into which a chaperone (OmPDI1/OmERO1/OmKar2) has been introduced and in which the aoxl gene has been disrupted (a KEX2-producing strain derived from the Δaox1+PEK strain); 3: a strain into which a chaperone (OmPDI1/OmERO1/OmKar2) has been introduced and in which the prbl gene has been interrupted (a KEX2-producing strain derived from the NBRC10746+PEK dprbl strain); and 4: a strain in which the prbl gene has been interrupted, into which a chaperone (OmPDI1/OmERO1/OmKar2) has been introduced, and in which the aoxl gene has been disrupted (a KEX2-producing strain derived from the Δaox1+PEK dprbl strain).

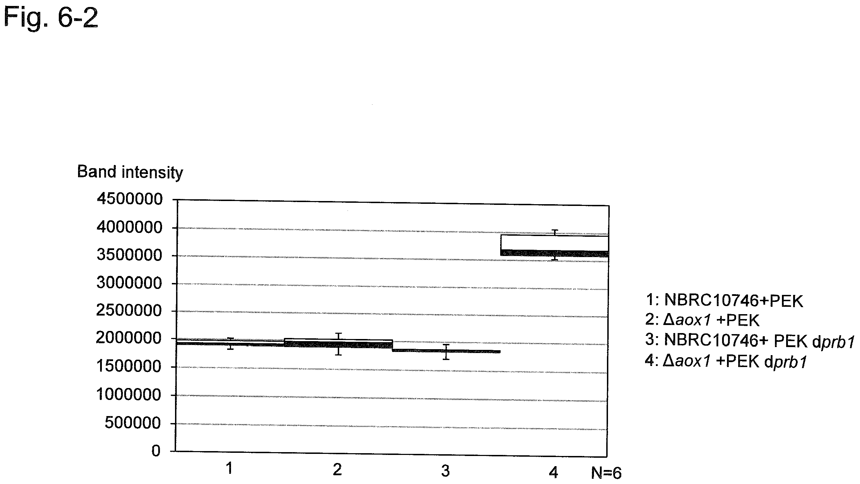

[Fig. 6-2] Fig. 6-2 shows a comparison of enzymatic activity of the KEX2 protein (1: the NBRC 10746 into which a chaperone (OmPDI1/OmERO1/OmKar2) has been introduced (the KEX2-producing strain derived from the NBRC10746+PEK strain); 2: a strain into which a chaperone (OmPDI1/OmERO1/OmKar2) has been introduced and in which the aoxl gene has been disrupted (the KEX2-producing strain derived from the Δaox1+PEK strain); 3: a strain into which a chaperone (OmPDI1/OmERO1/OmKar2) has been introduced and in which the prbl gene has been interrupted (the KEX2-producing strain derived from the NBRC10746+PEK dprbl strain); and 4: a strain in which the prbl gene has been interrupted, into which a chaperone (OmPDI1/OmERO1/OmKar2) has been introduced, and in which the aoxl gene has been disrupted (the KEX2-producing strain derived from the Δaox1+PEK dprbl strain); (white bar: third quartile-median; black bar: median-first quartile).
[Fig. 7] Fig. 7 shows a method for producing the hsa expression plasmid (hsa expression plasmid: pOMEA-Z1-HSA; hsa gene expression cassette).

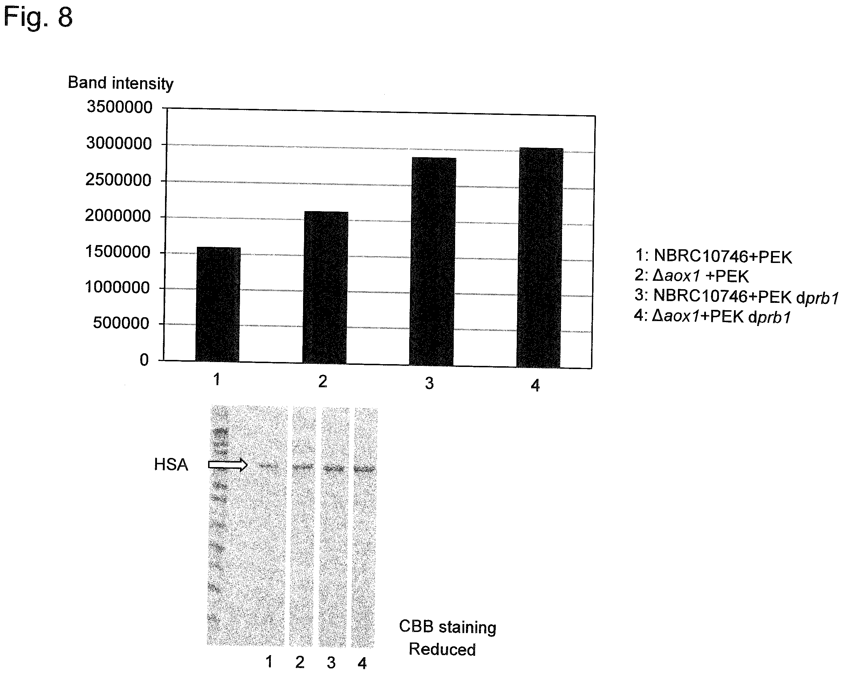

[Fig. 8] Fig. 8 shows the secretory production amount of the HSA protein induced by methanol (deep well plate scale) (1: the NBRC 10746 strain into which a chaperone (OmPDI1/OmERO1/OmKar2) has been introduced (the HSA-producing strain derived from the NBRC10746+PEK strain); 2: a strain into which a chaperone (OmPDI1/OmERO1/OmKar2) has been introduced and in which the aoxl gene has been disrupted (the HSA-producing strain derived from the Δaox1+PEK strain); 3: a strain into which a chaperone (OmPDI1/OmERO1/OmKar2) has been introduced and in which the prbl gene has been interrupted (the HSA-producing strain derived from the NBRC10746+PEK dprbl strain); and 4: a strain in which the prbl gene has been interrupted, into which a chaperone (OmPDI1/OmERO1/OmKar2) has been introduced, and in which the aoxl gene has been disrupted (the HSA-producing strain derived from the Δaox1+PEK dprbl strain).

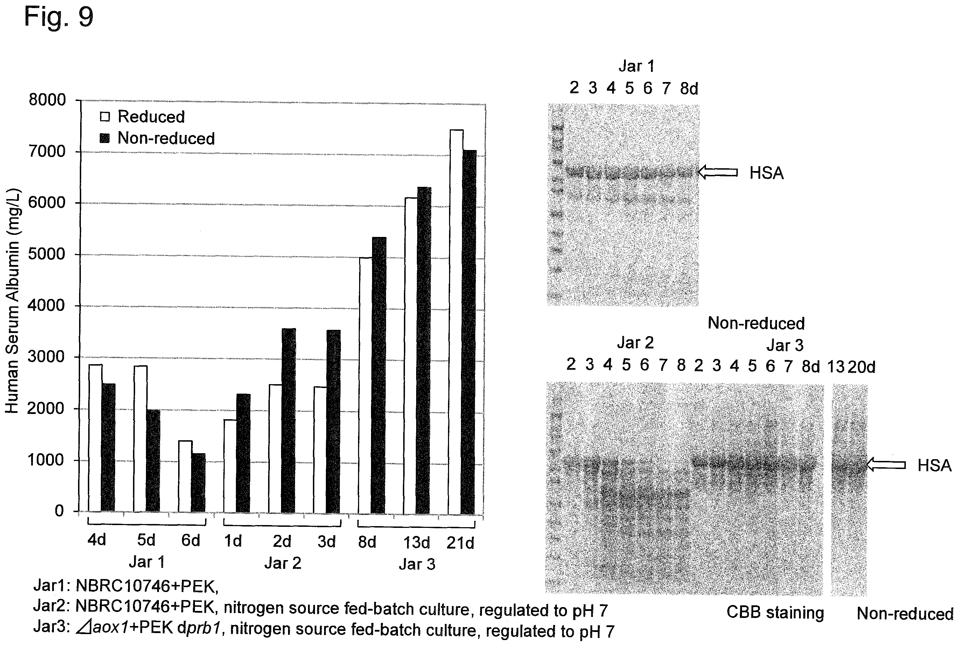

[Fig. 9] Fig. 9 shows the secretory production amount of the HSA protein induced by methanol (3L Jar scale) [Jar1: the NBRC 10746 strain into which a chaperone (OmPDI1/OmERO1/OmKar2) has been introduced (the HSA-producing strain derived from the NBRC10746+PEK strain); Jar2: the NBRC 10746 strain into which a chaperone (OmPDI1/OmERO1/OmKar2) has been introduced (the HSA-producing strain derived from the NBRC10746+PEK strain) (nitrogen source fed-batch culture; pH 7 control); Jar3: a strain in which the prb1 gene has been interrupted, into which a chaperone (OmPDI1/OmERO1/OmKar2) has been introduced, and in which the aoxl gene has been disrupted (the HSA-producing strain derived from the Δaox1+PEK dprbl strain) (nitrogen source fed-batch culture; pH 7 control).

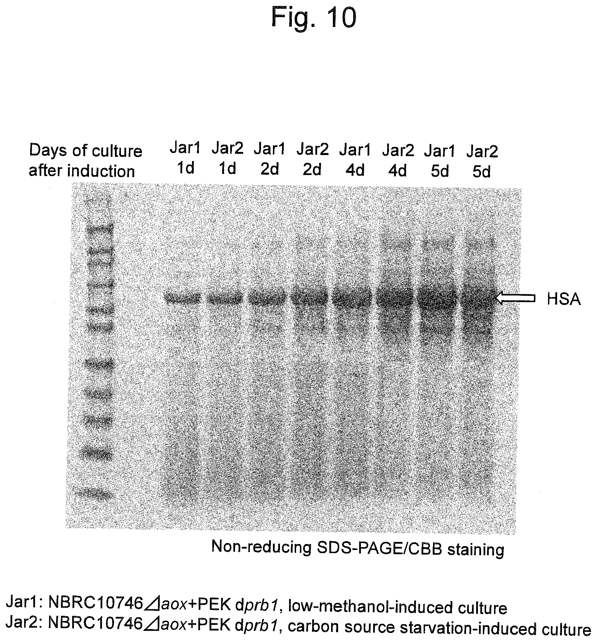

[Fig. 10] Fig. 10 shows the secretory production amount of the HSA protein achieved by carbon source starvation-induced culture (3L Jar scale) [Jar1: a strain in which the prbl gene has been interrupted, into which a chaperone (OmPDI1/OmERO1/OmKar2) has been introduced, and in which the aox 1 gene has been disrupted (the HSA-producing strain derived from the Δaox1+PEK dprbl strain) (low methanol-induced culture); Jar2: a strain in which the prbl gene has been interrupted, into which a chaperone (OmPDI1/OmERO1/OmKar2) has been introduced, and in which the aoxl gene has been disrupted (the HSA-producing strain derived from the Δaox1+PEK dprb1 strain) (carbon source starvation-induced culture).

### Embodiments for Carrying out the Invention

### 1. Transformed yeast

The transformed yeast of the present invention is obtained by introduction of a a chaperone gene, disruption of the aoxl gene, and disruption of the protease gene. Specifically, a transformed yeast into which a chaperone gene has been introduced and in which the aox1 gene has been disrupted, a transformed yeast into which a chaperone gene has been introduced and in which the protease gene has been disrupted, and a transformed yeast into which a chaperone gene has been introduced and in which the aoxl gene and a protease gene have been disrupted are within the scope of the transformed yeast of the present disclosure.

### (Host cells)

Host cells to be transformed are preferably yeast strains. Examples of yeast strains include methhylotrophic yeast strains such as *Ogataea minuta, Pichia lindneri, Pichia pastoris, Hansenulla polymorpha (Pichia angusta*), and *Candida boidinii* and yeast strains such as *Saccharomyces cerevisiae, Kluyveromyces lactis, Yarowia lipolytica,* and *Shizosaccharomyces pombe,* with methhylotrophic yeast strains being preferable. A specific example of the *Ogataea minuta* strain is the *Ogataea minuta* YK3 strain (Δoch1Δpep4Δprb1Δyps1Δura3Δade1), and a specific example of the *Saccharomyces cerevisiae* strain is the *Saccharomyces cerevisiae* BY4741 strain (MATa Δhis3Δleu2Δmet15Δura3), although yeast strains are not limited thereto.

### (Introduction of chaperone gene)

Examples of chaperone genes used in the present invention include genes encoding PDI1 (SEQ ID NO: 35 (the base sequence); SEQ ID NO: 36 (the amino acid sequence)), EROl (SEQ ID NO: 43 (the base sequence); SEQ ID NO: 44 (the amino acid sequence)), Kar2 (SEQ ID NO: 47 (the base sequence); SEQ ID NO: 48 (the amino acid sequence)), MPD1 (SEQ ID NO: 37 (the base sequence); SEQ ID NO: 38 (the amino acid sequence)), SCJ1 (SEQ ID NO: 39 (the base sequence); SEQ ID NO: 40 (the amino acid sequence)), EUG1 (SEQ ID NO: 41 (the base sequence); SEQ ID NO: 42 (the amino acid sequence)), and HSP104 (SEQ ID NO: 45 (the base sequence); SEQ ID NO: 46 (the amino acid sequence)) derived from *Ogataea minuta* (*O. minuta*).

The chaperone gene used in the present invention may be a chaperone gene derived from another organism species, such as other types of yeast, mold, or a human.

As a chaperone gene derived from another type of yeast, for example, a chaperone gene derived from *Saccharomyces cerevisiae* can be used. Specific examples include genes encoding PDI1 (Primary SGDID: S000000548; SEQ ID NO: 49 (the base sequence); SEQ ID NO: 50 (the amino acid sequence)), MPD1 (Primary SGDID: S000005814; SEQ ID NO: 51 (the base sequence); SEQ ID NO: 52 (the amino acid sequence)), SCJ1 (Primary SGDID: S000004827; SEQ ID NO: 53 (the base sequence); SEQ ID NO: 54 (the amino acid sequence)), ERO1 (Primary SGDID: S000004599; SEQ ID NO: 55 (the base sequence); SEQ ID NO: 56 (the amino acid sequence)), FKB2 (Primary SGDID: S000002927; SEQ ID NO: 57 (the base sequence); SEQ ID NO: 58 (the amino acid sequence)), JEM1 (Primary SGDID: S000003609; SEQ ID NO: 59 (the base sequence); SEQ ID NO: 60 (the amino acid sequence)), LHS1 (Primary SGDID: S000001556; SEQ ID NO: 61 (the base sequence); SEQ ID NO: 62 (the amino acid sequence)), MPD2 (Primary SGDID: S000005448; SEQ ID NO: 63 (the base sequence); SEQ ID NO: 64 (the amino acid sequence)), ERJ5 (Primary SGDID: S000001937; SEQ ID NO: 65 (the base sequence); SEQ ID NO: 66 (the amino acid sequence)), and EUG1 (Primary SGDID: S000002926; SEQ ID NO: 67 (the base sequence); SEQ ID NO: 68 (the amino acid sequence)). Sequence information regarding genes derived from *Saccharomyces cerevisiae* is available from SGD *(Saccharomyces* genome database: http://www.yeastgenome.org/).

Examples of chaperone genes derived from a human include genes encoding PDI (GenBank Accession No. BC010859; SEQ ID NO: 69 (the base sequence); SEQ ID NO: 70 (the amino acid sequence)), ERO1-Lα (GenBank Accession No. AF081886; SEQ ID NO: 71 (the base sequence); SEQ ID NO: 72 (the amino acid sequence)), ERO1-Lβ (GenBank Accession No. BC044573; SEQ ID NO: 73 (the base sequence); SEQ ID NO: 74 (the amino acid sequence)), and GRP78 (GenBank Accession No. AL354710; SEQ ID NO: 75 (the base sequence); SEQ ID NO: 76 (the amino acid sequence)).

The chaperone gene used in the present invention may be a gene encoding a protein that consists of an amino acid sequence derived from any of the amino acid sequences described above by deletion, substitution, and/or addition of one or several amino acids, provided that such gene has activity of promoting foreign protein secretion. The number of amino acids that may be deleted, substituted, and/or added is preferably 1 to several. The number represented by the term "several" is not particularly limited. For example, such number may be 50 or less, 40 or less, 30 or less, 25 or less, 20 or less, 15 or less, 12 or less, 10 or less, 9 or less, 8 or less, 7 or less, 6 or less, 5 or less, 4 or less, 3 or less, or 2. The term "mutation" used herein primarily refers to a mutation that is artificially introduced via a known method for preparing a mutant protein, and the term may refer to a mutation that is similar to one existing in nature. The term "foreign protein" is used in the same sense as the term "target protein" herein.

Also, the chaperone gene used in the present invention may be a gene encoding a protein that consists of an amino acid sequence having at least 80% sequence identity with any of the amino acid sequences described above and has activity of promoting foreign protein secretion. Specific examples include a gene that consists of a base sequence having at least 80% sequence identity with the base sequence as shown in SEQ ID NO: 35, 37, 39, 41, 43, 45, 47, 49, 51, 53, 55, 57, 59, 61, 63, 65, 67, 69, 71, 73, or 75 and encodes a protein having activity of promoting foreign protein secretion; and a gene encoding a protein that consists of an amino acid sequence having at least 80% sequence identity with the amino acid sequence as shown in SEQ ID NO: 36, 38, 40, 42, 44, 46, 48, 50, 52, 54, 56, 58, 60, 62, 64, 66, 68, 70, 72, 74, or 76 and has activity of promoting foreign protein secretion. The term "at least 80% sequence identity" preferably refers to at least 85% sequence identity, more preferably at least 90% sequence identity, further preferably at least 95%, and most preferably at least 99% sequence identity. A protein homology search can be carried out with the use of, for example, the DNA Databank of Japan (DDBJ) via FASTA, BLAST, or another program.

The chaperone gene used in the present invention may be a gene that hybridizes under stringent conditions to DNA consisting of a base sequence complementary to DNA consisting of any of the base sequences described above and encodes a protein having activity of promoting foreign protein secretion. Under the aforementioned "stringent conditions," a so-called specific hybrid is formed, but a non-specific hybrid is not formed. Under such conditions, for example, complementary strands of a nucleic acid exhibiting a high degree of sequence identity, i.e., a nucleic acid consisting of a base sequence having at least 80%, preferably at least 85%, more preferably at least 90%, further preferably at least 95%, and most preferably at least 99% sequence identity with any of the base sequences above, undergo hybridization, but complementary strands of a nucleic acid having lesser degrees of sequence identity do not undergo hybridization. More specifically, the sodium salt concentration is 15 to 750 mM, preferably 50 to 750 mM, and more preferably 300 to 750 mM, the temperature is 25°C to 70°C, preferably 50°C to 70°C, and more preferably 55°C to 65°C, and the formamide concentration is 0% to 50%, preferably 20% to 50%, and more preferably 35% to 45%. Under stringent conditions, further, a filter is generally washed at a sodium salt concentration of 15 to 600 mM, preferably 50 to 600 mM, and more preferably 300 to 600 mM, and a temperature of 50°C to 70°C, preferably 55°C to 70°C, and more preferably 60°C to 65°C, after hybridization.

A person skilled in the art can easily obtain such homologous genes by referring to, for example, Molecular Cloning (Sambrook, J. et al., Molecular Cloning: A Laboratory Manual 2nd ed., Cold Spring Harbor Laboratory Press, 10 Skyline Drive Plainview, N.Y., 1989). Also, a base sequence identity search can be carried out via FASTA, BLAST, or other programs.

The amino acid mutation mentioned above, such as deletion, substitution, and/or addition, can be introduced via a technique known in the art, such as the Kunkel method or the Gapped duplex method, or via a technique in accordance therewith. For example, mutagenesis kits utilizing site-directed mutagenesis, such as a Mutant-K (Takara Bio Inc.), Mutant-G (Takara Bio Inc.), or LA PCR in vitro Mutagenesis series kit (Takara Bio Inc.), can be used.

Also, chaperone genes derived from other organism species may be codon-modified genes that are modified so as to improve translation efficiency via substitution of a base sequence with a codon that is frequently used in a host cell. A specific example is a codon-modified gene of a gene encoding PDI derived from a human. DNA having a modified base sequence can be artificially synthesized. In the case of a long DNA sequence, the sequence is first divided into several fragments, fragments are synthesized in advance, and the resultants are then bound to each other at the end.

In the present invention, one or more types of the aforementioned chaperone genes are used in combination. When two or more genes are used in combination, such genes may be derived from the same or different organism species.

Preferable examples of the chaperone genes that are used in the present invention include the pdi1 gene derived from *O. minuta,* the ero1 gene derived from *O. minuta,* the kar2 gene derived from *O. minuta,* the pdil gene derived from *S. cerevisiae,* the pdi gene derived from a human, the ero1 gene derived from a human, and a gene encoding a protein that consists of an amino acid sequence having at least 80%, preferably at least 85%, more preferably at least 90%, further preferably at least 95%, and most preferably at least 99% sequence identity with any of the amino acid sequences above and has activity of promoting foreign protein secretion.

Further preferable examples of the chaperone genes that are used in the present invention include a combination of the pdi1 gene, the ero1 gene, and the kar2 gene derived from *O. minuta,* a combination of the pdi1 gene and the ero1 gene derived from *O. minuta,* a combination of the pdi1 gene and the kar2 gene derived from *O. minuta,* a combination of the pdi gene derived from a human and the ero1 gene derived from *O. minuta,* a combination of the pdi gene, the ero1-Lβ gene, and the grp78 gene derived from a human, a combination of the pdi gene derived from a human, the ero1 gene derived from *O. minuta,* and the grp78 gene derived from a human, and a combination of genes each encoding a protein that consists of an amino acid sequence having at least 80%, preferably at least 85%, more preferably at least 90%, further preferably at least 95%, and most preferably at least 99% sequence identity with any of the amino acid sequences of the combinations of the chaperones described above and has activity of promoting foreign protein secretion.

The most preferable examples of the chaperone genes that are used in the present invention include a combination of the pdi1 gene, the ero1 gene, and the kar2 gene derived from *O. minuta,* or a combination of genes each encoding a protein that consists of an amino acid sequence having at least 80% sequence identity with any of the amino acid sequences of PDI1, ERO1, and Kar2 derived from *O. minuta* and has activity of promoting foreign protein secretion (e.g., a combination of genes each that consists of the base sequence having at least 80%, preferably at least 85%, more preferably at least 90%, further preferably at least 95%, and most preferably at least 99% sequence identity with the base sequence as shown in SEQ ID NO: 35, 43, or 47 and encodes a protein having activity of promoting foreign protein secretion; or a combination of genes each encoding a protein that consists of an amino acid sequence having at least 80%, preferably at least 85%, more preferably at least 90%, further preferably at least 95%, and most preferably at least 99% sequence identity with the amino acid sequence as shown in SEQ ID NO: 36, 44, or 48 and has activity of promoting foreign protein secretion). Regarding the gene-related notation used herein, for example, the term "a gene encoding PDI1" is used in the same sense as the term "the pdi1 gene."

The chaperone gene is introduced into yeast, which is a host cell, with the use of an expression vector. In the present invention, an expression vector can be introduced into a host cell by any method, provided that the introduced gene is stably present and adequately expressed in a yeast host. Examples of methods that are generally employed include the calcium phosphate method (Ito et al., Agric. Biol. Chem., 48, 341, 1984), electroporation (Becker, D. M. et al., 1990; Methods. Enzymol., 194, 182-187), use of spheroplasts (Creggh et al., Mol. Cell. Biol., 5, 3376, 1985), the lithium acetate method (Itoh, H., 1983; J. Bacteriol. 153, 163-168), and lipofection.

### (Disruption of aoxl gene and/or disruption of protease gene)

The transformed yeast of the present disclosure is obtained by, in addition to the introduction of a chaperone gene, disruption of the aoxl gene endogenous in the host genome, disruption of the protease gene endogenous in the host genome, or disruption of both the aoxl gene and the protease gene endogenous in the host genome.

Examples of the aox1 genes include a gene encoding AOX1 derived from *O. minuta* (SEQ ID NO: 27 (the base sequence); SEQ ID NO: 28 (the amino acid sequence)), a gene encoding AOX1 derived from *Pichia pastoris* (GenBank accession number: U96967), and a gene encoding AOX1 derived from *Candida boidinii* (GenBank accession number: Q00922). The aoxl gene is not limited thereto, provided that the gene encodes AOX1 derived from yeast.

Examples of the protease genes include the prbl gene (SEQ ID NO: 31 (the base sequence); SEQ ID NO: 32 (the amino acid sequence)) and the pep4 gene (GenBank accession number: AB236164) derived from *O. minuta,* the prbl gene (GenBank accession number: AB060541) and the pep4 gene (JP 2000-078978 A) derived from *Candida boidinii,* the prbl gene and the pep4 gene derived from *Pichia pastoris,* the prbl gene (GenBank accession number: M18097) derived from *Saccharomyces cerevisiae,* and the prb1 gene (GenBank accession number: A75534) derived from *Kluyveromyces lactis.* The protease gene is not limited thereto, provided that the gene is derived from yeast.

Accordingly, the transformed yeast of the present disclosure is preferably obtained by, in addition to the introduction of a chaperone gene, disruption of the aox1 gene endogenous in the host genome or disruption of the prb1 gene endogenous in the host genome. More preferably, the transformed yeast is obtained by, in addition to the introduction of a chaperone gene, disruption of both the aox1 gene and the prb1 gene endogenous in the host genome. It is most preferable that, in addition to the introduction of, as the the chaperon gene, a combination of the pdi1 gene, the ero1 gene, and the kar2 gene derived from *O. minuta* or a combination of genes each encoding a protein that consists of an amino acid sequence having at least 80%, preferably at least 85%, more preferably at least 90%, further preferably at least 95%, and most preferably at least 99% sequence identity with any of the amino acid sequences of PDI1, ERO1, and Kar2 and has activity of promoting foreign protein secretion, both the aoxl gene and the prbl gene endogenous in the host genome are disrupted.

In the present invention, the term "gene disruption" refers to "gene deletion" whereby all or a part of the target gene is deleted from the chromosome, substitution of the target gene, and "gene interruption" that inhibits the expression of a functional protein encoded by a target gene by interruption of the target gene without deleting such gene. From the viewpoint of disruption of functions of the target gene, gene disruption may take the form of mutagenesis or expression inhibition of a gene causing functional deficiency. A means for gene disruption is not particularly limited, provided that the expression or functions of a protein encoded by the target gene is/are inhibited or deleted.

Typically, the target gene can be disrupted via homologous recombination. At the outset, the target gene is interrupted or partially deleted, an adequate selection marker gene is inserted thereinto, and a DNA construct comprising a selection marker flanked by the upstream region and the downstream region of the target gene is prepared. Subsequently, this construct is introduced into a yeast strain, so as to perform recombination in homologous regions at both ends of the introduced fragment (a DNA construct comprising a selection marker) and the target gene in the chromosome, and the target gene in the chromosome is then substituted with the introduced fragment. In such a case, a selection marker used for gene disruption can be an auxotrophic marker or a drug-tolerant marker, as described below.

An embodiment involving the use of the ura3 gene as a selection marker is specifically described. A plasmid comprising the ura3 gene having repeat structures before and after the structural gene is constructed, the gene cassette is cleaved with a restriction enzyme, and the resultant is inserted into the target gene of a plasmid, so as to construct the disrupted alleles. This plasmid is substituted with the target gene of the chromosome, so as to obtain a gene-disrupted strain. The ura3 gene inserted into the chromosome has repeat structures before and after the ura3 gene, homologous recombination takes place between repeat sequences, and the ura3 gene is thus deleted from the chromosome. The deleted strain can be selected with the use of 5-fluoroorotic acid (5-FOA). The ura3 variant is resistant to 5-FOA (Boeke et al., Mol. Gen. Genet., 197, 345-346, 1984; Boeke et al., Methods Enzymol., 154, 165-174, 1987), and strains having URA+ phenotypes cannot grow in a 5-FOA medium. If a strain exhibiting tolerance is separated with the use of a medium supplemented with 5-FOA, accordingly, the use of the ura3 gene marker becomes possible again. In general, use of a selection marker is necessary in order to disrupt a gene. With the use of the ura3 gene, however, ura3 traits can be efficiently reproduced.

A mutation aimed at causing functional defects can be introduced into a gene by modifying a gene via mutagenesis, such as site-directed mutagenesis. Specifically, a gene mutation aimed at causing functional defects at a particular site is, for example, a mutation that has been caused by frame-shift or amino acid substitution at the active center resulting from insertion or deletion of nucleotides into or from ORF, and the gene is mutated to encode a protein that has been inactivated. When gene expression is suppressed, the expression level of the relevant gene is lowered or lost. Examples of methods for suppressing gene expression include a method involving the use of antisense RNA or RNAi and a method comprising attenuating a promoter.

### (Expression vector)

The chaperone gene is introduced into yeast with the use of an expression vector. Examples of such expression vector include a vector comprising a single type of chaperone gene, a vector comprising two or more copies of a single type of chaperone gene, and a vector comprising a combination of two or more types of chaperone genes. In order to express the chaperone gene in yeast, a vector comprising a single gene may be used to carry out transformation. Alternatively, a vector comprising a plurality of genes may be used to carry out transformation. Also, such expression vector may comprise a gene encoding a foreign protein. Alternatively, aiming high expression and secretion, expression vectors comprising a gene encoding a foreign protein may be prepared separately. In such a case, vectors are cotransfected into a host cell.

A gene encoding a foreign protein is not particularly limited. Examples include: various enzyme genes, such as the lysozyme gene, the α-amylase gene, and the α-galactosidase gene, and in particular, glycosyltransferase genes that are necessary for production of pharmaceutically useful glycoproteins, such as the erythropoietin (EPO) gene and granulocyte-colony stimulating factor (G-CSF) genes; various interferon genes that are pharmaceutically useful and physiologically active proteins, such as interferon α and interferon γ genes; various interleukin genes, such as IL1 and IL2 genes; various cytokine genes, such as the erythropoietin (EPO) gene and the granulocyte-colony stimulating factor (G-CSF) gene; growth factor genes; and various vaccine antigens such as influenza. Such genes may be obtained via any means.

The present invention is particularly effective on a protein that is highly hydrophobic and a protein whose secretory production is insufficient due to composite formation. Thus, the aforementioned foreign protein includes a multimeric protein, such as an antibody or a functional fragment thereof; i.e., a heteromultimer.

An expression regulation region may be adequately added to the chaperone gene or a gene encoding a foreign protein to constitute an expression vector as a protein expression unit. A protein expression unit comprises, in the direction of a transcription reading frame, at least a promoter region, the above gene, and a transcription terminator region. A promoter that can be used herein may be an inducible expression promoter or constitutive expression promoter. Examples of inducible expression promoters include a promoter of a gene encoding alcohol oxidase (AOX), a promoter of a gene encoding dihydroxyacetone synthase (DAS), and a promoter of a gene encoding formate dehydrogenase (FDH) involved in the methanol metabolism of methhylotrophic yeast. An example of another inducible promoter that can be used is a copper-inducible promoter (CUP). Examples of constitutive expression promoters include promoters of the genes encoding glyceraldehyde-3-phosphate dehydrogenase (TDH, GAP), phosphoglycerokinase (PGK), phosphotriose isomerase (TPI), enolase (ENO), actin (ACT), cytochrome c (CYC), trehalose synthase (TPS), and alcohol dehydrogenase (ADH). Also, a transcription terminator may be a sequence having activity of terminating transcription from a promoter. It may have the same or a different sequence as the gene of the promoter.

Also, an expression vector may comprise DNA encoding a secretory signal sequence that functions in a yeast cell added to a gene encoding a foreign protein. Thus, secretory production becomes possible, and a foreign protein of interest can be easily isolated and purified. Examples of secretory signal sequences include a secretory signal sequence of an *S. cerevisiae-derived* α-mating factor (αMF), a secretory signal sequence of *S. cerevisiae-*derived invertase (SUC2), and a secretory signal sequence of human-derived α-galactosidase.

The expression vector can comprise a selection marker for selecting a transformant. For example, yeast expression vectors can comprise auxotrophic marker genes selected from among his1, his2, his3, his4, his5, his6, leu2, arg1, arg2, arg3, trp1, lys2, adel, ade2, ura3, and ura5 genes.

As selection markers, drug-resistant markers that impart resistance to drugs such as cerulenin, aureobasidin, Zeocin, canavanine, cycloheximide, hygromycin, blasticidin, tetracycline, kanamycin, ampicillin, tetracycline, and neomycin can be used, in addition to the aforementioned auxotrophic markers. Thus, transformants can be selected. Also, genes that impart solvent resistance to ethanol, osmotic resistance to glycerol or salt, metal ion resistance to copper, and the like may be used as markers, so that transformants can be selected.

### 2. Method for producing protein

In the present invention, proteins can be produced by culturing the transformed yeast as claimed obtained in 1. above via a conventional technique and sampling the proteins from the culture product, followed by purification. The term "culture product" used herein refers to culture cells, cultured strains, or disrupted cells or strains, in addition to a culture supernatant.

When the host cell is yeast, either a natural or synthetic medium may be used for culture, provided that it contains carbon sources, nitrogen sources, and inorganic salts assimilable by the yeast and permits efficient culture of the transformed yeast. Examples of carbon sources that can be used include: carbohydrates such as glucose, fructose, sucrose, and starch; organic acids such as acetic acid, lactic acid, citric acid, and propionic acid; and alcohols such as methanol, ethanol, propanol, and glycerol. Examples of nitrogen sources include: ammonia; ammonium salts of inorganic or organic acids such as ammonium chloride, ammonium sulfate, ammonium acetate, ammonium phosphate, and ammonium carbonate; other nitrogen-containing compounds such as urea; nitrogenous organic substances such as amino acids, yeast extracts, peptone, meat extracts, corn steep liquor, casein hydrolysate, soybean cake, and soybean cake hydrolysate. Examples of inorganic salts include: monopotassium phosphate, dipotassium phosphate, magnesium phosphate, magnesium sulfate, sodium chloride, iron(I) sulfate, manganese sulfate, copper sulfate, and calcium carbonate. In the case of an auxotrophic yeast, nutritive substances necessary for the growth thereof may be added to a medium. Examples of such nutritive substances include amino acids, vitamins, nucleic acids, and salts. In accordance with the type of selection marker, an antibiotic agent, such as aureobasidin, ampicillin, or tetracycline, may be adequately added to a medium. Alternatively, an amino acid that can be supplied by a gene complementing auxotrophy (e.g., leu, ura, or trp) may be removed.

When culturing yeast transformed with the use of an expression vector comprising an inducible promoter, an inducer may be added to the medium, according to need. When culturing yeast transformed with the use of an expression vector comprising a methanol-inducible promoter (e.g., aox, das, or mox), for example, methanol is added to the medium. When culturing yeast transformed with the use of an expression vector comprising a GAL promoter, galactose is added to the medium.

During culture, an inhibitor of PMT activity may be added to the medium, so as to inhibit addition of an O-sugar chain peculiar to yeast. Examples of inhibitors of PMT activity include the rhodanine-3-acetic acid derivative (5-[[3,4-(1-phenylmethoxy)phenyl]methylene]-4-oxo-2-thioxo-3-thiazolidineacetic acid, compound (1c) described in Bioorganic & Medicinal Chemistry Letters, Vol. 14, p. 3975, 2004) and {(5Z)-4-oxo-5-[3-(1-phenylethoxy)-4-(2-phenylethoxy)benzylidene]-2-thioxo-1,3-thiazolidin-3-yl}acetic acid (compound (5a) described in Bioorganic & Medicinal Chemistry Letters, Vol. 14, p. 3975, 2004).

Culture is carried out at about 20°C to 30°C for 24 to 1,000 hours. Culture can be carried out via batch culture or continuous culture, such as static, shake, agitation, or aeration culture.

When the transformed yeast of the present invention has been transformed with the use of an expression vector comprising a methanol-inducible promoter, it is cultured in a medium supplemented with methanol. Methanol may be added to the medium with the use of a pump or by other means while observing the growth of yeast. Such addition may be continuously or intermittently carried out, and it is not necessary to perform addition over the entire period of culture. When culturing a transformed yeast strain (i.e., a strain in which the aoxl gene has been disrupted), for example, methanol-induced culture is initiated in a medium supplemented with methanol at a concentration of preferably 0.3% to 2% (v/v) and more preferably 0.5% to 1% (v/v). When the methanol concentration is reduced to about 0.2% to 0.3% (v/v), methanol may be intermittently added, preferably at a final concentration of 0.1% to 0.5% (v/v), and more preferably at a final concentration of 0.2% to 0.4% (v/v) per day.

When carbon sources are depleted from the medium used for the growth of the transformed yeast and the medium is brought to a carbon-starved state, a methanol-inducible promoter is strongly activated. After the medium has been depleted of the carbon sources, accordingly, the carbon source content in the medium may be adequately adjusted. Thus, culture can be conducted without the addition of methanol or at a low methanol concentration of 0.2% (v/v) or lower. Examples of "carbon sources" include glycerin, alanine, mannitol, sorbitol, trehalose, and lactose. When "the carbon source content in the medium may be adequately adjusted," the carbon sources may be added to the medium at the lowest concentration necessary for the growth of the transformed yeast of the present invention and protein expression. Specifically, culture may be initiated under carbon source-depleted conditions (carbon source-starvation conditions) when the target cell density is attained. When culturing a transformed yeast strain (a strain in which the aox gene has been disrupted), for example, whether or not the cell density has reached the target level and carbon sources have been depleted is confirmed. At the same time, glycerin and sorbitol may be continuously added at a concentration of preferably 0.5% to 6% (w/v), and more preferably 1% to 4% (w/v) per day.

It is preferable that culture be carried out under conditions in which protease activity is inhibited. Thus, degradation of the target protein that is secreted and produced by yeast is inhibited, and the secretory production amount significantly increases. Protease activity can be inhibited by disrupting the proteinase B gene of yeast as described above, and it can be inhibited by regulating the pH level of the medium. The pH level of the medium is preferably 6.0 to 7.5, so that activity of acidic protease such as proteinase A in the medium is inhibited and the growth of the yeast is not affected. The pH level is regulated with the use of, for example, inorganic acid, organic acid, or an alkaline solution.

Further, culture is preferably carried out under conditions in which nitrogen sources are continuously added. By supplying nitrogen sources during culture, secretory production of proteins and maintenance thereof are remarkably improved. The final concentration of the nitrogen sources to be added per day is preferably 0.1% to 0.75% (w/v) in the case of a yeast extract and 0.05% to 0.15% (w/v) in the case of L-histidine monohydrochloride monohydrate, and it is more preferably 0.3% to 0.5% (w/v) in the case of a yeast extract and 0.1% to 0.13% (w/v) in the case of L-histidine monohydrochloride monohydrate. Nitrogen sources can be added with the use of a mixture of a yeast extract and L-histidine monohydrochloride monohydrate.

A transformed yeast into which a chaperone gene has been introduced and in which the aoxl gene and the protease gene have been disrupted, which is an embodiment of the transformed yeast of the present invention, is cultured in a medium with the pH level within the aforementioned range supplemented with nitrogen sources. Thus, the amount of methanol to be added can be reduced to 4% to 7% of the amount of methanol added, compared with the case of a transformed yeast into which only the chaperone gene has been introduced.

The expression product of a gene of a foreign protein from the culture product (i.e., a culture solution or culture cells) can be identified via SDS-PAGE, Western blotting, ELISA, or the like. The produced proteins may be isolated and purified via conventional techniques for protein isolation and purification. When target proteins are produced in the cells after culture, the cells may be pulverized using, for example, an ultrasonic pulverizer, a French press, a Manton-Gaulin homogenizer, or a Dyno-mil, to obtain target proteins. When the target proteins are produced outside the cells, the culture solution is used as it is, or the cells are removed via centrifugation or the like. Thereafter, the target proteins are collected via extraction using an organic solvent, subjected to various chromatography techniques (e.g., hydrophobic, reversed-phase, affinity, or ion-exchange chromatography), gel filtration using molecular sieves, electrophoresis using polyacrylamide gel, or the like, according to need. These techniques may be employed solely or in combinations of two or more.

The above culture and purification techniques are examples, and methods are not limited thereto. The amino acid sequence of the purified gene product can be confirmed by a conventional amino acid analysis method, such as automated amino acid sequencing via the Edman degradation technique.

### Examples

Hereafter, the present invention is described in detail with reference to the examples, although the technical scope of the present invention is not limited to the examples. Plasmids, restriction enzymes, DNA modifying enzymes, and the like that are used in the examples of the present invention are commercially available products, and these products can be used in accordance with conventional techniques. Also, procedures of DNA cloning, nucleotide sequencing, yeast transformation, culture of transformed yeast, and the like are well-known in the art or can be learned through existing publications.

### [Example 1] Construction of vector for foreign gene expression

### (1) Construction of vector for foreign gene introduction carrying a Zeocin-resistant gene as a selection marker and comprising the aoxl gene promoter of NBRC 10746 (O. minuta, Biological Resource Center, NITE) and the terminator cassette

NBRC 10746 AOX1 (GenBank Accession Number AB242209) comprises an amino acid sequence of 663 amino acids encoded by a 1,992-bp base sequence (SEQ ID NO: 27, SEQ ID NO: 28). PCR was carried out using the genomic DNA of NBRC 10746 prepared with the use of the Y-DER Yeast DNA Extraction Reagent (78870, PIERCE) as a template, the Hd AOXp Fw primer (5'-GCAAGCTTTCTTTCGCAAACAGCTCTTTG-3': SEQ ID NO: 1), the AOXp rv primer (5'-GAACCCGGGAACAGAATCTAGATTTTTTCGTAAGTCGTAAG-3': SEQ ID NO: 2), and the PrimeSTAR Max DNA Polymerase (RO45A, Takara Bio Inc.) at 98°C for 10 seconds, 55°C for 5 seconds, and 72°C for 15 seconds, and this cycle was repeated 30 times. Thus, an aoxl promoter region-contaning DNA fragment comprising the aoxl promoter region of about 2.4 kbp and a spacer region of 22 bp was amplified. Also, PCR was carried out using DNA of NBRC 10746 as a template, the AOXt fw primer (5'-CTGTTCCCGGGTTCCTGGATCCGAGACGGTGCCCGACTC-3': SEQ ID NO: 3), and the Kp AOXt Rv primer (5'-GCGGTACCGTTAGTGGTACGGGCAG-3': SEQ ID NO: 4) at 98°C for 10 seconds, 55°C for 5 seconds, and 72°C for 5 seconds, and this cycle was repeated 30 times. Thus, an aoxl terminator region-contaning DNA fragment comprising the aoxl terminator region of about 0.8 kbp and a spacer region of 22 bp was amplified. PCR was carried out using these DNA fragments as templates, the Hd AOXp Fw primer, and the Kp AOXt Rv primer at 98°C for 10 seconds, 55°C for 5 seconds, and 72°C for 15 seconds, and this cycle was repeated 30 times. Thus, the aoxl promoter region of about 2.4 kbp was ligated to the terminator region of about 0.8 kbp, and the resultant was then amplified. The ligated DNA fragment was subjected to agarose electrophoresis, recovered, and then cloned into pCR-Blunt II-TOPO. The cloned plasmid was subjected to double digestion with the restriction enzymes *Hind*III and *Kpn*I to obtain a DNA fragment comprising the aoxl gene promoter and the terminator cassette.

The pOMexGPlZ plasmid described in WO 2009/057813 (i.e., a vector for foreign gene expression carrying a Zeocin-resistant gene as a selection marker and comprising the gap gene promoter and the terminator cassette) was subjected to double digestion with the restriction enzymes *Hind*III and *Kpn*I to obtain a DNA fragment comprising a Zeocin-resistant gene marker. The DNA fragment comprising the aoxl gene promoter and the terminator cassette was introduced into the fragment obtained. Thus, the pOMEA-Z1 plasmid was obtained.

### [Example 2] Preparation of strain in which the ura3 gene has been disrupted

### (1) Preparation of DNA fragment for ura3 gene disruption

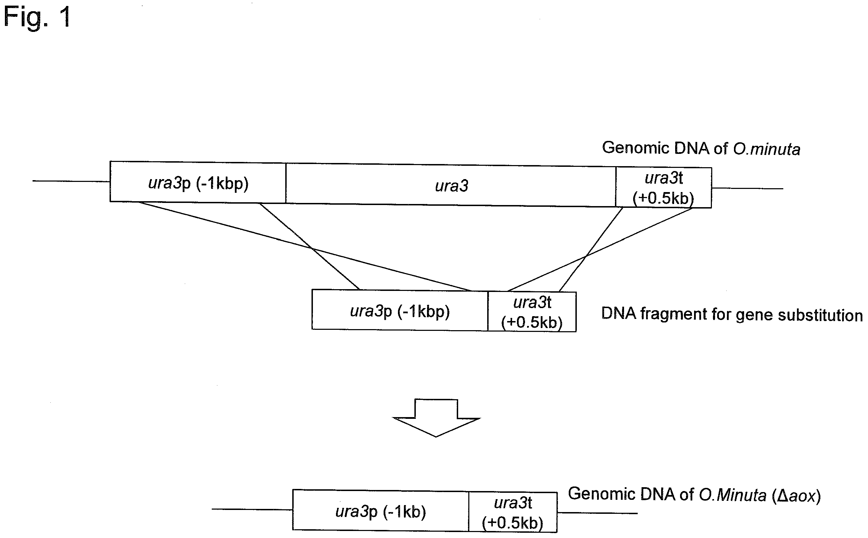

Fig. 1 shows forms of gene disruption using a DNA fragment comprising the ura3 ORF promoter and the terminator. NBRC 10746 URA3 (GenBank Accession Number AB242207) comprises an amino acid sequence of 265 amino acids encoded by a 798-bp base sequence (SEQ ID NO: 29, SEQ ID NO: 30). PCR was carried out using the genomic DNA of NBRC 10746 prepared with the use of the Y-DER Yeast DNA Extraction Reagent (78870, PIERCE) as a template, the dURA Fw primer (5'-GGTACCAGTACTGGAAA-3': SEQ ID NO: 5), the dURA rv primer (5'-CAGATAAACAGGCGACT TTTCGGGTCACGTGACT-3': SEQ ID NO: 6), and the PrimeSTAR Max DNA Polymerase (RO45A, Takara Bio Inc.) at 98°C for 10 seconds, 55°C for 5 seconds, and 72°C for 5 seconds, and this cycle was repeated 30 times. Thus, a ura3 terminator region-contaning DNA fragment comprising the ura3 terminator region of about 0.5 kbp and a ura3 promoter region of 17 bp was amplified. Also, PCR was carried out using DNA of NBRC 10746 as a template, the dURA fw primer (5'-AGTCACGTGACCCGAAA AGTCGCCTGTTTATCTG-3': SEQ ID NO: 7), and the dURA Rv primer (5'-CCAAGGAGGAAGAAATT-3': SEQ ID NO: 8) at 98°C for 10 seconds, 55°C for 5 seconds, and 72°C for 5 seconds, and this cycle was repeated 30 times. Thus, a ura3 promotor region- -contaning DNA fragment comprising the ura3 promoter region of about 1.2 kbp and a ura3 terminator region of 17 bp was amplified. PCR was carried out using these DNA fragments as templates, the dURA Fw primer, and the dURA Rv primer at 98°C for 10 seconds, 55°C for 5 seconds, and 72°C for 5 seconds, and this cycle was repeated 30 times. Thus, the ura3 promoter region of about 1.2 kbp was ligated to the terminator region of about 0.5 kbp, and the resultant was then amplified. The ligated DNA fragment was subjected to agarose electrophoresis, recovered, and then designated as a fragment for ura3 gene disruption.

### (2) Preparation of a strain in which the ura3 gene has been disrupted

A fragment for ura3 gene disruption was introduced into the NBRC 10746 strain via electroporation. The resultant was inoculated into 5 ml of YPD medium and cultured at 28°C for 12 to 14 hours up to the logarithmic growth phase (OD₆₀₀ = about 0.5 to 4). The strains were recovered via centrifugation at 1400 × g for 5 minutes and washed once with 10 ml of ice-cooled sterile water and then washed once with 4 ml of ice-cooled sterile water. The strains were suspended in 2 ml of LC buffer (100 mM LiCl, 50 mM potassium phosphate buffer, pH 7.5), the suspension was shaken at 28°C for 45 minutes, 0.05 ml of 1 M DTT was added thereto, and the resultant was shaken for an additional 15 minutes. The strains were recovered via centrifugation at 1400 × g for 5 minutes, the recovered strains were washed with 8 ml of ice-cooled STM buffer (270 mM sucrose, 10 mM Tris-HCl buffer, pH 7.5, 1 mM MgCl₂) and then with 1 ml of STM buffer, and the resultants were suspended in 0.05 ml of ice-cooled STM buffer. Transformation experiment via an electric pulse method was carried out using the Electro Cell Manipulator ECM 600 (BTX). After 0.05 ml of the cell suspension was mixed with 0.005 ml (3 µg) of a DNA sample of a fragment for ura3 gene disruption, the mixture was introduced into a 0.2-cm disposable cuvette, and electric pulses were applied under adequate conditions (voltage: 1.5 kV, 100-200 Ω). Immediately thereafter, YPD medium containing 1 ml of ice-cooled 1 M sorbitol was added, and shake culture was conducted at 28°C for 4 to 6 hours. After culture, the strains were applied to YPD selection medium containing an adequate amount of antibiotics, and the plate was subjected to culture at 28°C to obtain transformed colonies. After electroporation, the resultant was applied to a YPAD agar medium (10 g/l yeast extract, 20 g/l peptone, 20 g/l glucose, 40 mg/l adenine-HCl, and 20 g/l agar) containing 5-FOA (5-fluoroorotic acid) at a final concentration of 0.1% (w/v), and the transformants were allowed to proliferate at 28°C for about 3 days. The proliferated transformants were allowed to proliferate again on YPAD agar medium containing 5-FOA (5-fluoroorotic acid) at a final concentration of 0.1% (w/v). Transformants in which the URA3 gene has been disrupted were selected via colony PCR. Some yeast strains that had proliferated on the YPAD agar medium containing 5-FOA at a final concentration of 0.1% (w/v) were suspended in 10 µl of a 0.25% SDS solution, 90 µl of sterile water was added, and strains were removed via centrifugation at 3,100 × g and 4°C for 5 minutes. The resulting supernatant as a DNA solution was inspected with the use of the dURA check -1.5 kbp primer designed in the upstream sequence of the ura3 promoter (5'-ATCACAGGAAAGCGCAT-3': SEQ ID NO: 9) and the dURA check +1kbp primer designed in the downstream sequence of the ura3 terminator (5'-ATTCGAGCATCGCCGTG-3': SEQ ID NO: 10), and a strain in which a region of about 2.7 kbp without the ura3 coding region has been amplified was designated as the strain in which the ura3 gene has been disrupted (Δura3 strain).

### [Example 3] Preparation of a strain in which the aoxl gene has been disrupted

### (1) Preparation of vector for aoxl gene disruption

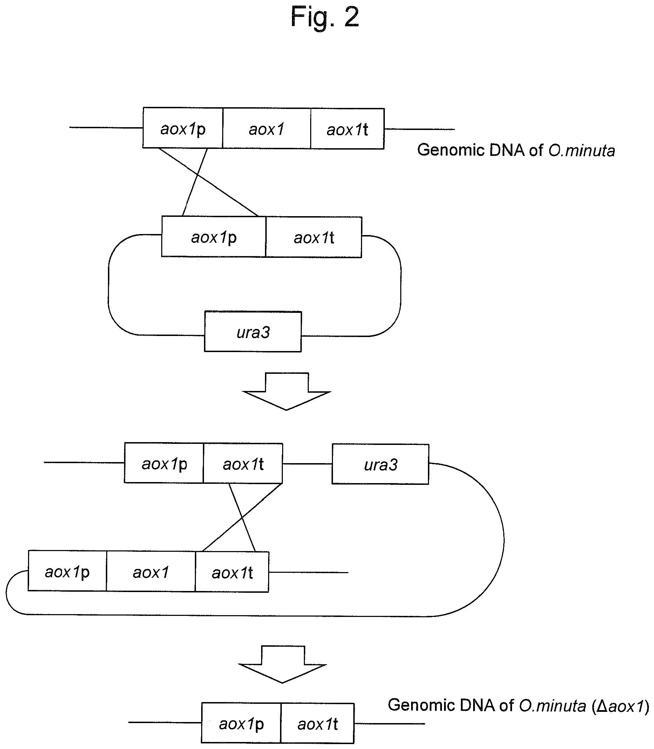

Fig. 2 shows forms of gene disruption using a DNA fragment comprising the aoxl ORF promoter and the terminator region. pOMEA-Z1 constructed in Example 1 was subjected to double digestion with *Hind*III and *Kpn*I to obtain a DNA fragment comprising the aoxl gene promoter and the terminator cassette. The DNA fragment comprising the aoxl gene promoter and the terminator cassette was introduced into the DNA fragment obtained via double digestion of the onaP09007 plasmid described in WO 2009/057813 (i.e., a constant expression vector for a gene encoding OmKar2) with *Hind*III and *Kpn*I to obtain the pOMEU1 plasmid.

### (2) Preparation of a strain in which the aoxl gene has been disrupted

The pOMEU1 plasmid was introduced into the strain in which the ura3 gene has been disrupted (Δura3 strain) described in Example 2 via electroporation under the conditions described in Example 2. The pOMEU1 was digested with the restriction enzyme *Bgnl*II*,* followed by ethanol precipitation, and the resultant DNA (3 µg) was used. After electroporation, the resultant was applied onto a casamino acid-U-A agar medium (6.7 g/l yeast nitrogen base w/o amino acids, 0.5 g/l casamino acid, 20 g/l glucose, 20 mg/l L-tryptophan, and 20 g/l agar), and it was then allowed to proliferate at 28°C for about 3 days. The proliferated transformants were allowed to proliferate again on the casamino acid-U-A agar medium to obtain a strain into which the aoxl gene-disrupting plasmid had been introduced.

Subsequently, the strain into which the aoxl gene-disrupting plasmid had been introduced was inoculated into a 50-ml polypropyrene centrifuge tube (227241, Greiner) containing 5 ml of YPAD medium (10 g/l yeast extract, 20 g/l peptone, 20 g/l glucose, and 40 mg/l adenine-HCl), and the upper part of the centrifuge tube was sealed with CO₂ permeable plate seal (676051, Greiner). After the reciprocal shake culture was conducted at an agitation speed of 250 rpm, an amplitude of 25 mm, and a temperature of 28°C for 2 days, the culture product was applied onto a YPAD medium (1 g/l yeast extract, 2 g/l peptone, 20 g/l glucose, 40 mg/l adenine-HCl, and 20 g/l agar) containing 5-FOA at a final concentration of 0.1% (w/v), and it was then allowed to proliferate at 28°C for about 3 days. The proliferated transformants were allowed to proliferate again on the YPAD medium containing 5-FOA at a final concentration of 0.1% (w/v), and transformants in which the aoxl gene has been disrupted were selected via colony PCR. Some yeast strains that had proliferated on the YPAD medium containing 5-FOA at a final concentration of 0.1% (w/v) were suspended in 10 µl of a 0.25% SDS solution, 90 µl of sterile water was added thereto, and strains were then removed via centrifugation at 3,100 × g and 4°C for 5 minutes. The resulting supernatant as a DNA solution was inspected with the use of the AOX ORF fw primer (5'-ATGGCTATTCCTGACGAATT-3': SEQ ID NO: 11) and the AOX ORF rv primer (5'-TTAGAATCTAGCCAGACCCTTC-3': SEQ ID NO: 12) designed within the aoxl ORF sequence, and a strain in which DNA amplification was not observed was designated as a strain in which the aoxl gene has been disrupted (Δaox1 strain).

### [Example 4] Preparation of strain into which OmPDI1, OmEROl, and OmKar2 chaperones have been introduced

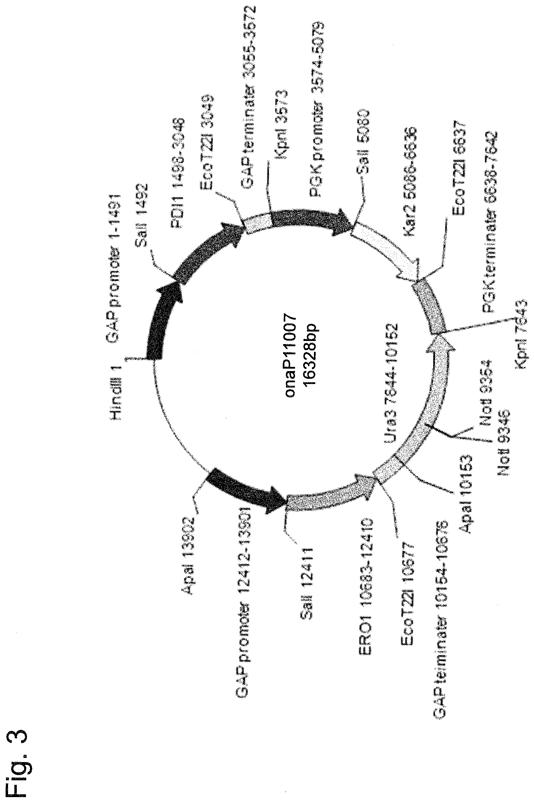

The onaP1 1007 coexpression vector for OmPDI1, OmERO1, and OmKar2 described in WO 2009/057813 was used (Fig. 3). The onaP11007 plasmid was introduced into the NBRC 10746 strain via electroporation. The onaP11007 plasmid was digested with the restriction enzyme *Not*I, followed by ethanol precipitation, and the resultant DNA (1 µg) was used. The constructed onaP11007 was digested with the restriction enzyme *Not*I and then introduced via electroporation described in Example 2. After electroporation, the resultant was applied onto a casamino acid-U-A agar medium (6.7 g/l yeast nitrogen base w/o amino acids, 0.5 g/l casamino acid, 20 g/l glucose, 20 mg/l L-tryptophan, and 20 g/l agar), and it was then allowed to proliferate at 28°C for about 3 days. The proliferated transformants were allowed to proliferate again on the casamino acid-U-A agar medium, and transformants into which a chaperone has been introduced were selected via colony PCR. Some yeast strains that had proliferated on the casamino acid-U-A agar medium were suspended in 10 µl of a 0.25% SDS solution, 90 µl of sterile water was added thereto, and strains were then removed via centrifugation at 3,100 × g and 4°C for 5 minutes. The resulting supernatant as a DNA solution was inspected in the manner described below. That is, introduction of the pdi 1 gene was confirmed by detecting amplification of a DNA fragment of about 1.6 kbp with the use of the GAPpforS-F primer (5'-GATCTCAGGCCGAGTCAAGAC-3': SEQ ID NO: 13) and the OmPDI-END Rv primer (5'-TTACAACTCGTCGTGAGCC-3': SEQ ID NO: 14) designed within the gap promoter sequence. Introduction of the erol gene was confirmed by detecting amplification of a DNA fragment of about 1.6 kbp with the use of the GAPpforS-F primer (5'-GATCTCAGGCCGAGTCAAGAC-3': SEQ ID NO: 13) and the OmERO-END Rv primer (5'-TTATAGCTCCAAACGATACAG-3': SEQ ID NO: 15) designed within the gap promoter sequence. Introduction of the kar2 gene was confirmed by detecting amplification of a DNA fragment of about 2 kbp with the use of the PGKp-END Fw primer (5'-TAAACACTAACGCCGCAT-3': SEQ ID NO: 16) and the OmKar-END Rv primer (5'-TCACAGCTCATCATGATCC-3': SEQ ID NO: 17) designed within the pgk promoter sequence. PCR was carried out using TaKaRa LA Taq™ with GC Buffer (RR02AG, TaKaRa Bio) to amplify a target fragment (a cycle of 94°C for 30 seconds, 55°C for 30 seconds, and 72°C for 120 seconds was repeated 30 times). A transformant in which amplification of interest was observed was designated as a strain into which a chaperone gene had been introduced (ura3: pdil/erol/kar2 strain, NBRC10746+PEK strain).

### [Example 5] Preparation of a strain in which the prbl gene has been interrupted

### (1) Preparation of a vector for interrupting the prb1 gene

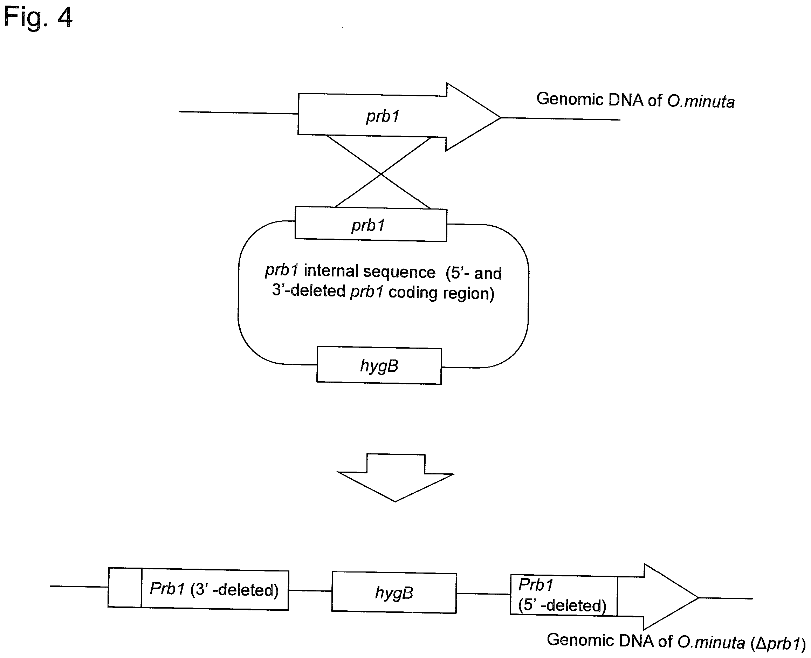

Fig. 4 shows forms of gene disruption using an internal sequence of prbl ORF. NBRC 10746 PRB1 comprises an amino acid sequence of 539 amino acids encoded by a 1,620-bp base sequence (SEQ ID NO: 31, SEQ ID NO: 32). The pOMexPGHy plasmid described in WO 2009/057813 (a vector for foreign gene expression carrying a hygromycin B-resistant gene as a selection marker and comprising a phosphoglycerin kinase (PGK1) promoter and a terminator) was subjected to double digestion with the restriction enzymes *Hin*dIII and *Kpn*I to obtain a DNA fragment comprising the hygromycin-resistant gene marker. PCR was carried out using the genomic DNA of NBRC 10746 prepared with the use of the Y-DER Yeast DNA Extraction Reagent (78870, PIERCE) as a template, the DoprblF primer (5'-CAAGCTTCGTTGGCAGCAGTGGAG-3': SEQ ID NO: 18) and the DoprblR primer (5'-CGGTACCCGATGGAATCTCAGACA-3': SEQ ID NO: 19) designed within PRB1 ORF, and PrimeStarmax Polymerase (12344-024, TaKaRa Bio) at 98°C for 10 seconds, 55°C for 5 seconds, and 72°C for 5 seconds, and this cycle was repeated 30 times. Thus, a target DNA fragment of about 1.4 kbp as shown in SEQ ID NO: 20 was amplified and then cloned into pCR2.1-TOPO. The base sequence of the inserted DNA fragment was inspected, and it was confirmed to have a prb1 internal sequence. A DNA fragment comprising the prb1 internal sequence was recovered via *Hind*III*-Kpn*I digestion from the plasmid carrying the DNA fragment in which the base sequence had been inspected with the use of the restriction enzyme *Hind*III site that had been introduced into the DoprblF primer and the restriction enzyme *Kpn*I site that had been introduced into the DoprblR primer. The DNA fragment comprising the prb1 internal sequence was introduced into the DNA fragment comprising the hygromycin-resistant gene marker to obtain the pdPRB1 plasmid.

### (2) Preparation of a strain in which the prbl gene has been interrupted

The pdPRB 1 plasmid was introduced into the NBRC 10746 strain via electroporation. The pdPRB 1 was digested with the restriction enzyme *Age*I, followed by ethanol precipitation, and the resultant DNA (5 µg) was used. After electroporation had been conducted under the conditions described in Example 2, the resultant was applied onto the casamino acid-U-A agar medium (6.7 g/l yeast nitrogen base w/o amino acids, 0.5 g/l casamino acid, 20 g/l glucose, 20 mg/l L-tryptophan, and 20 g/l agar) containing hygromycin B at a final concentration of 200 µg/ml, and it was then allowed to proliferate at 28°C for about 3 days. The proliferated transformants were allowed to proliferate again on the casamino acid-U-A agar medium containing hygromycin B at a final concentration of 200 µg/ml, and transformants into which the pdPRB 1 plasmid had been introduced were selected via colony PCR. Some yeast strains that had proliferated on the casamino acid-U-A agar medium containing hygromycin B at a final concentration of 200 µg/ml were suspended in 10 µl of a 0.25% (w/v) SDS solution, 90 µl of sterile water was added thereto, and strains were then removed via centrifugation at 3,100 × g and 4°C for 5 minutes. The resulting supernatant as a DNA solution was inspected with the use of the M13 RV primer (5'-CAGGAAACAGCTATGAC-3': SEQ ID NO: 21) designed within the PRB1 ORF sequence and the dprbl check rv primer (5'-CTAATCGAACAAATCAGCAACC-3': SEQ ID NO: 22) designed within the pdPRB1 sequence, and a strain in which DNA amplification of about 1.5 kbp was observed was identified. Also, the DNA solution was inspected with the use of the dprbl check fw primer (5'-ATGAAGTTATCCCAGTCTGCTG-3': SEQ ID NO: 23) designed within the prbl ORF sequence and the Hyg-t primer (5'-CAAAGGAATAGATCCCCCAT-3': SEQ ID NO: 24) designed in the hygromycin-resistant gene within the pdPRB 1 sequence, and a strain in which DNA amplification of about 2 kbp was observed was identified. These identified strains were designated as strains in which the prbl gene had been interrupted (prb1::hyg, dprbl strain).

### [Example 6] Preparation of a strain into which a chaperone gene has been introduced and in which the prb1 gene has been interrupted

The pdPRB1 plasmid was introduced into the NBRC10746+PEK strain described in Example 4 via electroporation. The pdPRB 1 was digested with the restriction enzyme *Age*I*,* followed by ethanol precipitation, and the resultant DNA (5 µg) was used. After electroporation had been conducted under the conditions described in Example 2, the resultant was applied onto the casamino acid-U-A agar medium (6.7 g/l yeast nitrogen base w/o amino acids, 0.5 g/l casamino acid, 20 g/l glucose, 20 mg/l L-tryptophan, and 20 g/l agar) containing hygromycin B at a final concentration of 200 µg/ml, and it was then allowed to proliferate at 28°C for about 3 days. The proliferated transformants were allowed to proliferate again on the casamino acid-U-A agar medium containing hygromycin B at a final concentration of 200 µg/ml, and transformants into which the pdPRB1 plasmid had been introduced were selected via colony PCR. Some yeast strains that had proliferated on the casamino acid-U-A agar medium containing hygromycin B at a final concentration of 200 µg/ml were suspended in 10 µl of a 0.25% (w/v) SDS solution, 90 µl of sterile water was added thereto, and strains were then removed via centrifugation at 3,100 × g and 4°C for 5 minutes. The resulting supernatant as a DNA solution was inspected with the use of the M13 RV primer (5'-CAGGAAACAGCTATGAC-3': SEQ ID NO: 21) designed within the prbl ORF sequence and the dprbl check rv primer (5'-CTAATCGAACAAATCAGCAACC-3': SEQ ID NO: 22) designed within the pdPRB 1 sequence, and a strain in which DNA amplification of about 1.5 kbp was observed was identified. Also, the DNA solution was inspected with the use of the dprbl check fw primer (5'-ATGAAGTTATCCCAGTCTGCTG-3': SEQ ID NO: 23) designed within the prbl ORF sequence and the Hyg-t primer (5'-CAAAGGAATAGATCCCCCAT-3': SEQ ID NO: 24) designed in the hygromycin-resistant gene within the pdPRB 1 sequence, and a strain in which DNA amplification of about 2 kbp was observed was identified. These identified strains were designated as strains into which a chaperone had been introduced and the prb1 gene had been interrupted (ura3::pdi1/ero1/kar2 prb 1::hyg, NBRC 10746+PEK dprb1 strains).

### [Example 7] Preparation of a strain in which the aoxl gene has been disrupted and the chaperone has been introduced

The onaP11007 plasmid was introduced into the Δaox1 strain described in Example 3 via electroporation. The onaP11007 was digested with the restriction enzyme *Not*I*,* followed by ethanol precipitation, and the resultant DNA (1 µg) was used. After electroporation had been conducted under the conditions described in Example 2, the resultant was applied onto the casamino acid-U-A agar medium (6.7 g/l yeast nitrogen base w/o amino acids, 0.5 g/l casamino acid, 20 g/l glucose, 20 mg/l L-tryptophan, and 20 g/l agar), and it was then allowed to proliferate at 28°C for about 3 days. The proliferated transformants were allowed to proliferate again on the casamino acid-U-A agar medium, and transformants into which a chaperone gene had been introduced were selected via colony PCR. Some yeast strains that had proliferated on the casamino acid-U-A agar medium were suspended in 10 µl of a 0.25% SDS solution, 90 µl of sterile water was added thereto, and strains were then removed via centrifugation at 3,100 × g and 4°C for 5 minutes.

The resulting supernatant as a DNA solution was inspected in the manner described below. That is, introduction of the pdi1 gene was confirmed by detecting amplification of a DNA fragment of about 1.6 kbp with the use of the GAPpforS-F primer (5'-GATCTCAGGCCGAGTCAAGAC-3': SEQ ID NO: 13) and the OmPDI-END Rv primer (5'-TTACAACTCGTCGTGAGCC-3': SEQ ID NO: 14) designed within the gap promoter sequence. Introduction of the ero1 gene was confirmed by detecting amplification of a DNA fragment of about 1.6 kbp with the use of the GAPpforS-F primer (5'-GATCTCAGGCCGAGTCAAGAC-3': SEQ ID NO: 13) and the OmERO-END Rv primer (5'-TTATAGCTCCAAACGATACAG-3': SEQ ID NO: 15) designed within the gap promoter sequence. Introduction of the kar2 gene was confirmed by detecting amplification of a DNA fragment of about 2 kbp with the use of the PGKp-END Fw primer (5'-TAAACACTAACGCCGCAT-3': SEQ ID NO: 16) and the OmKar-END Rv primer (5'-TCACAGCTCATCATGATCC-3': SEQ ID NO: 17) designed within the pgk promoter sequence. PCR was carried out using TaKaRa LA Taq™ with GC Buffer (RR02AG, TaKaRa Bio) to amplify a target fragment (a cycle of 94°C for 30 seconds, 55°C for 30 seconds, and 72°C for 120 seconds was repeated 30 times). Transformants in which amplification of interest was observed were designated as strains in which the aox1 gene had been disrupted and the chaperone gene had been introduced (Δaox1, ura3::pdi1/ero1/kar2, Δaox1+PEK strains).

### [Example 8] Preparation of a strain in which the aoxl gene has been disrupted, the chaperone has been introduced, and the prb1 gene has been interrupted

The pdPRB1 plasmid was introduced into the Δaox1+PEK strain described in Example 7 via electroporation. The pdPRB1 plasmid was digested with the restriction enzyme *Age*I*,* followed by ethanol precipitation, and the resultant DNA (5 µg) was used. After electroporation had been conducted under the conditions described in Example 2, the resultant was applied onto the casamino acid-U-A agar medium (6.7 g/l yeast nitrogen base w/o amino acids, 0.5 g/l casamino acid, 20 g/l glucose, 20 mg/l L-tryptophan, and 20 g/l agar) containing hygromycin B at a final concentration of 200 µg/ml, and it was then allowed to proliferate at 28°C for about 3 days. The proliferated transformants were allowed to proliferate again on the casamino acid-U-A agar medium containing hygromycin B at a final concentration of 200 µg/ml, and transformants into which the pdPRB1 plasmid had been introduced were selected via colony PCR. Some yeast strains that had proliferated on the casamino acid-U-A agar medium containing hygromycin B at a final concentration of 200 µg/ml were suspended in 10 µl of a 0.25% (w/v) SDS solution, 90 µl of sterile water was added thereto, and strains were then removed via centrifugation at 3,100 × g and 4°C for 5 minutes.

The resulting supernatant as a DNA solution was inspected with the use of the M13 RV primer (5'-CAGGAAACAGCTATGAC-3': SEQ ID NO: 21) designed within the prbl ORF sequence and the dprbl check rv primer (5'-CTAATCGAACAAATCAGCAACC-3': SEQ ID NO: 22) designed within the pdPRB1 sequence, and a strain in which DNA amplification of about 1.5 kbp was observed was identified. Also, the DNA solution was inspected with the use of the dprbl check fw primer (5'-ATGAAGTTATCCCAGTCTGCTG-3': SEQ ID NO: 23) designed within the prbl ORF sequence and the Hyg-t primer (5'-CAAAGGAATAGATCCCCCAT-3': SEQ ID NO: 24) designed in the hygromycin-resistant gene within the pdPRB1 sequence, and a strain in which DNA amplification of about 2 kbp was observed was identified. These identified strains were designated as strains in which the aoxl gene had been disrupted, into which the chaperone had been introduced, and in which the prbl gene had been interrupted (Δaox1, ura3::pdi1/ero1/kar2, prb1::hyg, Δaox1+PEK dprbl strains).

### [Example 9] Amount of KEX2 protein secretion induced by methanol at deep well plate scale

### (1) Preparation of kex2 expression plasmid

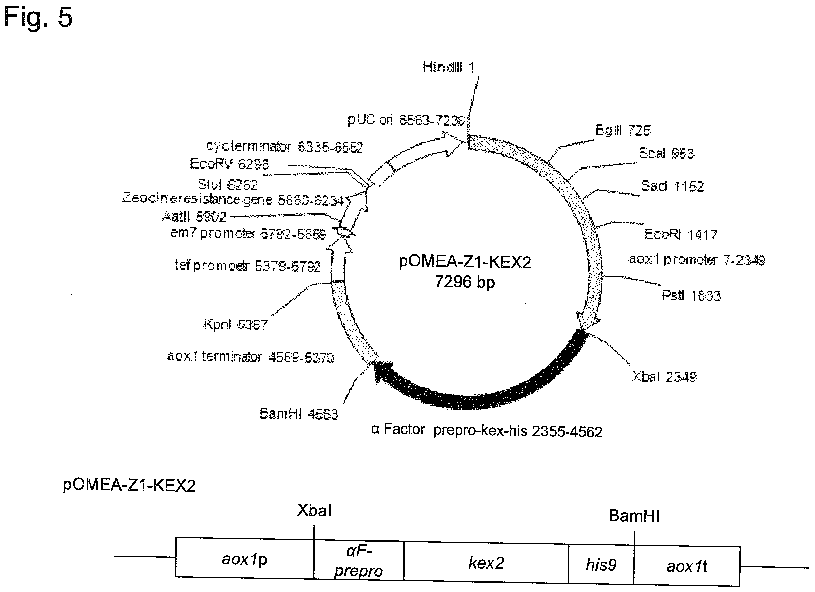

The KEX2 protein derived from *S. cerevisiae* (GenBank Accession Number M22870.1) comprises an amino acid sequence of 814 amino acid residues (SEQ ID NO: 33). In order to express an α-factor pre-sequence, an α-factor pro-sequence, KEX2 (amino acids 24 to 660 of SEQ ID NO: 33), and His9-tag with 9 His tags in the form of a fusion protein, the base sequence as shown in SEQ ID NO: 25 was artificially synthesized at Life technologies in accordance with the codon usage frequency of *P. pastoris.* A vector comprising the artificially synthesized base sequence was digested with the restriction enzymes *Xba*I and *Bam*HI*,* and the resultant was introduced into a fragment obtained by digesting pOMEA-Z1 prepared in Example 1 with the restriction enzymes *Xba*I and *Bam*HI*.* The resulting plasmid was designated as the kex2 expression plasmid (pOMEA-Z1-KEX2) (Fig. 5).

### (2) Preparation of KEX2-producing strain

### (2-1) Preparation of KEX2-producing strain derived from chaperone-introduced strain

The kex2 expression plasmid was introduced into the NBRC10746+PEK strain described in Example 4 via electroporation. The kex2 expression plasmid was digested with the restriction enzyme *Bgl*II, followed by ethanol precipitation, and the resultant DNA (1 µg) was used. After electroporation had been conducted under the conditions described in Example 2, the resultant was applied onto the casamino acid-U-A agar medium (6.7 g/l yeast nitrogen base w/o amino acids, 0.5 g/l casamino acid, 20 g/l glucose, 20 mg/l L-tryptophan, and 20 g/l agar) containing Zeocin at a final concentration of 200 µg/ml, and it was then allowed to proliferate at 28°C for about 3 days. The proliferated transformants were allowed to proliferate again on the casamino acid-U-A agar medium containing Zeocin at a final concentration of 200 µg/ml.

Strains capable of high levels of production of KEX2 were selected by conducting culture in the manner described below. A 2xYP-P6-dp medium [wherein the 2xYP-P6-dp medium was prepared by dissolving 20 g of a yeast extract and 40 g of peptone in 900 ml of pure water, subjecting the solution to high-pressure steam sterilization, and adding 100 ml of a separately sterilized 10x phosphate buffer (pH 6.0) (1 M KH₂PO₄, 0.15 M (NH₄)₂SO₄, 0.355 N KOH), 10 ml of a separately sterilized 50% glucose solution, and 6.25 ml of separately sterilized 80% glycerin] was used. The 2xYP-P6-dpn medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days, 100 µl of the 2xYP-P6-dpn medium containing 40 µM rhodanine-3-acetic acid derivative 1c and 10%(v/v) methanol [wherein the 2xYP-P6-dpn medium was prepared by dissolving 20 g of a yeast extract and 40 g of peptone in 900 ml of pure water, subjecting the solution to high-pressure steam sterilization, and adding 100 ml of a separately sterilized 10x phosphate buffer (pH 6.0) (1 M KH₂PO₄, 0.15 M (NH₄)₂SO₄, 0.355 N KOH)] was added, and 100 µl of the 2xYP-P6-dpn medium containing 40 µM rhodanine-3-acetic acid derivative 1c and 10%(v/v) methanol was further added 3 days after the initiation of culture. The strains were removed from the culture solution via centrifugation at 3,100 × g and 4°C for 5 minutes 4 days after the initiation of culture, and the resulting culture supernatant was designated as a KEX2-producing sample. Quantitative assays of the KEX2 that had been secreted and produced were performed in accordance with the dot blot technique. An SDS-PAGE buffer (SDS/β-mercaptoethanol) was added, the reaction was allowed to proceed at 100°C for 5 minutes, 1 µl of the resulting culture supernatant was added dropwise to a nitrocellulose membrane to adsorb the proteins in the culture supernatant, and the KEX2-producing strain was selected via luminescence detection using the ECL Select™ Western Blotting Detection Reagent (RPN2235, GE Healthcare) and the peroxidase-labeled penta-His-specific antibody (Penta-His HRP Conjugate Kit, 34460, QIAGEN). The selected KEX2-producing strain was designated as the KEX2-producing strain derived from the chaperone-introduced strain (ura3::pdi1/ero1/kar2, NBRC10746+PEK strain).

### (2-2) Preparation of KEX2-producing strain derived from a strain into which a chaperone had been introduced and in which the prbl gene had been interrupted

The kex2 expression plasmid was introduced into the NBRC10746+PEK dprbl strain described in Example 6 via electroporation. The kex2 expression plasmid was digested with the restriction enzyme *Bgl*II*,* followed by ethanol precipitation, and the resultant DNA (1 µg) was used. After electroporation had been conducted under the conditions described in Example 2, the resultant was applied onto the casamino acid-U-A agar medium containing Zeocin at a final concentration of 200 µg/ml and hygromycin B at a final concentration of 200 µg/ml, and it was then allowed to proliferate at 28°C for about 2 or 3 days. The proliferated transformants were allowed to proliferate again on the casamino acid-U-A agar medium containing Zeocin at a final concentration of 200 µg/ml and hygromycin B at a final concentration of 200 µg/ml.

Strains capable of high levels of production of KEX2 were selected by conducting culture in the manner described below. The 2xYP-P6-dpn medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days, 100 µl of the 2xYP-P6-dpn medium containing 40 µM rhodanine-3-acetic acid derivative 1c and 10%(v/v) methanol was added, and 100 µl of the 2xYP-P6-dpn medium containing 40 µM rhodanine-3-acetic acid derivative 1c and 10%(v/v) methanol was further added 3 days after the initiation of culture. The strains were removed from the culture solution via centrifugation at 3,100 × g and 4°C for 5 minutes 4 days after the initiation of culture, and the resulting culture supernatant was designated as a KEX2-producing sample. Quantitative assays of the KEX2 that had been secreted and produced were performed in accordance with the dot blot technique. An SDS-PAGE buffer (SDS/β-mercaptoethanol) was added, the reaction was allowed to proceed at 100°C for 5 minutes, 1 µl of the resulting culture supernatant was added dropwise to a nitrocellulose membrane to adsorb the proteins in the culture supernatant, and the KEX2-producing strain was selected via luminescence detection using the ECL Select™ Western Blotting Detection Reagent (RPN2235, GE Healthcare) and the peroxidase-labeled penta-His-specific antibody (Penta-His HRP Conjugate Kit, 34460, QIAGEN). The selected KEX2-producing strain was designated as the KEX2-producing strain derived from the strain into which a chaperone had been introduced and in which the prbl gene had been interrupted (ura3::pdi1/ero1/kar2, prb1::hyg, NBRC10746+PEK dprbl).

### (2-3) Preparation of KEX2-producing strain derived from a strain in which the aoxl gene had been disrupted and into which the chaperone had been introduced

The kex2 expression plasmid was introduced into the Δaox1+PEK strain described in Example 7 via electroporation. The kex2 expression plasmid was digested with the restriction enzyme *Bgl*II, followed by ethanol precipitation, and the resultant DNA (1 µg) was used. After electroporation had been conducted under the conditions described in Example 2, the resultant was applied onto the casamino acid-U-A agar medium containing Zeocin at a final concentration of 200 µg/ml, and it was then allowed to proliferate at 28°C for about 2 or 3 days. The proliferated transformants were allowed to proliferate again on the casamino acid-U-A agar medium containing Zeocin at a final concentration of 200 µg/ml.

Strains capable of high levels of production of KEX2 were selected by conducting culture in the manner described below. The 2xYP-P6-dp medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days, 100 µl of the 2xYP-P6-dpn medium containing 40 µM rhodanine-3-acetic acid derivative 1c, 5%(w/v) glycerin, and 5%(v/v) methanol was added, and 100 µl of the 2xYP-P6-dpn medium containing 40 µM rhodanine-3-acetic acid derivative 1c and 5%(w/v) glycerin was further added 3 days after the initiation of culture. The strains were removed from the culture solution via centrifugation at 3,100 × g and 4°C for 5 minutes 4 days after the initiation of culture, and the resulting culture supernatant was designated as a KEX2-producing sample. Quantitative assays of the KEX2 that had been secreted and produced were performed in accordance with the dot blot technique. The Tris-SDS β-MF sample treatment solution (5 µl, 423437, Cosmo Bio, Co., Ltd.) was added to 5 µl of the culture supernatant, 1 µl of the culture supernatant resulting from a reaction conducted at 100°C for 5 minutes was added dropwise to a nitrocellulose membrane to adsorb the proteins in the culture supernatant, and the KEX2-producing strain was selected via luminescence detection using the ECL Select™ Western Blotting Detection Reagent (RPN2235, GE Healthcare) and the peroxidase-labeled penta-His-specific antibody (Penta-His HRP Conjugate Kit, 34460, QIAGEN). The selected KEX2-producing strain was designated as the KEX2-producing strain derived from the strain in which the aoxl gene had been disrupted and the chaperone had been introduced (Δaox1, ura3::pdi1/ero1/kar2, Δaox1+PEK strain).

### (2-4) Preparation of KEX2-producing strain derived from a strain in which the aoxl gene had been disrupted, into which the chaperone had been introduced, and in which the prbl gene had been interrupted

The kex2 expression plasmid was introduced into the Δaox1+PEK dprbl strain described in Example 8 via electroporation. The kex2 expression plasmid was digested with the restriction enzyme *Bgl*II, followed by ethanol precipitation, and the resultant DNA (1 µg) was used. After electroporation had been conducted under the conditions described in Example 2, the resultant was applied onto the casamino acid-U-A agar medium containing Zeocin at a final concentration of 200 µg/ml and hygromycin B at a final concentration of 200 µg/ml, and it was then allowed to proliferate at 28°C for about 2 or 3 days. The proliferated transformants were allowed to proliferate again on the casamino acid-U-A agar medium containing Zeocin at a final concentration of 200 µg/ml and hygromycin B at a final concentration of 200 µg/ml.

Strains capable of high levels of production of KEX2 were selected by conducting culture in the manner described below. The 2xYP-P6-dp medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days, 100 µl of the 2xYP-P6-dpn medium containing 40 µM rhodanine-3-acetic acid derivative 1c, 5%(w/v) glycerin, and 5%(v/v) methanol was added, and 100 µl of the 2xYP-P6-dpn medium containing 40 µM rhodanine-3-acetic acid derivative 1c and 5% glycerin was further added 3 days after the initiation of culture. The strains were removed from the culture solution via centrifugation at 3,100 × g and 4°C for 5 minutes 4 days after the initiation of culture, and the resulting culture supernatant was designated as a KEX2-producing sample. Quantitative assays of the KEX2 that had been secreted and produced were performed in accordance with the dot blot technique. The Tris-SDS β-ME sample treatment solution (5 µl, 423437, Cosmo Bio, Co., Ltd.) was added to 5 µl of the culture supernatant, 1 µl of the culture supernatant resulting from a reaction conducted at 100°C for 5 minutes was added dropwise to a nitrocellulose membrane to adsorb the proteins in the culture supernatant, and the KEX2-producing strain was selected via luminescence detection using the ECL Select™ Western Blotting Detection Reagent (RPN2235, GE Healthcare) and the peroxidase-labeled penta-His-specific antibody (Penta-His HRP Conjugate Kit, 34460, QIAGEN). The selected KEX2-producing strain was designated as the KEX2-producing strain derived from the strain in which the aoxl gene had been disrupted, into which the chaperone had been introduced, and in which the prb1 gene had been interrupted (Δaox1, ura3::pdi1/ero1/kar2, prb1::hyg, Δaox1+PEK dprb1 strain).

### (3) Comparison of secretory production amounts of KEX2

The KEX2-secreting and producing strains obtained above were applied onto the casamino acid-U-A agar medium containing Zeocin at a final concentration of 200 µg/ml (in the case of dprbl-derived strain, contaning Zeocin at a final concentration of 200 µg/ml and hygromycin B at a final concentration of 200 µg/ml), and the strains were allowed to proliferate at 28°C for about 2 days. The 2xYP-P6-dp medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days. In the case of the NBRC10746+PEK- and NBRC10746+PEK dprbl-derived strains, thereafter, 100 µl of the 2xYP-P6-dpn medium containing 40 µM of rhodanine-3-acetic acid derivative 1c and 10%(v/v) methanol was added, and 100 µl of the 2xYP-P6-dpn medium containing 40 µM of rhodanine-3-acetic acid derivative 1c and 10%(v/v) methanol was further added 3 days after the initiation of culture. In the case of the Δaox1+PEK- and Δaox1+PEK dprbl-derived strains, 100 µl of the 2xYP-P6-dpn medium containing 40 µM rhodanine-3-acetic acid derivative 1c, 5%(w/v) glycerin, and 5%(v/v) methanol was added, and 100 µl of the 2xYP-P6-dpn medium containing 40 µM rhodanine-3-acetic acid derivative 1c and 5%(w/v) glycerin was further added 3 days after the initiation of culture. The strains were removed from the culture solution via centrifugation at 3,100 × g and 4°C for 5 minutes 4 days after the initiation of culture, and the resulting culture supernatant was designated as a KEX2-producing sample.

The KEX2-producing samples were compared by removing the N-linked sugar chain added to the produced KEX2 with the aid of a sugar chain cleavage enzyme (Endo H, P0702S, NEW ENGLAND Bio Labs) and observing the band intensities of the samples via SDS-PAGE/CBB staining. Band intensity was determined by photographing the SDS-PAGE gel using a Light-Capture (ATTO) and analyzing the photographs using analytical software (Cool Saver 2, Version 1.01.1058, ATTO). As shown in Fig. 6-1, the control sample (NBRC10746+PEK strain) exhibited a band intensity of 81178, and the NBRC10746+PEK dprbl strain exhibited a band intensity of 93277. That is, the NBRC10746+PEK dprbl strain exhibited improvement in secretory production that was about 1.1 times greater than that of the control sample. Meanwhile, the Δaox1+PEK dprbl strain exhibited a band intensity of 143819 with the addition of methanol at a lower concentration than that in the case of the NBRC10746+PEK strain and the NBRC10746+PEK dprbl strain. That is, the Δaox1+PEK dprbl strain exhibited improvement in secretory production that was about 1.8 times greater than that of the control sample. In particular, the Δaox1+PEK dprbl strain exhibited high-level secretory productivity with the addition of methanol at a lower concentration than that in the case of the control sample.

### [Example 10] Comparison of KEX2 protein enzyme activity

Fig. 6-2 shows enzyme activity of KEX2 secreted and produced in Example 9. Enzyme activity was evaluated by fluorescence detection of AMC (7-amino-4-methylcoumarin) released upon the reaction of KEX2 with Boc-Leu-Arg-Arg-MCA (4-methylcoumaryl-7-amide) (#3140-v, Peptide Institute, Inc.) as a substrate. The strains were removed from the culture supernatant via centrifugation at 3,100 × g and 4°C for 5 minutes, and the resulting culture supernatant was designated as a KEX2-producing sample. After the sample had been adequately diluted with 100 mM Tris (pH 7.0), 100 µl of the diluted sample was mixed with 100 µl of the substrate solution (400 mM Tris (pH 7.0), 2 mM CaCl₂, 0.2% lubrol, 100 µM BOC-Leu-Arg-Arg-MCA), the mixture was subjected to reaction at 28°C for 30 minutes, and 50 µl of a reaction terminator (5 mM EGTA (Na)) was added to terminate the reaction. Thereafter, AMC was quantified using a fluorescence plate reader (excitation wavelength: 355 nm; measurement wavelength: 460 nm). The AMC standard sample (#3099-v, manufactured by Peptide Institute, Inc.) was diluted and subjected to detection in the same manner, so as to prepare a calibration curve, and the KEX2 protease activity in the sample was determined. A unit of KEX2 activity was defined as the KEX2 content that releases 1 pmol of AMC every minute under the reaction conditions described above. The Δaox1+PEK dprb1 strain exhibited improvement in enzyme activity that was about 1.8 times greater than that of the control (the NBRC 10746+PEK strain).

### [Example 11] Amount of HSA protein secretion induced by methanol at deep well plate scale

### (1) Preparation of hsa expression plasmid

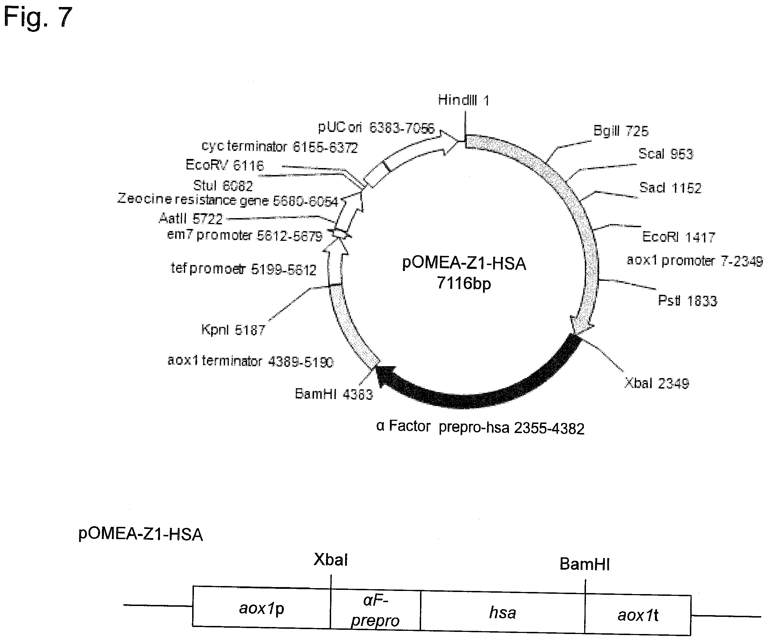

HSA (human serum albumin) (GenBank Accession Number NP000468) comprises an amino acid sequence of 609 amino acids (SEQ ID NO: 34). In order to express an α-factor pre-sequence and an α-factor pro-sequence derived from *S. cerevisiae* and HSA in the form of a fusion protein, DNA as shown in SEQ ID NO: 26 was artificially synthesized at Life technologies in accordance with the codon usage frequency of *P. pastoris.* A vector comprising the artificially synthesized DNA was digested with the restriction enzymes *Xba*I and *Bam*HI*,* and the resultant was introduced into a fragment obtained by digesting pOMEA-Z1 with the restriction enzymes *Xba*I and *Bam*HI*.* The resulting plasmid was designated as the hsa expression plasmid (pOMEA-Zl-HSA) (Fig. 7).

### (2) Preparation of HSA-producing strain

### (2-1) Preparation of HSA-producing strain derived from chaperone-introduced strain

The hsa expression plasmid was introduced into the NBRC10746+PEK strain described in Example 4 via electroporation. The hsa expression plasmid was digested with the restriction enzyme *BglII,* followed by ethanol precipitation, and the resultant DNA (1 µg) was used. After electroporation had been conducted under the conditions described in Example 2, the resultant was applied onto the casamino acid-U-A agar medium (6.7 g/l yeast nitrogen base W/O amino acids, 0.5 g/l casamino acid, 20 g/l glucose, 20 mg/l L-tryptophan, and 20 g/l agar) containing Zeocin at a final concentration of 200 µg/ml, and it was then allowed to proliferate at 28°C for about 2 or 3 days. The proliferated transformants were allowed to proliferate again on the casamino acid-U-A agar medium containing Zeocin at a final concentration of 200 µg/ml.

Strains capable of high levels of production of HSA were selected by conducting culture in the manner described below. A 2xYP-P6-dp medium [wherein the 2xYP-P6-dp medium was prepared by dissolving 20 g of a yeast extract and 40 g of peptone in 900 ml of pure water, subjecting the solution to high-pressure steam sterilization, and adding 100 ml of a separately sterilized 10x phosphate buffer (pH 6.0) (1 M KH₂PO₄, 0.15 M (NH₄)₂SO₄, 0.355 N KOH), 10 ml of a separately sterilized 50% glucose solution, and 6.25 ml of separately sterilized 80% glycerin] was used. The 2xYP-P6-dp medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days, 100 µl of the 2xYP-P6-dpn medium containing 40 µM rhodanine-3-acetic acid derivative 1c and 10%(v/v) methanol [wherein the 2xYP-P6-dp medium was prepared by dissolving 20 g of a yeast extract and 40 g of peptone in 900 ml of pure water, subjecting the solution to high-pressure steam sterilization, and adding 100 ml of a separately sterilized 10x phosphate buffer (pH 6.0) (1 M KH₂PO₄, 0.15 M (NH₄)₂SO₄, 0.355 N KOH)] was added, and 100 µl of the 2xYP-P6-dpn medium containing 40 µM rhodanine-3-acetic acid derivative 1c and 10%(v/v) methanol was further added 3 days after the initiation of culture. The strains were removed from the culture solution via centrifugation at 3,100 × g and 4°C for 5 minutes 4 days after the initiation of culture, and the resulting culture supernatant was designated as a HSA-producing sample. Quantitative assays of the HSA that had been secreted and produced were performed in accordance with the dot blot technique. The Tris-SDS b-ME sample treatment solution (5 µl, 423437, Cosmo Bio, Co., Ltd.) was added to 5 µl of the culture supernatant, 1 µl of the culture supernatant resulting from a reaction conducted at 100°C for 5 minutes was added dropwise to a nitrocellulose membrane to adsorb the proteins in the culture supernatant, and the HSA-producing strain was selected via luminescence detection using the ECL Select™ Western Blotting Detection Reagent (RPN2235, GE Healthcare) and the peroxidase-labeled human albumin-specific antibody (Goat anti-Human Albumin-HRP Conjugated) (A80-129P, BETHYL). The selected HSA-producing strain was designated as the HSA-producing strain derived from the chaperone-introduced strain (ura3::pdi1/ero1/kar2, NBRC10746+PEK strain).

### (2-2) Preparation of HSA-producing strain derived from a strain into which a chaperone had been introduced and in which the prb1 gene had been interrupted

The hsa expression plasmid was introduced into the NBRC10746+PEK dprbl strain described in Example 6 via electroporation. The hsa expression plasmid was digested with the restriction enzyme *Bgl*II, followed by ethanol precipitation, and the resultant DNA (1 µg) was used. After electroporation had been conducted under the conditions described in Example 2, the resultant was applied onto the casamino acid-U-A agar medium containing Zeocin at a final concentration of 200 µg/ml and hygromycin B at a final concentration of 200 µg/ml, and it was then allowed to proliferate at 28°C for about 2 or 3 days. The proliferated transformants were allowed to proliferate again on the casamino acid-U-A agar medium containing Zeocin at a final concentration of 200 µg/ml and hygromycin B at a final concentration of 200 µg/ml.

Strains capable of high levels of production of HSA were selected by conducting culture in the manner described below. The 2xYP-P6-dp medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days, 100 µl of the 2xYP-P6-dpn medium containing 40 µM rhodanine-3-acetic acid derivative 1c and 10%(v/v) methanol was added, and 100 µl of the 2xYP-P6-dpn medium containing 40 µM rhodanine-3-acetic acid derivative 1c and 10%(v/v) methanol was further added 3 days after the initiation of culture. The strains were removed from the culture solution via centrifugation at 3,100 × g and 4°C for 5 minutes 4 days after the initiation of culture, and the resulting culture supernatant was designated as a HSA-producing sample. Quantitative assays of the HSA that had been secreted and produced were performed in accordance with the dot blot technique. An SDS-PAGE buffer (SDS/β-mercaptoethanol) was added, the reaction was allowed to proceed at 100°C for 5 minutes, 1 µl of the resulting culture supernatant was added dropwise to a nitrocellulose membrane to adsorb the proteins in the culture supernatant, and the HSA-producing strain was selected via luminescence detection using the ECL Select™ Western Blotting Detection Reagent (RPN2235, GE Healthcare) and the peroxidase-labeled human albumin-specific antibody (Goat anti-Human Albumin-HRP Conjugated) (A80-129P, BETHYL). The selected HSA-producing strain was designated as the HSA-producing strain derived from the strain into which a chaperone had been introduced and the prbl gene had been interrupted (ura3::pdi1/ero1/kar2, prb1::hyg, NBRC10746+PEK dprbl).

### (2-3) Preparation of HSA-producing strain derived from a strain in which the aoxl gene had been disrupted and into which the chaperone had been introduced

The hsa expression plasmid was introduced into the Δaox1+PEK strain described in Example 7 via electroporation. The hsa expression plasmid was digested with the restriction enzyme *Bgl*II, followed by ethanol precipitation, and the resultant DNA (1 µg) was used. After electroporation had been conducted under the conditions described in Example 2, the resultant was applied onto the casamino acid-U-A agar medium containing Zeocin at a final concentration of 200 µg/ml, and it was then allowed to proliferate at 28°C for about 2 or 3 days. The proliferated transformants were allowed to proliferate again on the casamino acid-U-A agar medium containing Zeocin at a final concentration of 200 µg/ml.

Strains capable of high levels of production of HSA were selected by conducting culture in the manner described below. The 2xYP-P6-dp medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days, 100 µl of the 2xYP-P6-dpn medium containing 40 µM rhodanine-3-acetic acid derivative 1c, 5%(w/v) glycerin, and 5%(v/v) methanol was added, and 100 µl of the 2xYP-P6-dpn medium containing 40 µM rhodanine-3-acetic acid derivative 1c and 5%(w/v) glycerin was further added 3 days after the initiation of culture. The strains were removed from the culture solution via centrifugation at 3,100 × g and 4°C for 5 minutes 4 days after the initiation of culture, and the resulting culture supernatant was designated as a HSA-producing sample. Quantitative assays of the HSA that had been secreted and produced were performed in accordance with the dot blot technique. The Tris-SDS β-ME sample treatment solution (5 µl, 423437, Cosmo Bio, Co., Ltd.) was added to 5 µl of the culture supernatant, 1 µl of the culture supernatant resulting from a reaction conducted at 100°C for 5 minutes was added dropwise to a nitrocellulose membrane to adsorb the proteins in the culture supernatant, and the HSA-producing strain was selected via luminescence detection using the ECL Select™ Western Blotting Detection Reagent (RPN2235, GE Healthcare) and the peroxidase-labeled human albumin-specific antibody (Goat anti-Human Albumin-HRP Conjugated) (A80-129P, BETHYL). The selected HSA-producing strain was designated as the HSA-producing strain derived from the strain in which the aoxl gene had been disrupted and into which the chaperone had been introduced (Δaox1, ura3::pdi1/ero1/kar2, Δaox1+PEK strain).

### (2-4) Preparation of HSA-producing strain derived from a strain in which the aoxl gene had been disrupted, into which the chaperone had been introduced, and in which the prb1 gene had been interrupted

The hsa expression plasmid was introduced into the Δaox1+PEK dprbl strain described in Example 8 via electroporation. The hsa expression plasmid was digested with the restriction enzyme *Bgl*II, followed by ethanol precipitation, and the resultant DNA (1 µg) was used. After electroporation had been conducted under the conditions described in Example 2, the resultant was applied onto the casamino acid-U-A agar medium containing Zeocin at a final concentration of 200 µg/ml and hygromycin B at a final concentration of 200 µg/ml, and it was then allowed to proliferate at 28°C for about 2 or 3 days. The proliferated transformants were allowed to proliferate again on the casamino acid-U-A agar medium containing Zeocin at a final concentration of 200 µg/ml and hygromycin B at a final concentration of 200 µg/ml.

Strains capable of high levels of production of HSA were selected by conducting culture in the manner described below. The 2xYP-P6-dp medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days, 100 µl of the 2xYP-P6-dpn medium containing 40 µM rhodanine-3-acetic acid derivative 1c, 5%(w/v) glycerin, and 5%(v/v) methanol was added, and 100 µl of the 2xYP-P6-dpn medium containing 40 µM rhodanine-3-acetic acid derivative 1c and 5% glycerin was further added 3 days after the initiation of culture. The strains were removed from the culture solution via centrifugation at 3,100 × g and 4°C for 5 minutes 4 days after the initiation of culture, and the resulting culture supernatant was designated as a HSA-producing sample. Quantitative assays of the HSA that had been secreted and produced were performed in accordance with the dot blot technique. An SDS-PAGE buffer (SDS/β-mercaptoethanol) was added, the reaction was allowed to proceed at 100°C for 5 minutes, 1 µl of the resulting culture supernatant was added dropwise to a nitrocellulose membrane to adsorb the proteins in the culture supernatant, and the HSA-producing strain was selected via luminescence detection using the ECL Select™ Western Blotting Detection Reagent (RPN2235, GE Healthcare) and the peroxidase-labeled human albumin-specific antibody (Goat anti-Human Albumin-HRP Conjugated) (A80-129P, BETHYL). The selected HSA-producing strain was designated as the HSA-producing strain derived from the strain in which the aoxl gene had been disrupted, into which the chaperone had been introduced, and in which the prbl gene had been interrupted (Δaox1, ura3::pdi1/ero1/kar2, prb1::hyg, Δaox1+PEK dprb1 strain).

### (3) Comparison of secretory production amounts of HSA

The HSA-secreting and producing strains obtained above were applied onto the casamino acid-U-A agar medium containing Zeocin at a final concentration of 200 µg/ml (in the case of the dprbl-derived strain, contaning Zeocin at a final concentration of 200 µg/ml and hygromycin B at a final concentration of 200 µg/ml), and the strains were allowed to proliferate at 28°C for about 2 days. The 2xYP-P6-dp medium (800 µl) was introduced into a 96-deep well plate (780271, Greiner), the strains were introduced thereinto using a toothpick, and the upper part of the plate was sealed with CO₂ permeable plate seal (676051, Greiner). Culture was conducted at an agitation speed of 310 rpm, an amplitude of 25 mm, and a temperature of 28°C for 2 days. In the case of the NBRC10746+PEK- and NBRC10746+PEK dprbl-derived strains, 100 µl of the 2xYP-P6-dpn medium containing 40 µM rhodanine-3-acetic acid derivative 1c and 10%(v/v) methanol was added, and 100 µl of the 2xYP-P6-dpn medium containing 40 µM rhodanine-3-acetic acid derivative 1c and 10%(v/v) methanol was further added 3 days after the initiation of culture. In the case of theΔaox1+PEK- and Δaox1+PEK dprb1-derived strains, 100 µl of the 2xYP-P6-dpn medium containing 40 µM rhodanine-3-acetic acid derivative 1c, 5%(w/v) glycerin, and 5%(v/v) methanol was added, and 100 µl of the 2xYP-P6-dpn medium containing 40 µM rhodanine-3-acetic acid derivative 1c and 5%(w/v) glycerin was further added 3 days after the initiation of culture. The strains were removed from the culture solution via centrifugation at 3,100 × g and 4°C for 5 minutes 4 days after the initiation of culture, and the resulting culture supernatant was designated as a HSA-producing sample. The samples were compared based on the band intensity obtained from SDS-PAGE/CBB staining. Band intensity was determined by photographing the SDS-PAGE gel using a Light-Capture (ATTO) and analyzing the photographs using analytical software (Cool Saver 2, Version 1.01.1058, ATTO). As shown in Fig. 8, the control sample (NBRC10746+PEK strain) exhibited a band intensity of 1585061, and the NBRC10746+PEK dprbl strain exhibited a band intensity of 2882752. That is, the NBRC10746+PEK dprbl strain exhibited improvement in secretory production that was about 1.8 times greater than that of the control sample. The Δaox1+PEK strain exhibited a band intensity of 2111007, which is 1.3 times greater than that of the control sample. The Δaox1+PEK dprbl strain exhibited a band intensity of 3041627, which is 1.9 times greater than that of the control sample and the greatest improvement among these strains. The Δaox1+PEK strain, especially, the Δaox1+PEK dprbl strain exhibited high secretory productivity with the addition of methanol at a lower concentration than the case of the control sample (i.e., the NBRC10746+PEK strain).

### [Example 12] Amount of secretion and production of HSA protein induced by methanol at 3-L aeration-agitation culture scale

The HSA-producing strains obtained in Example 11 (NBRC10746+PEK strain and Δaox1+PEK dprbl strain) were compared in terms of the secretory production amount of the HSA protein induced by methanol at 3-L aeration-agitation culture scale. 3-L aeration-agitation culture was conducted in the manner described below. The HSA-producing strains obtained in Example 11 (NBRC10746+PEK strain and Δaox1+PEK dprbl strain) were applied onto the casamino acid-U-A agar medium (6.7 g/l yeast nitrogen base W/O amino acids, 0.5 g/l casamino acid, 20 g/l glucose, 20 mg/l L-tryptophan, and 20 g/l agar) containing Zeocin at a final concentration of 200 µg/ml (in the case of the dprbl-derived strain, containing Zeocin at a final concentration of 200 µg/ml and hygromycin B at a final concentration of 200 µg/ml), and the strains were allowed to proliferate at 28°C for about 2 days. The proliferated strains were inoculated into a 50-ml polypropyrene centrifuge tube (227241, Greiner) containing 5 ml of the casamino acid-U-A agar medium (6.7 g/l yeast nitrogen base W/O amino acids, 0.5 g/l casamino acid, 20 g/l glucose, and 20 mg/l L-tryptophan) containing Zeocin at a final concentration of 25 µg/ml (in the case of the dprbl-derived strain, contaning Zeocin at a final concentration of 25 µg/ml and hygromycin B at a final concentration of 50 µg/ml), and the upper part of the centrifuge tube was sealed with plate seal (676051, Greiner). The reciprocal shake culture was conducted at an agitation speed of 250 rpm, an amplitude of 25 mm, and a temperature of 28°C for 24 hours, and the resulting culture solution was designated as the first-type culture solution.

Subsequently, 10 ml of the first-type culture solution was inoculated into a 500-ml baffle flask (355123, BD Falcon) containing 40 ml of the 2x YP-P6 seed medium containing Zeocin at a final concentration of 25 µg/ml (in the case of the dprbl-derived strain, containing Zeocin at a final concentration of 25 µg/ml and hygromycin B at a final concentration of 50 µg/ml) [wherein the 2x YP-P6 seed medium was prepared by dissolving 20 g of a yeast extract and 40 g of peptone in 900 ml of pure water, subjecting the solution to high-pressure steam sterilization, and adding 100 ml of a separately sterilized 10x phosphate buffer (pH 6.0) (1 M KH₂PO₄, 0.15 M (NH₄)₂SO₄, 0.355 N KOH), 12.5 ml of a separately sterilized 50% glucose solution, and 62.5 ml of separately sterilized 80% glycerin] was applied onto the medium containing Zeocin at a final concentration of 25 µg/ml and hygromycin B at a final concentration of 50 µg/ml), and the upper part was sealed with plate seal (676051, Greiner). The reciprocal shake culture was conducted at an agitation speed of 180 rpm, an amplitude of 50 mm, and a temperature of 28°C for 24 hours, and the resulting culture solution was designated as the second-type culture solution.

Subsequently, 60 ml of the second-type culture solution was inoculated into a 3-liter jar fermentor (BMS 03PI and BMS-03PII; ABLE Corporation) containing the 3x YP-P6 medium, which was prepared by dissolving 36 g of a yeast extract and 72 g of peptone in 1080 ml of pure water, subjecting the solution to high-pressure steam sterilization, and adding 120 ml of a separately sterilized 10x phosphate buffer (pH 6.0) (1 M KH₂PO₄, 0.15 M (NH₄)₂SO₄, 0.355 N KOH), 12 ml of a separately sterilized 50%(w/v) glucose solution, 60 ml of separately sterilized 80%(w/v) glycerin, and 100 µg of a defoaming agent (CB-442) (1.2 ml of 50 mg/ml hygromycin B was added in the case of the dprbl-derived strain). Culture was conducted at the culture temperature of 28°C, the internal pressure of 0.1 MPa, and DO of 2 ppm (automatically regulated via agitation). The feeding of an aqueous solution of 50%(w/v) glycerin was initiated 8 hours after the initiation of culture and the feeding was continued at 3m/hr up to 24 hours after the initiation of culture. After the depletion of glycerin from the medium was confirmed, 780 µl of the 40 mM rhodanine-3-acetic acid derivative 1c was added.

Subsequently, methanol-induced culture was conducted in the manner described below. The control sample (NBRC10746+PEK strain: Jarl) was subjected to methanol-induced culture in a medium prepared by adding 32 ml of sterile water and 12 ml of 100% methanol to the medium that was confirmed to have been deprived of glycerin. The addition of 20 ml of sterile water and 86 ml of 100% methanol per day was initiated 2 hours after the initiation of methanol-induced culture and continued until the end of the culture period. The NBRC10746+PEK strain (Jar2) and the Δaox1+PEK dprbl strain (Jar3) were subjected to pH-controlled, nitrogen-source-fed, and methanol-induced culture in the manner described below. The NBRC10746+PEK strain (Jar2) was subjected to methanol-induced culture in the medium that was confirmed to have been deprived of glycerin by adding 32 ml of 10%(w/v) L-histidine monohydrochloride monohydrate, initiating automatic control of the pH level with 14% (v/v) ammonia water, and adding 12 ml of 100% methanol. The Δaox1+PEK dprbl strain (Jar3) was subjected to methanol-induced culture in the medium that was confirmed to have been deprived of glycerin by adding 32 ml of 10%(w/v) L-histidine monohydrochloride monohydrate and 1.2 ml of 50 mg/ml hygromycin B, initiating automatic control of the pH level with 14% (v/v) ammonia water, and adding 6.5 ml of 100% methanol. In the case of the NBRC10746+PEK strain (Jar2), the pH level of the culture solution was adjusted to 6.75 three hours after the initiation of methanol-induced culture and it was adjusted to 7 six hours after the initiation of culture and thereafter. Immediately after the initiation of methanol-induced culture, the feeding of 20 ml of the nitrogen source feeding solution (300 g/l yeast extract and 80 g/l L-histidine monohydrochloride monohydrate) and 86 ml of 100% methanol per day was initiated and it was continued until the end of the culture period. In the case of the Δaox1+PEK dprbl strain (Jar3), the pH level of the culture solution was adjusted to 6.75 three hours after the initiation of methanol-induced culture and it was adjusted to 7 six hours after the initiation of culture and thereafter. Further, the feeding of 20 ml of a nitrogen source feeding solution, 30 ml of 80%(w/v) glycerin, and 56 ml of sterile water per day was initiated immediately after the initiation of methanol-induced culture and it was continued until the end of the culture period. Also, 1.2 ml of 50 mg/ml hygromycin B and methanol at a final concentration of 0.2-0.5%(v/v) were intermittently fed per day from 24 hours after the initiation of methanol-induced culture. From 9 days after the initiation of methanol-induced culture, 100 ml of the culture was allowed to overflow every day.

The culture solutions were subjected to centrifugation at 3,000 × g and 4°C for 5 minutes to remove the strains, and the resulting culture supernatant was designated as a HSA-producing sample. Quantitative assays of HSA secreted and produced were carried out by comparing the samples based on the band intensity obtained by SDS-PAGE/CBB staining. Band intensity was determined by photographing the SDS-PAGE gel using a Light-Capture (ATTO) and analyzing the photographs using analytical software (Cool Saver 2, Version 1.01.1058, ATTO). HSA was quantified on the basis of the calibration curve indicating the band intensity of the Albumin Standard (232209, Thermo scientific) as the standard sample.

Fig. 9 shows the results of quantification of the secretory production amount of HSA. The control sample (NBRC10746+PEK strain: Jar1) exhibited the productivity peak 4 or 5 days after the initiation of methanol-induced culture. In the case of pH-controlled and nitrogen source fed-batch culture, the productivity peak of the NBRC10746+PEK strain (Jar2) was observed 2 or 3 days after the initiation of methanol-induced culture, and degradation of the secreted and produced HSA became significant 4 days after the initiation of culture and thereafter. In contrast, productivity of the Δaox1+PEK dprbl strain (Jar3) was maintained up to 21 days after the initiation of culture, degradation was not substantially observed, and it was thus found to be a protein production system capable of long-term culture. In the case of pH-controlled and nitrogen source fed-batch culture, the amount of HSA secreted and produced by the NBRC10746+PEK strain (Jar2) was improved by about 1.4 times compared with the control sample (NBRC10746+PEK strain: Jar1) (improved from 2486 mg/l to 3594 mg/1). Secretory productivity was improved by about 2.9 times in the case of the Δaox1+PEK dprbl strain (Jar3) (improved from 2486 mg/l to 7104 mg/l). The amount of methanol added when culturing the Δaox1+PEK dprbl strain (Jar3) was reduced to about 1/17 (5.8%) that of the control sample (NBRC10746+PEK strain: Jar1) and the NBRC10746+PEK strain (Jar2) up to 7 days after the initiation of methanol-induced culture. Accordingly, the Δ aox1+PEK dprbl strain (Jar3) that had been subjected to pH-controlled and nitrogen source fed-batch culture was found to have realized a significant reduction of the amount of methanol added and high-level secretory productivity, compared with the control sample (NBRC10746+PEK strain: Jar1) and the NBRC10746+PEK strain (Jar2).

### [Example13] Secretory productivity of HSA protein under carbon source-starvation-induction at 3-L aeration-agitation culture scale

The amount of the HSA protein secreted and produced by the HSA-producing strain derived from the Δaox1+PEK dprbl strain obtained in Example 11 under carbon source-starvation-induction was compared with that under methanol-induction at the 3-L aeration-agitation culture scale. 3-L aeration-agitation culture was conducted in the manner described below. The HSA-producing strain derived from the Δaox1+PEK dprb1 strain obtained in Example 11 was applied onto the casamino acid-U-A agar medium (6.7 g/l yeast nitrogen base W/O amino acids, 0.5 g/l casamino acid, 20 g/l glucose, 20 mg/l L-tryptophan, and 20 g/l agar) containing Zeocin at a final concentration of 200 µg/ml and hygromycin B at a final concentration of 200 µg/ml, and the strains were allowed to proliferate at 28°C for about 2 days. The proliferated strains were inoculated into a 50-ml polypropyrene centrifuge tube (227241, Greiner) containing 5 ml of the casamino acid-U-A agar medium (6.7 g/l yeast nitrogen base W/O amino acids, 0.5 g/l casamino acid, 20 g/l glucose, and 20 mg/l L-tryptophan) containing Zeocin at a final concentration of 25 µg/ml and hygromycin B at a final concentration of 50 µg/ml, and the upper part was sealed with plate seal (676051, Greiner). The reciprocal shake culture was conducted at an agitation speed of 250 rpm, an amplitude of 25 mm, and a temperature of 28°C for 24 hours, and the resulting culture solution was designated as the first-type culture solution.

Subsequently, 10 ml of the first-type culture solution was inoculated into a 500-ml baffle flask (355123, BD Falcon) containing 40 ml of the 2x YP-P6 seed medium containing Zeocin at a final concentration of 31.25 µg/ml and hygromycin B at a final concentration of 62.5 µg/ml [wherein the 2x YP-P6 seed medium was prepared by dissolving 20 g of a yeast extract and 40 g of peptone in 900 ml of pure water, subjecting the solution to high-pressure steam sterilization, and adding 100 ml of a separately sterilized 10x phosphate buffer (pH 6.0) (1 M KH₂PO₄, 0.15 M (NH₄)₂SO₄, 0.355 N KOH), 12.5 ml of a separately sterilized 50% glucose solution, and 62.5 ml of separately sterilized 80% glycerin], and the upper part was sealed with plate seal (676051, Greiner). The reciprocal shake culture was conducted at an agitation speed of 180 rpm, an amplitude of 50 mm, and a temperature of 28°C for 24 hours, and the resulting culture solution was designated as the second-type culture solution.

Subsequently, 75 ml of the second-type culture solution was inoculated into a 3-liter jar fermentor (BMS 03PI and BMS-03PII; ABLE Corporation) containing the 3x YP-P6 medium, which was prepared by dissolving 45 g of a yeast extract and 90 g of peptone in 1350 ml of pure water, subjecting the solution to high-pressure steam sterilization, and adding 150 ml of a separately sterilized 10x phosphate buffer (pH 6.0) (1 M KH₂PO₄, 0.15 M (NH₄)₂SO₄, 0.355 N KOH), 15 ml of a separately sterilized 50%(w/v) glucose solution, 75 ml of separately sterilized 80%(w/v) glycerin, 1.5 ml of 50 mg/ml hygromycin B, and 100 µg of a defoaming agent (CB-442). Culture was conducted at the culture temperature of 28°C, the internal pressure of 0.1 MPa, and DO of 2 ppm (automatically regulated via agitation). The feeding of an aqueous solution of 50%(w/v) glycerin was initiated 8 hours after the initiation of culture and the feeding was continued at 4.3 ml/hr up to 22 hours after the initiation of culture.

After the depletion of glycerin from the medium was confirmed, subsequently, methanol-induced culture, carbon source-starvation culture, and induction culture from the aox promoter were conducted in the manner described below. Methanol-induced culture was initiated in the medium that was confirmed to have been deprived of glycerin by adding 54 ml of sterile water, 40 ml of 10%(w/v) L-histidine monohydrochloride monohydrate, and 1.5 ml of 50 mg/ml of hygromycin B, regulating the pH level of the culture solution to 6.5 with 14%(v/v) ammonia water, and adding 8.5 ml of 100% methanol. Thereafter, the pH level was adjusted to 6.75 three hours after the initiation of methanol-induced culture and it was adjusted to 7 six and a half hours after the initiation of culture and thereafter. Immediately after the initiation of methanol-induced culture, the feeding of 12.5 ml of the nitrogen source feeding solution (300 g/l yeast extract and 80 g/l L-histidine monohydrochloride monohydrate) and 37.5 ml of 80%(w/v) glycerin per day was initiated, it was continued until the end of the culture period, and 5.1 to 5.7 ml of 100% methanol (final concentration: 0.25-0.35%(v/v)) was intermittently fed per day from 24 hours after the initiation of methanol-induced culture and thereafter. Further, 1.5 ml of 50 mg/ml hygromycin B was added 48, 96, and 144 hours after the initiation of methanol-induced culture.

Meanwhile, starvation-induced culture comprising carbon source depletion was initiated in the medium that was confirmed to have been deprived of glycerin by adding 40 ml of 10%(w/v) L-histidine monohydrochloride monohydrate and 1.5 ml of 50 mg/ml hygromycin B, and regulating the pH level to 6.5 with 14%(v/v) ammonia water (hereafter, it is referred to as "carbon source starvation-induced culture"). The pH level was adjusted to 6.75 three hours after the initiation of carbon source starvation-induced culture and it was adjusted to 7 six and a half hours after the initiation of culture and thereafter. Immediately after the initiation of carbon source starvation-induced culture, continuous feeding of 12.5 ml of the nitrogen source feeding solution (300 g/l yeast extract and 80 g/l L-histidine monohydrochloride monohydrate) and 92 ml of 65%(w/v) sorbitol per day was initiated. The nitrogen source feeding solution (12.5 ml) and 37.5 ml of 80%(w/v) glycerin were continuously fed per day from 24 hours after the initiation of methanol-induced culture up to the end of the culture period. Further, 1.5 ml of 50 mg/ml hygromycin B was intermittently fed per day 48, 96, and 144 hours after the initiation of carbon source starvation-induced culture.

The culture solutions were subjected to centrifugation at 3,000 × g and 4°C for 5 minutes to remove the strains, and the resulting culture supernatant was designated as a HSA-producing sample. Quantitative comparison assays of HSA secreted and produced were carried out by comparing the samples based on the SDS-PAGE/CBB staining intensity. Fig. 10 shows the results of quantification of the secretory production amount of HSA. It was found that the productivity attained via carbon source starvation-induced culture was substantially equivalent to that attained via methanol-induced culture.

### Industrial Applicability

The present invention is applicable to the field of production of protein pharmaceuticals, such as antigen or antibody.

## Claims

1. A transformed yeast into which a chaperone gene has been introduced and in which
the aox1 gene has been disrupted, and
a protease gene has been disrupted, wherein optionally the protease gene is a prb1 gene.

2. The transformed yeast according to claim 1, wherein the chaperone gene is at least one gene selected from the group consisting of genes (a) to (d) below:
(a) a gene encoding PDI1, ERO1, Kar2, MPD1, SCJ1, EUG1, or HSP104 derived from *Ogataea minuta* (*O. minuta*);
(b) a gene encoding PDI1, MPD1, SCJ1, ERO1, FKB2, JEM1, LHS1, MPD2, ERJ5, or EUG1 derived from *Saccharomyces cerevisiae* (*S. cerevisia*);
(c) a gene encoding PDI, ERO1-Lα, ERO1-Lβ, or GRP78 derived from a human; and
(d) a gene exhibiting 95% or higher sequence homology to a base sequence of any of the genes (a) to (c).

3. The transformed yeast according to claim 1, wherein the chaperone gene is at least one gene selected from the group consisting of genes (a) to (g) below:
(a) a gene encoding PDI1 derived from *O*. *minuta;*
(b) a gene encoding ERO1 derived from *O*. *minuta;*
(c) a gene encoding Kar2 derived from *O*. *minuta;*
(d) a gene encoding PDI1 derived from *S. cerevisiae;*
(e) a gene encoding PDI derived from a human;
(f) a gene encoding ERO1 derived from a human; and
(g) a gene exhibiting 95% or higher sequence homology to a base sequence of any of the genes (a) to (f).

4. The transformed yeast according to claim 1, wherein the chaperone gene is any of the chaperone genes (a) to (g) below:
(a) a combination of a gene encoding PDI1, a gene encoding ERO1, and a gene encoding Kar2 derived from *O*. *minuta;*
(b) a combination of a gene encoding PDI1 and a gene encoding Kar2 derived from *O. minuta;*
(c) a combination of a gene encoding PDI derived from a human and a gene encoding ERO1 derived from *O*. *minuta*;
(d) a combination of a gene encoding PDI1 and a gene encoding ERO1 derived from *O. minuta;*
(e) a combination of a gene encoding PDI derived from a human, a gene encoding ERO1-Lβ derived from a human, and a gene encoding GRP78 derived from a human;
(f) a combination of a gene encoding PDI derived from a human, a gene encoding ERO1 derived from *O*. *minuta,* and a gene encoding GRP78 derived from a human; and
(g) a gene exhibiting 95% or higher sequence homology to a base sequence of any of the genes (a) to (f).

5. The transformed yeast according to any of claims 1 to 4, wherein the yeast is a methylotrophic yeast.

6. The transformed yeast according to any of claims 1 to 5, which comprises a gene encoding a target protein introduced thereinto.

7. Use of the transformed yeast according to any of claims 1 to 6 for the production of a target protein.

8. A method for producing a protein comprising culturing the transformed yeast according to claim 6 in a medium and sampling a target protein from the culture product.

9. The method for producing a protein according to claim 8, wherein culture is conducted under conditions in which protease activity is inhibited.

10. The method for producing a protein according to claim 8 or 9, wherein culture is conducted in a medium with a pH of 6.0 to 7.5.

11. The method for producing a protein according to any of claims 8 to 10, wherein a nitrogen source is added to the medium.

12. The method for producing a protein according to claims 8 to 11, wherein (a) methanol is not added to the medium or (b) the amount of methanol added to the medium is 2% (v/v) or less.

13. A method for producing a transformed yeast comprising step (i) in addition to both step (ii) and (iii):
(i) a step of introducing a chaperone gene into yeast; and
(ii) a step of disrupting the aox1 gene in yeast; and
(iii) a step of disrupting the prb1 gene in yeast,
optionally further comprising a step of introducing a gene encoding a target protein.

## Patentansprüche

1. Transformierte Hefe, in die ein Chaperon-Gen eingebracht wurde und in der das aox1-Gen unterbrochen wurde und
ein Protease-Gen unterbrochen wurde, wobei das Protease-Gen optional ein prb1-Gen ist.

2. Transformierte Hefe nach Anspruch 1, wobei das Chaperon-Gen wenigstens ein Gen ist, ausgewählt aus der Gruppe, bestehend aus den Genen (a) bis (d) unten:
(a) ein Gen, welches PDI1, ERO1, Kar2, MPD1, SCJ1, EUG1 oder HSP104, abgeleitet von *Ogataea minuta* (*O. minuta),* codiert;
(b) ein Gen, welches PDI1, MPD1, SCJ1, ERO1, FKB2, JEM1, LHS1, MPD2, ERJ5 oder EUG1, abgeleitet von *Saccharomyces cerevisiae* (*S. cerevisiae),* codiert;
(c) ein Gen, welches PDI, ERO1-Lα, ERO1-Lβ oder GRP78, abgeleitet von einem Menschen, codiert; und
(d) ein Gen, welches 95% oder mehr Sequenzhomologie zu einer Basensequenz irgendeines der Gene (a) bis (c) zeigt.

3. Transformierte Hefe gemäß Anspruch 1, wobei das Chaperon-Gen wenigstens ein Gen ist, ausgewählt aus der Gruppe, bestehend aus den Genen (a) bis (g) unten:
(a) ein Gen, welches PDI1, abgeleitet von *O*. *minuta,* codiert;
(b) ein Gen, welches ERO1, abgeleitet von *O*. *minuta,* codiert;
(c) ein Gen, welches Kar2, abgeleitet von *O*. *minuta,* codiert;
(d) ein Gen, welches PDI1, abgeleitet von *S. cerevisiae,* codiert;
(e) ein Gen, welches PDI, abgeleitet von einem Menschen, codiert;
(f) ein Gen, welches ERO1, abgeleitet von einem Menschen, codiert; und
(g) ein Gen, welches 95% oder mehr Sequenzhomologie zu einer Basensequenz irgendeines der Gene (a) bis (f) zeigt.

4. Transformierte Hefe gemäß Anspruch 1, wobei das Chaperon-Gen irgendeines der Chaperon-Gene (a) bis (g) unten ist:
(a) eine Kombination aus einem Gen, welches PDI1 codiert, einem Gen, welches ERO1 codiert, und einem Gen, welches Kar2 codiert, abgeleitet von *O*. *minuta;*
(b) eine Kombination aus einem Gen, welches PDI1 codiert, und einem Gen, welches Kar2 codiert, abgeleitet von *O*. *minuta;*
(c) eine Kombination aus einem Gen, welches PDI, abgeleitet von einem Menschen, codiert, und einem Gen, welches ERO1, abgeleitet von *O*. *minuta,* codiert;
(d) eine Kombination aus einem Gen, welches PDI1 codiert, und einem Gen, welches ERO1 codiert, abgeleitet von *O*. *minuta;*
(e) eine Kombination aus einem Gen, welches PDI, abgeleitet von einem Menschen, codiert, einem Gen, welches ERO1-Lβ, abgeleitet von einem Menschen, codiert, und einem Gen, welches GRP78, abgeleitet von einem Menschen, codiert;
(f) eine Kombination aus einem Gen, welches PDI, abgeleitet von einem Menschen, codiert, einem Gen, welches ERO1, abgeleitet von *O*. *minuta,* codiert, und einem Gen, welches GRP78, abgeleitet von einem Menschen, codiert; und
(g) ein Gen, welches 95% oder mehr Sequenzhomologie zu einer Basensequenz irgendeines der Gene (a) bis (f) zeigt.

5. Transformierte Hefe gemäß irgendeinem der Ansprüche 1 bis 4, wobei die Hefe eine methylotrophe Hefe ist.

6. Transformierte Hefe gemäß irgendeinem der Ansprüche 1 bis 5, welche ein Gen umfasst, das ein dort eingebrachtes Zielprotein codiert.

7. Verwendung der transformierten Hefe gemäß irgendeinem der Ansprüche 1 bis 6 zur Produktion eines Zielproteins.

8. Verfahren zum Produzieren eines Proteins, umfassend das Kultivieren der transformierten Hefe gemäß Anspruch 6 in einem Medium und Entnahme eines Zielproteins aus dem Kulturprodukt.

9. Verfahren zum Produzieren eines Proteins gemäß Anspruch 8, wobei die Kultur unter Bedingungen durchgeführt wird, in denen Proteaseaktivität inhibiert ist.

10. Verfahren zum Produzieren eines Proteins gemäß Anspruch 8 oder 9, wobei die Kultur in einem Medium mit einem pH-Wert von 6,0 bis 7,5 durchgeführt wird.

11. Verfahren zum Produzieren eines Proteins gemäß irgendeinem der Ansprüche 8 bis 10, wobei eine Stickstoffquelle zu dem Medium hinzugefügt wird.

12. Verfahren zum Produzieren eines Proteins gemäß den Ansprüchen 8 bis 11, wobei (a) Methanol nicht zu dem Medium hinzugefügt wird oder (b) die Menge an zu dem Medium hinzugefügtem Methanol 2% (v/v) oder weniger ist.

13. Verfahren zum Produzieren einer transformierten Hefe, umfassend Schritt (i) zusätzlich zu sowohl Schritt (ii) als auch Schritt (iii):
(i) einen Schritt des Einbringens eines Chaperon-Gens in Hefe; und
(ii) einen Schritt des Unterbrechens des aox1-Gens in Hefe; und
(iii) einen Schritt des Unterbrechens des prb1-Gens in Hefe,
optional weiterhin umfassend einen Schritt des Einbringens eines ein Zielprotein codierenden Gens.

## Revendications

1. Levure transformée dans laquelle un gène chaperon a été introduit et dans laquelle le gène aox1 a été interrompu, et
un gène de protéase a été interrompu, dans laquelle facultativement le gène de protéase est un gène prb1.

2. Levure transformée selon la revendication 1, dans laquelle le gène chaperon est au moins un gène sélectionné dans le groupe consistant en les gènes (a) à (d) ci-dessous :
(a) un gène codant pour PDI1, ERO1, Kar2, MPD1, SCJ1, EUG1, ou HSP104 dérivé de *Ogataea minuta* (*O. minuta*) *;*
(b) un gène codant pour PDI1, MPD1, SCJ1, ERO1, FKB2, JEM1, LHS1, MPD2, ERJ5, ou EUG1 dérivé de *Saccharomyces cerevisiae* (*S. cerevisia*) *;*
(c) un gène codant pour PDI, ERO1-Lα, ERO1-Lβ, ou GRP78 dérivé d'un humain ; et
(d) un gène présentant 95 % ou plus d'homologie de séquence avec une séquence de base de l'un quelconque des gènes (a) à (c).

3. Levure transformée selon la revendication 1, dans laquelle le gène chaperon est au moins un gène sélectionné dans le groupe consistant en les gènes (a) à (g) ci-dessous :
(a) un gène codant pour PDI1 dérivé de *O*. *minuta ;*
(b) un gène codant pour ERO1 dérivé de *O*. *minuta ;*
(c) un gène codant pour Kar2 dérivé de *O*. *minuta ;*
(d) un gène codant pour PDI1 dérivé de *S. cerevisiae ;*
(e) un gène codant pour la PDI dérivé d'un humain ;
(f) un gène codant pour ERO1 dérivé d'un humain ; et
(g) un gène présentant 95 % ou plus d'homologie de séquence avec une séquence de bases de l'un quelconque des gènes (a) à (f).

4. Levure transformée selon la revendication 1, dans laquelle le gène chaperon est l'un quelconque des gènes chaperon (a) à (g) ci-dessous :
(a) une combinaison d'un gène codant pour PDI1, d'un gène codant pour ERO1, et d'un gène codant pour Kar2 dérivés de *O*. *minuta ;*
(b) une combinaison d'un gène codant pour PDI1 et d'un gène codant pour Kar2 dérivés de *O*. *minuta ;*
(c) une combinaison d'un gène codant pour PDI dérivé d'un humain et d'un gène codant pour ERO1 dérivé de *O*. *minuta ;*
(d) une combinaison d'un gène codant pour PDI1 et d'un gène codant pour ERO1 dérivés de *O*. *minuta ;*
(e) une combinaison d'un gène codant pour PDI dérivé d'un humain, d'un gène codant pour ERO1-Lβ dérivé d'un humain, et d'un gène codant pour GRP78 dérivé d'un humain ;
(f) une combinaison d'un gène codant pour PDI dérivé d'un humain, d'un gène codant pour ERO1 dérivé de *O*. *minuta,* et d'un gène codant pour GRP78 dérivé d'un humain ; et
(g) un gène présentant 95 % ou plus d'homologie de séquence avec une séquence de bases de l'un quelconque des gènes (a) à (f).

5. Levure transformée selon l'une quelconque des revendications 1 à 4, dans laquelle la levure est une levure méthylotrophe.

6. Levure transformée selon l'une quelconque des revendications 1 à 5, qui comprend un gène codant pour une protéine cible introduite dans celui-ci.

7. Utilisation de la levure transformée selon l'une quelconque des revendications 1 à 6 pour la production d'une protéine cible.

8. Procédé de production d'une protéine comprenant la mise en culture de la levure transformée selon la revendication 6 dans un milieu et l'échantillonnage d'une protéine cible à partir du produit de culture.

9. Procédé de production d'une protéine selon la revendication 8, dans lequel la culture est menée dans des conditions dans lesquelles l'activité des protéases est inhibée.

10. Procédé de production d'une protéine selon la revendication 8 ou 9, dans lequel la culture est menée dans un milieu ayant un pH de 6,0 à 7,5.

11. Procédé de production d'une protéine selon l'une quelconque des revendications 8 à 10, dans lequel une source d'azote est ajoutée au milieu.

12. Procédé de production d'une protéine selon les revendications 8 à 11, dans lequel (a) du méthanol n'est pas ajouté au milieu ou (b) la quantité de méthanol ajoutée au milieu est de 2 % (v/v) ou moins.

13. Procédé de production d'une levure transformée comprenant l'étape (i) en plus des deux étapes (ii) et (iii) :
(i) une étape d'introduction d'un gène chaperon dans la levure ; et
(ii) une étape d'interruption du gène aox1 dans la levure ; et
(iii) une étape d'interruption du gène prb1 dans la levure,
facultativement comprenant en outre une étape d'introduction d'un gène codant pour une protéine cible.


### SEQUENCE LISTING

<110> DAIICHI SANKYO COMPANY, LIMITED
   National Institute of Advanced Industrial Science and Technology
<120> An improved method for high-yield secretory production of proteins
<130> PH-6230-PCT
<150> JP2014-155272
   <151> 2014-07-30
<160> 76
<170> PatentIn version 3.5
<210> 1
   <211> 29
   <212> DNA
   <213> Artificial
<220>
   <223> primer
<400> 1
   gcaagctttc tttcgcaaac agctctttg 29
<210> 2
   <211> 41
   <212> DNA
   <213> Artificial
<220>
   <223> primer
<400> 2
   gaacccggga acagaatcta gattttttcg taagtcgtaa g 41
<210> 3
   <211> 39
   <212> DNA
   <213> Artificial
<220>
   <223> primer
<400> 3
   ctgttcccgg gttcctggat ccgagacggt gcccgactc 39
<210> 4
   <211> 25
   <212> DNA
   <213> Artificial
<220>
   <223> primer
<400> 4
   gcggtaccgt tagtggtacg ggcag 25
<210> 5
   <211> 17
   <212> DNA
   <213> Artificial
<220>
   <223> primer
<400> 5
   ggtaccagta ctggaaa 17
<210> 6
   <211> 34
   <212> DNA
   <213> Artificial
<220>
   <223> primer
<400> 6
   cagataaaca ggcgactttt cgggtcacgt gact 34
<210> 7
   <211> 34
   <212> DNA
   <213> Artificial
<220>
   <223> primer
<400> 7
   agtcacgtga cccgaaaagt cgcctgttta tctg 34
<210> 8
   <211> 17
   <212> DNA
   <213> Artificial
<220>
   <223> primer
<400> 8
   ccaaggagga agaaatt 17
<210> 9
   <211> 17
   <212> DNA
   <213> Artificial
<220>
   <223> primer
<400> 9
   atcacaggaa agcgcat 17
<210> 10
   <211> 17
   <212> DNA
   <213> Artificial
<220>
   <223> primer
<400> 10
   attcgagcat cgccgtg 17
<210> 11
   <211> 20
   <212> DNA
   <213> Artificial
<220>
   <223> primer
<400> 11
   atggctattc ctgacgaatt 20
<210> 12
   <211> 22
   <212> DNA
   <213> Artificial
<220>
   <223> primer
<400> 12
   ttagaatcta gccagaccct tc 22
<210> 13
   <211> 21
   <212> DNA
   <213> Artificial
<220>
   <223> primer
<400> 13
   gatctcaggc cgagtcaaga c 21
<210> 14
   <211> 19
   <212> DNA
   <213> Artificial
<220>
   <223> primer
<400> 14
   ttacaactcg tcgtgagcc 19
<210> 15
   <211> 21
   <212> DNA
   <213> Artificial
<220>
   <223> primer
<400> 15
   ttatagctcc aaacgataca g 21
<210> 16
   <211> 18
   <212> DNA
   <213> Artificial
<220>
   <223> primer
<400> 16
   taaacactaa cgccgcat 18
<210> 17
   <211> 19
   <212> DNA
   <213> Artificial
<220>
   <223> primer
<400> 17
   tcacagctca tcatgatcc 19
<210> 18
   <211> 24
   <212> DNA
   <213> Artificial
<220>
   <223> primer
<400> 18
   caagcttcgt tggcagcagt ggag 24
<210> 19
   <211> 24
   <212> DNA
   <213> Artificial
<220>
   <223> primer
<400> 19
   cggtacccga tggaatctca gaca 24
<210> 20
   <211> 1384
   <212> DNA
   <213> Ogataea minuta
<400> 20
   caagcttcgt tggcagcagt ggaggccttg gtcatcccgt tatttgacga cttgccagca 60
   gagtttgccc ttgttccaat ggatgcgaaa gcggaagtca tttctgacgt tcctgtcgac      120
   tcggccatta gtgatgctcc tatcgcggca ctaaatgatg ctccaagccc tctcgtcaca      180
   tcgctgatcg catctcaaaa tttgattcca aactcttata ttgtcgtttt caagaatggc      240
   ctagcttccg gggcagttga cttccacatg gagtggctca aggaaacgca ctcccaaacc      300
   ctggctgctt tgtctaagga catgccagca gaagaattgg ccgccgaagg tttcgtttcc      360
   gaaagcattg atcttactga ggtgtttagc atctccgatt tgttcagtgg atataccgga      420
   tacttcccgg agaaggtggt tgacctcatc agaagacacc ctgacgtggc gttcgttgag      480
   caggactcga gagttttcgc cgataagtcg tctactcaaa acggtgctcc ttggggtttg      540
   tctagaatct ctcacagaga gcctctcagt ctcggcaatt tcaacgagta cgtttacgac      600
   gatcttgctg gagatggcgt cacggcttat gtcattgata ccggtatcaa tgtgaagcac      660
   gagcagttcg gtggcagagc agagtggggt aagaccatcc caaccggtga tgatgatatt      720
   gacggaaacg gtcacggtac tcactgcgct ggtacaattg gctcggaaga ttatggagtt      780
   tctaagaact ccaaaattgt cgcagtgaag gttttgagat ctaacggttc tggttccatg      840
   tctgacgtga tcaagggtgt tgaattcgct gcaaatgatc acgttgccaa gtctaaagcc      900
   aagaaggacg gtttcaaggg atcgactgcc aacatgtctt tgggaggtgg caagtctcct      960
   gctcttgact tggctgtcaa tgccgctgtc aaagctggtt tacactttgc tgttgccgct     1020
   ggtaacgaca atgctgacgc atgcaactat tctcctgctg ctgcagagaa cgcagtcact     1080
   gttggtgcgt ccactttgtc tgactctaga gcttactttt ccaactatgg taaatgtgtt     1140
   gacatttttg ctccgggctt gaacatcctt tccacctaca taggttctga cactgccacc     1200
   gccactcttt ctggtacatc gatggcctcc cctcacgttt gtggtctgtt gacctacttt     1260
   ttgagcttgc aaccagaatc gtcgtcgttg ttttcttcgg cagctatctc ccctgctcag     1320
   ctgaagaaga acctgatcaa gtttggtacg aagaacgttt tgtctgagat tccatcgggt     1380
   accg                                                                  1384
<210> 21
   <211> 17
   <212> DNA
   <213> Artificial
<220>
   <223> primer
<400> 21
   caggaaacag ctatgac 17
<210> 22
   <211> 22
   <212> DNA
   <213> Artificial
<220>
   <223> primer
<400> 22
   ctaatcgaac aaatcagcaa cc 22
<210> 23
   <211> 22
   <212> DNA
   <213> Artificial
<220>
   <223> primer
<400> 23
   atgaagttat cccagtctgc tg 22
<210> 24
   <211> 20
   <212> DNA
   <213> Artificial
<220>
   <223> primer
<400> 24
   caaaggaata gatcccccat 20
<210> 25
   <211> 2220
   <212> DNA
   <213> Artificial
<220>
   <223> synthetic nucleotide
<400> 25
   tctagaatga gattcccatc catcttcacc gccgttttgt tcgctgcttc ctccgctttg      60
   gctgctccag ttaacaccac caccgaggac gagaccgctc aaatcccagc tgaggctgtt     120
   atcggttact ccgacttgga gggtgacttc gacgttgctg ttttgccatt ctccaactcc     180
   accaacaacg gtttgttgtt catcaacacc accatcgctt ccatcgctgc taaggaggag     240
   ggtgtttcct tggagaagag agaggctgag gctcaacaaa tcccattgaa ggaccacacc     300
   tccagacaat acttcgctgt tgagtccaac gagaccttgt ccagattgga ggagatgcac     360
   ccaaactgga agtacgagca cgacgttaga ggtttgccaa accactacgt tttctccaag     420
   gagttgttga agttgggtaa gagatcctcc ttggaggagt tgcaaggtga caacaacgac     480
   cacatcttgt ccgttcacga cttgttccca agaaacgact tgttcaagag attgccagtt     540
   ccagctccac caatggactc ctccttgttg ccagttaagg aggctgagga caagttgtcc     600
   atcaacgacc cattgttcga gagacaatgg cacttggtta acccatcctt cccaggttcc     660
   gacatcaacg ttttggactt gtggtacaac aacatcaccg gtgctggtgt tgttgctgct 720
   atcgttgacg acggtttgga ctacgagaac gaggacttga aggacaactt ctgtgctgag 780
   ggttcctggg acttcaacga caacaccaac ttgccaaagc caagattgtc cgacgactac 840
   cacggtacca gatgtgctgg tgagatcgct gctaagaagg gtaacaactt ctgtggtgtt 900
   ggtgttggtt acaacgctaa gatctccggt atcagaatct tgtccggtga catcaccacc 960
   gaggacgagg ctgcttcctt gatctacggt ttggacgtta acgacatcta ctcctgttcc 1020
   tggggtccag ctgacgacgg tagacacttg caaggtccat ccgacttggt taagaaggct 1080
   ttggttaagg gtgttaccga gggtagagac tccaagggtg ctatctacgt tttcgcttcc 1140
   ggtaacggtg gtaccagagg tgacaactgt aactacgacg gttacaccaa ctccatctac 1200
   tccatcacca tcggtgctat cgaccacaag gacttgcacc caccatactc cgagggttgt 1260
   tccgctgtta tggctgttac ctactcctcc ggttccggtg agtacatcca ctcctccgac 1320
   atcaacggta gatgttccaa ctcccacggt ggtacctccg ctgctgctcc attggctgct 1380
   ggtgtttaca ccttgttgtt ggaggctaac ccaaacttga cctggagaga cgttcaatac 1440
   ttgtccatct tgtccgctgt tggtttggag aagaacgctg acggtgactg gagagactcc 1500
   gctatgggta agaagtactc ccacagatac ggtttcggta agatcgacgc tcacaagttg 1560
   atcgagatgt ccaagacctg ggagaacgtt aacgctcaaa cctggttcta cttgccaacc 1620
   ttgtacgttt cccaatccac caactccacc gaggagacct tggagtccgt tatcaccatc 1680
   tccgagaagt ccttgcaaga cgctaacttc aagagaatcg agcacgttac cgttaccgtt 1740
   gacatcgaca ccgagatcag aggtaccacc accgttgact tgatctcccc agctggtatc 1800
   atctccaact tgggtgttgt tagaccaaga gacgtttcct ccgagggttt caaggactgg 1860
   accttcatgt ccgttgctca ctggggtgag aacggtgttg gtgactggaa gatcaaggtt 1920
   aagaccaccg agaacggtca cagaatcgac ttccactcct ggagattgaa gttgttcggt 1980
   gagtccatcg actcctccaa gaccgagacc ttcgttttcg gtaacgacaa ggaggaggtt 2040
   gagccagctg ctaccgagtc caccgtttcc caatactccg cttcctccac ctccatctcc 2100
   atctccgcta cctccacctc ctccatctcc atcggtgttg agacctccgc tatcccacaa 2160
   accaccaccg cttccaccga cccacaccac caccaccacc accaccacca ctaaggatcc 2220
<210> 26
   <211> 2040
   <212> **DNA**
   <213> Artificial
<220>
   <223> synthetic nucleotide
<400> 26
   tctagaatga gattcccatc catcttcact gctgttttgt tcgctgcttc ttccgctttg 60
   gctgctccag ttaacactac tactgaggac gagactgctc agattccagc tgaagctgtt 120
   attggttact ccgacttgga aggtgacttc gacgttgctg ttttgccatt ctccaactcc 180
   actaacaacg gtttgttgtt catcaacact acaatcgctt ccattgctgc taaagaagag 240
   ggagtttcct tggagaagag agaagctgag gctgacgctc acaagtctga agttgctcac 300
   agattcaagg acttgggtga agagaacttc aaggctttgg ttttgatcgc tttcgctcag 360
   tacttgcagc agtgtccatt cgaggatcac gttaagttgg ttaacgaggt tactgagttc 420
   gctaagactt gtgttgctga cgaatccgct gagaactgtg ataagtcctt gcacactttg 480
   ttcggtgaca agttgtgtac tgttgctact ttgagagaaa cttacggtga gatggctgac 540
   tgttgtgcta agcaagagcc agaaagaaac gagtgtttct tgcagcacaa ggacgacaac 600
   ccaaacttgc caagattggt tagaccagag gttgacgtta tgtgtactgc tttccacgac 660
   aacgaagaga ctttcttgaa gaagtacttg tacgagatcg ctagaagaca cccatacttc 720
   tacgctccag agttgttgtt ctttgctaag agatacaagg ctgctttcac tgagtgttgt 780
   caggctgctg acaaagctgc ttgtttgttg ccaaagttgg acgagttgag agatgagggt 840
   aaggcttctt ctgctaagca gagattgaag tgtgcttcct tgcagaagtt cggagagaga 900
   gcttttaagg cttgggctgt tgctagattg tcccagagat ttccaaaggc tgaattcgct 960
   gaggtttcca aattggttac agacttgact aaggttcaca cagaatgttg tcacggtgac 1020
   ttgttggaat gtgctgatga cagagctgac ttggctaagt acatctgtga aaaccaggac 1080
   tccatctcct ccaagttgaa agaatgttgt gagaagcctt tgttggagaa gtcccactgt 1140
   attgctgagg ttgagaacga cgaaatgcct gctgatttgc cttccttggc tgctgacttc 1200
   gttgaatcca aggacgtttg taagaactac gctgaggcta aggatgtttt cttgggtatg 1260
   ttcttgtatg aatatgctag aagacatcca gactactccg ttgttttgtt gttgagattg 1320
   gctaaaactt acgagactac tttggaaaag tgttgtgctg ctgctgaccc acacgaatgt 1380
   tacgctaagg ttttcgacga gttcaagcca ttggttgagg aaccacagaa cttgattaag 1440
   caaaactgtg agttgttcga gcagttgggt gagtacaagt tccagaacgc tttgttggtt 1500
   agatacacta agaaggttcc acaggtttcc actcctactt tggttgaagt ttccagaaac 1560
   ttgggtaagg ttggttccaa gtgttgtaag cacccagagg ctaagagaat gccatgtgct 1620
   gaagattact tgtctgttgt tttgaaccag ttgtgtgttt tgcacgaaaa gactccagtt 1680
   tccgacagag ttacaaaatg ttgtactgag tccttggtta acagaagacc ttgtttctct 1740
   gctttggaag ttgacgagac ttacgttcca aaagagttca acgctgagac tttcactttc 1800
   cacgctgaca tctgtacttt gtccgagaaa gagagacaga tcaagaagca gactgctttg 1860
   gttgagttgg ttaagcacaa gccaaaggct acaaaagagc aattgaaggc tgttatggac 1920
   gactttgctg ctttcgttga gaaatgttgt aaggctgacg acaaagagac ttgtttcgct 1980
   gaagagggta agaagttggt tgctgcttct caagctgctt tgggtttgta gtaaggatcc 2040
<210> 27
   <211> 1992
   <212> DNA
   <213> Ogataea minuta
<220>
   <221> CDS
   <222> (1)..(1992)
<400> 27
   atg gct att cct gac gaa ttc gat atc atc gtt gtg ggt gga ggc tca        48
   Met Ala Ile Pro Asp Glu Phe Asp Ile Ile Val Val Gly Gly Gly Ser
   1               5                   10                  15
   tgc ggc tgc gcc atc gcc ggt aga ctc ggt aac ctc gac ccg gac gtt        96
   Cys Gly Cys Ala Ile Ala Gly Arg Leu Gly Asn Leu Asp Pro Asp Val
               20                  25                  30
   act gtg gct ctc atc gag ggt ggt gag aac aac atc aat aac cca tgg       144
   Thr Val Ala Leu Ile Glu Gly Gly Glu Asn Asn Ile Asn Asn Pro Trp
           35                  40                  45
   gtc tac ctt cct ggt gtc tat cca aga aac atg aga ctc gac tcc aag       192
   Val Tyr Leu Pro Gly Val Tyr Pro Arg Asn Met Arg Leu Asp Ser Lys
       50                  55                  60
   acg gct acc ttc tac aac tcg aga cca tcc aag cac ctg aac ggc aga       240
   Thr Ala Thr Phe Tyr Asn Ser Arg Pro Ser Lys His Leu Asn Gly Arg
   65                  70                  75                  80
   agg gcc att gtc ccc tgc gct aac att ctt ggt gga ggt tcc tcc atc       288
   Arg Ala Ile Val Pro Cys Ala Asn Ile Leu Gly Gly Gly Ser Ser Ile
                   85                  90                  95
   aac ttc ctc atg tac acc aga gcc tcg gcc tcc gac tac gac gac tgg       336
   Asn Phe Leu Met Tyr Thr Arg Ala Ser Ala Ser Asp Tyr Asp Asp Trp
               100                 105                 110
   gag caa gag gga tgg acc acc gac gag ctg ctt ccg ctc atg aag aag       384
   Glu Gln Glu Gly Trp Thr Thr Asp Glu Leu Leu Pro Leu Met Lys Lys
           115                 120                 125
   ctc gag acg tat caa cgt cct tgc aac aac agg gag gtg cac ggt ttc       432
   Leu Glu Thr Tyr Gln Arg Pro Cys Asn Asn Arg Glu Val His Gly Phe
       130                 135                 140
   gac ggt ccg atc aag gtc tcc ttc ggt aac tac acc tac cca act gcc       480
   Asp Gly Pro Ile Lys Val Ser Phe Gly Asn Tyr Thr Tyr Pro Thr Ala
   145                 150                 155                 160
   caa gac ttc ctg aga gcc tgc gag tcg cag ggt att cct ttc aac gac       528
   Gln Asp Phe Leu Arg Ala Cys Glu Ser Gln Gly Ile Pro Phe Asn Asp
                   165                 170                 175
   gat ctt gaa gac ctc aag gcc tcg cac gga gct gag tac tgg ctc aag       576
   Asp Leu Glu Asp Leu Lys Ala Ser His Gly Ala Glu Tyr Trp Leu Lys
               180                 185                 190
   tgg atc aac agg gat ctc ggt aga aga tcg gac tcg gca cac gcc tac         624
   Trp Ile Asn Arg Asp Leu Gly Arg Arg Ser Asp Ser Ala His Ala Tyr
           195                 200                 205
   atc cac cct acc atg aga aac aag agc aat ctg ttc ctc att acg tcc         672
   Ile His Pro Thr Met Arg Asn Lys Ser Asn Leu Phe Leu Ile Thr Ser
       210                 215                 220
   acc aag gct gac aag gtg atc att gag aac ggc gtt gct gtc ggt gtc         720
   Thr Lys Ala Asp Lys Val Ile Ile Glu Asn Gly Val Ala Val Gly Val
   225                 230                 235                 240
   agg acc gtt cca atg aag ccg gtc gag acc aaa aac cct cca agc agg         768
   Arg Thr Val Pro Met Lys Pro Val Glu Thr Lys Asn Pro Pro Ser Arg
                   245                 250                 255
   atc ttc aag gcc aga aag caa att gtg gtt tcg tgc ggt acg atc tcc         816
   Ile Phe Lys Ala Arg Lys Gln Ile Val Val Ser Cys Gly Thr Ile Ser
               260                 265                 270
   tct cca ttg gtg ctg caa aga tct ggt atc ggt gcg gcc cac aag ctg         864
   Ser Pro Leu Val Leu Gln Arg Ser Gly Ile Gly Ala Ala His Lys Leu
           275                 280                 285
   aga caa gcg ggc atc aag ccg atc gtc gac ttg cct ggt gtc ggt gag         912
   Arg Gln Ala Gly Ile Lys Pro Ile Val Asp Leu Pro Gly Val Gly Glu
       290                 295                 300
   aac ttc cag gac cac tac tgc ttc ttc acc cca tac tat tcc aag cca         960
   Asn Phe Gln Asp His Tyr Cys Phe Phe Thr Pro Tyr Tyr Ser Lys Pro
   305                 310                 315                 320
   gag gtt cca acc ttt gac gac ttt gtc aga ggt gac cca gtc gct caa        1008
   Glu Val Pro Thr Phe Asp Asp Phe Val Arg Gly Asp Pro Val Ala Gln
                   325                 330                 335
   aag tcc gcc ttt gac cag tgg tac tcc aac aag gac ggt cct ctt acc        1056
   Lys Ser Ala Phe Asp Gln Trp Tyr Ser Asn Lys Asp Gly Pro Leu Thr
               340                 345                 350
   acc aac ggt atc gag gct ggt gtc aag atc aga cca acc gac gag gag        1104
   Thr Asn Gly Ile Glu Ala Gly Val Lys Ile Arg Pro Thr Asp Glu Glu
           355                 360                 365
   ttg gcc acg gct gac gat gac ttc atc caa ggg tac cac gag tac ttt        1152
   Leu Ala Thr Ala Asp Asp Asp Phe Ile Gln Gly Tyr His Glu Tyr Phe
       370                 375                 380
   gac aac aag cca gac aag cca ctg atg cat tac tct gtc att tcc ggt        1200
   Asp Asn Lys Pro Asp Lys Pro Leu Met His Tyr Ser Val Ile Ser Gly
   385                 390                 395                 400
   ttc ttc ggt gac cac acc aag att cca aac ggc aag ttc ttc acc atg        1248
   Phe Phe Gly Asp His Thr Lys Ile Pro Asn Gly Lys Phe Phe Thr Met
                   405                 410                 415
   ttc cac ttt ttg gag tac cca ttt tcg aga ggt ttc gtt tat gct gtt        1296
   Phe His Phe Leu Glu Tyr Pro Phe Ser Arg Gly Phe Val Tyr Ala Val
               420                 425                 430
   tcc cca gac cca tac gaa gct cca gac ttt gat cca ggt ttc ctg aac        1344
   Ser Pro Asp Pro Tyr Glu Ala Pro Asp Phe Asp Pro Gly Phe Leu Asn
           435                 440                 445
   gat tcc aga gac atg tgg cct atg gtt tgg tct tac aag aag tcg aga      1392
   Asp Ser Arg Asp Met Trp Pro Met Val Trp Ser Tyr Lys Lys Ser Arg
       450                 455                 460
   cag aca gcc aga aga atg gag tcg ttt gct ggt gaa gtc acc tcg cac      1440
   Gln Thr Ala Arg Arg Met Glu Ser Phe Ala Gly Glu Val Thr Ser His
   465                 470                 475                 480
   cac cca ctc tac ccg gtt gac tct cca gcc cgt gcc aag gac ttg gat      1488
   His Pro Leu Tyr Pro Val Asp Ser Pro Ala Arg Ala Lys Asp Leu Asp
                   485                 490                 495
   ctc gag aca tgc aag gca ttt gct gga cca aac cac ttc acc gcc aac      1536
   Leu Glu Thr Cys Lys Ala Phe Ala Gly Pro Asn His Phe Thr Ala Asn
               500                 505                 510
   ttg tac cac ggt tcc tgg act gtt cca att gag aag cca acg cca aag      1584
   Leu Tyr His Gly Ser Trp Thr Val Pro Ile Glu Lys Pro Thr Pro Lys
           515                 520                 525
   aac gac tcg cac gtg acc tgc aac cag gtc gag atc ttc tcc gac att      1632
   Asn Asp Ser His Val Thr Cys Asn Gln Val Glu Ile Phe Ser Asp Ile
       530                 535                 540
   gac tac tct gcc gag gac gat gag gct att gtc aag tac atc aag gag      1680
   Asp Tyr Ser Ala Glu Asp Asp Glu Ala Ile Val Lys Tyr Ile Lys Glu
   545                 550                 555                 560
   cac act gag acc acc tgg cac tgt ttg gga acc tgt tcg atg gct cca      1728
   His Thr Glu Thr Thr Trp His Cys Leu Gly Thr Cys Ser Met Ala Pro
                   565                 570                 575
   caa gaa ggt agc aag atc gct cca aag ggt ggt gtt gtc gat gcc aga      1776
   Gln Glu Gly Ser Lys Ile Ala Pro Lys Gly Gly Val Val Asp Ala Arg
               580                 585                 590
   ttg aac gtg tac gaa gtg aag aac ctc aag gtt gcc gac ctg tcg atc      1824
   Leu Asn Val Tyr Glu Val Lys Asn Leu Lys Val Ala Asp Leu Ser Ile
           595                 600                 605
   tgc cca gat aac gtt gga tgt aat act tac tcc act gct ctt ctg att      1872
   Cys Pro Asp Asn Val Gly Cys Asn Thr Tyr Ser Thr Ala Leu Leu Ile
       610                 615                 620
   ggt gag aag gct gcc act ttg gtc gcc gag gac ctg gga tac tca gga      1920
   Gly Glu Lys Ala Ala Thr Leu Val Ala Glu Asp Leu Gly Tyr Ser Gly
   625                 630                 635                 640
   tct gat ctc gcc atg acc att cca aac ttc aag cta ggt act tac gag      1968
   Ser Asp Leu Ala Met Thr Ile Pro Asn Phe Lys Leu Gly Thr Tyr Glu
                   645                 650                 655
   gag aag ggt ctg gct aga ttc taa                                      1992
   Glu Lys Gly Leu Ala Arg Phe
               660
<210> 28
   <211> 663
   <212> PRT
   <213> Ogataea minuta
<400> 28
   Met Ala Ile Pro Asp Glu Phe Asp Ile Ile Val Val Gly Gly Gly Ser
   1               5                   10                  15
   Cys Gly Cys Ala Ile Ala Gly Arg Leu Gly Asn Leu Asp Pro Asp Val
               20                  25                  30
   Thr Val Ala Leu Ile Glu Gly Gly Glu Asn Asn Ile Asn Asn Pro Trp
           35                  40                  45
   Val Tyr Leu Pro Gly Val Tyr Pro Arg Asn Met Arg Leu Asp Ser Lys
       50                  55                  60
   Thr Ala Thr Phe Tyr Asn Ser Arg Pro Ser Lys His Leu Asn Gly Arg
   65                  70                  75                  80
   Arg Ala Ile Val Pro Cys Ala Asn Ile Leu Gly Gly Gly Ser Ser Ile
                   85                  90                  95
   Asn Phe Leu Met Tyr Thr Arg Ala Ser Ala Ser Asp Tyr Asp Asp Trp
               100                 105                 110
   Glu Gln Glu Gly Trp Thr Thr Asp Glu Leu Leu Pro Leu Met Lys Lys
           115                 120                 125
   Leu Glu Thr Tyr Gln Arg Pro Cys Asn Asn Arg Glu Val His Gly Phe
       130                 135                 140
   Asp Gly Pro Ile Lys Val Ser Phe Gly Asn Tyr Thr Tyr Pro Thr Ala
   145                 150                 155                 160
   Gln Asp Phe Leu Arg Ala Cys Glu Ser Gln Gly Ile Pro Phe Asn Asp
                   165                 170                 175
   Asp Leu Glu Asp Leu Lys Ala Ser His Gly Ala Glu Tyr Trp Leu Lys
               180                 185                 190
   Trp Ile Asn Arg Asp Leu Gly Arg Arg Ser Asp Ser Ala His Ala Tyr
           195                 200                 205
   Ile His Pro Thr Met Arg Asn Lys Ser Asn Leu Phe Leu Ile Thr Ser
       210                 215                 220
   Thr Lys Ala Asp Lys Val Ile Ile Glu Asn Gly Val Ala Val Gly Val
   225                 230                 235                 240
   Arg Thr Val Pro Met Lys Pro Val Glu Thr Lys Asn Pro Pro Ser Arg
                   245                 250                 255
   Ile Phe Lys Ala Arg Lys Gln Ile Val Val Ser Cys Gly Thr Ile Ser
               260                 265                 270
   Ser Pro Leu Val Leu Gln Arg Ser Gly Ile Gly Ala Ala His Lys Leu
           275                 280                 285
   Arg Gln Ala Gly Ile Lys Pro Ile Val Asp Leu Pro Gly Val Gly Glu
       290                 295                 300
   Asn Phe Gln Asp His Tyr Cys Phe Phe Thr Pro Tyr Tyr Ser Lys Pro
   305                 310                 315                 320
   Glu Val Pro Thr Phe Asp Asp Phe Val Arg Gly Asp Pro Val Ala Gln
                   325                 330                 335
   Lys Ser Ala Phe Asp Gln Trp Tyr Ser Asn Lys Asp Gly Pro Leu Thr
               340                 345                 350
   Thr Asn Gly Ile Glu Ala Gly Val Lys Ile Arg Pro Thr Asp Glu Glu
           355                 360                 365
   Leu Ala Thr Ala Asp Asp Asp Phe Ile Gln Gly Tyr His Glu Tyr Phe
       370                 375                 380
   Asp Asn Lys Pro Asp Lys Pro Leu Met His Tyr Ser Val Ile Ser Gly
   385                 390                 395                 400
   Phe Phe Gly Asp His Thr Lys Ile Pro Asn Gly Lys Phe Phe Thr Met
                   405                 410                 415
   Phe His Phe Leu Glu Tyr Pro Phe Ser Arg Gly Phe Val Tyr Ala Val
               420                 425                 430
   Ser Pro Asp Pro Tyr Glu Ala Pro Asp Phe Asp Pro Gly Phe Leu Asn
           435                 440                 445
   Asp Ser Arg Asp Met Trp Pro Met Val Trp Ser Tyr Lys Lys Ser Arg
       450                 455                 460
   Gln Thr Ala Arg Arg Met Glu Ser Phe Ala Gly Glu Val Thr Ser His
   465                 470                 475                 480
   His Pro Leu Tyr Pro Val Asp Ser Pro Ala Arg Ala Lys Asp Leu Asp
                   485                 490                 495
   Leu Glu Thr Cys Lys Ala Phe Ala Gly Pro Asn His Phe Thr Ala Asn
   500 505 510
   Leu Tyr His Gly Ser Trp Thr Val Pro Ile Glu Lys Pro Thr Pro Lys
   515 520 525
   Asn Asp Ser His Val Thr Cys Asn Gln Val Glu Ile Phe Ser Asp Ile
   530 535 540
   Asp Tyr Ser Ala Glu Asp Asp Glu Ala Ile Val Lys Tyr Ile Lys Glu
   545 550 555 560
   His Thr Glu Thr Thr Trp His Cys Leu Gly Thr Cys Ser Met Ala Pro
   565 570 575
   Gln Glu Gly Ser Lys Ile Ala Pro Lys Gly Gly Val Val Asp Ala Arg
   580 585 590
   Leu Asn Val Tyr Glu Val Lys Asn Leu Lys Val Ala Asp Leu Ser Ile
   595 600 605
   Cys Pro Asp Asn Val Gly Cys Asn Thr Tyr Ser Thr Ala Leu Leu Ile
   610 615 620
   Gly Glu Lys Ala Ala Thr Leu Val Ala Glu Asp Leu Gly Tyr Ser Gly
   625 630 635 640
   Ser Asp Leu Ala Met Thr Ile Pro Asn Phe Lys Leu Gly Thr Tyr Glu
   645 650 655
   Glu Lys Gly Leu Ala Arg Phe
   660
<210> 29
   <211> 798
   <212> DNA
   <213> Ogataea minuta
<220>
   <221> CDS
   <222> (1)..(798)
<400> 29
   atg tcc tcg act aag aca tac gcg caa agg gcg gcg gct cat ccg tcg          48
   Met Ser Ser Thr Lys Thr Tyr Ala Gln Arg Ala Ala Ala His Pro Ser
   1               5                   10                  15
   cct gtg gcc aga aga ctg ctg aac ttg atg gaa tcc aag aag acg aac          96
   Pro Val Ala Arg Arg Leu Leu Asn Leu Met Glu Ser Lys Lys Thr Asn
               20                  25                  30
   ttg tgt gcc tcg gtc gat ctc acc tct aca aag gac ctt ttg gag ctg      144
   Leu Cys Ala Ser Val Asp Leu Thr Ser Thr Lys Asp Leu Leu Glu Leu
           35                  40                  45
   ttg gac aag ctg gga ccg ttc att tgt ctg gtc aag aca cac atc gac      192
   Leu Asp Lys Leu Gly Pro Phe Ile Cys Leu Val Lys Thr His Ile Asp
       50                  55                  60
   att gtg gaa gac ttt tcg tac gaa aac acc gtg gtg ccg ctg ctg aaa      240
   Ile Val Glu Asp Phe Ser Tyr Glu Asn Thr Val Val Pro Leu Leu Lys
   65                  70                  75                  80
   ctg gcc aag aaa cac aac ttc atg atc ttc gag gac cga aaa ttt gcc      288
   Leu Ala Lys Lys His Asn Phe Met Ile Phe Glu Asp Arg Lys Phe Ala
                   85                  90                  95
   gat ata ggc aac acc gtc aaa ctc cag tac aag gga gga gtt tac caa      336
   Asp Ile Gly Asn Thr Val Lys Leu Gln Tyr Lys Gly Gly Val Tyr Gln
               100                 105                 110
   atc gca aag tgg gcc gat atc acc aac gcc cac gga gtg acc ggc tcg      384
   Ile Ala Lys Trp Ala Asp Ile Thr Asn Ala His Gly Val Thr Gly Ser
           115                 120                 125
   cga att gtc tcg ggt ctc aga cag gct gcc cag gag acc acc gac gag      432
   Arg Ile Val Ser Gly Leu Arg Gln Ala Ala Gln Glu Thr Thr Asp Glu
       130                 135                 140
   cca aga ggt ctg ctc atg ctg gct gag ctg tcg tct gaa ggc tcg ctc      480
   Pro Arg Gly Leu Leu Met Leu Ala Glu Leu Ser Ser Glu Gly Ser Leu
   145                 150                 155                 160
   gcg tac gga gag tac acc aaa aag acg gtt gaa atc gca aag tcc gac      528
   Ala Tyr Gly Glu Tyr Thr Lys Lys Thr Val Glu Ile Ala Lys Ser Asp
                   165                 170                 175
   aga gat ttt gtg atc ggt ttc att gcg caa aac gac atg ggt ggc cgc      576
   Arg Asp Phe Val Ile Gly Phe Ile Ala Gln Asn Asp Met Gly Gly Arg
               180                 185                 190
   gat gag ggc ttc gac tgg ctc atc atg acc cca ggt gtc gga ctc gac      624
   Asp Glu Gly Phe Asp Trp Leu Ile Met Thr Pro Gly Val Gly Leu Asp
           195                 200                 205
   gac acc ggt gac gct ctg ggc cag cag tac cgc acg gtc agc gcc gtt      672
   Asp Thr Gly Asp Ala Leu Gly Gln Gln Tyr Arg Thr Val Ser Ala Val
       210                 215                 220
   atg aag acg gga act gac atc ata atc gtg ggc agg gga ctg ttc ggc      720
   Met Lys Thr Gly Thr Asp Ile Ile Ile Val Gly Arg Gly Leu Phe Gly
   225                 230                 235                 240
   aag gga aga gac cct gtc gtg gaa ggc gaa aga tac aga aag gct gga      768
   Lys Gly Arg Asp Pro Val Val Glu Gly Glu Arg Tyr Arg Lys Ala Gly
                   245                 250                 255
   tgg gac gct tat ttg agt cgt gtc gca tga                              798
   Trp Asp Ala Tyr Leu Ser Arg Val Ala
               260                 265
<210> 30
   <211> 265
   <212> PRT
   <213> Ogataea minuta
<400> 30
   Met Ser Ser Thr Lys Thr Tyr Ala Gln Arg Ala Ala Ala His Pro Ser
   1               5                   10                  15
   Pro Val Ala Arg Arg Leu Leu Asn Leu Met Glu Ser Lys Lys Thr Asn
               20                  25                  30
   Leu Cys Ala Ser Val Asp Leu Thr Ser Thr Lys Asp Leu Leu Glu Leu
           35                  40                  45
   Leu Asp Lys Leu Gly Pro Phe Ile Cys Leu Val Lys Thr His Ile Asp
       50                  55                  60
   Ile Val Glu Asp Phe Ser Tyr Glu Asn Thr Val Val Pro Leu Leu Lys
   65                  70                  75                  80
   Leu Ala Lys Lys His Asn Phe Met Ile Phe Glu Asp Arg Lys Phe Ala
                   85                  90                  95
   Asp Ile Gly Asn Thr Val Lys Leu Gln Tyr Lys Gly Gly Val Tyr Gln
               100                 105                 110
   Ile Ala Lys Trp Ala Asp Ile Thr Asn Ala His Gly Val Thr Gly Ser
           115                 120                 125
   Arg Ile Val Ser Gly Leu Arg Gln Ala Ala Gln Glu Thr Thr Asp Glu
       130                 135                 140
   Pro Arg Gly Leu Leu Met Leu Ala Glu Leu Ser Ser Glu Gly Ser Leu
   145                 150                 155                 160
   Ala Tyr Gly Glu Tyr Thr Lys Lys Thr Val Glu Ile Ala Lys Ser Asp
                   165                 170                 175
   Arg Asp Phe Val Ile Gly Phe Ile Ala Gln Asn Asp Met Gly Gly Arg
               180                 185                 190
   Asp Glu Gly Phe Asp Trp Leu Ile Met Thr Pro Gly Val Gly Leu Asp
           195                 200                 205
   Asp Thr Gly Asp Ala Leu Gly Gln Gln Tyr Arg Thr Val Ser Ala Val
       210                 215                 220
   Met Lys Thr Gly Thr Asp Ile Ile Ile Val Gly Arg Gly Leu Phe Gly
   225 230 235 240
   Lys Gly Arg Asp Pro Val Val Glu Gly Glu Arg Tyr Arg Lys Ala Gly
   245 250 255
   Trp Asp Ala Tyr Leu Ser Arg Val Ala
   260 265
<210> 31
   <211> 1620
   <212> DNA
   <213> Ogataea minuta
<220>
   <221> CDS
   <222> (1)..(1620)
<400> 31
   atg aag tta tcc cag tct gct gcg gtg gct att ctg tct tcg ttg gca        48
   Met Lys Leu Ser Gln Ser Ala Ala Val Ala Ile Leu Ser Ser Leu Ala
   1               5                   10                  15
   gca gtg gag gcc ttg gtc atc ccg tta ttt gac gac ttg cca gca gag        96
   Ala Val Glu Ala Leu Val Ile Pro Leu Phe Asp Asp Leu Pro Ala Glu
               20                  25                  30
   ttt gcc ctt gtt cca atg gat gcg aaa gcg gaa gtc att tct gac gtt       144
   Phe Ala Leu Val Pro Met Asp Ala Lys Ala Glu Val Ile Ser Asp Val
           35                  40                  45
   cct gtc gac tcg gcc att agt gat gct cct atc gcg gca cta aat gat       192
   Pro Val Asp Ser Ala Ile Ser Asp Ala Pro Ile Ala Ala Leu Asn Asp
       50                  55                  60
   gct cca agc cct ctc gtc aca tcg ctg atc gca tct caa aat ttg att       240
   Ala Pro Ser Pro Leu Val Thr Ser Leu Ile Ala Ser Gln Asn Leu Ile
   65                  70                  75                  80
   cca aac tct tat att gtc gtt ttc aag aat ggc cta gct tcc ggg gca       288
   Pro Asn Ser Tyr Ile Val Val Phe Lys Asn Gly Leu Ala Ser Gly Ala
                   85                  90                  95
   gtt gac ttc cac atg gag tgg ctc aag gaa acg cac tcc caa acc ctg       336
   Val Asp Phe His Met Glu Trp Leu Lys Glu Thr His Ser Gln Thr Leu
               100                 105                 110
   gct gct ttg tct aag gac atg cca gca gaa gaa ttg gcc gcc gaa ggt       384
   Ala Ala Leu Ser Lys Asp Met Pro Ala Glu Glu Leu Ala Ala Glu Gly
           115                 120                 125
   ttc gtt tcc gaa agc att gat ctt act gag gtg ttt agc atc tcc gat       432
   Phe Val Ser Glu Ser Ile Asp Leu Thr Glu Val Phe Ser Ile Ser Asp
       130                 135                 140
   ttg ttc agt gga tat acc gga tac ttc ccg gag aag gtg gtt gac ctc       480
   Leu Phe Ser Gly Tyr Thr Gly Tyr Phe Pro Glu Lys Val Val Asp Leu
   145                 150                 155                 160
   atc aga aga cac cct gac gtg gcg ttc gtt gag cag gac tcg aga gtt       528
   Ile Arg Arg His Pro Asp Val Ala Phe Val Glu Gln Asp Ser Arg Val
                   165                 170                 175
   ttc gcc gat aag tcg tct act caa aac ggt gct cct tgg ggt ttg tct      576
   Phe Ala Asp Lys Ser Ser Thr Gln Asn Gly Ala Pro Trp Gly Leu Ser
               180                 185                 190
   aga atc tct cac aga gag cct ctc agt ctc ggc aat ttc aac gag tac      624
   Arg Ile Ser His Arg Glu Pro Leu Ser Leu Gly Asn Phe Asn Glu Tyr
           195                 200                 205
   gtt tac gac gat ctt gct gga gat ggc gtc acg gct tat gtc att gat      672
   Val Tyr Asp Asp Leu Ala Gly Asp Gly Val Thr Ala Tyr Val Ile Asp
       210                 215                 220
   acc ggt atc aat gtg aag cac gag cag ttc ggt ggc aga gca gag tgg      720
   Thr Gly Ile Asn Val Lys His Glu Gln Phe Gly Gly Arg Ala Glu Trp
   225                 230                 235                 240
   ggt aag acc atc cca acc ggt gat gat gat att gac gga aac ggt cac      768
   Gly Lys Thr Ile Pro Thr Gly Asp Asp Asp Ile Asp Gly Asn Gly His
                   245                 250                 255
   ggt act cac tgc gct ggt aca att ggc tcg gaa gat tat gga gtt tct      816
   Gly Thr His Cys Ala Gly Thr Ile Gly Ser Glu Asp Tyr Gly Val Ser
               260                 265                 270
   aag aac tcc aaa att gtc gca gtg aag gtt ttg aga tct aac ggt tct      864
   Lys Asn Ser Lys Ile Val Ala Val Lys Val Leu Arg Ser Asn Gly Ser
           275                 280                 285
   ggt tcc atg tct gac gtg atc aag ggt gtt gaa ttc gct gca aat gat      912
   Gly Ser Met Ser Asp Val Ile Lys Gly Val Glu Phe Ala Ala Asn Asp
       290                 295                 300
   cac gtt gcc aag tct aaa gcc aag aag gac ggt ttc aag gga tcg act      960
   His Val Ala Lys Ser Lys Ala Lys Lys Asp Gly Phe Lys Gly Ser Thr
   305                 310                 315                 320
   gcc aac atg tct ttg gga ggt ggc aag tct cct gct ctt gac ttg gct     1008
   Ala Asn Met Ser Leu Gly Gly Gly Lys Ser Pro Ala Leu Asp Leu Ala
                   325                 330                 335
   gtc aat gcc gct gtc aaa gct ggt tta cac ttt gct gtt gcc gct ggt     1056
   Val Asn Ala Ala Val Lys Ala Gly Leu His Phe Ala Val Ala Ala Gly
               340                 345                 350
   aac gac aat gct gac gca tgc aac tat tct cct gct gct gca gag aac     1104
   Asn Asp Asn Ala Asp Ala Cys Asn Tyr Ser Pro Ala Ala Ala Glu Asn
           355                 360                 365
   gca gtc act gtt ggt gcg tcc act ttg tct gac tct aga gct tac ttt     1152
   Ala Val Thr Val Gly Ala Ser Thr Leu Ser Asp Ser Arg Ala Tyr Phe
       370                 375                 380
   tcc aac tat ggt aaa tgt gtt gac att ttt gct ccg ggc ttg aac atc     1200
   Ser Asn Tyr Gly Lys Cys Val Asp Ile Phe Ala Pro Gly Leu Asn Ile
   385                 390                 395                 400
   ctt tcc acc tac ata ggt tct gac act gcc acc gcc act ctt tct ggt     1248
   Leu Ser Thr Tyr Ile Gly Ser Asp Thr Ala Thr Ala Thr Leu Ser Gly
                   405                 410                 415
   aca tcg atg gcc tcc cct cac gtt tgt ggt ctg ttg acc tac ttt ttg      1296
   Thr Ser Met Ala Ser Pro His Val Cys Gly Leu Leu Thr Tyr Phe Leu
               420                 425                 430
   agc ttg caa cca gaa tcg tcg tcg ttg ttt tct tcg gca gct atc tcc      1344
   Ser Leu Gln Pro Glu Ser Ser Ser Leu Phe Ser Ser Ala Ala Ile Ser
           435                 440                 445
   cct gct cag ctg aag aag aac ctg atc aag ttt ggt acg aag aac gtt      1392
   Pro Ala Gln Leu Lys Lys Asn Leu Ile Lys Phe Gly Thr Lys Asn Val
       450                 455                 460
   ttg tct gag att cca tcg gac gga acc cca aat att ctc att tac aac      1440
   Leu Ser Glu Ile Pro Ser Asp Gly Thr Pro Asn Ile Leu Ile Tyr Asn
   465                 470                 475                 480
   ggt gct ggc aag aac atc agt gac ttc tgg gcg ttt gaa gac gag gcc      1488
   Gly Ala Gly Lys Asn Ile Ser Asp Phe Trp Ala Phe Glu Asp Glu Ala
                   485                 490                 495
   tcg gcc aag tcc gac ttg aag aag gct gtc gat att gcc aca agt gtt      1536
   Ser Ala Lys Ser Asp Leu Lys Lys Ala Val Asp Ile Ala Thr Ser Val
               500                 505                 510
   gac tta gac ctg caa gat atc aag gag aag ttc aac cat att ttg gag      1584
   Asp Leu Asp Leu Gln Asp Ile Lys Glu Lys Phe Asn His Ile Leu Glu
           515                 520                 525
   gag gtc gcc gaa gag gtt gct gat ttg ttc gat tag                      1620
   Glu Val Ala Glu Glu Val Ala Asp Leu Phe Asp
       530                 535
<210> 32
   <211> 539
   <212> PRT
   <213> Ogataea minuta
<400> 32
   Met Lys Leu Ser Gln Ser Ala Ala Val Ala Ile Leu Ser Ser Leu Ala
   1               5                   10                  15
   Ala Val Glu Ala Leu Val Ile Pro Leu Phe Asp Asp Leu Pro Ala Glu
               20                  25                  30
   Phe Ala Leu Val Pro Met Asp Ala Lys Ala Glu Val Ile Ser Asp Val
           35                  40                  45
   Pro Val Asp Ser Ala Ile Ser Asp Ala Pro Ile Ala Ala Leu Asn Asp
       50                  55                  60
   Ala Pro Ser Pro Leu Val Thr Ser Leu Ile Ala Ser Gln Asn Leu Ile
   65                  70                  75                  80
   Pro Asn Ser Tyr Ile Val Val Phe Lys Asn Gly Leu Ala Ser Gly Ala
                   85                  90                  95
   Val Asp Phe His Met Glu Trp Leu Lys Glu Thr His Ser Gln Thr Leu
               100                 105                 110
   Ala Ala Leu Ser Lys Asp Met Pro Ala Glu Glu Leu Ala Ala Glu Gly
           115                 120                 125
   Phe Val Ser Glu Ser Ile Asp Leu Thr Glu Val Phe Ser Ile Ser Asp
       130                 135                 140
   Leu Phe Ser Gly Tyr Thr Gly Tyr Phe Pro Glu Lys Val Val Asp Leu
   145                 150                 155                 160
   Ile Arg Arg His Pro Asp Val Ala Phe Val Glu Gln Asp Ser Arg Val
                   165                 170                 175
   Phe Ala Asp Lys Ser Ser Thr Gln Asn Gly Ala Pro Trp Gly Leu Ser
               180                 185                 190
   Arg Ile Ser His Arg Glu Pro Leu Ser Leu Gly Asn Phe Asn Glu Tyr
           195                 200                 205
   Val Tyr Asp Asp Leu Ala Gly Asp Gly Val Thr Ala Tyr Val Ile Asp
       210                 215                 220
   Thr Gly Ile Asn Val Lys His Glu Gln Phe Gly Gly Arg Ala Glu Trp
   225                 230                 235                 240
   Gly Lys Thr Ile Pro Thr Gly Asp Asp Asp Ile Asp Gly Asn Gly His
                   245                 250                 255
   Gly Thr His Cys Ala Gly Thr Ile Gly Ser Glu Asp Tyr Gly Val Ser
               260                 265                 270
   Lys Asn Ser Lys Ile Val Ala Val Lys Val Leu Arg Ser Asn Gly Ser
           275                 280                 285
   Gly Ser Met Ser Asp Val Ile Lys Gly Val Glu Phe Ala Ala Asn Asp
       290                 295                 300
   His Val Ala Lys Ser Lys Ala Lys Lys Asp Gly Phe Lys Gly Ser Thr
   305                 310                 315                 320
   Ala Asn Met Ser Leu Gly Gly Gly Lys Ser Pro Ala Leu Asp Leu Ala
                   325                 330                 335
   Val Asn Ala Ala Val Lys Ala Gly Leu His Phe Ala Val Ala Ala Gly
               340                 345                 350
   Asn Asp Asn Ala Asp Ala Cys Asn Tyr Ser Pro Ala Ala Ala Glu Asn
           355                 360                 365
   Ala Val Thr Val Gly Ala Ser Thr Leu Ser Asp Ser Arg Ala Tyr Phe
       370                 375                 380
   Ser Asn Tyr Gly Lys Cys Val Asp Ile Phe Ala Pro Gly Leu Asn Ile
   385                 390                 395                 400
   Leu Ser Thr Tyr Ile Gly Ser Asp Thr Ala Thr Ala Thr Leu Ser Gly
                   405                 410                 415
   Thr Ser Met Ala Ser Pro His Val Cys Gly Leu Leu Thr Tyr Phe Leu
               420                 425                 430
   Ser Leu Gln Pro Glu Ser Ser Ser Leu Phe Ser Ser Ala Ala Ile Ser
           435                 440                 445
   Pro Ala Gln Leu Lys Lys Asn Leu Ile Lys Phe Gly Thr Lys Asn Val
       450                 455                 460
   Leu Ser Glu Ile Pro Ser Asp Gly Thr Pro Asn Ile Leu Ile Tyr Asn
   465                 470                 475                 480
   Gly Ala Gly Lys Asn Ile Ser Asp Phe Trp Ala Phe Glu Asp Glu Ala
                   485                 490                 495
   Ser Ala Lys Ser Asp Leu Lys Lys Ala Val Asp Ile Ala Thr Ser Val
               500                 505                 510
   Asp Leu Asp Leu Gln Asp Ile Lys Glu Lys Phe Asn His Ile Leu Glu
           515                 520                 525
   Glu Val Ala Glu Glu Val Ala Asp Leu Phe Asp
       530                 535
<210> 33
   <211> 814
   <212> PRT
   <213> Saccharomyces cerevisiae
<400> 33
   Met Lys Val Arg Lys Tyr Ile Thr Leu Cys Phe Trp Trp Ala Phe Ser
   1               5                   10                  15
   Thr Ser Ala Leu Val Ser Ser Gln Gln Ile Pro Leu Lys Asp His Thr
               20                  25                  30
   Ser Arg Gln Tyr Phe Ala Val Glu Ser Asn Glu Thr Leu Ser Arg Leu
           35                  40                  45
   Glu Glu Met His Pro Asn Trp Lys Tyr Glu His Asp Val Arg Gly Leu
       50                  55                  60
   Pro Asn His Tyr Val Phe Ser Lys Glu Leu Leu Lys Leu Gly Lys Arg
   65                  70                  75                  80
   Ser Ser Leu Glu Glu Leu Gln Gly Asp Asn Asn Asp His Ile Leu Ser
                   85                  90                  95
   Val His Asp Leu Phe Pro Arg Asn Asp Leu Phe Lys Arg Leu Pro Val
               100                 105                 110
   Pro Ala Pro Pro Met Asp Ser Ser Leu Leu Pro Val Lys Glu Ala Glu
           115                 120                 125
   Asp Lys Leu Ser Ile Asn Asp Pro Leu Phe Glu Arg Gln Trp His Leu
       130                 135                 140
   Val Asn Pro Ser Phe Pro Gly Ser Asp Ile Asn Val Leu Asp Leu Trp
   145                 150                 155                 160
   Tyr Asn Asn Ile Thr Gly Ala Gly Val Val Ala Ala Ile Val Asp Asp
                   165                 170                 175
   Gly Leu Asp Tyr Glu Asn Glu Asp Leu Lys Asp Asn Phe Cys Ala Glu
               180                 185                 190
   Gly Ser Trp Asp Phe Asn Asp Asn Thr Asn Leu Pro Lys Pro Arg Leu
           195                 200                 205
   Ser Asp Asp Tyr His Gly Thr Arg Cys Ala Gly Glu Ile Ala Ala Lys
       210                 215                 220
   Lys Gly Asn Asn Phe Cys Gly Val Gly Val Gly Tyr Asn Ala Lys Ile
   225                 230                 235                 240
   Ser Gly Ile Arg Ile Leu Ser Gly Asp Ile Thr Thr Glu Asp Glu Ala
                   245                 250                 255
   Ala Ser Leu Ile Tyr Gly Leu Asp Val Asn Asp Ile Tyr Ser Cys Ser
               260                 265                 270
   Trp Gly Pro Ala Asp Asp Gly Arg His Leu Gln Gly Pro Ser Asp Leu
           275                 280                 285
   Val Lys Lys Ala Leu Val Lys Gly Val Thr Glu Gly Arg Asp Ser Lys
       290                 295                 300
   Gly Ala Ile Tyr Val Phe Ala Ser Gly Asn Gly Gly Thr Arg Gly Asp
   305                 310                 315                 320
   Asn Cys Asn Tyr Asp Gly Tyr Thr Asn Ser Ile Tyr Ser Ile Thr Ile
                   325                 330                 335
   Gly Ala Ile Asp His Lys Asp Leu His Pro Pro Tyr Ser Glu Gly Cys
               340                 345                 350
   Ser Ala Val Met Ala Val Thr Tyr Ser Ser Gly Ser Gly Glu Tyr Ile
           355                 360                 365
   His Ser Ser Asp Ile Asn Gly Arg Cys Ser Asn Ser His Gly Gly Thr
       370                 375                 380
   Ser Ala Ala Ala Pro Leu Ala Ala Gly Val Tyr Thr Leu Leu Leu Glu
   385                 390                 395                 400
   Ala Asn Pro Asn Leu Thr Trp Arg Asp Val Gln Tyr Leu Ser Ile Leu
                   405                 410                 415
   Ser Ala Val Gly Leu Glu Lys Asn Ala Asp Gly Asp Trp Arg Asp Ser
               420                 425                 430
   Ala Met Gly Lys Lys Tyr Ser His Arg Tyr Gly Phe Gly Lys Ile Asp
           435                 440                 445
   Ala His Lys Leu Ile Glu Met Ser Lys Thr Trp Glu Asn Val Asn Ala
       450                 455                 460
   Gln Thr Trp Phe Tyr Leu Pro Thr Leu Tyr Val Ser Gln Ser Thr Asn
   465                 470                 475                 480
   Ser Thr Glu Glu Thr Leu Glu Ser Val Ile Thr Ile Ser Glu Lys Ser
                   485                 490                 495
   Leu Gln Asp Ala Asn Phe Lys Arg Ile Glu His Val Thr Val Thr Val
               500                 505                 510
   Asp Ile Asp Thr Glu Ile Arg Gly Thr Thr Thr Val Asp Leu Ile Ser
           515                 520                 525
   Pro Ala Gly Ile Ile Ser Asn Leu Gly Val Val Arg Pro Arg Asp Val
       530                 535                 540
   Ser Ser Glu Gly Phe Lys Asp Trp Thr Phe Met Ser Val Ala His Trp
   545                 550                 555                 560
   Gly Glu Asn Gly Val Gly Asp Trp Lys Ile Lys Val Lys Thr Thr Glu
                   565                 570                 575
   Asn Gly His Arg Ile Asp Phe His Ser Trp Arg Leu Lys Leu Phe Gly
               580                 585                 590
   Glu Ser Ile Asp Ser Ser Lys Thr Glu Thr Phe Val Phe Gly Asn Asp
           595                 600                 605
   Lys Glu Glu Val Glu Pro Ala Ala Thr Glu Ser Thr Val Ser Gln Tyr
       610                 615                 620
   Ser Ala Ser Ser Thr Ser Ile Ser Ile Ser Ala Thr Ser Thr Ser Ser
   625                 630                 635                 640
   Ile Ser Ile Gly Val Glu Thr Ser Ala Ile Pro Gln Thr Thr Thr Ala
                   645                 650                 655
   Ser Thr Asp Pro Asp Ser Asp Pro Asn Thr Pro Lys Lys Leu Ser Ser
               660                 665                 670
   Pro Arg Gln Ala Met His Tyr Phe Leu Thr Ile Phe Leu Ile Gly Ala
           675                 680                 685
   Thr Phe Leu Val Leu Tyr Phe Met Phe Phe Met Lys Ser Arg Arg Arg
       690                 695                 700
   Ile Arg Arg Ser Arg Ala Glu Thr Tyr Glu Phe Asp Ile Ile Asp Thr
   705                 710                 715                 720
   Asp Ser Glu Tyr Asp Ser Thr Leu Asp Asn Gly Thr Ser Gly Ile Thr
                   725                 730                 735
   Glu Pro Glu Glu Val Glu Asp Phe Asp Phe Asp Leu Ser Asp Glu Asp
               740                 745                 750
   His Leu Ala Ser Leu Ser Ser Ser Glu Asn Gly Asp Ala Glu His Thr
           755                 760                 765
   Ile Asp Ser Val Leu Thr Asn Glu Asn Pro Phe Ser Asp Pro Ile Lys
       770                 775                 780
   Gln Lys Phe Pro Asn Asp Ala Asn Ala Glu Ser Ala Ser Asn Lys Leu
   785 790 795 800
   Gln Glu Leu Gln Pro Asp Val Pro Pro Ser Ser Gly Arg Ser
   805 810
<210> 34
   <211> 609
   <212> PRT
   <213> Homo sapiens
<400> 34
<210> 35
   <211> 1551
   <212> DNA
   <213> Ogataea minuta
<220>
   <221> CDS
   <222> (1)..(1551)
<400> 35
   atg aag tta ttt gga ttg act aca ttg acc agc atc ctg gcc gcc ctc      48
   Met Lys Leu Phe Gly Leu Thr Thr Leu Thr Ser Ile Leu Ala Ala Leu
   1               5                   10                  15
   aca gtg gtg agc gcc acc gaa gag cca gca gtt gcc tcg cca gac tcg      96
   Thr Val Val Ser Ala Thr Glu Glu Pro Ala Val Ala Ser Pro Asp Ser
                   20                  25                  30
   gcg gtg gtg aag ctg acg gcc gac act ttc gag agc ttc atc aag gag      144
   Ala Val Val Lys Leu Thr Ala Asp Thr Phe Glu Ser Phe Ile Lys Glu
               35                  40                  45
   aac cca ttg gtt ctg gcg gag ttt ttt gcg cca tgg tgt ggc cac tgc      192
   Asn Pro Leu Val Leu Ala Glu Phe Phe Ala Pro Trp Cys Gly His Cys
           50                  55                  60
   aag aag ctg ggt cca gaa ttc agc gca gcc gca gac cag ctg gtg gag      240
   Lys Lys Leu Gly Pro Glu Phe Ser Ala Ala Ala Asp Gln Leu Val Glu
   65                  70                  75                  80
   aag aac atc aag ctt gca cag atc gac tgt acc gag gaa aga gat ctg      288
   Lys Asn Ile Lys Leu Ala Gln Ile Asp Cys Thr Glu Glu Arg Asp Leu
                   85                  90                  95
   tgt tcg tcg cac gga atc aga gga tac cca act ttg aag gtg ttc agg      336
   Cys Ser Ser His Gly Ile Arg Gly Tyr Pro Thr Leu Lys Val Phe Arg
               100                 105                 110
   ggc gct agt gag cct gct gac tac caa ggc gcc aga gaa cag gaa gct      384
   Gly Ala Ser Glu Pro Ala Asp Tyr Gln Gly Ala Arg Glu Gln Glu Ala
           115                 120                 125
   att gtc agt caa atg atc aag ctt tct tta cct gct gtt tcc gtc att      432
   Ile Val Ser Gln Met Ile Lys Leu Ser Leu Pro Ala Val Ser Val Ile
       130                 135                 140
   gag gat tct gcc gac ctg ttt gat acc att gca gaa gtc tcc gac gcc      480
   Glu Asp Ser Ala Asp Leu Phe Asp Thr Ile Ala Glu Val Ser Asp Ala
   145                 150                 155                 160
   ctc att gtg caa gtg ttt cct gcg gga gct gct cag tct tcc aac gag      528
   Leu Ile Val Gln Val Phe Pro Ala Gly Ala Ala Gln Ser Ser Asn Glu
                   165                 170                 175
   acg ttc tac gaa gtc gcc aac gaa ctg aga aac gac ttt gtt ttt gtc      576
   Thr Phe Tyr Glu Val Ala Asn Glu Leu Arg Asn Asp Phe Val Phe Val
               180                 185                 190
   tcc acc act aac gag ggg tac gtg aaa aag tac gcg aag gac tca aag      624
   Ser Thr Thr Asn Glu Gly Tyr Val Lys Lys Tyr Ala Lys Asp Ser Lys
           195                 200                 205
   tca cct gct tat gtc atc ttc agg caa gga gaa aag gtt gaa gat gcg      672
   Ser Pro Ala Tyr Val Ile Phe Arg Gln Gly Glu Lys Val Glu Asp Ala
       210                 215                 220
   tcc aca tac acc gga aag act gtt gac gac act cac ttg aag cag ttc      720
   Ser Thr Tyr Thr Gly Lys Thr Val Asp Asp Thr His Leu Lys Gln Phe
   225                 230                 235                 240
   atc aat acc gaa acc aaa cct ctg ttt ggt gaa atc acc ggc aac act      768
   Ile Asn Thr Glu Thr Lys Pro Leu Phe Gly Glu Ile Thr Gly Asn Thr
                   245                 250                 255
   ttc aag acc tac atg gag gcc gag ctt cct ttg gcg tac ttt ttc tgg      816
   Phe Lys Thr Tyr Met Glu Ala Glu Leu Pro Leu Ala Tyr Phe Phe Trp
               260                 265                 270
   gac gaa gag tct caa agg gcc gag gtc gct gac atc atc acc gag ctg      864
   Asp Glu Glu Ser Gln Arg Ala Glu Val Ala Asp Ile Ile Thr Glu Leu
           275                 280                 285
   gcc aag aag ttt aga gga gag atg aac ttt gtt ggt ttg gaa gcc aag      912
   Ala Lys Lys Phe Arg Gly Glu Met Asn Phe Val Gly Leu Glu Ala Lys
       290                 295                 300
   aga tac ggt atg cat gcc aag aac ctc aac atg gag gaa aag ttc ccc      960
   Arg Tyr Gly Met His Ala Lys Asn Leu Asn Met Glu Glu Lys Phe Pro
   305                 310                 315                 320
   ttg ttc gcc atc cac gat ttg acc gga aac ctc aag tac ggt att tct     1008
   Leu Phe Ala Ile His Asp Leu Thr Gly Asn Leu Lys Tyr Gly Ile Ser
                   325                 330                 335
   caa gag tct gat ctt gac gtc aag gaa atc cct aag ttc gtt gag gat     1056
   Gln Glu Ser Asp Leu Asp Val Lys Glu Ile Pro Lys Phe Val Glu Asp
               340                 345                 350
   ttc aag aag ggc aag ttg caa gca att gtc aag tct gag cca att cca     1104
   Phe Lys Lys Gly Lys Leu Gln Ala Ile Val Lys Ser Glu Pro Ile Pro
           355                 360                 365
   gaa gtc caa gag gag tcc gtg tac cac ctg gtt gga cac gag cac gac     1152
   Glu Val Gln Glu Glu Ser Val Tyr His Leu Val Gly His Glu His Asp
       370                 375                 380
   gcc atc acc aag cag aag aag gac gtt ttg gtt gag tac tac gcc cca     1200
   Ala Ile Thr Lys Gln Lys Lys Asp Val Leu Val Glu Tyr Tyr Ala Pro
   385                 390                 395                 400
   tgg tgt gga cac tgc aag aag ctg gct cca act tac gaa att ttg gcc     1248
   Trp Cys Gly His Cys Lys Lys Leu Ala Pro Thr Tyr Glu Ile Leu Ala
                   405                 410                 415
   agc atc tac cag aac gac act gat gcc aag gaa aag gtt gtg att gcc     1296
   Ser Ile Tyr Gln Asn Asp Thr Asp Ala Lys Glu Lys Val Val Ile Ala
               420                 425                 430
   aag att gac cac act gcc aac gat gtt gcc ggt gtc gac atc gcc ggt     1344
   Lys Ile Asp His Thr Ala Asn Asp Val Ala Gly Val Asp Ile Ala Gly
           435                 440                 445
   tat cca acc atc atc ttg tat cct ggt gac gaa tct gag ccg gtt gtg     1392
   Tyr Pro Thr Ile Ile Leu Tyr Pro Gly Asp Glu Ser Glu Pro Val Val
       450                 455                 460
   tac gag ggt tct aga act cta gag gct ctc agt tca ttc atc aag gag     1440
   Tyr Glu Gly Ser Arg Thr Leu Glu Ala Leu Ser Ser Phe Ile Lys Glu
   465                 470                 475                 480
   aag ggt tcg aac ggc gtt gac gct ttg tcc atc aag gaa tcg cgt gtt     1488
   Lys Gly Ser Asn Gly Val Asp Ala Leu Ser Ile Lys Glu Ser Arg Val
                   485                 490                 495
   gaa aaa gaa gcc gat gct caa gcc gat gct cct gac gct ggc gtg gct     1536
   Glu Lys Glu Ala Asp Ala Gln Ala Asp Ala Pro Asp Ala Gly Val Ala
               500                 505                 510
   cac gac gag ttg taa                                                 1551
   His Asp Glu Leu
           515
<210> 36
   <211> 516
   <212> PRT
   <213> Ogataea minuta
<400> 36
   Met Lys Leu Phe Gly Leu Thr Thr Leu Thr Ser Ile Leu Ala Ala Leu
   1               5                   10                  15
   Thr Val Val Ser Ala Thr Glu Glu Pro Ala Val Ala Ser Pro Asp Ser
               20                  25                  30
   Ala Val Val Lys Leu Thr Ala Asp Thr Phe Glu Ser Phe Ile Lys Glu
           35                  40                  45
   Asn Pro Leu Val Leu Ala Glu Phe Phe Ala Pro Trp Cys Gly His Cys
       50                  55                  60
   Lys Lys Leu Gly Pro Glu Phe Ser Ala Ala Ala Asp Gln Leu Val Glu
   65                  70                  75                  80
   Lys Asn Ile Lys Leu Ala Gln Ile Asp Cys Thr Glu Glu Arg Asp Leu
                   85                  90                  95
   Cys Ser Ser His Gly Ile Arg Gly Tyr Pro Thr Leu Lys Val Phe Arg
               100                 105                 110
   Gly Ala Ser Glu Pro Ala Asp Tyr Gln Gly Ala Arg Glu Gln Glu Ala
           115                 120                 125
   Ile Val Ser Gln Met Ile Lys Leu Ser Leu Pro Ala Val Ser Val Ile
       130                 135                 140
   Glu Asp Ser Ala Asp Leu Phe Asp Thr Ile Ala Glu Val Ser Asp Ala
   145                 150                 155                 160
   Leu Ile Val Gln Val Phe Pro Ala Gly Ala Ala Gln Ser Ser Asn Glu
                   165                 170                 175
   Thr Phe Tyr Glu Val Ala Asn Glu Leu Arg Asn Asp Phe Val Phe Val
               180                 185                 190
   Ser Thr Thr Asn Glu Gly Tyr Val Lys Lys Tyr Ala Lys Asp Ser Lys
           195                 200                 205
   Ser Pro Ala Tyr Val Ile Phe Arg Gln Gly Glu Lys Val Glu Asp Ala
       210                 215                 220
   Ser Thr Tyr Thr Gly Lys Thr Val Asp Asp Thr His Leu Lys Gln Phe
   225                 230                 235                 240
   Ile Asn Thr Glu Thr Lys Pro Leu Phe Gly Glu Ile Thr Gly Asn Thr
                   245                 250                 255
   Phe Lys Thr Tyr Met Glu Ala Glu Leu Pro Leu Ala Tyr Phe Phe Trp
               260                 265                 270
   Asp Glu Glu Ser Gln Arg Ala Glu Val Ala Asp Ile Ile Thr Glu Leu
           275                 280                 285
   Ala Lys Lys Phe Arg Gly Glu Met Asn Phe Val Gly Leu Glu Ala Lys
       290                 295                 300
   Arg Tyr Gly Met His Ala Lys Asn Leu Asn Met Glu Glu Lys Phe Pro
   305                 310                 315                 320
   Leu Phe Ala Ile His Asp Leu Thr Gly Asn Leu Lys Tyr Gly Ile Ser
                   325                 330                 335
   Gln Glu Ser Asp Leu Asp Val Lys Glu Ile Pro Lys Phe Val Glu Asp
               340                 345                 350
   Phe Lys Lys Gly Lys Leu Gln Ala Ile Val Lys Ser Glu Pro Ile Pro
           355                 360                 365
   Glu Val Gln Glu Glu Ser Val Tyr His Leu Val Gly His Glu His Asp
       370                 375                 380
   Ala Ile Thr Lys Gln Lys Lys Asp Val Leu Val Glu Tyr Tyr Ala Pro
   385                 390                 395                 400
   Trp Cys Gly His Cys Lys Lys Leu Ala Pro Thr Tyr Glu Ile Leu Ala
                   405                 410                 415
   Ser Ile Tyr Gln Asn Asp Thr Asp Ala Lys Glu Lys Val Val Ile Ala
               420                 425                 430
   Lys Ile Asp His Thr Ala Asn Asp Val Ala Gly Val Asp Ile Ala Gly
           435                 440                 445
   Tyr Pro Thr Ile Ile Leu Tyr Pro Gly Asp Glu Ser Glu Pro Val Val
       450                 455                 460
   Tyr Glu Gly Ser Arg Thr Leu Glu Ala Leu Ser Ser Phe Ile Lys Glu
   465                 470                 475                 480
   Lys Gly Ser Asn Gly Val Asp Ala Leu Ser Ile Lys Glu Ser Arg Val
               485                 490                 495
   Glu Lys Glu Ala Asp Ala Gln Ala Asp Ala Pro Asp Ala Gly Val Ala
           500                 505                 510
   His Asp Glu Leu
       515
<210> 37
   <211> 936
   <212> DNA
   <213> Ogataea minuta
<220>
   <221> CDS
   <222> (1)..(936)
<400> 37
   atg aaa gtg gca agt ttg ata gca ttg gtc gtt acg ccg att att gct       48
   Met Lys Val Ala Ser Leu Ile Ala Leu Val Val Thr Pro Ile Ile Ala
   1               5                   10                  15
   gcc act ggg gtg gta gca gat ccg cag cag cag gcc aaa aga cct ggt       96
   Ala Thr Gly Val Val Ala Asp Pro Gln Gln Gln Ala Lys Arg Pro Gly
               20                  25                  30
   ttt tac aag aat tca aag cat atc tac aat ctc act ccc cag aac ttt      144
   Phe Tyr Lys Asn Ser Lys His Ile Tyr Asn Leu Thr Pro Gln Asn Phe
           35                  40                  45
   gac gac gtg gtc ctg caa acc aac cat acg tct gtc gtg gag ttc tat      192
   Asp Asp Val Val Leu Gln Thr Asn His Thr Ser Val Val Glu Phe Tyr
       50                  55                  60
   gcg cca tgg tgt ggc tat tgc gca gag ttt gag agc cag tac cgc aaa      240
   Ala Pro Trp Cys Gly Tyr Cys Ala Glu Phe Glu Ser Gln Tyr Arg Lys
   65                  70                  75                  80
   gca gca aag atc gga tcg gag ttc gtg aat ttt gcg gcc gtt aac tgc      288
   Ala Ala Lys Ile Gly Ser Glu Phe Val Asn Phe Ala Ala Val Asn Cys
                   85                  90                  95
   gac gaa gac aag aac aaa cca ttg tgc aac aag tac cgc gtc gaa ggg      336
   Asp Glu Asp Lys Asn Lys Pro Leu Cys Asn Lys Tyr Arg Val Glu Gly
               100                 105                 110
   ttc ccg acg gtg atg gtt ttc cgt cca gcg aag gtc aac tcg gcg gga      384
   Phe Pro Thr Val Met Val Phe Arg Pro Ala Lys Val Asn Ser Ala Gly
           115                 120                 125
   tcc aac ggc aac agg cct cat tcc tcc gaa acg tat cgg ggc gag aga      432
   Ser Asn Gly Asn Arg Pro His Ser Ser Glu Thr Tyr Arg Gly Glu Arg
       130                 135                 140
   acg gcg gct aag ttg ctc gag cat gtc aag ggc cgt gtg gtg aat tac      480
   Thr Ala Ala Lys Leu Leu Glu His Val Lys Gly Arg Val Val Asn Tyr
   145                 150                 155                 160
   gtg aag aga atc aag ctc aac aaa ctt gat gaa ttt ctc aaa ccg aat      528
   Val Lys Arg Ile Lys Leu Asn Lys Leu Asp Glu Phe Leu Lys Pro Asn
                   165                 170                 175
   gaa aag agc aga gtc ttg ctg gtg act tca aaa agc act ctt tcg ccg      576
   Glu Lys Ser Arg Val Leu Leu Val Thr Ser Lys Ser Thr Leu Ser Pro
               180                 185                 190
   gtt ttc aag agc ctg tcg atc gat ttt ctc gac tca gtc acg ttg gca      624
   Val Phe Lys Ser Leu Ser Ile Asp Phe Leu Asp Ser Val Thr Leu Ala
           195                 200                 205
   tac ctc act ctg agc gaa aac gac tcc gaa ggt aga gac aaa ctg ctg      672
   Tyr Leu Thr Leu Ser Glu Asn Asp Ser Glu Gly Arg Asp Lys Leu Leu
       210                 215                 220
   gaa aag att cct gcc ctc aaa gcg gac ttc aaa gtc ccg act tta ctc      720
   Glu Lys Ile Pro Ala Leu Lys Ala Asp Phe Lys Val Pro Thr Leu Leu
   225                 230                 235                 240
   gcc atc gac aag gga acg aaa aat gtg acg gtt tat gat tcc gaa tcg      768
   Ala Ile Asp Lys Gly Thr Lys Asn Val Thr Val Tyr Asp Ser Glu Ser
                   245                 250                 255
   atg tcg aaa aaa gag ctg acg aag ttc atg tct aag ttc ggc cag cca      816
   Met Ser Lys Lys Glu Leu Thr Lys Phe Met Ser Lys Phe Gly Gln Pro
               260                 265                 270
   caa gag ggg gca atg agc gaa aga ggg ggc atc ttg aaa gga atc aag      864
   Gln Glu Gly Ala Met Ser Glu Arg Gly Gly Ile Leu Lys Gly Ile Lys
           275                 280                 285
   aag ggt gct tac aag agc ttc aaa gat tac aaa aag aag atg caa caa      912
   Lys Gly Ala Tyr Lys Ser Phe Lys Asp Tyr Lys Lys Lys Met Gln Gln
       290                 295                 300
   gct ctt gaa aaa gat gag cta tga                                     936
   Ala Leu Glu Lys Asp Glu Leu
   305                 310
<210> 38
   <211> 311
   <212> PRT
   <213> Ogataea minuta
<400> 38
   Met Lys Val Ala Ser Leu Ile Ala Leu Val Val Thr Pro Ile Ile Ala
   1               5                   10                  15
   Ala Thr Gly Val Val Ala Asp Pro Gln Gln Gln Ala Lys Arg Pro Gly
               20                  25                  30
   Phe Tyr Lys Asn Ser Lys His Ile Tyr Asn Leu Thr Pro Gln Asn Phe
           35                  40                  45
   Asp Asp Val Val Leu Gln Thr Asn His Thr Ser Val Val Glu Phe Tyr
   50 55 60
   Ala Pro Trp Cys Gly Tyr Cys Ala Glu Phe Glu Ser Gln Tyr Arg Lys
   65 70 75 80
   Ala Ala Lys Ile Gly Ser Glu Phe Val Asn Phe Ala Ala Val Asn Cys
   85 90 95
   Asp Glu Asp Lys Asn Lys Pro Leu Cys Asn Lys Tyr Arg Val Glu Gly
   100 105 110
   Phe Pro Thr Val Met Val Phe Arg Pro Ala Lys Val Asn Ser Ala Gly
   115 120 125
   Ser Asn Gly Asn Arg Pro His Ser Ser Glu Thr Tyr Arg Gly Glu Arg
   130 135 140
   Thr Ala Ala Lys Leu Leu Glu His Val Lys Gly Arg Val Val Asn Tyr
   145 150 155 160
   Val Lys Arg Ile Lys Leu Asn Lys Leu Asp Glu Phe Leu Lys Pro Asn
   165 170 175
   Glu Lys Ser Arg Val Leu Leu Val Thr Ser Lys Ser Thr Leu Ser Pro
   180 185 190
   Val Phe Lys Ser Leu Ser Ile Asp Phe Leu Asp Ser Val Thr Leu Ala
   195 200 205
   Tyr Leu Thr Leu Ser Glu Asn Asp Ser Glu Gly Arg Asp Lys Leu Leu
   210 215 220
   Glu Lys Ile Pro Ala Leu Lys Ala Asp Phe Lys Val Pro Thr Leu Leu
   225 230 235 240
   Ala Ile Asp Lys Gly Thr Lys Asn Val Thr Val Tyr Asp Ser Glu Ser
   245 250 255
   Met Ser Lys Lys Glu Leu Thr Lys Phe Met Ser Lys Phe Gly Gln Pro
   260 265 270
   Gln Glu Gly Ala Met Ser Glu Arg Gly Gly Ile Leu Lys Gly Ile Lys
   275 280 285
   Lys Gly Ala Tyr Lys Ser Phe Lys Asp Tyr Lys Lys Lys Met Gln Gln
   290 295 300
   Ala Leu Glu Lys Asp Glu Leu
   305 310
<210> 39
   <211> 930
   <212> DNA
   <213> Ogataea minuta
<220>
   <221> CDS
   <222> (1)..(930)
<400> 39
   atg ttt atg gag atc gga gag gcg tac gag gtg ctg atg gat tca gaa 48
   Met Phe Met Glu Ile Gly Glu Ala Tyr Glu Val Leu Met Asp Ser Glu
   1 5 10 15
   aag aga tcc ata tat gac agg tac gga gag gaa ggc ttg aaa ggc ggt 96
   Lys Arg Ser Ile Tyr Asp Arg Tyr Gly Glu Glu Gly Leu Lys Gly Gly
   20 25 30
   gca ggg ggc gga gga gga gga caa cac cac gat ccg ttc gac atg ttt 144
   Ala Gly Gly Gly Gly Gly Gly Gln His His Asp Pro Phe Asp Met Phe
   35 40 45
   gcc aac ttt ttc ggc ggc gcc ggt ggg cgt caa caa gca cgg gga gtt 192
   Ala Asn Phe Phe Gly Gly Ala Gly Gly Arg Gln Gln Ala Arg Gly Val
   50 55 60
   cca aga ggg tcg gac att gcc acg gag atg gag ttt acc ttg aaa gag 240
   Pro Arg Gly Ser Asp Ile Ala Thr Glu Met Glu Phe Thr Leu Lys Glu
   65 70 75 80
   ttt tac aac gga gtg aat agc gac ttt tca ctc gaa ctg caa gac atc 288
   Phe Tyr Asn Gly Val Asn Ser Asp Phe Ser Leu Glu Leu Gln Asp Ile
   85 90 95
   tgt gac cgt tgt gac gga agc ggg tcg cag gac ggg aaa gtg cac aag 336
   Cys Asp Arg Cys Asp Gly Ser Gly Ser Gln Asp Gly Lys Val His Lys
   100 105 110
   tgt tct cga tgc aat ggt cgt ggc cgg gtg tta gtg aag aga cag ttg 384
   Cys Ser Arg Cys Asn Gly Arg Gly Arg Val Leu Val Lys Arg Gln Leu
   115 120 125
   ggt cct ggc atg ttc cag cag atg gag tcg gcg tgt ccc gac tgt cgt 432
   Gly Pro Gly Met Phe Gln Gln Met Glu Ser Ala Cys Pro Asp Cys Arg
   130 135 140
   gga gca gga aaa cag att act cac cat tgc aag aag tgt cgg ggt ggc 480
   Gly Ala Gly Lys Gln Ile Thr His His Cys Lys Lys Cys Arg Gly Gly
   145 150 155 160
   ggg gtt gtc cgt gga att cgc aac ttc aac atc cac ctt gag cca gga 528
   Gly Val Val Arg Gly Ile Arg Asn Phe Asn Ile His Leu Glu Pro Gly
   165 170 175
   act ccg cgc gac cac gtc gaa gtg tac gag ggt cag tcc gac agg tct 576
   Thr Pro Arg Asp His Val Glu Val Tyr Glu Gly Gln Ser Asp Arg Ser
   180 185 190
   ccg gag tgg gag gct ggt aac tta cgt ctg agc gtc aga gag aag aaa      624
   Pro Glu Trp Glu Ala Gly Asn Leu Arg Leu Ser Val Arg Glu Lys Lys
           195                 200                 205
   agc gga aac ctt ggg tat cgt cgg atc gga aac aat ctg tac cgc aca      672
   Ser Gly Asn Leu Gly Tyr Arg Arg Ile Gly Asn Asn Leu Tyr Arg Thr
       210                 215                 220
   gag atc ttg acg ctg agc gag tct ctg aag ggt ggt tgg gtc cgc gag      720
   Glu Ile Leu Thr Leu Ser Glu Ser Leu Lys Gly Gly Trp Val Arg Glu
   225                 230                 235                 240
   atc ccg ttt ctg gac aac tac gac gcc gtc tta aag ctg gaa aga cca      768
   Ile Pro Phe Leu Asp Asn Tyr Asp Ala Val Leu Lys Leu Glu Arg Pro
                   245                 250                 255
   ctc gga agt gtt gtt acc agc ggg gaa gtg cag gtg gtg aaa gga aag      816
   Leu Gly Ser Val Val Thr Ser Gly Glu Val Gln Val Val Lys Gly Lys
               260                 265                 270
   ggt atg ccg att gcc aac tcc gtg gat cag ttt ggc gat ctg tat gtg      864
   Gly Met Pro Ile Ala Asn Ser Val Asp Gln Phe Gly Asp Leu Tyr Val
           275                 280                 285
   gag tat gtg gtg ttg tat ccg gga gga agc ccg aag aag gtg agc aag      912
   Glu Tyr Val Val Leu Tyr Pro Gly Gly Ser Pro Lys Lys Val Ser Lys
       290                 295                 300
   ttg cac gac gag ctg tga                                              930
   Leu His Asp Glu Leu
   305
<210> 40
   <211> 309
   <212> PRT
   <213> Ogataea minuta
<400> 40
   Met Phe Met Glu Ile Gly Glu Ala Tyr Glu Val Leu Met Asp Ser Glu
   1               5                   10                  15
   Lys Arg Ser Ile Tyr Asp Arg Tyr Gly Glu Glu Gly Leu Lys Gly Gly
               20                  25                  30
   Ala Gly Gly Gly Gly Gly Gly Gln His His Asp Pro Phe Asp Met Phe
           35                  40                  45
   Ala Asn Phe Phe Gly Gly Ala Gly Gly Arg Gln Gln Ala Arg Gly Val
       50                  55                  60
   Pro Arg Gly Ser Asp Ile Ala Thr Glu Met Glu Phe Thr Leu Lys Glu
   65                  70                  75                  80
   Phe Tyr Asn Gly Val Asn Ser Asp Phe Ser Leu Glu Leu Gln Asp Ile
                   85                  90                  95
   Cys Asp Arg Cys Asp Gly Ser Gly Ser Gln Asp Gly Lys Val His Lys
               100                 105                 110
   Cys Ser Arg Cys Asn Gly Arg Gly Arg Val Leu Val Lys Arg Gln Leu
           115                 120                 125
   Gly Pro Gly Met Phe Gln Gln Met Glu Ser Ala Cys Pro Asp Cys Arg
       130                 135                 140
   Gly Ala Gly Lys Gln Ile Thr His His Cys Lys Lys Cys Arg Gly Gly
   145                 150                 155                 160
   Gly Val Val Arg Gly Ile Arg Asn Phe Asn Ile His Leu Glu Pro Gly
                   165                 170                 175
   Thr Pro Arg Asp His Val Glu Val Tyr Glu Gly Gln Ser Asp Arg Ser
               180                 185                 190
   Pro Glu Trp Glu Ala Gly Asn Leu Arg Leu Ser Val Arg Glu Lys Lys
           195                 200                 205
   Ser Gly Asn Leu Gly Tyr Arg Arg Ile Gly Asn Asn Leu Tyr Arg Thr
       210                 215                 220
   Glu Ile Leu Thr Leu Ser Glu Ser Leu Lys Gly Gly Trp Val Arg Glu
   225                 230                 235                 240
   Ile Pro Phe Leu Asp Asn Tyr Asp Ala Val Leu Lys Leu Glu Arg Pro
                   245                 250                 255
   Leu Gly Ser Val Val Thr Ser Gly Glu Val Gln Val Val Lys Gly Lys
               260                 265                 270
   Gly Met Pro Ile Ala Asn Ser Val Asp Gln Phe Gly Asp Leu Tyr Val
           275                 280                 285
   Glu Tyr Val Val Leu Tyr Pro Gly Gly Ser Pro Lys Lys Val Ser Lys
       290                 295                 300
   Leu His Asp Glu Leu
   305
<210> 41
   <211> 1137
   <212> DNA
   <213> Ogataea minuta
<220>
   <221> CDS
   <222> (1)..(1137)
<400> 41
   atg aaa gtc acg tct atc tgg caa gtc ctg ttt atg ttg atc gcc ggc      48
   Met Lys Val Thr Ser Ile Trp Gln Val Leu Phe Met Leu Ile Ala Gly
   1               5                   10                  15
   gtt atg gcg tca gcg tcc aaa gtg atg gag ctg aac gac aag aac ttt      96
   Val Met Ala Ser Ala Ser Lys Val Met Glu Leu Asn Asp Lys Asn Phe
               20                  25                  30
   gac gag gtg gtt ctc aac tcc gga aag acc tcg cta gtg gaa ttc tac      144
   Asp Glu Val Val Leu Asn Ser Gly Lys Thr Ser Leu Val Glu Phe Tyr
           35                  40                  45
   gcg tcg tgg tgc agt cac tgc aag aag ttg gag cct act tgg gaa gag      192
   Ala Ser Trp Cys Ser His Cys Lys Lys Leu Glu Pro Thr Trp Glu Glu
       50                  55                  60
   ctg gcc tcg gcg tac gga aac aag aac gat atc cag atc gtc aag atc      240
   Leu Ala Ser Ala Tyr Gly Asn Lys Asn Asp Ile Gln Ile Val Lys Ile
   65                  70                  75                  80
   gac gct gac gaa aac gga aac gtg gga aga aaa ttc gga atc aag gga      288
   Asp Ala Asp Glu Asn Gly Asn Val Gly Arg Lys Phe Gly Ile Lys Gly
                   85                  90                  95
   ttt ccc acg ctg aaa ctg ttc aaa aaa gat gat ctc aac aac cca gtg      336
   Phe Pro Thr Leu Lys Leu Phe Lys Lys Asp Asp Leu Asn Asn Pro Val
               100                 105                 110
   gaa ttt gaa ggc tcc agg gac ttc cat tct ttc acc aac ttc att gct      384
   Glu Phe Glu Gly Ser Arg Asp Phe His Ser Phe Thr Asn Phe Ile Ala
           115                 120                 125
   gca cac acg ggt atc aag gct gcc aac gcg gtt ccc act gag ccg tcc      432
   Ala His Thr Gly Ile Lys Ala Ala Asn Ala Val Pro Thr Glu Pro Ser
       130                 135                 140
   aaa gtg gtg gaa ctg cac gat gga aat ttg gag gag ctt gtt aag gag      480
   Lys Val Val Glu Leu His Asp Gly Asn Leu Glu Glu Leu Val Lys Glu
   145                 150                 155                 160
   cag gga aaa aat gct ctt ttt gca atc acc gca gag tgg tgt ggt tac      528
   Gln Gly Lys Asn Ala Leu Phe Ala Ile Thr Ala Glu Trp Cys Gly Tyr
                   165                 170                 175
   tgc aag aag ctc aag cct aca tgg gag cag ctg gct gcc gtt ttc caa      576
   Cys Lys Lys Leu Lys Pro Thr Trp Glu Gln Leu Ala Ala Val Phe Gln
               180                 185                 190
   ggc gac gag gaa aac atc ttg att gga cag gtc caa acc acc ggc gat      624
   Gly Asp Glu Glu Asn Ile Leu Ile Gly Gln Val Gln Thr Thr Gly Asp
           195                 200                 205
   aac cca aca gaa tgg atc cag gag aaa tac aac ctc cag tcg ttc ccc      672
   Asn Pro Thr Glu Trp Ile Gln Glu Lys Tyr Asn Leu Gln Ser Phe Pro
       210                 215                 220
   aca ata gtc ttc atc gag aag ggc aac ctg gac gag cct gtg ttc tat      720
   Thr Ile Val Phe Ile Glu Lys Gly Asn Leu Asp Glu Pro Val Phe Tyr
   225                 230                 235                 240
   ccg tac gga aga gag ctg gga gac ctg gtt gag ttc gtt aac acc cag      768
   Pro Tyr Gly Arg Glu Leu Gly Asp Leu Val Glu Phe Val Asn Thr Gln
                   245                 250                 255
   gcc gga act cac cgg aac gag aaa ggc gag ctg gac tcc gag gcc ggg      816
   Ala Gly Thr His Arg Asn Glu Lys Gly Glu Leu Asp Ser Glu Ala Gly
               260                 265                 270
   ctg ata cac gca gtc gac gag ctg gtt gag cag ttt gtc ggt tcc tcg      864
   Leu Ile His Ala Val Asp Glu Leu Val Glu Gln Phe Val Gly Ser Ser
           275                 280                 285
   agc agc ggc aga aaa aat ctg gtt ccg aaa ttc ttg gaa gct ttg aaa      912
   Ser Ser Gly Arg Lys Asn Leu Val Pro Lys Phe Leu Glu Ala Leu Lys
       290                 295                 300
   tcg gct gac acc gac aat gca ttg tcg aaa gaa gtg aaa tac tac aac      960
   Ser Ala Asp Thr Asp Asn Ala Leu Ser Lys Glu Val Lys Tyr Tyr Asn
   305                 310                 315                 320
   aag atc atc cat acg atg gtc aac ggt ccc ttt gac ttc gtc gcg aaa     1008
   Lys Ile Ile His Thr Met Val Asn Gly Pro Phe Asp Phe Val Ala Lys
                   325                 330                 335
   gaa acc gct aga ctg gag tcg cta ctg aag tcg gat ctg tct tcc cga     1056
   Glu Thr Ala Arg Leu Glu Ser Leu Leu Lys Ser Asp Leu Ser Ser Arg
               340                 345                 350
   gcc aga gac tca gct tcc ttt aga ctc aac atc ctc aag ttt ttc agc     1104
   Ala Arg Asp Ser Ala Ser Phe Arg Leu Asn Ile Leu Lys Phe Phe Ser
           355                 360                 365
   gat cct gcc cct cca gcc aag gat gag ctg tga                         1137
   Asp Pro Ala Pro Pro Ala Lys Asp Glu Leu
       370                 375
<210> 42
   <211> 378
   <212> PRT
   <213> Ogataea minuta
<400> 42
   Met Lys Val Thr Ser Ile Trp Gln Val Leu Phe Met Leu Ile Ala Gly
   1               5                   10                  15
   Val Met Ala Ser Ala Ser Lys Val Met Glu Leu Asn Asp Lys Asn Phe
               20                  25                  30
   Asp Glu Val Val Leu Asn Ser Gly Lys Thr Ser Leu Val Glu Phe Tyr
           35                  40                  45
   Ala Ser Trp Cys Ser His Cys Lys Lys Leu Glu Pro Thr Trp Glu Glu
       50                  55                  60
   Leu Ala Ser Ala Tyr Gly Asn Lys Asn Asp Ile Gln Ile Val Lys Ile
   65                  70                  75                  80
   Asp Ala Asp Glu Asn Gly Asn Val Gly Arg Lys Phe Gly Ile Lys Gly
                   85                  90                  95
   Phe Pro Thr Leu Lys Leu Phe Lys Lys Asp Asp Leu Asn Asn Pro Val
               100                 105                 110
   Glu Phe Glu Gly Ser Arg Asp Phe His Ser Phe Thr Asn Phe Ile Ala
           115                 120                 125
   Ala His Thr Gly Ile Lys Ala Ala Asn Ala Val Pro Thr Glu Pro Ser
       130                 135                 140
   Lys Val Val Glu Leu His Asp Gly Asn Leu Glu Glu Leu Val Lys Glu
   145                 150                 155                 160
   Gln Gly Lys Asn Ala Leu Phe Ala Ile Thr Ala Glu Trp Cys Gly Tyr
                   165                 170                 175
   Cys Lys Lys Leu Lys Pro Thr Trp Glu Gln Leu Ala Ala Val Phe Gln
               180                 185                 190
   Gly Asp Glu Glu Asn Ile Leu Ile Gly Gln Val Gln Thr Thr Gly Asp
           195                 200                 205
   Asn Pro Thr Glu Trp Ile Gln Glu Lys Tyr Asn Leu Gln Ser Phe Pro
       210                 215                 220
   Thr Ile Val Phe Ile Glu Lys Gly Asn Leu Asp Glu Pro Val Phe Tyr
   225                 230                 235                 240
   Pro Tyr Gly Arg Glu Leu Gly Asp Leu Val Glu Phe Val Asn Thr Gln
                   245                 250                 255
   Ala Gly Thr His Arg Asn Glu Lys Gly Glu Leu Asp Ser Glu Ala Gly
               260                 265                 270
   Leu Ile His Ala Val Asp Glu Leu Val Glu Gln Phe Val Gly Ser Ser
           275                 280                 285
   Ser Ser Gly Arg Lys Asn Leu Val Pro Lys Phe Leu Glu Ala Leu Lys
       290                 295                 300
   Ser Ala Asp Thr Asp Asn Ala Leu Ser Lys Glu Val Lys Tyr Tyr Asn
   305                 310                 315                 320
   Lys Ile Ile His Thr Met Val Asn Gly Pro Phe Asp Phe Val Ala Lys
   325 330 335
   Glu Thr Ala Arg Leu Glu Ser Leu Leu Lys Ser Asp Leu Ser Ser Arg
   340 345 350
   Ala Arg Asp Ser Ala Ser Phe Arg Leu Asn Ile Leu Lys Phe Phe Ser
   355 360 365
   Asp Pro Ala Pro Pro Ala Lys Asp Glu Leu
   370 375
<210> 43
   <211> 1728
   <212> DNA
   <213> Ogataea minuta
<220>
   <221> CDS
   <222> (1)..(1728)
<400> 43
   atg aag cac gtg ata agt ggc cga tcc cgg gtg gcg ttg ggc tgg att       48
   Met Lys His Val Ile Ser Gly Arg Ser Arg Val Ala Leu Gly Trp Ile
   1               5                   10                  15
   ctg acg tgg ttg atc tgt gcg att tgc gct gtt ccc gga gct ggg ttg       96
   Leu Thr Trp Leu Ile Cys Ala Ile Cys Ala Val Pro Gly Ala Gly Leu
               20                  25                  30
   cag gag att tcg tcc ttg gag agg aaa ccg gct tac ctg tcg ccg cag      144
   Gln Glu Ile Ser Ser Leu Glu Arg Lys Pro Ala Tyr Leu Ser Pro Gln
           35                  40                  45
   tat gag tac gac aac att cac gag ttt gag tcg acg cct ttc cgc gat      192
   Tyr Glu Tyr Asp Asn Ile His Glu Phe Glu Ser Thr Pro Phe Arg Asp
       50                  55                  60
   tac gag acg ttc acg ggg tcc aag gtg agt gag agt tcg aac gtg act      240
   Tyr Glu Thr Phe Thr Gly Ser Lys Val Ser Glu Ser Ser Asn Val Thr
   65                  70                  75                  80
   ttt ggc cag atc aac gcg ctc aac aac gaa atc aga ccg gtt ttg cac      288
   Phe Gly Gln Ile Asn Ala Leu Asn Asn Glu Ile Arg Pro Val Leu His
                   85                  90                  95
   gat ctg att aac gag aac ttc ttc aaa atc ttt cga ctc aac ctg tac      336
   Asp Leu Ile Asn Glu Asn Phe Phe Lys Ile Phe Arg Leu Asn Leu Tyr
               100                 105                 110
   aag gag tgt ccg ttc tgg tcg agt tcg gag gga ttt tgc atg cac aag      384
   Lys Glu Cys Pro Phe Trp Ser Ser Ser Glu Gly Phe Cys Met His Lys
           115                 120                 125
   agc tgt gcc gtg gac acc att gac gac tgg aaa gat ctt ccg gag ata      432
   Ser Cys Ala Val Asp Thr Ile Asp Asp Trp Lys Asp Leu Pro Glu Ile
       130                 135                 140
   tgg cag ccc gag gct ctg ggt cgg atc gag tcg ttg acg cga gaa ccg      480
   Trp Gln Pro Glu Ala Leu Gly Arg Ile Glu Ser Leu Thr Arg Glu Pro
   145                 150                 155                 160
   cct acg acg ata tct gac gcg gga aat ggc tcg tgt gtc gct gca ggc      528
   Pro Thr Thr Ile Ser Asp Ala Gly Asn Gly Ser Cys Val Ala Ala Gly
                   165                 170                 175
   gga cgg agc acg cgg gat tac tgc gaa ctg gac gag gtc aac gag gac      576
   Gly Arg Ser Thr Arg Asp Tyr Cys Glu Leu Asp Glu Val Asn Glu Asp
               180                 185                 190
   tcg gta tac gtg aat ctg gtg gac aat ccc gag aga ttc acg ggt tac      624
   Ser Val Tyr Val Asn Leu Val Asp Asn Pro Glu Arg Phe Thr Gly Tyr
           195                 200                 205
   gga gga gat cag tcg ttc caa att tgg cgc agc att tac aac gag aac      672
   Gly Gly Asp Gln Ser Phe Gln Ile Trp Arg Ser Ile Tyr Asn Glu Asn
       210                 215                 220
   tgt ttc aat ttg ggc tcg gat caa tgt ctc gag aag aac ttt ttc tac      720
   Cys Phe Asn Leu Gly Ser Asp Gln Cys Leu Glu Lys Asn Phe Phe Tyr
   225                 230                 235                 240
   aag ttg atc agt gga atg cac tcg tcg atc tcg act cat ctg acc aac      768
   Lys Leu Ile Ser Gly Met His Ser Ser Ile Ser Thr His Leu Thr Asn
                   245                 250                 255
   gag tac ctg aac ttc aag acc aag cag tat gga cag gat ctc aag cag      816
   Glu Tyr Leu Asn Phe Lys Thr Lys Gln Tyr Gly Gln Asp Leu Lys Gln
               260                 265                 270
   ttc atg atc cgg gtg ggg gac ttc cct gac cgg ttc gag aac ttg tat      864
   Phe Met Ile Arg Val Gly Asp Phe Pro Asp Arg Phe Glu Asn Leu Tyr
           275                 280                 285
   ctg aac tac gtt ctg gtg gtg aag tcc ttg atc aag ctg gag cag tcg      912
   Leu Asn Tyr Val Leu Val Val Lys Ser Leu Ile Lys Leu Glu Gln Ser
       290                 295                 300
   ggt gtg ctg gac aac ctc cag ttc tgt gac gag gag gtg ttt cag acg      960
   Gly Val Leu Asp Asn Leu Gln Phe Cys Asp Glu Glu Val Phe Gln Thr
   305                 310                 315                 320
   aag gag aag gag cta aaa cgc gag ctg aag gag atg att tct ccg ttc     1008
   Lys Glu Lys Glu Leu Lys Arg Glu Leu Lys Glu Met Ile Ser Pro Phe
                   325                 330                 335
   tac cag ctg gcg gaa ggt ggg aaa gtg gac gag tgt ctg ttc aac gag     1056
   Tyr Gln Leu Ala Glu Gly Gly Lys Val Asp Glu Cys Leu Phe Asn Glu
               340                 345                 350
   cac tcg ctg ttc cag agc gag gac tcg acg tat ctg aag gac gag ttc     1104
   His Ser Leu Phe Gln Ser Glu Asp Ser Thr Tyr Leu Lys Asp Glu Phe
           355                 360                 365
   agt gag aac ttc agg aac gtg tcg cgg atc atg gat tgt gtc cac tgc     1152
   Ser Glu Asn Phe Arg Asn Val Ser Arg Ile Met Asp Cys Val His Cys
       370                 375                 380
   gac agg tgc aga ctg tgg ggg aag gtg cag acc acc ggg tac gga act     1200
   Asp Arg Cys Arg Leu Trp Gly Lys Val Gln Thr Thr Gly Tyr Gly Thr
   385                 390                 395                 400
   gcg ctg aag att ttg ttt gag ctg gat gca agc gac agc cac gag ctg     1248
   Ala Leu Lys Ile Leu Phe Glu Leu Asp Ala Ser Asp Ser His Glu Leu
                   405                 410                 415
   ggc aag aat ttc cag atc tcc aaa atc gag ctg gtc gcc ctg atc aac     1296
   Gly Lys Asn Phe Gln Ile Ser Lys Ile Glu Leu Val Ala Leu Ile Asn
               420                 425                 430
   acg ttt gac agg ctg tcc aag agc gtg cac gcc atc gga aac ttc aaa     1344
   Thr Phe Asp Arg Leu Ser Lys Ser Val His Ala Ile Gly Asn Phe Lys
           435                 440                 445
   caa ctg tac gat ctg aga atg aaa cag gag gaa gaa ggg ggg tcc atg     1392
   Gln Leu Tyr Asp Leu Arg Met Lys Gln Glu Glu Glu Gly Gly Ser Met
       450                 455                 460
   att act gca gac acg ttt gac ttg gag caa ttg ctg ttg aca gac cag     1440
   Ile Thr Ala Asp Thr Phe Asp Leu Glu Gln Leu Leu Leu Thr Asp Gln
   465                 470                 475                 480
   acg gtg gac gta ttc ggc cag agc act tct gag cca gaa acc ccg tcg     1488
   Thr Val Asp Val Phe Gly Gln Ser Thr Ser Glu Pro Glu Thr Pro Ser
                   485                 490                 495
   gac gtt aga tat ccc gac aga aca cgg ggc tcg ctc gtt ccc gag ggg     1536
   Asp Val Arg Tyr Pro Asp Arg Thr Arg Gly Ser Leu Val Pro Glu Gly
               500                 505                 510
   ctc ggc gag gcg ttc aag aca gag ctg tac agc gtt tat cag gcg ttc     1584
   Leu Gly Glu Ala Phe Lys Thr Glu Leu Tyr Ser Val Tyr Gln Ala Phe
           515                 520                 525
   tac ttt gtc gtg acc agc tac acc atg ttc ccc aag ctg atc tac aac     1632
   Tyr Phe Val Val Thr Ser Tyr Thr Met Phe Pro Lys Leu Ile Tyr Asn
       530                 535                 540
   tac ctg ctg atc cgg gtg gtg tac tgg tgg aac atc ttt gtg ggt cat     1680
   Tyr Leu Leu Ile Arg Val Val Tyr Trp Trp Asn Ile Phe Val Gly His
   545                 550                 555                 560
   gta cac gag gac ttt gac gtg gat cgc ctg tat cgt ttg gag cta taa     1728
   Val His Glu Asp Phe Asp Val Asp Arg Leu Tyr Arg Leu Glu Leu
                   565                 570                 575
<210> 44
   <211> 575
   <212> PRT
   <213> Ogataea minuta
<400> 44
   Met Lys His Val Ile Ser Gly Arg Ser Arg Val Ala Leu Gly Trp Ile
   1               5                   10                  15
   Leu Thr Trp Leu Ile Cys Ala Ile Cys Ala Val Pro Gly Ala Gly Leu
               20                  25                  30
   Gln Glu Ile Ser Ser Leu Glu Arg Lys Pro Ala Tyr Leu Ser Pro Gln
           35                  40                  45
   Tyr Glu Tyr Asp Asn Ile His Glu Phe Glu Ser Thr Pro Phe Arg Asp
       50                  55                  60
   Tyr Glu Thr Phe Thr Gly Ser Lys Val Ser Glu Ser Ser Asn Val Thr
   65                  70                  75                  80
   Phe Gly Gln Ile Asn Ala Leu Asn Asn Glu Ile Arg Pro Val Leu His
                   85                  90                  95
   Asp Leu Ile Asn Glu Asn Phe Phe Lys Ile Phe Arg Leu Asn Leu Tyr
               100                 105                 110
   Lys Glu Cys Pro Phe Trp Ser Ser Ser Glu Gly Phe Cys Met His Lys
           115                 120                 125
   Ser Cys Ala Val Asp Thr Ile Asp Asp Trp Lys Asp Leu Pro Glu Ile
       130                 135                 140
   Trp Gln Pro Glu Ala Leu Gly Arg Ile Glu Ser Leu Thr Arg Glu Pro
   145                 150                 155                 160
   Pro Thr Thr Ile Ser Asp Ala Gly Asn Gly Ser Cys Val Ala Ala Gly
                   165                 170                 175
   Gly Arg Ser Thr Arg Asp Tyr Cys Glu Leu Asp Glu Val Asn Glu Asp
               180                 185                 190
   Ser Val Tyr Val Asn Leu Val Asp Asn Pro Glu Arg Phe Thr Gly Tyr
           195                 200                 205
   Gly Gly Asp Gln Ser Phe Gln Ile Trp Arg Ser Ile Tyr Asn Glu Asn
       210                 215                 220
   Cys Phe Asn Leu Gly Ser Asp Gln Cys Leu Glu Lys Asn Phe Phe Tyr
   225                 230                 235                 240
   Lys Leu Ile Ser Gly Met His Ser Ser Ile Ser Thr His Leu Thr Asn
                   245                 250                 255
   Glu Tyr Leu Asn Phe Lys Thr Lys Gln Tyr Gly Gln Asp Leu Lys Gln
               260                 265                 270
   Phe Met Ile Arg Val Gly Asp Phe Pro Asp Arg Phe Glu Asn Leu Tyr
           275                 280                 285
   Leu Asn Tyr Val Leu Val Val Lys Ser Leu Ile Lys Leu Glu Gln Ser
       290                 295                 300
   Gly Val Leu Asp Asn Leu Gln Phe Cys Asp Glu Glu Val Phe Gln Thr
   305                 310                 315                 320
   Lys Glu Lys Glu Leu Lys Arg Glu Leu Lys Glu Met Ile Ser Pro Phe
                   325                 330                 335
   Tyr Gln Leu Ala Glu Gly Gly Lys Val Asp Glu Cys Leu Phe Asn Glu
               340                 345                 350
   His Ser Leu Phe Gln Ser Glu Asp Ser Thr Tyr Leu Lys Asp Glu Phe
           355                 360                 365
   Ser Glu Asn Phe Arg Asn Val Ser Arg Ile Met Asp Cys Val His Cys
       370                 375                 380
   Asp Arg Cys Arg Leu Trp Gly Lys Val Gln Thr Thr Gly Tyr Gly Thr
   385                 390                 395                 400
   Ala Leu Lys Ile Leu Phe Glu Leu Asp Ala Ser Asp Ser His Glu Leu
                   405                 410                 415
   Gly Lys Asn Phe Gln Ile Ser Lys Ile Glu Leu Val Ala Leu Ile Asn
               420                 425                 430
   Thr Phe Asp Arg Leu Ser Lys Ser Val His Ala Ile Gly Asn Phe Lys
           435                 440                 445
   Gln Leu Tyr Asp Leu Arg Met Lys Gln Glu Glu Glu Gly Gly Ser Met
       450                 455                 460
   Ile Thr Ala Asp Thr Phe Asp Leu Glu Gln Leu Leu Leu Thr Asp Gln
   465                 470                 475                 480
   Thr Val Asp Val Phe Gly Gln Ser Thr Ser Glu Pro Glu Thr Pro Ser
                   485                 490                 495
   Asp Val Arg Tyr Pro Asp Arg Thr Arg Gly Ser Leu Val Pro Glu Gly
               500                 505                 510
   Leu Gly Glu Ala Phe Lys Thr Glu Leu Tyr Ser Val Tyr Gln Ala Phe
           515                 520                 525
   Tyr Phe Val Val Thr Ser Tyr Thr Met Phe Pro Lys Leu Ile Tyr Asn
       530                 535                 540
   Tyr Leu Leu Ile Arg Val Val Tyr Trp Trp Asn Ile Phe Val Gly His
   545 550 555 560
   Val His Glu Asp Phe Asp Val Asp Arg Leu Tyr Arg Leu Glu Leu
   565 570 575
<210> 45
   <211> 2700
   <212> DNA
   <213> Ogataea minuta
<220>
   <221> CDS
   <222> (1)..(2700)
<400> 45
   atg gat tct acg caa ttt aca gac aga gct cta gac ata gtg acg acg      48
   Met Asp Ser Thr Gln Phe Thr Asp Arg Ala Leu Asp Ile Val Thr Thr
   1               5                   10                  15
   gct cag aag cta tgt cag cag aac tca aac gca cag ata gtg cca ttg      96
   Ala Gln Lys Leu Cys Gln Gln Asn Ser Asn Ala Gln Ile Val Pro Leu
               20                  25                  30
   cac ttt ctg gcg gca atg act ccg acc tcc agt gag ggg gag gca atc     144
   His Phe Leu Ala Ala Met Thr Pro Thr Ser Ser Glu Gly Glu Ala Ile
           35                  40                  45
   tat ctc aag acg ttg att gag aga ggt cga ttt gac tgg act gct ttt     192
   Tyr Leu Lys Thr Leu Ile Glu Arg Gly Arg Phe Asp Trp Thr Ala Phe
       50                  55                  60
   gaa aga gcc gtt aat aaa gca gtg gtg cgg ctc cca agc gtg gcc ggt     240
   Glu Arg Ala Val Asn Lys Ala Val Val Arg Leu Pro Ser Val Ala Gly
   65                  70                  75                  80
   tcc aac acc gag cca agc att tcc gcc tcc gcc gcc agc att atc acc     288
   Ser Asn Thr Glu Pro Ser Ile Ser Ala Ser Ala Ala Ser Ile Ile Thr
                   85                  90                  95
   aac gca tcc aag atc aag gca cag cag aaa gac tcg tac atc ggc caa     336
   Asn Ala Ser Lys Ile Lys Ala Gln Gln Lys Asp Ser Tyr Ile Gly Gln
               100                 105                 110
   gac cac att ctc tcg gcc ctt ttg gac gat tct agt atc cag gct gtg     384
   Asp His Ile Leu Ser Ala Leu Leu Asp Asp Ser Ser Ile Gln Ala Val
           115                 120                 125
   ctg aag gaa gcg aac gtc aag ccc gac gca ttg aag aca cag atc gtt     432
   Leu Lys Glu Ala Asn Val Lys Pro Asp Ala Leu Lys Thr Gln Ile Val
       130                 135                 140
   gaa ctc aga ggc aat cag aga att gat tct cgt caa gct gat tcg tct     480
   Glu Leu Arg Gly Asn Gln Arg Ile Asp Ser Arg Gln Ala Asp Ser Ser
   145                 150                 155                 160
   cag aag ttt gag ttt ctg tcc aag tac gcc ctt gat ctt acc gag cag     528
   Gln Lys Phe Glu Phe Leu Ser Lys Tyr Ala Leu Asp Leu Thr Glu Gln
                   165                 170                 175
   gcg ttg cag ggt aag att gac cct gtg ata ggc aga gag gag gag atc      576
   Ala Leu Gln Gly Lys Ile Asp Pro Val Ile Gly Arg Glu Glu Glu Ile
                   180                 185                 190
   aga agg gcc att cgg gtt ctt tcg aga cgg gcc aag tcg aac ccg tgt      624
   Arg Arg Ala Ile Arg Val Leu Ser Arg Arg Ala Lys Ser Asn Pro Cys
           195                 200                 205
   ctg att gga gat cct ggt gtt ggt aag act agt att gtt gag gga gtc      672
   Leu Ile Gly Asp Pro Gly Val Gly Lys Thr Ser Ile Val Glu Gly Val
       210                 215                 220
   gca cag agg ata gtg gac aac gat gtt cct acc gtt tta cag ggg tgc      720
   Ala Gln Arg Ile Val Asp Asn Asp Val Pro Thr Val Leu Gln Gly Cys
   225                 230                 235                 240
   aag ctg tac tcg ctg gac ttg ggt gcg ttg aaa gcg gga gcc aag tac      768
   Lys Leu Tyr Ser Leu Asp Leu Gly Ala Leu Lys Ala Gly Ala Lys Tyr
                   245                 250                 255
   cag ggt gag ttc gag gaa aga ttg aag gga gtt ttg agc gac atc gaa      816
   Gln Gly Glu Phe Glu Glu Arg Leu Lys Gly Val Leu Ser Asp Ile Glu
               260                 265                 270
   agc tcg aac tcg atg atc atc ttg ttc atc gac gag atc cac atg ttg      864
   Ser Ser Asn Ser Met Ile Ile Leu Phe Ile Asp Glu Ile His Met Leu
           275                 280                 285
   atg ggc gat gga aag tcg gac gct gcc aac ttg ctg aag cct gct ctg      912
   Met Gly Asp Gly Lys Ser Asp Ala Ala Asn Leu Leu Lys Pro Ala Leu
       290                 295                 300
   gcg aga ggt cag ttc cac tgc atc ggt gcg act acc gtg acc gag tac      960
   Ala Arg Gly Gln Phe His Cys Ile Gly Ala Thr Thr Val Thr Glu Tyr
   305                 310                 315                 320
   aga aag cac atc gag aag gac ggt gct ttc gag aga aga ttt cag aga     1008
   Arg Lys His Ile Glu Lys Asp Gly Ala Phe Glu Arg Arg Phe Gln Arg
                   325                 330                 335
   atc gac gtg aga gag ccg acc atc aga gaa acc gtt gcc att ctc aga     1056
   Ile Asp Val Arg Glu Pro Thr Ile Arg Glu Thr Val Ala Ile Leu Arg
               340                 345                 350
   gga ctg cag cca agg tac gag atc cac cac ggt gtc cga atc ctg gac     1104
   Gly Leu Gln Pro Arg Tyr Glu Ile His His Gly Val Arg Ile Leu Asp
           355                 360                 365
   agt gcc ctg gtg acg gct gct caa ctg gcc tcc aga tac ctc acc tac     1152
   Ser Ala Leu Val Thr Ala Ala Gln Leu Ala Ser Arg Tyr Leu Thr Tyr
       370                 375                 380
   aga aag ctt ccg gac tct gca gtg gat ctg att gac gag tcc gcc gcg     1200
   Arg Lys Leu Pro Asp Ser Ala Val Asp Leu Ile Asp Glu Ser Ala Ala
   385                 390                 395                 400
   gga gtc gct gtt gcc agg gac tcc aag ccg gag gag ctg gac tcc aag     1248
   Gly Val Ala Val Ala Arg Asp Ser Lys Pro Glu Glu Leu Asp Ser Lys
                   405                 410                 415
   gag aga cag cta cag ctg atc gag gtt gag atc aat gct ctg gaa aga     1296
   Glu Arg Gln Leu Gln Leu Ile Glu Val Glu Ile Asn Ala Leu Glu Arg
               420                 425                 430
   gac cag gac gcc gac acg tcc acc aag gag aga ctg gag cag gcc aga     1344
   Asp Gln Asp Ala Asp Thr Ser Thr Lys Glu Arg Leu Glu Gln Ala Arg
           435                 440                 445
   cag aga aga cag aac ctg gag gaa gag ctg gct cca ttg agg gag aag     1392
   Gln Arg Arg Gln Asn Leu Glu Glu Glu Leu Ala Pro Leu Arg Glu Lys
       450                 455                 460
   tac cag cag gaa aga gcg ggc cac gag gag ctg act gct gcc aag aga     1440
   Tyr Gln Gln Glu Arg Ala Gly His Glu Glu Leu Thr Ala Ala Lys Arg
   465                 470                 475                 480
   aag tta gac gac ctc gaa gtt aag gcg caa gat gcg gag aga aga cac     1488
   Lys Leu Asp Asp Leu Glu Val Lys Ala Gln Asp Ala Glu Arg Arg His
                   485                 490                 495
   gac tct cag acc att gcg gac ctg cgg atg ttt gcc att ccg gac gtg     1536
   Asp Ser Gln Thr Ile Ala Asp Leu Arg Met Phe Ala Ile Pro Asp Val
               500                 505                 510
   aaa cgc aga att gag gag ttg gaa cag aaa gtg gtt gaa gaa gag gcc     1584
   Lys Arg Arg Ile Glu Glu Leu Glu Gln Lys Val Val Glu Glu Glu Ala
           515                 520                 525
   act tct gaa gat ttc atg gtg aag aac gtt gtt ggt tcg gag caa gtt     1632
   Thr Ser Glu Asp Phe Met Val Lys Asn Val Val Gly Ser Glu Gln Val
       530                 535                 540
   tcc gag acc gcg gcc agg ttg acc ggt att ccg gtc agc aag ctg acg     1680
   Ser Glu Thr Ala Ala Arg Leu Thr Gly Ile Pro Val Ser Lys Leu Thr
   545                 550                 555                 560
   cag gca gaa aat gct aag ctg atc acc atg gag aag gag ctg tcc gct     1728
   Gln Ala Glu Asn Ala Lys Leu Ile Thr Met Glu Lys Glu Leu Ser Ala
                   565                 570                 575
   gcc gtt gtc gga cag ggc gag gct gtc aag gct gtt tcg aac tcg atc     1776
   Ala Val Val Gly Gln Gly Glu Ala Val Lys Ala Val Ser Asn Ser Ile
               580                 585                 590
   aga ttg tcg aga tct ggt ctg gcc aat cct aac cag ccg gcc tcg ttc     1824
   Arg Leu Ser Arg Ser Gly Leu Ala Asn Pro Asn Gln Pro Ala Ser Phe
           595                 600                 605
   ctg ttc ctg gga ctc tcg ggt tcc ggt aag acg gag ctg gcc aaa aag     1872
   Leu Phe Leu Gly Leu Ser Gly Ser Gly Lys Thr Glu Leu Ala Lys Lys
       610                 615                 620
   ctg gca ggg ttc ctg ttc tcg gat gag aag gcc atg atc aga atc gac     1920
   Leu Ala Gly Phe Leu Phe Ser Asp Glu Lys Ala Met Ile Arg Ile Asp
   625                 630                 635                 640
   tgt tca gaa ctg atg gag aag tac tcg gtt tcc aag ctg ctg ggc tcc     1968
   Cys Ser Glu Leu Met Glu Lys Tyr Ser Val Ser Lys Leu Leu Gly Ser
                   645                 650                 655
   acc gcc ggt tac gtt ggc tac gag gag ggt ggt atg ttg acc aac cag     2016
   Thr Ala Gly Tyr Val Gly Tyr Glu Glu Gly Gly Met Leu Thr Asn Gln
               660                 665                 670
   ctt ttg aga aga ccg tac tct gtg gtg ttg ttc gac gag gtc gag aag     2064
   Leu Leu Arg Arg Pro Tyr Ser Val Val Leu Phe Asp Glu Val Glu Lys
           675                 680                 685
   gcc gct ccc gag gtg ctg aac att ctc ctg cag atg ttg gac gac gga      2112
   Ala Ala Pro Glu Val Leu Asn Ile Leu Leu Gln Met Leu Asp Asp Gly
       690                 695                 700
   aga atc acc gcc gcc aac ggt act ctc gtc aac tgc tcg aac gct atc      2160
   Arg Ile Thr Ala Ala Asn Gly Thr Leu Val Asn Cys Ser Asn Ala Ile
   705                 710                 715                 720
   gtg atc atg acg tcc aac ctc ggc gcg gag tac atc aac gca tca aag      2208
   Val Ile Met Thr Ser Asn Leu Gly Ala Glu Tyr Ile Asn Ala Ser Lys
                   725                 730                 735
   gga acg aag gtg acc gac gag gtc aga agc ctg gtg atg aac act gtc      2256
   Gly Thr Lys Val Thr Asp Glu Val Arg Ser Leu Val Met Asn Thr Val
               740                 745                 750
   aag gga cat ttc aga ccg gag ttc ctc aac aga atc tcg gcc acc gtg      2304
   Lys Gly His Phe Arg Pro Glu Phe Leu Asn Arg Ile Ser Ala Thr Val
           755                 760                 765
   gtg ttc aac aga ctc tcc aga cat gcc atc gcc aag atc gtg cgg ctg      2352
   Val Phe Asn Arg Leu Ser Arg His Ala Ile Ala Lys Ile Val Arg Leu
       770                 775                 780
   aga ctg aag gaa atc gaa gag agg ttc gag gcc aac ggc aag tcg atc      2400
   Arg Leu Lys Glu Ile Glu Glu Arg Phe Glu Ala Asn Gly Lys Ser Ile
   785                 790                 795                 800
   aaa ctg aac gtg gac gat ggg gca ctc gag tac ctg tgc aaa aag gga      2448
   Lys Leu Asn Val Asp Asp Gly Ala Leu Glu Tyr Leu Cys Lys Lys Gly
                   805                 810                 815
   tac tct ccc gac ttg ggc gct aga ccg ctg aac aga ctg atc cag agt      2496
   Tyr Ser Pro Asp Leu Gly Ala Arg Pro Leu Asn Arg Leu Ile Gln Ser
               820                 825                 830
   gaa atc ctg aac cat ctg gcg gtg atg gtg ttg aac gga cag gtg ttg      2544
   Glu Ile Leu Asn His Leu Ala Val Met Val Leu Asn Gly Gln Val Leu
           835                 840                 845
   gac aaa gag gaa gtt cag att acg acg ggc agc aag gga ctg tct gtg      2592
   Asp Lys Glu Glu Val Gln Ile Thr Thr Gly Ser Lys Gly Leu Ser Val
       850                 855                 860
   gtt cct aac cat gac atc gag gac gaa gct atg gac gtg gac gtg gac      2640
   Val Pro Asn His Asp Ile Glu Asp Glu Ala Met Asp Val Asp Val Asp
   865                 870                 875                 880
   gag tgg acc gac gcc gcc gac gac gac gac tcg ggc tac ggc agt cct      2688
   Glu Trp Thr Asp Ala Ala Asp Asp Asp Asp Ser Gly Tyr Gly Ser Pro
                   885                 890                 895
   gat ctc gat taa                                                      2700
   Asp Leu Asp
<210> 46
   <211> 899
   <212> PRT
   <213> Ogataea minuta
<400> 46
   Met Asp Ser Thr Gln Phe Thr Asp Arg Ala Leu Asp Ile Val Thr Thr
   1               5                   10                  15
   Ala Gln Lys Leu Cys Gln Gln Asn Ser Asn Ala Gln Ile Val Pro Leu
               20                  25                  30
   His Phe Leu Ala Ala Met Thr Pro Thr Ser Ser Glu Gly Glu Ala Ile
           35                  40                  45
   Tyr Leu Lys Thr Leu Ile Glu Arg Gly Arg Phe Asp Trp Thr Ala Phe
       50                  55                  60
   Glu Arg Ala Val Asn Lys Ala Val Val Arg Leu Pro Ser Val Ala Gly
   65                  70                  75                  80
   Ser Asn Thr Glu Pro Ser Ile Ser Ala Ser Ala Ala Ser Ile Ile Thr
                   85                  90                  95
   Asn Ala Ser Lys Ile Lys Ala Gln Gln Lys Asp Ser Tyr Ile Gly Gln
               100                 105                 110
   Asp His Ile Leu Ser Ala Leu Leu Asp Asp Ser Ser Ile Gln Ala Val
           115                 120                 125
   Leu Lys Glu Ala Asn Val Lys Pro Asp Ala Leu Lys Thr Gln Ile Val
       130                 135                 140
   Glu Leu Arg Gly Asn Gln Arg Ile Asp Ser Arg Gln Ala Asp Ser Ser
   145                 150                 155                 160
   Gln Lys Phe Glu Phe Leu Ser Lys Tyr Ala Leu Asp Leu Thr Glu Gln
                   165                 170                 175
   Ala Leu Gln Gly Lys Ile Asp Pro Val Ile Gly Arg Glu Glu Glu Ile
               180                 185                 190
   Arg Arg Ala Ile Arg Val Leu Ser Arg Arg Ala Lys Ser Asn Pro Cys
           195                 200                 205
   Leu Ile Gly Asp Pro Gly Val Gly Lys Thr Ser Ile Val Glu Gly Val
       210                 215                 220
   Ala Gln Arg Ile Val Asp Asn Asp Val Pro Thr Val Leu Gln Gly Cys
   225                 230                 235                 240
   Lys Leu Tyr Ser Leu Asp Leu Gly Ala Leu Lys Ala Gly Ala Lys Tyr
   245 250 255
   Gln Gly Glu Phe Glu Glu Arg Leu Lys Gly Val Leu Ser Asp Ile Glu
   260 265 270
   Ser Ser Asn Ser Met Ile Ile Leu Phe Ile Asp Glu Ile His Met Leu
   275 280 285
   Met Gly Asp Gly Lys Ser Asp Ala Ala Asn Leu Leu Lys Pro Ala Leu
   290 295 300
   Ala Arg Gly Gln Phe His Cys Ile Gly Ala Thr Thr Val Thr Glu Tyr
   305 310 315 320
   Arg Lys His Ile Glu Lys Asp Gly Ala Phe Glu Arg Arg Phe Gln Arg
   325 330 335
   Ile Asp Val Arg Glu Pro Thr Ile Arg Glu Thr Val Ala Ile Leu Arg
   340 345 350
   Gly Leu Gln Pro Arg Tyr Glu Ile His His Gly Val Arg Ile Leu Asp
   355 360 365
   Ser Ala Leu Val Thr Ala Ala Gln Leu Ala Ser Arg Tyr Leu Thr Tyr
   370 375 380
   Arg Lys Leu Pro Asp Ser Ala Val Asp Leu Ile Asp Glu Ser Ala Ala
   385 390 395 400
   Gly Val Ala Val Ala Arg Asp Ser Lys Pro Glu Glu Leu Asp Ser Lys
   405 410 415
   Glu Arg Gln Leu Gln Leu Ile Glu Val Glu Ile Asn Ala Leu Glu Arg
   420 425 430
   Asp Gln Asp Ala Asp Thr Ser Thr Lys Glu Arg Leu Glu Gln Ala Arg
   435 440 445
   Gln Arg Arg Gln Asn Leu Glu Glu Glu Leu Ala Pro Leu Arg Glu Lys
   450 455 460
   Tyr Gln Gln Glu Arg Ala Gly His Glu Glu Leu Thr Ala Ala Lys Arg
   465 470 475 480
   Lys Leu Asp Asp Leu Glu Val Lys Ala Gln Asp Ala Glu Arg Arg His
   485 490 495
   Asp Ser Gln Thr Ile Ala Asp Leu Arg Met Phe Ala Ile Pro Asp Val
   500 505 510
   Lys Arg Arg Ile Glu Glu Leu Glu Gln Lys Val Val Glu Glu Glu Ala
   515 520 525
   Thr Ser Glu Asp Phe Met Val Lys Asn Val Val Gly Ser Glu Gln Val
   530 535 540
   Ser Glu Thr Ala Ala Arg Leu Thr Gly Ile Pro Val Ser Lys Leu Thr
   545 550 555 560
   Gln Ala Glu Asn Ala Lys Leu Ile Thr Met Glu Lys Glu Leu Ser Ala
   565 570 575
   Ala Val Val Gly Gln Gly Glu Ala Val Lys Ala Val Ser Asn Ser Ile
   580 585 590
   Arg Leu Ser Arg Ser Gly Leu Ala Asn Pro Asn Gln Pro Ala Ser Phe
   595 600 605
   Leu Phe Leu Gly Leu Ser Gly Ser Gly Lys Thr Glu Leu Ala Lys Lys
   610 615 620
   Leu Ala Gly Phe Leu Phe Ser Asp Glu Lys Ala Met Ile Arg Ile Asp
   625 630 635 640
   Cys Ser Glu Leu Met Glu Lys Tyr Ser Val Ser Lys Leu Leu Gly Ser
   645 650 655
   Thr Ala Gly Tyr Val Gly Tyr Glu Glu Gly Gly Met Leu Thr Asn Gln
   660 665 670
   Leu Leu Arg Arg Pro Tyr Ser Val Val Leu Phe Asp Glu Val Glu Lys
   675 680 685
   Ala Ala Pro Glu Val Leu Asn Ile Leu Leu Gln Met Leu Asp Asp Gly
   690 695 700
   Arg Ile Thr Ala Ala Asn Gly Thr Leu Val Asn Cys Ser Asn Ala Ile
   705 710 715 720
   Val Ile Met Thr Ser Asn Leu Gly Ala Glu Tyr Ile Asn Ala Ser Lys
   725 730 735
   Gly Thr Lys Val Thr Asp Glu Val Arg Ser Leu Val Met Asn Thr Val
   740 745 750
   Lys Gly His Phe Arg Pro Glu Phe Leu Asn Arg Ile Ser Ala Thr Val
             755                 760                 765
   Val Phe Asn Arg Leu Ser Arg His Ala Ile Ala Lys Ile Val Arg Leu
       770                 775                 780
   Arg Leu Lys Glu Ile Glu Glu Arg Phe Glu Ala Asn Gly Lys Ser Ile
   785                 790                 795                 800
   Lys Leu Asn Val Asp Asp Gly Ala Leu Glu Tyr Leu Cys Lys Lys Gly
                   805                 810                 815
   Tyr Ser Pro Asp Leu Gly Ala Arg Pro Leu Asn Arg Leu Ile Gln Ser
               820                 825                 830
   Glu Ile Leu Asn His Leu Ala Val Met Val Leu Asn Gly Gln Val Leu
           835                 840                 845
   Asp Lys Glu Glu Val Gln Ile Thr Thr Gly Ser Lys Gly Leu Ser Val
       850                 855                 860
   Val Pro Asn His Asp Ile Glu Asp Glu Ala Met Asp Val Asp Val Asp
   865                 870                 875                 880
   Glu Trp Thr Asp Ala Ala Asp Asp Asp Asp Ser Gly Tyr Gly Ser Pro
                   885                 890                 895
   Asp Leu Asp
<210> 47
   <211> 1998
   <212> DNA
   <213> Ogataea minuta
<220>
   <221> CDS
   <222> (1)..(1998)
<400> 47
   atg ttt aag ttc aac cgc tct gtg cta tct ata gct acg ata ctg tat       48
   Met Phe Lys Phe Asn Arg Ser Val Leu Ser Ile Ala Thr Ile Leu Tyr
   1               5                   10                  15
   gca gtg ctg ttg gta gtc cta cct ttg gct tca caa caa ttc gtg gaa       96
   Ala Val Leu Leu Val Val Leu Pro Leu Ala Ser Gln Gln Phe Val Glu
               20                  25                  30
   gca gag gcg aac gac aac tat ggt act gtt atc ggt atc gat ttg gga      144
   Ala Glu Ala Asn Asp Asn Tyr Gly Thr Val Ile Gly Ile Asp Leu Gly
           35                  40                  45
   acc act tac tca tgt gtg ggt gtg atg aaa gct ggt aga gtt gag atc      192
   Thr Thr Tyr Ser Cys Val Gly Val Met Lys Ala Gly Arg Val Glu Ile
       50                  55                  60
   ctt gcc aat gac cag ggt aac aga att act cca tct tat gtg gca ttt      240
   Leu Ala Asn Asp Gln Gly Asn Arg Ile Thr Pro Ser Tyr Val Ala Phe
   65                  70                  75                  80
   act gat gaa gag aga ctt gtc gga gat gcc gca aag aac cag att gcc      288
   Thr Asp Glu Glu Arg Leu Val Gly Asp Ala Ala Lys Asn Gln Ile Ala
                   85                  90                  95
   tcc aac cca agc aac aca atc ttc gat atc aag aga ctc ata gga cac      336
   Ser Asn Pro Ser Asn Thr Ile Phe Asp Ile Lys Arg Leu Ile Gly His
               100                 105                 110
   aga ttt gac gat aag gtt gtg caa aaa gag att gca cac ctc cct tac      384
   Arg Phe Asp Asp Lys Val Val Gln Lys Glu Ile Ala His Leu Pro Tyr
           115                 120                 125
   aag atc aga aac caa gag ggc aga ccg gtc gtt gag gcc act gtc aat      432
   Lys Ile Arg Asn Gln Glu Gly Arg Pro Val Val Glu Ala Thr Val Asn
       130                 135                 140
   gga gag gtg acc acg ttc acg gcc gaa gaa gtt tcg gcc atg atc ttg      480
   Gly Glu Val Thr Thr Phe Thr Ala Glu Glu Val Ser Ala Met Ile Leu
   145                 150                 155                 160
   gga aag atg aag caa att gct gaa gat tat ctc gga aag aag gtt acc      528
   Gly Lys Met Lys Gln Ile Ala Glu Asp Tyr Leu Gly Lys Lys Val Thr
                   165                 170                 175
   cat gct gtt gtc acg gtt cct gca tac ttt aac gac gcc caa aga cag      576
   His Ala Val Val Thr Val Pro Ala Tyr Phe Asn Asp Ala Gln Arg Gln
               180                 185                 190
   gcc act aag gat gct ggt acc att gcc ggt ctg gaa gtt ttg aga att      624
   Ala Thr Lys Asp Ala Gly Thr Ile Ala Gly Leu Glu Val Leu Arg Ile
           195                 200                 205
   gtt aac gag cct act gcc gct gca att gct tac ggt ctc gac aag acg      672
   Val Asn Glu Pro Thr Ala Ala Ala Ile Ala Tyr Gly Leu Asp Lys Thr
       210                 215                 220
   gac gaa gag aag cat atc att gtt tac gat ttg ggt gga gga act ttt      720
   Asp Glu Glu Lys His Ile Ile Val Tyr Asp Leu Gly Gly Gly Thr Phe
   225                 230                 235                 240
   gat gtt tct ctg ttg aca att gca ggt gga gct ttc gag gtt cgt gcc      768
   Asp Val Ser Leu Leu Thr Ile Ala Gly Gly Ala Phe Glu Val Arg Ala
                   245                 250                 255
   acc gct ggt gat acc cat ctt ggt ggt gag gac ttt gat tac aga gtt      816
   Thr Ala Gly Asp Thr His Leu Gly Gly Glu Asp Phe Asp Tyr Arg Val
               260                 265                 270
   gtc aga cat ttc atc aag gtg ttt aag aag aag cat ggc att gat atc      864
   Val Arg His Phe Ile Lys Val Phe Lys Lys Lys His Gly Ile Asp Ile
           275                 280                 285
   agt gat aat cca aag gct ctt gct aaa ttg aag aga gaa gtt gaa aaa      912
   Ser Asp Asn Pro Lys Ala Leu Ala Lys Leu Lys Arg Glu Val Glu Lys
       290                 295                 300
   gct aag aga acc ttg tct tct caa atg tcc acc aga att gag att gac      960
   Ala Lys Arg Thr Leu Ser Ser Gln Met Ser Thr Arg Ile Glu Ile Asp
   305                 310                 315                 320
   tcg ttc gct gac ggt att gac ttc tcc gag tcc tta tcc agg gcc aag     1008
   Ser Phe Ala Asp Gly Ile Asp Phe Ser Glu Ser Leu Ser Arg Ala Lys
                   325                 330                 335
   ttc gag gaa ttg aac att gag ttg ttc aaa aag acc ttg aag cct gtt     1056
   Phe Glu Glu Leu Asn Ile Glu Leu Phe Lys Lys Thr Leu Lys Pro Val
               340                 345                 350
   caa cgt gtt ctt gaa gac gcc aaa ttc aag gtt tca gaa att gat gac     1104
   Gln Arg Val Leu Glu Asp Ala Lys Phe Lys Val Ser Glu Ile Asp Asp
           355                 360                 365
   att gtc ttg gtt ggt ggt tcc acg aga att cca aag gtg caa gag ttg     1152
   Ile Val Leu Val Gly Gly Ser Thr Arg Ile Pro Lys Val Gln Glu Leu
       370                 375                 380
   ctg gaa agt tac ttc aac ggc aag caa gtg tcc aag gga att aac cca     1200
   Leu Glu Ser Tyr Phe Asn Gly Lys Gln Val Ser Lys Gly Ile Asn Pro
   385                 390                 395                 400
   gat gaa gct gtt gct tac ggt gcg gct gtt caa gct ggt gtc ctc tct     1248
   Asp Glu Ala Val Ala Tyr Gly Ala Ala Val Gln Ala Gly Val Leu Ser
                   405                 410                 415
   ggt gaa gaa ggc gtt gaa gac att gtt ttg att gat gtg aat cca tta     1296
   Gly Glu Glu Gly Val Glu Asp Ile Val Leu Ile Asp Val Asn Pro Leu
               420                 425                 430
   act ttg ggt atc gag acc tcc ggc ggt gtc atg acc act ttg att aag     1344
   Thr Leu Gly Ile Glu Thr Ser Gly Gly Val Met Thr Thr Leu Ile Lys
           435                 440                 445
   aga aac act gca att cca acc aag aag tct caa att ttc tct act gct     1392
   Arg Asn Thr Ala Ile Pro Thr Lys Lys Ser Gln Ile Phe Ser Thr Ala
       450                 455                 460
   gct gac aat caa cct gtt gtc ttg atc caa gtc tat gaa ggt gag aga     1440
   Ala Asp Asn Gln Pro Val Val Leu Ile Gln Val Tyr Glu Gly Glu Arg
   465                 470                 475                 480
   gcc atg gca aag gat aac aat ttg cta gga aag ttc gag ttg aag gat     1488
   Ala Met Ala Lys Asp Asn Asn Leu Leu Gly Lys Phe Glu Leu Lys Asp
                   485                 490                 495
   att cct cca gcc cca aga ggt acc cca caa att gag gtg act ttc act     1536
   Ile Pro Pro Ala Pro Arg Gly Thr Pro Gln Ile Glu Val Thr Phe Thr
               500                 505                 510
   ctg gac tcc aac gga atc ctg aag gtt gct gcc act gat aaa ggt act     1584
   Leu Asp Ser Asn Gly Ile Leu Lys Val Ala Ala Thr Asp Lys Gly Thr
           515                 520                 525
   ggt aag tct aac tct atc aca atc aca aac gac aag ggt aga ctt tcg     1632
   Gly Lys Ser Asn Ser Ile Thr Ile Thr Asn Asp Lys Gly Arg Leu Ser
       530                 535                 540
   aag gag gag att gag aag aag gtt gag gag gcc gaa cag tat gct caa     1680
   Lys Glu Glu Ile Glu Lys Lys Val Glu Glu Ala Glu Gln Tyr Ala Gln
   545                 550                 555                 560
   caa gat aag gag gtc aga gag aag atc gag agc aga aac gga ctt gag     1728
   Gln Asp Lys Glu Val Arg Glu Lys Ile Glu Ser Arg Asn Gly Leu Glu
                   565                 570                 575
   aac tac gcc cac tcg ttg aaa aac caa gtg aac gat gag acc gga ttc     1776
   Asn Tyr Ala His Ser Leu Lys Asn Gln Val Asn Asp Glu Thr Gly Phe
               580                 585                 590
   ggc tcc aag ctt gat gag gat gac aag gaa act ttg ttg gat gcc atc     1824
   Gly Ser Lys Leu Asp Glu Asp Asp Lys Glu Thr Leu Leu Asp Ala Ile
           595                 600                 605
   aac gag gca ttg gag tac ttg gac gac aac ttt gag acc gca aca aag     1872
   Asn Glu Ala Leu Glu Tyr Leu Asp Asp Asn Phe Glu Thr Ala Thr Lys
       610                 615                 620
   caa gac ttt gag gat cag aag gaa aaa ttg agt aag gtt gct tac cca     1920
   Gln Asp Phe Glu Asp Gln Lys Glu Lys Leu Ser Lys Val Ala Tyr Pro
   625                 630                 635                 640
   att act tca aag ttg tat gat acg cca cct act agt gac gaa gat gat     1968
   Ile Thr Ser Lys Leu Tyr Asp Thr Pro Pro Thr Ser Asp Glu Asp Asp
                   645                 650                 655
   gag gat gac tgg gat cat gat gag ctg tga                             1998
   Glu Asp Asp Trp Asp His Asp Glu Leu
               660                 665
<210> 48
   <211> 665
   <212> PRT
   <213> Ogataea minuta
<400> 48
   Met Phe Lys Phe Asn Arg Ser Val Leu Ser Ile Ala Thr Ile Leu Tyr
   1               5                   10                  15
   Ala Val Leu Leu Val Val Leu Pro Leu Ala Ser Gln Gln Phe Val Glu
               20                  25                  30
   Ala Glu Ala Asn Asp Asn Tyr Gly Thr Val Ile Gly Ile Asp Leu Gly
           35                  40                  45
   Thr Thr Tyr Ser Cys Val Gly Val Met Lys Ala Gly Arg Val Glu Ile
       50                  55                  60
   Leu Ala Asn Asp Gln Gly Asn Arg Ile Thr Pro Ser Tyr Val Ala Phe
   65                  70                  75                  80
   Thr Asp Glu Glu Arg Leu Val Gly Asp Ala Ala Lys Asn Gln Ile Ala
                   85                  90                  95
   Ser Asn Pro Ser Asn Thr Ile Phe Asp Ile Lys Arg Leu Ile Gly His
               100                 105                 110
   Arg Phe Asp Asp Lys Val Val Gln Lys Glu Ile Ala His Leu Pro Tyr
           115                 120                 125
   Lys Ile Arg Asn Gln Glu Gly Arg Pro Val Val Glu Ala Thr Val Asn
       130                 135                 140
   Gly Glu Val Thr Thr Phe Thr Ala Glu Glu Val Ser Ala Met Ile Leu
   145                 150                 155                 160
   Gly Lys Met Lys Gln Ile Ala Glu Asp Tyr Leu Gly Lys Lys Val Thr
                   165                 170                 175
   His Ala Val Val Thr Val Pro Ala Tyr Phe Asn Asp Ala Gln Arg Gln
               180                 185                 190
   Ala Thr Lys Asp Ala Gly Thr Ile Ala Gly Leu Glu Val Leu Arg Ile
           195                 200                 205
   Val Asn Glu Pro Thr Ala Ala Ala Ile Ala Tyr Gly Leu Asp Lys Thr
       210                 215                 220
   Asp Glu Glu Lys His Ile Ile Val Tyr Asp Leu Gly Gly Gly Thr Phe
   225                 230                 235                 240
   Asp Val Ser Leu Leu Thr Ile Ala Gly Gly Ala Phe Glu Val Arg Ala
                   245                 250                 255
   Thr Ala Gly Asp Thr His Leu Gly Gly Glu Asp Phe Asp Tyr Arg Val
               260                 265                 270
   Val Arg His Phe Ile Lys Val Phe Lys Lys Lys His Gly Ile Asp Ile
           275                 280                 285
   Ser Asp Asn Pro Lys Ala Leu Ala Lys Leu Lys Arg Glu Val Glu Lys
       290                 295                 300
   Ala Lys Arg Thr Leu Ser Ser Gln Met Ser Thr Arg Ile Glu Ile Asp
   305                 310                 315                 320
   Ser Phe Ala Asp Gly Ile Asp Phe Ser Glu Ser Leu Ser Arg Ala Lys
                   325                 330                 335
   Phe Glu Glu Leu Asn Ile Glu Leu Phe Lys Lys Thr Leu Lys Pro Val
               340                 345                 350
   Gln Arg Val Leu Glu Asp Ala Lys Phe Lys Val Ser Glu Ile Asp Asp
           355                 360                 365
   Ile Val Leu Val Gly Gly Ser Thr Arg Ile Pro Lys Val Gln Glu Leu
       370                 375                 380
   Leu Glu Ser Tyr Phe Asn Gly Lys Gln Val Ser Lys Gly Ile Asn Pro
   385                 390                 395                 400
   Asp Glu Ala Val Ala Tyr Gly Ala Ala Val Gln Ala Gly Val Leu Ser
                   405                 410                 415
   Gly Glu Glu Gly Val Glu Asp Ile Val Leu Ile Asp Val Asn Pro Leu
               420                 425                 430
   Thr Leu Gly Ile Glu Thr Ser Gly Gly Val Met Thr Thr Leu Ile Lys
           435                 440                 445
   Arg Asn Thr Ala Ile Pro Thr Lys Lys Ser Gln Ile Phe Ser Thr Ala
       450                 455                 460
   Ala Asp Asn Gln Pro Val Val Leu Ile Gln Val Tyr Glu Gly Glu Arg
   465                 470                 475                 480
   Ala Met Ala Lys Asp Asn Asn Leu Leu Gly Lys Phe Glu Leu Lys Asp
                   485                 490                 495
   Ile Pro Pro Ala Pro Arg Gly Thr Pro Gln Ile Glu Val Thr Phe Thr
               500                 505                 510
   Leu Asp Ser Asn Gly Ile Leu Lys Val Ala Ala Thr Asp Lys Gly Thr
           515                 520                 525
   Gly Lys Ser Asn Ser Ile Thr Ile Thr Asn Asp Lys Gly Arg Leu Ser
       530                 535                 540
   Lys Glu Glu Ile Glu Lys Lys Val Glu Glu Ala Glu Gln Tyr Ala Gln
   545                 550                 555                 560
   Gln Asp Lys Glu Val Arg Glu Lys Ile Glu Ser Arg Asn Gly Leu Glu
                   565                 570                 575
   Asn Tyr Ala His Ser Leu Lys Asn Gln Val Asn Asp Glu Thr Gly Phe
               580                 585                 590
   Gly Ser Lys Leu Asp Glu Asp Asp Lys Glu Thr Leu Leu Asp Ala Ile
   595 600 605
   Asn Glu Ala Leu Glu Tyr Leu Asp Asp Asn Phe Glu Thr Ala Thr Lys
   610 615 620
   Gln Asp Phe Glu Asp Gln Lys Glu Lys Leu Ser Lys Val Ala Tyr Pro
   625 630 635 640
   Ile Thr Ser Lys Leu Tyr Asp Thr Pro Pro Thr Ser Asp Glu Asp Asp
   645 650 655
   Glu Asp Asp Trp Asp His Asp Glu Leu
   660 665
<210> 49
   <211> 1569
   <212> DNA
   <213> Saccharomyces cerevisiae
<220>
   <221> CDS
   <222> (1)..(1569)
<400> 49
   atg aag ttt tct gct ggt gcc gtc ctg tca tgg tcc tcc ctg ctg ctc       48
   Met Lys Phe Ser Ala Gly Ala Val Leu Ser Trp Ser Ser Leu Leu Leu
   1               5                   10                  15
   gcc tcc tct gtt ttc gcc caa caa gag gct gtg gcc cct gaa gac tcc       96
   Ala Ser Ser Val Phe Ala Gln Gln Glu Ala Val Ala Pro Glu Asp Ser
               20                  25                  30
   gct gtc gtt aag ttg gcc acc gac tcc ttc aat gag tac att cag tcg      144
   Ala Val Val Lys Leu Ala Thr Asp Ser Phe Asn Glu Tyr Ile Gln Ser
           35                  40                  45
   cac gac ttg gtg ctt gcg gag ttt ttt gct cca tgg tgt ggc cac tgt      192
   His Asp Leu Val Leu Ala Glu Phe Phe Ala Pro Trp Cys Gly His Cys
       50                  55                  60
   aag aac atg gct cct gaa tac gtt aaa gcc gcc gag act tta gtt gag      240
   Lys Asn Met Ala Pro Glu Tyr Val Lys Ala Ala Glu Thr Leu Val Glu
   65                  70                  75                  80
   aaa aac att acc ttg gcc cag atc gac tgt act gaa aac cag gat ctg      288
   Lys Asn Ile Thr Leu Ala Gln Ile Asp Cys Thr Glu Asn Gln Asp Leu
                   85                  90                  95
   tgt atg gaa cac aac att cca ggg ttc cca agc ttg aag att ttc aaa      336
   Cys Met Glu His Asn Ile Pro Gly Phe Pro Ser Leu Lys Ile Phe Lys
               100                 105                 110
   aac agc gat gtt aac aac tcg atc gat tac gag gga cct aga act gcc      384
   Asn Ser Asp Val Asn Asn Ser Ile Asp Tyr Glu Gly Pro Arg Thr Ala
           115                 120                 125
   gag gcc att gtc caa ttc atg atc aag caa agc caa ccg gct gtc gcc      432
   Glu Ala Ile Val Gln Phe Met Ile Lys Gln Ser Gln Pro Ala Val Ala
       130                 135                 140
   gtt gtt gct gat cta cca gct tac ctt gct aac gag act ttt gtc act      480
   Val Val Ala Asp Leu Pro Ala Tyr Leu Ala Asn Glu Thr Phe Val Thr
   145                 150                 155                 160
   cca gtt atc gtc caa tcc ggt aag att gac gcc gac ttc aac gcc acc      528
   Pro Val Ile Val Gln Ser Gly Lys Ile Asp Ala Asp Phe Asn Ala Thr
                   165                 170                 175
   ttt tac tcc atg gcc aac aaa cac ttc aac gac tac gac ttt gtc tcc      576
   Phe Tyr Ser Met Ala Asn Lys His Phe Asn Asp Tyr Asp Phe Val Ser
               180                 185                 190
   gct gaa aac gca gac gat gat ttc aag ctt tct att tac ttg ccc tcc      624
   Ala Glu Asn Ala Asp Asp Asp Phe Lys Leu Ser Ile Tyr Leu Pro Ser
           195                 200                 205
   gcc atg gac gag cct gta gta tac aac ggt aag aaa gcc gat atc gct      672
   Ala Met Asp Glu Pro Val Val Tyr Asn Gly Lys Lys Ala Asp Ile Ala
       210                 215                 220
   gac gct gat gtt ttt gaa aaa tgg ttg caa gtg gaa gcc ttg ccc tac      720
   Asp Ala Asp Val Phe Glu Lys Trp Leu Gln Val Glu Ala Leu Pro Tyr
   225                 230                 235                 240
   ttt ggt gaa atc gac ggt tcc gtt ttc gcc caa tac gtc gaa agc ggt      768
   Phe Gly Glu Ile Asp Gly Ser Val Phe Ala Gln Tyr Val Glu Ser Gly
                   245                 250                 255
   ttg cct ttg ggt tac tta ttc tac aat gac gag gaa gaa ttg gaa gaa      816
   Leu Pro Leu Gly Tyr Leu Phe Tyr Asn Asp Glu Glu Glu Leu Glu Glu
               260                 265                 270
   tac aag cct ctc ttt acc gag ttg gcc aaa aag aac aga ggt cta atg      864
   Tyr Lys Pro Leu Phe Thr Glu Leu Ala Lys Lys Asn Arg Gly Leu Met
           275                 280                 285
   aac ttt gtt agc atc gat gcc aga aaa ttc ggc aga cac gcc ggc aac      912
   Asn Phe Val Ser Ile Asp Ala Arg Lys Phe Gly Arg His Ala Gly Asn
       290                 295                 300
   ttg aac atg aag gaa caa ttc cct cta ttt gcc atc cac gac atg act      960
   Leu Asn Met Lys Glu Gln Phe Pro Leu Phe Ala Ile His Asp Met Thr
   305                 310                 315                 320
   gaa gac ttg aag tac ggt ttg cct caa ctc tct gaa gag gcg ttt gac     1008
   Glu Asp Leu Lys Tyr Gly Leu Pro Gln Leu Ser Glu Glu Ala Phe Asp
                   325                 330                 335
   gaa ttg agc gac aag atc gtg ttg gag tct aag gct att gaa tct ttg     1056
   Glu Leu Ser Asp Lys Ile Val Leu Glu Ser Lys Ala Ile Glu Ser Leu
               340                 345                 350
   gtt aag gac ttc ttg aaa ggt gat gcc tcc cca atc gtg aag tcc caa     1104
   Val Lys Asp Phe Leu Lys Gly Asp Ala Ser Pro Ile Val Lys Ser Gln
           355                 360                 365
   gag atc ttc gag aac caa gat tcc tct gtc ttc caa ttg gtc ggt aag     1152
   Glu Ile Phe Glu Asn Gln Asp Ser Ser Val Phe Gln Leu Val Gly Lys
       370                 375                 380
   aac cat gac gaa atc gtc aac gac cca aag aag gac gtt ctt gtt ttg     1200
   Asn His Asp Glu Ile Val Asn Asp Pro Lys Lys Asp Val Leu Val Leu
   385                 390                 395                 400
   tac tat gcc cca tgg tgt ggt cac tgt aag aga ttg gcc cca act tac     1248
   Tyr Tyr Ala Pro Trp Cys Gly His Cys Lys Arg Leu Ala Pro Thr Tyr
                   405                 410                 415
   caa gaa cta gct gat acc tac gcc aac gcc aca tcc gac gtt ttg att     1296
   Gln Glu Leu Ala Asp Thr Tyr Ala Asn Ala Thr Ser Asp Val Leu Ile
               420                 425                 430
   gct aaa cta gac cac act gaa aac gat gtc aga ggc gtc gta att gaa     1344
   Ala Lys Leu Asp His Thr Glu Asn Asp Val Arg Gly Val Val Ile Glu
           435                 440                 445
   ggt tac cca aca atc gtc tta tac cca ggt ggt aag aag tcc gaa tct     1392
   Gly Tyr Pro Thr Ile Val Leu Tyr Pro Gly Gly Lys Lys Ser Glu Ser
       450                 455                 460
   gtt gtg tac caa ggt tca aga tcc ttg gac tct tta ttc gac ttc atc     1440
   Val Val Tyr Gln Gly Ser Arg Ser Leu Asp Ser Leu Phe Asp Phe Ile
   465                 470                 475                 480
   aag gaa aac ggt cac ttc gac gtc gac ggt aag gcc ttg tac gaa gaa     1488
   Lys Glu Asn Gly His Phe Asp Val Asp Gly Lys Ala Leu Tyr Glu Glu
                   485                 490                 495
   gcc cag gaa aaa gct gct gag gaa gcc gat gct gac gct gaa ttg gct     1536
   Ala Gln Glu Lys Ala Ala Glu Glu Ala Asp Ala Asp Ala Glu Leu Ala
               500                 505                 510
   gac gaa gaa gat gcc att cac gat gaa ttg taa                         1569
   Asp Glu Glu Asp Ala Ile His Asp Glu Leu
           515                 520
<210> 50
   <211> 522
   <212> PRT
   <213> Saccharomyces cerevisiae
<400> 50
   Met Lys Phe Ser Ala Gly Ala Val Leu Ser Trp Ser Ser Leu Leu Leu
   1               5                   10                  15
   Ala Ser Ser Val Phe Ala Gln Gln Glu Ala Val Ala Pro Glu Asp Ser
               20                  25                  30
   Ala Val Val Lys Leu Ala Thr Asp Ser Phe Asn Glu Tyr Ile Gln Ser
           35                  40                  45
   His Asp Leu Val Leu Ala Glu Phe Phe Ala Pro Trp Cys Gly His Cys
       50                  55                  60
   Lys Asn Met Ala Pro Glu Tyr Val Lys Ala Ala Glu Thr Leu Val Glu
   65                  70                  75                  80
   Lys Asn Ile Thr Leu Ala Gln Ile Asp Cys Thr Glu Asn Gln Asp Leu
   85 90 95
   Cys Met Glu His Asn Ile Pro Gly Phe Pro Ser Leu Lys Ile Phe Lys
   100 105 110
   Asn Ser Asp Val Asn Asn Ser Ile Asp Tyr Glu Gly Pro Arg Thr Ala
   115 120 125
   Glu Ala Ile Val Gln Phe Met Ile Lys Gln Ser Gln Pro Ala Val Ala
   130 135 140
   Val Val Ala Asp Leu Pro Ala Tyr Leu Ala Asn Glu Thr Phe Val Thr
   145 150 155 160
   Pro Val Ile Val Gln Ser Gly Lys Ile Asp Ala Asp Phe Asn Ala Thr
   165 170 175
   Phe Tyr Ser Met Ala Asn Lys His Phe Asn Asp Tyr Asp Phe Val Ser
   180 185 190
   Ala Glu Asn Ala Asp Asp Asp Phe Lys Leu Ser Ile Tyr Leu Pro Ser
   195 200 205
   Ala Met Asp Glu Pro Val Val Tyr Asn Gly Lys Lys Ala Asp Ile Ala
   210 215 220
   Asp Ala Asp Val Phe Glu Lys Trp Leu Gln Val Glu Ala Leu Pro Tyr
   225 230 235 240
   Phe Gly Glu Ile Asp Gly Ser Val Phe Ala Gln Tyr Val Glu Ser Gly
   245 250 255
   Leu Pro Leu Gly Tyr Leu Phe Tyr Asn Asp Glu Glu Glu Leu Glu Glu
   260 265 270
   Tyr Lys Pro Leu Phe Thr Glu Leu Ala Lys Lys Asn Arg Gly Leu Met
   275 280 285
   Asn Phe Val Ser Ile Asp Ala Arg Lys Phe Gly Arg His Ala Gly Asn
   290 295 300
   Leu Asn Met Lys Glu Gln Phe Pro Leu Phe Ala Ile His Asp Met Thr
   305 310 315 320
   Glu Asp Leu Lys Tyr Gly Leu Pro Gln Leu Ser Glu Glu Ala Phe Asp
   325 330 335
   Glu Leu Ser Asp Lys Ile Val Leu Glu Ser Lys Ala Ile Glu Ser Leu
               340                 345                 350
   Val Lys Asp Phe Leu Lys Gly Asp Ala Ser Pro Ile Val Lys Ser Gln
           355                 360                 365
   Glu Ile Phe Glu Asn Gln Asp Ser Ser Val Phe Gln Leu Val Gly Lys
       370                 375                 380
   Asn His Asp Glu Ile Val Asn Asp Pro Lys Lys Asp Val Leu Val Leu
   385                 390                 395                 400
   Tyr Tyr Ala Pro Trp Cys Gly His Cys Lys Arg Leu Ala Pro Thr Tyr
                   405                 410                 415
   Gln Glu Leu Ala Asp Thr Tyr Ala Asn Ala Thr Ser Asp Val Leu Ile
               420                 425                 430
   Ala Lys Leu Asp His Thr Glu Asn Asp Val Arg Gly Val Val Ile Glu
           435                 440                 445
   Gly Tyr Pro Thr Ile Val Leu Tyr Pro Gly Gly Lys Lys Ser Glu Ser
       450                 455                 460
   Val Val Tyr Gln Gly Ser Arg Ser Leu Asp Ser Leu Phe Asp Phe Ile
   465                 470                 475                 480
   Lys Glu Asn Gly His Phe Asp Val Asp Gly Lys Ala Leu Tyr Glu Glu
                   485                 490                 495
   Ala Gln Glu Lys Ala Ala Glu Glu Ala Asp Ala Asp Ala Glu Leu Ala
               500                 505                 510
   Asp Glu Glu Asp Ala Ile His Asp Glu Leu
           515                 520
<210> 51
   <211> 957
   <212> DNA
   <213> Saccharomyces cerevisiae
<220>
   <221> CDS
   <222> (1)..(957)
<400> 51
   atg tta ttt ctt aat att att aag ctc ctt ttg gga ctt ttt att atg      48
   Met Leu Phe Leu Asn Ile Ile Lys Leu Leu Leu Gly Leu Phe Ile Met
   1               5                   10                  15
   aat gaa gta aag gcg caa aac ttt tac gat tcc gat cct cat ata tca      96
   Asn Glu Val Lys Ala Gln Asn Phe Tyr Asp Ser Asp Pro His Ile Ser
               20                  25                  30
   gag tta acg cca aaa agc ttc gat aaa gcg atc cat aac aca aat tac     144
   Glu Leu Thr Pro Lys Ser Phe Asp Lys Ala Ile His Asn Thr Asn Tyr
           35                  40                  45
   aca tca tta gtg gaa ttt tat gct ccg tgg tgc ggc cat tgt aag aag     192
   Thr Ser Leu Val Glu Phe Tyr Ala Pro Trp Cys Gly His Cys Lys Lys
       50                  55                  60
   ctc tct agt acg ttc cgc aag gca gca aaa aga ttg gat ggt gta gtc     240
   Leu Ser Ser Thr Phe Arg Lys Ala Ala Lys Arg Leu Asp Gly Val Val
   65                  70                  75                  80
   caa gtt gct gct gta aac tgt gac ctt aac aag aat aag gct ttg tgt     288
   Gln Val Ala Ala Val Asn Cys Asp Leu Asn Lys Asn Lys Ala Leu Cys
                   85                  90                  95
   gct aaa tac gac gta aac gga ttt ccc acg tta atg gta ttt agg ccc     336
   Ala Lys Tyr Asp Val Asn Gly Phe Pro Thr Leu Met Val Phe Arg Pro
               100                 105                 110
   cca aaa att gac cta tct aag cca ata gat aac gcc aaa aaa agt ttc     384
   Pro Lys Ile Asp Leu Ser Lys Pro Ile Asp Asn Ala Lys Lys Ser Phe
           115                 120                 125
   agc gct cat gcc aat gaa gtg tac tca ggt gca aga act ctc gcg cct     432
   Ser Ala His Ala Asn Glu Val Tyr Ser Gly Ala Arg Thr Leu Ala Pro
       130                 135                 140
   att gtt gat ttt tct ctt tca aga ata agg tca tat gtc aaa aag ttt     480
   Ile Val Asp Phe Ser Leu Ser Arg Ile Arg Ser Tyr Val Lys Lys Phe
   145                 150                 155                 160
   gtc cgt ata gat aca ctt ggc tct tta ctt aga aag tca ccc aaa ctt     528
   Val Arg Ile Asp Thr Leu Gly Ser Leu Leu Arg Lys Ser Pro Lys Leu
                   165                 170                 175
   tcc gtg gtg ttg ttt tcc aaa caa gac aaa att tca ccg gtt tat aaa     576
   Ser Val Val Leu Phe Ser Lys Gln Asp Lys Ile Ser Pro Val Tyr Lys
               180                 185                 190
   agc att gcc ctt gat tgg tta gga aag ttc gat ttt tat tca att tca     624
   Ser Ile Ala Leu Asp Trp Leu Gly Lys Phe Asp Phe Tyr Ser Ile Ser
           195                 200                 205
   aac aaa aaa ctc aag caa cta acc gat atg aac cca aca tat gaa aaa     672
   Asn Lys Lys Leu Lys Gln Leu Thr Asp Met Asn Pro Thr Tyr Glu Lys
       210                 215                 220
   act cct gag att ttc aaa tat ttg cag aag gtc att cct gaa cag cga     720
   Thr Pro Glu Ile Phe Lys Tyr Leu Gln Lys Val Ile Pro Glu Gln Arg
   225                 230                 235                 240
   cag agc gat aaa agt aag ctt gtc gtt ttt gat gca gac aaa gat aaa     768
   Gln Ser Asp Lys Ser Lys Leu Val Val Phe Asp Ala Asp Lys Asp Lys
                   245                 250                 255
   ttt tgg gag tat gaa ggg aac tca atc aac aaa aat gac att tcc aaa     816
   Phe Trp Glu Tyr Glu Gly Asn Ser Ile Asn Lys Asn Asp Ile Ser Lys
               260                 265                 270
   ttt ctg cgg gac act ttt agt att acc ccc aat gag ggt cct ttt agt      864
   Phe Leu Arg Asp Thr Phe Ser Ile Thr Pro Asn Glu Gly Pro Phe Ser
           275                 280                 285
   aga cgt tct gaa tat att gct tac tta aaa act ggc aag aag cca att      912
   Arg Arg Ser Glu Tyr Ile Ala Tyr Leu Lys Thr Gly Lys Lys Pro Ile
       290                 295                 300
   aaa aag aac cat tcc tcc tca gga aac aag cac gac gaa ttg tag          957
   Lys Lys Asn His Ser Ser Ser Gly Asn Lys His Asp Glu Leu
   305                 310                 315
<210> 52
   <211> 318
   <212> PRT
   <213> Saccharomyces cerevisiae
<400> 52
   Met Leu Phe Leu Asn Ile Ile Lys Leu Leu Leu Gly Leu Phe Ile Met
   1               5                   10                  15
   Asn Glu Val Lys Ala Gln Asn Phe Tyr Asp Ser Asp Pro His Ile Ser
               20                  25                  30
   Glu Leu Thr Pro Lys Ser Phe Asp Lys Ala Ile His Asn Thr Asn Tyr
           35                  40                  45
   Thr Ser Leu Val Glu Phe Tyr Ala Pro Trp Cys Gly His Cys Lys Lys
       50                  55                  60
   Leu Ser Ser Thr Phe Arg Lys Ala Ala Lys Arg Leu Asp Gly Val Val
   65                  70                  75                  80
   Gln Val Ala Ala Val Asn Cys Asp Leu Asn Lys Asn Lys Ala Leu Cys
                   85                  90                  95
   Ala Lys Tyr Asp Val Asn Gly Phe Pro Thr Leu Met Val Phe Arg Pro
               100                 105                 110
   Pro Lys Ile Asp Leu Ser Lys Pro Ile Asp Asn Ala Lys Lys Ser Phe
           115                 120                 125
   Ser Ala His Ala Asn Glu Val Tyr Ser Gly Ala Arg Thr Leu Ala Pro
       130                 135                 140
   Ile Val Asp Phe Ser Leu Ser Arg Ile Arg Ser Tyr Val Lys Lys Phe
   145                 150                 155                 160
   Val Arg Ile Asp Thr Leu Gly Ser Leu Leu Arg Lys Ser Pro Lys Leu
                   165                 170                 175
   Ser Val Val Leu Phe Ser Lys Gln Asp Lys Ile Ser Pro Val Tyr Lys
               180                 185                 190
   Ser Ile Ala Leu Asp Trp Leu Gly Lys Phe Asp Phe Tyr Ser Ile Ser
           195                 200                 205
   Asn Lys Lys Leu Lys Gln Leu Thr Asp Met Asn Pro Thr Tyr Glu Lys
       210                 215                 220
   Thr Pro Glu Ile Phe Lys Tyr Leu Gln Lys Val Ile Pro Glu Gln Arg
   225                 230                 235                 240
   Gln Ser Asp Lys Ser Lys Leu Val Val Phe Asp Ala Asp Lys Asp Lys
                   245                 250                 255
   Phe Trp Glu Tyr Glu Gly Asn Ser Ile Asn Lys Asn Asp Ile Ser Lys
               260                 265                 270
   Phe Leu Arg Asp Thr Phe Ser Ile Thr Pro Asn Glu Gly Pro Phe Ser
           275                 280                 285
   Arg Arg Ser Glu Tyr Ile Ala Tyr Leu Lys Thr Gly Lys Lys Pro Ile
       290                 295                 300
   Lys Lys Asn His Ser Ser Ser Gly Asn Lys His Asp Glu Leu
   305                 310                 315
<210> 53
   <211> 1134
   <212> DNA
   <213> Saccharomyces cerevisiae
<220>
   <221> CDS
   <222> (1)..(1134)
<400> 53
   atg att cca aaa tta tat ata cat ttg ata cta tct tta ttg ttg ttg      48
   Met Ile Pro Lys Leu Tyr Ile His Leu Ile Leu Ser Leu Leu Leu Leu
   1               5                   10                  15
   ccg cta att ttg gcg cag gat tat tat gca ata cta gag ata gac aaa      96
   Pro Leu Ile Leu Ala Gln Asp Tyr Tyr Ala Ile Leu Glu Ile Asp Lys
               20                  25                  30
   gat gcc act gag aag gaa atc aaa tca gcg tac aga caa ttg tct aag     144
   Asp Ala Thr Glu Lys Glu Ile Lys Ser Ala Tyr Arg Gln Leu Ser Lys
           35                  40                  45
   aag tac cat ccg gat aaa aat gct ggg agc gaa gaa gcc cat caa aaa      192
   Lys Tyr His Pro Asp Lys Asn Ala Gly Ser Glu Glu Ala His Gln Lys
       50                  55                  60
   ttc att gaa gtc ggc gag gca tac gat gta ttg agc gat cct gaa aag      240
   Phe Ile Glu Val Gly Glu Ala Tyr Asp Val Leu Ser Asp Pro Glu Lys
   65                  70                  75                  80
   aaa aag att tat gac cag ttt ggt gca gat gct gta aag aat ggc ggt      288
   Lys Lys Ile Tyr Asp Gln Phe Gly Ala Asp Ala Val Lys Asn Gly Gly
                   85                  90                  95
   ggc ggt ggc ggt cca gga ggc cct ggc gca ggt gga ttc cac gat ccg      336
   Gly Gly Gly Gly Pro Gly Gly Pro Gly Ala Gly Gly Phe His Asp Pro
               100                 105                 110
   ttt gac ata ttc gaa cgg atg ttt caa gga ggt cat gga ggt cct ggc      384
   Phe Asp Ile Phe Glu Arg Met Phe Gln Gly Gly His Gly Gly Pro Gly
           115                 120                 125
   ggc gga ttt ggc cag aga cag agg cag cgt ggt cca atg atc aag gtc      432
   Gly Gly Phe Gly Gln Arg Gln Arg Gln Arg Gly Pro Met Ile Lys Val
       130                 135                 140
   cag gaa aaa cta tct tta aag cag ttt tat tcc ggg tcc tcg ata gaa      480
   Gln Glu Lys Leu Ser Leu Lys Gln Phe Tyr Ser Gly Ser Ser Ile Glu
   145                 150                 155                 160
   ttt act tta aac cta aac gat gaa tgt gat gca tgc cat ggt agt ggc      528
   Phe Thr Leu Asn Leu Asn Asp Glu Cys Asp Ala Cys His Gly Ser Gly
                   165                 170                 175
   tct gca gat ggt aag ctg gcc caa tgt ccc gat tgt caa ggt cgt ggg      576
   Ser Ala Asp Gly Lys Leu Ala Gln Cys Pro Asp Cys Gln Gly Arg Gly
               180                 185                 190
   gtt ata ata caa gtg ctg cgc atg ggt att atg acg cag cag att caa      624
   Val Ile Ile Gln Val Leu Arg Met Gly Ile Met Thr Gln Gln Ile Gln
           195                 200                 205
   cag atg tgt ggt agg tgt ggt ggt acg gga caa att atc aaa aat gaa      672
   Gln Met Cys Gly Arg Cys Gly Gly Thr Gly Gln Ile Ile Lys Asn Glu
       210                 215                 220
   tgc aaa aca tgt cac ggc aaa aaa gtt acc aaa aag aac aag ttc ttc      720
   Cys Lys Thr Cys His Gly Lys Lys Val Thr Lys Lys Asn Lys Phe Phe
   225                 230                 235                 240
   cac gtt gac gtt cca cca ggc gca cca aga aac tac atg gac aca aga      768
   His Val Asp Val Pro Pro Gly Ala Pro Arg Asn Tyr Met Asp Thr Arg
                   245                 250                 255
   gtc ggc gag gct gaa aaa ggg cct gac ttt gac gcc ggt gac ttg gtc      816
   Val Gly Glu Ala Glu Lys Gly Pro Asp Phe Asp Ala Gly Asp Leu Val
               260                 265                 270
   ata gaa ttc aag gaa aag gat act gag aac atg ggt tac aga aga aga      864
   Ile Glu Phe Lys Glu Lys Asp Thr Glu Asn Met Gly Tyr Arg Arg Arg
           275                 280                 285
   ggc gac aat ctg tac aga aca gaa gtt ctt tct gct gcg gaa gcg cta      912
   Gly Asp Asn Leu Tyr Arg Thr Glu Val Leu Ser Ala Ala Glu Ala Leu
       290                 295                 300
   tac ggc gga tgg caa aga acg ata gaa ttc ctt gat gag aac aag ccc      960
   Tyr Gly Gly Trp Gln Arg Thr Ile Glu Phe Leu Asp Glu Asn Lys Pro
   305                 310                 315                 320
   gtt aag tta tct aga ccc gct cat gta gtt gtc tcc aat ggc gaa gtt     1008
   Val Lys Leu Ser Arg Pro Ala His Val Val Val Ser Asn Gly Glu Val
                   325                 330                 335
   gaa gtc gtg aag gga ttc ggc atg ccc aag ggt agc aag ggt tac ggt     1056
   Glu Val Val Lys Gly Phe Gly Met Pro Lys Gly Ser Lys Gly Tyr Gly
               340                 345                 350
   gat ttg tac ata gac tac gtc gtt gtc atg cca aag act ttc aaa tct     1104
   Asp Leu Tyr Ile Asp Tyr Val Val Val Met Pro Lys Thr Phe Lys Ser
           355                 360                 365
   ggg caa aat atg ctc aaa gat gag ttg tag                             1134
   Gly Gln Asn Met Leu Lys Asp Glu Leu
       370                 375
<210> 54
   <211> 377
   <212> PRT
   <213> Saccharomyces cerevisiae
<400> 54
   Met Ile Pro Lys Leu Tyr Ile His Leu Ile Leu Ser Leu Leu Leu Leu
   1               5                   10                  15
   Pro Leu Ile Leu Ala Gln Asp Tyr Tyr Ala Ile Leu Glu Ile Asp Lys
               20                  25                  30
   Asp Ala Thr Glu Lys Glu Ile Lys Ser Ala Tyr Arg Gln Leu Ser Lys
           35                  40                  45
   Lys Tyr His Pro Asp Lys Asn Ala Gly Ser Glu Glu Ala His Gln Lys
       50                  55                  60
   Phe Ile Glu Val Gly Glu Ala Tyr Asp Val Leu Ser Asp Pro Glu Lys
   65                  70                  75                  80
   Lys Lys Ile Tyr Asp Gln Phe Gly Ala Asp Ala Val Lys Asn Gly Gly
                   85                  90                  95
   Gly Gly Gly Gly Pro Gly Gly Pro Gly Ala Gly Gly Phe His Asp Pro
               100                 105                 110
   Phe Asp Ile Phe Glu Arg Met Phe Gln Gly Gly His Gly Gly Pro Gly
           115                 120                 125
   Gly Gly Phe Gly Gln Arg Gln Arg Gln Arg Gly Pro Met Ile Lys Val
       130                 135                 140
   Gln Glu Lys Leu Ser Leu Lys Gln Phe Tyr Ser Gly Ser Ser Ile Glu
   145                 150                 155                 160
   Phe Thr Leu Asn Leu Asn Asp Glu Cys Asp Ala Cys His Gly Ser Gly
                   165                 170                 175
   Ser Ala Asp Gly Lys Leu Ala Gln Cys Pro Asp Cys Gln Gly Arg Gly
               180                 185                 190
   Val Ile Ile Gln Val Leu Arg Met Gly Ile Met Thr Gln Gln Ile Gln
           195                 200                 205
   Gln Met Cys Gly Arg Cys Gly Gly Thr Gly Gln Ile Ile Lys Asn Glu
       210                 215                 220
   Cys Lys Thr Cys His Gly Lys Lys Val Thr Lys Lys Asn Lys Phe Phe
   225                 230                 235                 240
   His Val Asp Val Pro Pro Gly Ala Pro Arg Asn Tyr Met Asp Thr Arg
                   245                 250                 255
   Val Gly Glu Ala Glu Lys Gly Pro Asp Phe Asp Ala Gly Asp Leu Val
               260                 265                 270
   Ile Glu Phe Lys Glu Lys Asp Thr Glu Asn Met Gly Tyr Arg Arg Arg
           275                 280                 285
   Gly Asp Asn Leu Tyr Arg Thr Glu Val Leu Ser Ala Ala Glu Ala Leu
       290                 295                 300
   Tyr Gly Gly Trp Gln Arg Thr Ile Glu Phe Leu Asp Glu Asn Lys Pro
   305                 310                 315                 320
   Val Lys Leu Ser Arg Pro Ala His Val Val Val Ser Asn Gly Glu Val
                   325                 330                 335
   Glu Val Val Lys Gly Phe Gly Met Pro Lys Gly Ser Lys Gly Tyr Gly
               340                 345                 350
   Asp Leu Tyr Ile Asp Tyr Val Val Val Met Pro Lys Thr Phe Lys Ser
           355                 360                 365
   Gly Gln Asn Met Leu Lys Asp Glu Leu
       370                 375
<210> 55
   <211> 1692
   <212> DNA
   <213> Saccharomyces cerevisiae
<220>
   <221> CDS
   <222> (1)..(1692)
<400> 55
   atg aga tta aga acc gcc att gcc aca ctg tgc ctc acg gct ttt aca      48
   Met Arg Leu Arg Thr Ala Ile Ala Thr Leu Cys Leu Thr Ala Phe Thr
   1               5                   10                  15
   tct gca act tca aac aat agc tac atc gcc acc gac caa aca caa aat      96
   Ser Ala Thr Ser Asn Asn Ser Tyr Ile Ala Thr Asp Gln Thr Gln Asn
               20                  25                  30
   gcc ttt aat gac act cac ttt tgt aag gtc gac agg aat gat cac gtt     144
   Ala Phe Asn Asp Thr His Phe Cys Lys Val Asp Arg Asn Asp His Val
           35                  40                  45
   agt ccc agt tgt aac gta aca ttc aat gaa tta aat gcc ata aat gaa     192
   Ser Pro Ser Cys Asn Val Thr Phe Asn Glu Leu Asn Ala Ile Asn Glu
       50                  55                  60
   aac att aga gat gat ctt tcg gcg tta tta aaa tct gat ttc ttc aaa     240
   Asn Ile Arg Asp Asp Leu Ser Ala Leu Leu Lys Ser Asp Phe Phe Lys
   65                  70                  75                  80
   tac ttt cgg ctg gat tta tac aag caa tgt tca ttt tgg gac gcc aac     288
   Tyr Phe Arg Leu Asp Leu Tyr Lys Gln Cys Ser Phe Trp Asp Ala Asn
                   85                  90                  95
   gat ggt ctg tgc tta aac cgc gct tgc tct gtt gat gtc gta gag gac     336
   Asp Gly Leu Cys Leu Asn Arg Ala Cys Ser Val Asp Val Val Glu Asp
               100                 105                 110
   tgg gat aca ctg cct gag tac tgg cag cct gag atc ttg ggt agt ttc     384
   Trp Asp Thr Leu Pro Glu Tyr Trp Gln Pro Glu Ile Leu Gly Ser Phe
           115                 120                 125
   aat aat gat aca atg aag gaa gcg gat gat agc gat gac gaa tgt aag     432
   Asn Asn Asp Thr Met Lys Glu Ala Asp Asp Ser Asp Asp Glu Cys Lys
       130                 135                 140
   ttc tta gat caa cta tgt caa acc agt aaa aaa cct gta gat atc gaa     480
   Phe Leu Asp Gln Leu Cys Gln Thr Ser Lys Lys Pro Val Asp Ile Glu
   145                 150                 155                 160
   gac acc atc aac tac tgt gat gta aat gac ttt aac ggt aaa aac gcc     528
   Asp Thr Ile Asn Tyr Cys Asp Val Asn Asp Phe Asn Gly Lys Asn Ala
                   165                 170                 175
   gtt ctg att gat tta aca gca aat ccg gaa cga ttt aca ggt tat ggt     576
   Val Leu Ile Asp Leu Thr Ala Asn Pro Glu Arg Phe Thr Gly Tyr Gly
               180                 185                 190
   ggt aag caa gct ggt caa att tgg tct act atc tac caa gac aac tgt     624
   Gly Lys Gln Ala Gly Gln Ile Trp Ser Thr Ile Tyr Gln Asp Asn Cys
           195                 200                 205
   ttt aca att ggc gaa act ggt gaa tca ttg gcc aaa gat gca ttt tat     672
   Phe Thr Ile Gly Glu Thr Gly Glu Ser Leu Ala Lys Asp Ala Phe Tyr
       210                 215                 220
   aga ctt gta tcc ggt ttc cat gcc tct atc ggt act cac tta tca aag      720
   Arg Leu Val Ser Gly Phe His Ala Ser Ile Gly Thr His Leu Ser Lys
   225                 230                 235                 240
   gaa tat ttg aac acg aaa act ggt aaa tgg gag ccc aat ctg gat ttg      768
   Glu Tyr Leu Asn Thr Lys Thr Gly Lys Trp Glu Pro Asn Leu Asp Leu
                   245                 250                 255
   ttt atg gca aga atc ggg aac ttt cct gat aga gtg aca aac atg tat      816
   Phe Met Ala Arg Ile Gly Asn Phe Pro Asp Arg Val Thr Asn Met Tyr
               260                 265                 270
   ttc aat tat gct gtt gta gct aag gct ctc tgg aaa att caa cca tat      864
   Phe Asn Tyr Ala Val Val Ala Lys Ala Leu Trp Lys Ile Gln Pro Tyr
           275                 280                 285
   tta cca gaa ttt tca ttc tgt gat cta gtc aat aaa gaa atc aaa aac      912
   Leu Pro Glu Phe Ser Phe Cys Asp Leu Val Asn Lys Glu Ile Lys Asn
       290                 295                 300
   aaa atg gat aac gtt att tcc cag ctg gac aca aaa att ttt aac gaa      960
   Lys Met Asp Asn Val Ile Ser Gln Leu Asp Thr Lys Ile Phe Asn Glu
   305                 310                 315                 320
   gac tta gtt ttt gcc aac gac cta agt ttg act ttg aag gac gaa ttc     1008
   Asp Leu Val Phe Ala Asn Asp Leu Ser Leu Thr Leu Lys Asp Glu Phe
                   325                 330                 335
   aga tct cgc ttc aag aat gtc acg aag att atg gat tgt gtg caa tgt     1056
   Arg Ser Arg Phe Lys Asn Val Thr Lys Ile Met Asp Cys Val Gln Cys
               340                 345                 350
   gat aga tgt aga ttg tgg ggc aaa att caa act acc ggt tac gca act     1104
   Asp Arg Cys Arg Leu Trp Gly Lys Ile Gln Thr Thr Gly Tyr Ala Thr
           355                 360                 365
   gcc ttg aaa att ttg ttt gaa atc aac gac gct gat gaa ttc acc aaa     1152
   Ala Leu Lys Ile Leu Phe Glu Ile Asn Asp Ala Asp Glu Phe Thr Lys
       370                 375                 380
   caa cat att gtt ggt aag tta acc aaa tat gag ttg att gca cta tta     1200
   Gln His Ile Val Gly Lys Leu Thr Lys Tyr Glu Leu Ile Ala Leu Leu
   385                 390                 395                 400
   cag act ttc ggt aga tta tct gaa tct att gaa tct gtt aac atg ttc     1248
   Gln Thr Phe Gly Arg Leu Ser Glu Ser Ile Glu Ser Val Asn Met Phe
                   405                 410                 415
   gaa aaa atg tac ggg aaa agg tta aac ggt tct gaa aac agg tta agc     1296
   Glu Lys Met Tyr Gly Lys Arg Leu Asn Gly Ser Glu Asn Arg Leu Ser
               420                 425                 430
   tca ttc ttc caa aat aac ttc ttc aac att ttg aag gag gca ggc aaa     1344
   Ser Phe Phe Gln Asn Asn Phe Phe Asn Ile Leu Lys Glu Ala Gly Lys
           435                 440                 445
   tcg att cgt tac acc ata gag aac atc aat tcc act aaa gaa gga aag     1392
   Ser Ile Arg Tyr Thr Ile Glu Asn Ile Asn Ser Thr Lys Glu Gly Lys
       450                 455                 460
   aaa aag act aac aat tct caa tca cat gta ttt gat gat tta aaa atg     1440
   Lys Lys Thr Asn Asn Ser Gln Ser His Val Phe Asp Asp Leu Lys Met
   465                 470                 475                 480
   ccc aaa gca gaa ata gtt cca agg ccc tct aac ggt aca gta aat aaa     1488
   Pro Lys Ala Glu Ile Val Pro Arg Pro Ser Asn Gly Thr Val Asn Lys
                   485                 490                 495
   tgg aag aaa gct tgg aat act gaa gtt aac aac gtt tta gaa gca ttc     1536
   Trp Lys Lys Ala Trp Asn Thr Glu Val Asn Asn Val Leu Glu Ala Phe
               500                 505                 510
   aga ttt att tat aga agc tat ttg gat tta ccc agg aac atc tgg gaa     1584
   Arg Phe Ile Tyr Arg Ser Tyr Leu Asp Leu Pro Arg Asn Ile Trp Glu
           515                 520                 525
   tta tct ttg atg aag gta tac aaa ttt tgg aat aaa ttc atc ggt gtt     1632
   Leu Ser Leu Met Lys Val Tyr Lys Phe Trp Asn Lys Phe Ile Gly Val
       530                 535                 540
   gct gat tac gtt agt gag gag aca cga gag cct att tcc tat aag cta     1680
   Ala Asp Tyr Val Ser Glu Glu Thr Arg Glu Pro Ile Ser Tyr Lys Leu
   545                 550                 555                 560
   gat ata caa taa                                                     1692
   Asp Ile Gln
<210> 56
   <211> 563
   <212> PRT
   <213> Saccharomyces cerevisiae
<400> 56
   Met Arg Leu Arg Thr Ala Ile Ala Thr Leu Cys Leu Thr Ala Phe Thr
   1               5                   10                  15
   Ser Ala Thr Ser Asn Asn Ser Tyr Ile Ala Thr Asp Gln Thr Gln Asn
               20                  25                  30
   Ala Phe Asn Asp Thr His Phe Cys Lys Val Asp Arg Asn Asp His Val
           35                  40                  45
   Ser Pro Ser Cys Asn Val Thr Phe Asn Glu Leu Asn Ala Ile Asn Glu
       50                  55                  60
   Asn Ile Arg Asp Asp Leu Ser Ala Leu Leu Lys Ser Asp Phe Phe Lys
   65                  70                  75                  80
   Tyr Phe Arg Leu Asp Leu Tyr Lys Gln Cys Ser Phe Trp Asp Ala Asn
                   85                  90                  95
   Asp Gly Leu Cys Leu Asn Arg Ala Cys Ser Val Asp Val Val Glu Asp
               100                 105                 110
   Trp Asp Thr Leu Pro Glu Tyr Trp Gln Pro Glu Ile Leu Gly Ser Phe
   115 120 125
   Asn Asn Asp Thr Met Lys Glu Ala Asp Asp Ser Asp Asp Glu Cys Lys
   130 135 140
   Phe Leu Asp Gln Leu Cys Gln Thr Ser Lys Lys Pro Val Asp Ile Glu
   145 150 155 160
   Asp Thr Ile Asn Tyr Cys Asp Val Asn Asp Phe Asn Gly Lys Asn Ala
   165 170 175
   Val Leu Ile Asp Leu Thr Ala Asn Pro Glu Arg Phe Thr Gly Tyr Gly
   180 185 190
   Gly Lys Gln Ala Gly Gln Ile Trp Ser Thr Ile Tyr Gln Asp Asn Cys
   195 200 205
   Phe Thr Ile Gly Glu Thr Gly Glu Ser Leu Ala Lys Asp Ala Phe Tyr
   210 215 220
   Arg Leu Val Ser Gly Phe His Ala Ser Ile Gly Thr His Leu Ser Lys
   225 230 235 240
   Glu Tyr Leu Asn Thr Lys Thr Gly Lys Trp Glu Pro Asn Leu Asp Leu
   245 250 255
   Phe Met Ala Arg Ile Gly Asn Phe Pro Asp Arg Val Thr Asn Met Tyr
   260 265 270
   Phe Asn Tyr Ala Val Val Ala Lys Ala Leu Trp Lys Ile Gln Pro Tyr
   275 280 285
   Leu Pro Glu Phe Ser Phe Cys Asp Leu Val Asn Lys Glu Ile Lys Asn
   290 295 300
   Lys Met Asp Asn Val Ile Ser Gln Leu Asp Thr Lys Ile Phe Asn Glu
   305 310 315 320
   Asp Leu Val Phe Ala Asn Asp Leu Ser Leu Thr Leu Lys Asp Glu Phe
   325 330 335
   Arg Ser Arg Phe Lys Asn Val Thr Lys Ile Met Asp Cys Val Gln Cys
   340 345 350
   Asp Arg Cys Arg Leu Trp Gly Lys Ile Gln Thr Thr Gly Tyr Ala Thr
   355 360 365
   Ala Leu Lys Ile Leu Phe Glu Ile Asn Asp Ala Asp Glu Phe Thr Lys
   370 375 380
   Gln His Ile Val Gly Lys Leu Thr Lys Tyr Glu Leu Ile Ala Leu Leu
   385 390 395 400
   Gln Thr Phe Gly Arg Leu Ser Glu Ser Ile Glu Ser Val Asn Met Phe
   405 410 415
   Glu Lys Met Tyr Gly Lys Arg Leu Asn Gly Ser Glu Asn Arg Leu Ser
   420 425 430
   Ser Phe Phe Gln Asn Asn Phe Phe Asn Ile Leu Lys Glu Ala Gly Lys
   435 440 445
   Ser Ile Arg Tyr Thr Ile Glu Asn Ile Asn Ser Thr Lys Glu Gly Lys
   450 455 460
   Lys Lys Thr Asn Asn Ser Gln Ser His Val Phe Asp Asp Leu Lys Met
   465 470 475 480
   Pro Lys Ala Glu Ile Val Pro Arg Pro Ser Asn Gly Thr Val Asn Lys
   485 490 495
   Trp Lys Lys Ala Trp Asn Thr Glu Val Asn Asn Val Leu Glu Ala Phe
   500 505 510
   Arg Phe Ile Tyr Arg Ser Tyr Leu Asp Leu Pro Arg Asn Ile Trp Glu
   515 520 525
   Leu Ser Leu Met Lys Val Tyr Lys Phe Trp Asn Lys Phe Ile Gly Val
   530 535 540
   Ala Asp Tyr Val Ser Glu Glu Thr Arg Glu Pro Ile Ser Tyr Lys Leu
   545 550 555 560
   Asp Ile Gln
<210> 57
   <211> 408
   <212> DNA
   <213> Saccharomyces cerevisiae
<220>
   <221> CDS
   <222> (1)..(408)
<400> 57
   atg atg ttt aat att tac ctt ttc gtc act ttt ttt tcc acc att ctt        48
   Met Met Phe Asn Ile Tyr Leu Phe Val Thr Phe Phe Ser Thr Ile Leu
   1               5                   10                  15
   gca ggt tcc ctg tca gat ttg gaa atc ggt att atc aag aga ata ccg        96
   Ala Gly Ser Leu Ser Asp Leu Glu Ile Gly Ile Ile Lys Arg Ile Pro
               20                  25                  30
   gta gaa gat tgc tta att aag gca atg cca ggt gat aaa gtt aag gtt       144
   Val Glu Asp Cys Leu Ile Lys Ala Met Pro Gly Asp Lys Val Lys Val
           35                  40                  45
   cat tat aca gga tct tta tta gaa tcg gga act gta ttt gac tca agt       192
   His Tyr Thr Gly Ser Leu Leu Glu Ser Gly Thr Val Phe Asp Ser Ser
       50                  55                  60
   tat tca aga ggc tct cct atc gct ttt gaa ctt ggc gtt ggc aga gta       240
   Tyr Ser Arg Gly Ser Pro Ile Ala Phe Glu Leu Gly Val Gly Arg Val
   65                  70                  75                  80
   att aaa ggt tgg gat caa ggt gtt gcc ggc atg tgc gtt ggc gaa aaa       288
   Ile Lys Gly Trp Asp Gln Gly Val Ala Gly Met Cys Val Gly Glu Lys
                   85                  90                  95
   aga aag ctg caa att cca agt tct ttg gcc tac gga gaa aga ggt gtc       336
   Arg Lys Leu Gln Ile Pro Ser Ser Leu Ala Tyr Gly Glu Arg Gly Val
               100                 105                 110
   cca ggc gtc att cct cca agt gct gat ttg gtg ttt gat gtc gaa ttg       384
   Pro Gly Val Ile Pro Pro Ser Ala Asp Leu Val Phe Asp Val Glu Leu
           115                 120                 125
   gta gac gtg aaa tca gcc gcc tag                                       408
   Val Asp Val Lys Ser Ala Ala
       130                 135
<210> 58
   <211> 135
   <212> PRT
   <213> Saccharomyces cerevisiae
<400> 58
   Met Met Phe Asn Ile Tyr Leu Phe Val Thr Phe Phe Ser Thr Ile Leu
   1               5                   10                  15
   Ala Gly Ser Leu Ser Asp Leu Glu Ile Gly Ile Ile Lys Arg Ile Pro
               20                  25                  30
   Val Glu Asp Cys Leu Ile Lys Ala Met Pro Gly Asp Lys Val Lys Val
           35                  40                  45
   His Tyr Thr Gly Ser Leu Leu Glu Ser Gly Thr Val Phe Asp Ser Ser
       50                  55                  60
   Tyr Ser Arg Gly Ser Pro Ile Ala Phe Glu Leu Gly Val Gly Arg Val
   65                  70                  75                  80
   Ile Lys Gly Trp Asp Gln Gly Val Ala Gly Met Cys Val Gly Glu Lys
                   85                  90                  95
   Arg Lys Leu Gln Ile Pro Ser Ser Leu Ala Tyr Gly Glu Arg Gly Val
               100                 105                 110
   Pro Gly Val Ile Pro Pro Ser Ala Asp Leu Val Phe Asp Val Glu Leu
           115                 120                 125
   Val Asp Val Lys Ser Ala Ala
       130                 135
<210> 59
   <211> 1938
   <212> DNA
   <213> Saccharomyces cerevisiae
<220>
   <221> CDS
   <222> (1)..(1938)
<400> 59
   atg ata ctg atc tcg gga tac tgt ctt tta gtg tat agc gtt att ttg      48
   Met Ile Leu Ile Ser Gly Tyr Cys Leu Leu Val Tyr Ser Val Ile Leu
   1               5                   10                  15
   cca gta ctg ata tcg gct tct aag tta tgt gat ttg gct gag tta caa      96
   Pro Val Leu Ile Ser Ala Ser Lys Leu Cys Asp Leu Ala Glu Leu Gln
               20                  25                  30
   cga ttg aac aag aat tta aaa gta gac act gaa tcc ttg cca aaa tac     144
   Arg Leu Asn Lys Asn Leu Lys Val Asp Thr Glu Ser Leu Pro Lys Tyr
           35                  40                  45
   caa tgg atc gct ggg cag ttg gaa caa aac tgc atg act gcg gat cca     192
   Gln Trp Ile Ala Gly Gln Leu Glu Gln Asn Cys Met Thr Ala Asp Pro
       50                  55                  60
   gca agt gaa aat atg tca gac gta att caa cta gcc aat caa ata tac     240
   Ala Ser Glu Asn Met Ser Asp Val Ile Gln Leu Ala Asn Gln Ile Tyr
   65                  70                  75                  80
   tac aaa att ggg ctg atc caa tta tcc aac gat caa cat cta aga gct     288
   Tyr Lys Ile Gly Leu Ile Gln Leu Ser Asn Asp Gln His Leu Arg Ala
                   85                  90                  95
   att aac aca ttt gaa aaa atc gtt ttt aat gaa act tac aaa ggt tct     336
   Ile Asn Thr Phe Glu Lys Ile Val Phe Asn Glu Thr Tyr Lys Gly Ser
               100                 105                 110
   ttt ggg aag ctg gcg gaa aag agg cta caa gag ctg tat gtc gat ttt     384
   Phe Gly Lys Leu Ala Glu Lys Arg Leu Gln Glu Leu Tyr Val Asp Phe
           115                 120                 125
   ggg atg tgg gac aag gtg cat cag aag gat gat cag tat gcg aaa tat     432
   Gly Met Trp Asp Lys Val His Gln Lys Asp Asp Gln Tyr Ala Lys Tyr
       130                 135                 140
   ctg tcc ttg aat gaa acc atc aga aac aaa ata tca tcc aaa gac gtt      480
   Leu Ser Leu Asn Glu Thr Ile Arg Asn Lys Ile Ser Ser Lys Asp Val
   145                 150                 155                 160
   tct gtg gag gaa gat att tct gag ctg cta cgc ata acg ccg tac gat      528
   Ser Val Glu Glu Asp Ile Ser Glu Leu Leu Arg Ile Thr Pro Tyr Asp
                   165                 170                 175
   gtt aac gtc ctc tcc acg cac atc gat gtt ctt ttt cac aaa cta gct      576
   Val Asn Val Leu Ser Thr His Ile Asp Val Leu Phe His Lys Leu Ala
               180                 185                 190
   gaa gaa att gac gtt tcg tta gct gct gct atc att ttg gat tac gaa      624
   Glu Glu Ile Asp Val Ser Leu Ala Ala Ala Ile Ile Leu Asp Tyr Glu
           195                 200                 205
   aca atc ctc gac aag cat ttg gct agc tta agc ata gat aca aga ctt      672
   Thr Ile Leu Asp Lys His Leu Ala Ser Leu Ser Ile Asp Thr Arg Leu
       210                 215                 220
   tcg att cat tat gtc ata tct gtt tta cag acc ttt gta ctt aac tca      720
   Ser Ile His Tyr Val Ile Ser Val Leu Gln Thr Phe Val Leu Asn Ser
   225                 230                 235                 240
   gat gcg tcg ttc aat ata aga aaa tgc ctt tcc att gat atg gac tat      768
   Asp Ala Ser Phe Asn Ile Arg Lys Cys Leu Ser Ile Asp Met Asp Tyr
                   245                 250                 255
   gat aaa tgt aaa aaa cta agc ctg act att tcc aaa ttg aac aag gtg      816
   Asp Lys Cys Lys Lys Leu Ser Leu Thr Ile Ser Lys Leu Asn Lys Val
               260                 265                 270
   aat cca tca aaa aga cag atc ctg gat cca gca aca tat gca ttt gag      864
   Asn Pro Ser Lys Arg Gln Ile Leu Asp Pro Ala Thr Tyr Ala Phe Glu
           275                 280                 285
   aac aaa aag ttt aga agt tgg gat aga att att gaa ttt tat ttg aag      912
   Asn Lys Lys Phe Arg Ser Trp Asp Arg Ile Ile Glu Phe Tyr Leu Lys
       290                 295                 300
   gat aag aag cca ttt att aca cca atg aaa att ctt aac aaa gat aca      960
   Asp Lys Lys Pro Phe Ile Thr Pro Met Lys Ile Leu Asn Lys Asp Thr
   305                 310                 315                 320
   aac ttt aaa aac aac tac ttc ttt tta gag gaa att atc aaa caa ttg     1008
   Asn Phe Lys Asn Asn Tyr Phe Phe Leu Glu Glu Ile Ile Lys Gln Leu
                   325                 330                 335
   ata gaa gac gtt caa ctg tcg aga cct ttg gca aaa aat tta ttc gaa     1056
   Ile Glu Asp Val Gln Leu Ser Arg Pro Leu Ala Lys Asn Leu Phe Glu
               340                 345                 350
   gat ccc cca ata acc gat ggt ttt gtc aaa cca aaa tca tac tat cat     1104
   Asp Pro Pro Ile Thr Asp Gly Phe Val Lys Pro Lys Ser Tyr Tyr His
           355                 360                 365
   acc gat tat cta gta tac att gat tcc att ctt tgt cag gct tct agc     1152
   Thr Asp Tyr Leu Val Tyr Ile Asp Ser Ile Leu Cys Gln Ala Ser Ser
       370                 375                 380
   atg agt ccg gac gtc aag aga gct aaa ctg gct gcg ccg ttc tgt aaa     1200
   Met Ser Pro Asp Val Lys Arg Ala Lys Leu Ala Ala Pro Phe Cys Lys
   385                 390                 395                 400
   aag agt ttg agg cat tca cta aca cta gaa aca tgg aaa cac tat cag     1248
   Lys Ser Leu Arg His Ser Leu Thr Leu Glu Thr Trp Lys His Tyr Gln
                   405                 410                 415
   gat gct aag tcc gag caa aaa cct tta cct gag acg gta ttg agt gat     1296
   Asp Ala Lys Ser Glu Gln Lys Pro Leu Pro Glu Thr Val Leu Ser Asp
               420                 425                 430
   gta tgg aat tcc aat cct cat ttg ctg atg tat atg gta aac tca ata     1344
   Val Trp Asn Ser Asn Pro His Leu Leu Met Tyr Met Val Asn Ser Ile
           435                 440                 445
   ctt aat aaa agt agg tct aaa cct cat tca cag ttc aaa aag caa tta     1392
   Leu Asn Lys Ser Arg Ser Lys Pro His Ser Gln Phe Lys Lys Gln Leu
       450                 455                 460
   tat gac cag ata aac aaa ttt ttc caa gat aac ggc ctc tca gag tcg     1440
   Tyr Asp Gln Ile Asn Lys Phe Phe Gln Asp Asn Gly Leu Ser Glu Ser
   465                 470                 475                 480
   acc aat cca tac gtg atg aag aac ttc cga tta tta cag aaa caa tta     1488
   Thr Asn Pro Tyr Val Met Lys Asn Phe Arg Leu Leu Gln Lys Gln Leu
                   485                 490                 495
   caa acc tat aaa gag cat aaa cat cgg aat ttc aac cag caa tat ttc     1536
   Gln Thr Tyr Lys Glu His Lys His Arg Asn Phe Asn Gln Gln Tyr Phe
               500                 505                 510
   caa caa caa caa cag cag caa caa cac caa cga cat caa gca ccc cca     1584
   Gln Gln Gln Gln Gln Gln Gln Gln His Gln Arg His Gln Ala Pro Pro
           515                 520                 525
   gca gcg cct aac tac gac cca aaa aag gac tat tat aaa att ctt gga     1632
   Ala Ala Pro Asn Tyr Asp Pro Lys Lys Asp Tyr Tyr Lys Ile Leu Gly
       530                 535                 540
   gta tcg cct agt gct agt tcg aaa gaa ata agg aaa gca tat tta aat     1680
   Val Ser Pro Ser Ala Ser Ser Lys Glu Ile Arg Lys Ala Tyr Leu Asn
   545                 550                 555                 560
   tta acc aaa aaa tac cac cca gac aaa ata aag gcc aac cat aac gac     1728
   Leu Thr Lys Lys Tyr His Pro Asp Lys Ile Lys Ala Asn His Asn Asp
                   565                 570                 575
   aaa caa gaa tca att cac gaa act atg tca caa atc aat gaa gcg tac     1776
   Lys Gln Glu Ser Ile His Glu Thr Met Ser Gln Ile Asn Glu Ala Tyr
               580                 585                 590
   gaa aca tta agt gat gac gat aaa agg aag gaa tac gat ctt tcc aga     1824
   Glu Thr Leu Ser Asp Asp Asp Lys Arg Lys Glu Tyr Asp Leu Ser Arg
           595                 600                 605
   tca aac ccc cgc cgc aac act ttt cct cag ggg cct agg caa aat aac     1872
   Ser Asn Pro Arg Arg Asn Thr Phe Pro Gln Gly Pro Arg Gln Asn Asn
       610                 615                 620
   atg ttc aaa aat cca gga agt ggc ttc cca ttc gga aat ggc ttt aaa     1920
   Met Phe Lys Asn Pro Gly Ser Gly Phe Pro Phe Gly Asn Gly Phe Lys
   625                 630                 635                 640
   atg aat ttt ggg ctt tga                                          1938
   Met Asn Phe Gly Leu
                   645
<210> 60
   <211> 645
   <212> PRT
   <213> Saccharomyces cerevisiae
<400> 60
   Met Ile Leu Ile Ser Gly Tyr Cys Leu Leu Val Tyr Ser Val Ile Leu
   1               5                   10                  15
   Pro Val Leu Ile Ser Ala Ser Lys Leu Cys Asp Leu Ala Glu Leu Gln
               20                  25                  30
   Arg Leu Asn Lys Asn Leu Lys Val Asp Thr Glu Ser Leu Pro Lys Tyr
           35                  40                  45
   Gln Trp Ile Ala Gly Gln Leu Glu Gln Asn Cys Met Thr Ala Asp Pro
       50                  55                  60
   Ala Ser Glu Asn Met Ser Asp Val Ile Gln Leu Ala Asn Gln Ile Tyr
   65                  70                  75                  80
   Tyr Lys Ile Gly Leu Ile Gln Leu Ser Asn Asp Gln His Leu Arg Ala
                   85                  90                  95
   Ile Asn Thr Phe Glu Lys Ile Val Phe Asn Glu Thr Tyr Lys Gly Ser
               100                 105                 110
   Phe Gly Lys Leu Ala Glu Lys Arg Leu Gln Glu Leu Tyr Val Asp Phe
           115                 120                 125
   Gly Met Trp Asp Lys Val His Gln Lys Asp Asp Gln Tyr Ala Lys Tyr
       130                 135                 140
   Leu Ser Leu Asn Glu Thr Ile Arg Asn Lys Ile Ser Ser Lys Asp Val
   145                 150                 155                 160
   Ser Val Glu Glu Asp Ile Ser Glu Leu Leu Arg Ile Thr Pro Tyr Asp
                   165                 170                 175
   Val Asn Val Leu Ser Thr His Ile Asp Val Leu Phe His Lys Leu Ala
               180                 185                 190
   Glu Glu Ile Asp Val Ser Leu Ala Ala Ala Ile Ile Leu Asp Tyr Glu
           195                 200                 205
   Thr Ile Leu Asp Lys His Leu Ala Ser Leu Ser Ile Asp Thr Arg Leu
       210                 215                 220
   Ser Ile His Tyr Val Ile Ser Val Leu Gln Thr Phe Val Leu Asn Ser
   225                 230                 235                 240
   Asp Ala Ser Phe Asn Ile Arg Lys Cys Leu Ser Ile Asp Met Asp Tyr
                   245                 250                 255
   Asp Lys Cys Lys Lys Leu Ser Leu Thr Ile Ser Lys Leu Asn Lys Val
               260                 265                 270
   Asn Pro Ser Lys Arg Gln Ile Leu Asp Pro Ala Thr Tyr Ala Phe Glu
           275                 280                 285
   Asn Lys Lys Phe Arg Ser Trp Asp Arg Ile Ile Glu Phe Tyr Leu Lys
       290                 295                 300
   Asp Lys Lys Pro Phe Ile Thr Pro Met Lys Ile Leu Asn Lys Asp Thr
   305                 310                 315                 320
   Asn Phe Lys Asn Asn Tyr Phe Phe Leu Glu Glu Ile Ile Lys Gln Leu
                   325                 330                 335
   Ile Glu Asp Val Gln Leu Ser Arg Pro Leu Ala Lys Asn Leu Phe Glu
               340                 345                 350
   Asp Pro Pro Ile Thr Asp Gly Phe Val Lys Pro Lys Ser Tyr Tyr His
           355                 360                 365
   Thr Asp Tyr Leu Val Tyr Ile Asp Ser Ile Leu Cys Gln Ala Ser Ser
       370                 375                 380
   Met Ser Pro Asp Val Lys Arg Ala Lys Leu Ala Ala Pro Phe Cys Lys
   385                 390                 395                 400
   Lys Ser Leu Arg His Ser Leu Thr Leu Glu Thr Trp Lys His Tyr Gln
                   405                 410                 415
   Asp Ala Lys Ser Glu Gln Lys Pro Leu Pro Glu Thr Val Leu Ser Asp
               420                 425                 430
   Val Trp Asn Ser Asn Pro His Leu Leu Met Tyr Met Val Asn Ser Ile
           435                 440                 445
   Leu Asn Lys Ser Arg Ser Lys Pro His Ser Gln Phe Lys Lys Gln Leu
       450                 455                 460
   Tyr Asp Gln Ile Asn Lys Phe Phe Gln Asp Asn Gly Leu Ser Glu Ser
   465                 470                 475                 480
   Thr Asn Pro Tyr Val Met Lys Asn Phe Arg Leu Leu Gln Lys Gln Leu
                   485                 490                 495
   Gln Thr Tyr Lys Glu His Lys His Arg Asn Phe Asn Gln Gln Tyr Phe
               500                 505                 510
   Gln Gln Gln Gln Gln Gln Gln Gln His Gln Arg His Gln Ala Pro Pro
           515                 520                 525
   Ala Ala Pro Asn Tyr Asp Pro Lys Lys Asp Tyr Tyr Lys Ile Leu Gly
       530                 535                 540
   Val Ser Pro Ser Ala Ser Ser Lys Glu Ile Arg Lys Ala Tyr Leu Asn
   545                 550                 555                 560
   Leu Thr Lys Lys Tyr His Pro Asp Lys Ile Lys Ala Asn His Asn Asp
                   565                 570                 575
   Lys Gln Glu Ser Ile His Glu Thr Met Ser Gln Ile Asn Glu Ala Tyr
               580                 585                 590
   Glu Thr Leu Ser Asp Asp Asp Lys Arg Lys Glu Tyr Asp Leu Ser Arg
           595                 600                 605
   Ser Asn Pro Arg Arg Asn Thr Phe Pro Gln Gly Pro Arg Gln Asn Asn
       610                 615                 620
   Met Phe Lys Asn Pro Gly Ser Gly Phe Pro Phe Gly Asn Gly Phe Lys
   625                 630                 635                 640
   Met Asn Phe Gly Leu
                   645
<210> 61
   <211> 2646
   <212> DNA
   <213> Saccharomyces cerevisiae
<220>
   <221> CDS
   <222> (1)..(2646)
<400> 61
   atg cga aac gtt tta agg ctt tta ttt tta aca gct ttt gtt gct ata 48
   Met Arg Asn Val Leu Arg Leu Leu Phe Leu Thr Ala Phe Val Ala Ile
   1               5                   10                  15
   ggg tct tta gca gcc gtt tta ggt gtt gat tac ggt cag caa aat atc      96
   Gly Ser Leu Ala Ala Val Leu Gly Val Asp Tyr Gly Gln Gln Asn Ile
               20                  25                  30
   aag gcc att gtg gtt tct ccg caa gcc cca tta gaa ctt gtg ctc aca     144
   Lys Ala Ile Val Val Ser Pro Gln Ala Pro Leu Glu Leu Val Leu Thr
           35                  40                  45
   cca gag gca aaa cgg aag gag ata tct ggt ctt tcg ata aaa aga tta     192
   Pro Glu Ala Lys Arg Lys Glu Ile Ser Gly Leu Ser Ile Lys Arg Leu
       50                  55                  60
   cca ggt tat gga aag gat gat ccg aat ggg att gaa aga atc tac ggt     240
   Pro Gly Tyr Gly Lys Asp Asp Pro Asn Gly Ile Glu Arg Ile Tyr Gly
   65                  70                  75                  80
   tcc gct gtt ggc agt tta gca aca agg ttt ccc caa aac aca ttg ttg     288
   Ser Ala Val Gly Ser Leu Ala Thr Arg Phe Pro Gln Asn Thr Leu Leu
                   85                  90                  95
   cat ttg aaa ccg cta ctt ggg aaa tca cta gaa gat gaa acc act gta     336
   His Leu Lys Pro Leu Leu Gly Lys Ser Leu Glu Asp Glu Thr Thr Val
               100                 105                 110
   act ttg tat tca aaa caa cac ccc ggt tta gaa atg gta tca aca aat     384
   Thr Leu Tyr Ser Lys Gln His Pro Gly Leu Glu Met Val Ser Thr Asn
           115                 120                 125
   aga agt acc ata gcc ttt tta gtt gat aat gtg gaa tat cca ttg gaa     432
   Arg Ser Thr Ile Ala Phe Leu Val Asp Asn Val Glu Tyr Pro Leu Glu
       130                 135                 140
   gag tta gtg gca atg aat gtc caa gag att gcc aat aga gcc aat tca     480
   Glu Leu Val Ala Met Asn Val Gln Glu Ile Ala Asn Arg Ala Asn Ser
   145                 150                 155                 160
   ctg ttg aag gat aga gat gca aga act gag gac ttt gta aac aag atg     528
   Leu Leu Lys Asp Arg Asp Ala Arg Thr Glu Asp Phe Val Asn Lys Met
                   165                 170                 175
   agt ttt aca att cct gac ttt ttt gac caa cat caa agg aaa gca ctt     576
   Ser Phe Thr Ile Pro Asp Phe Phe Asp Gln His Gln Arg Lys Ala Leu
               180                 185                 190
   tta gat gcc agt tca ata acc aca gga atc gaa gag aca tat ctg gtt     624
   Leu Asp Ala Ser Ser Ile Thr Thr Gly Ile Glu Glu Thr Tyr Leu Val
           195                 200                 205
   agt gaa ggg atg tct gtt gca gtt aac ttt gta tta aag cag cgc caa     672
   Ser Glu Gly Met Ser Val Ala Val Asn Phe Val Leu Lys Gln Arg Gln
       210                 215                 220
   ttt cca cca ggt gaa cag cag cat tat atc gta tat gac atg ggg agc     720
   Phe Pro Pro Gly Glu Gln Gln His Tyr Ile Val Tyr Asp Met Gly Ser
   225                 230                 235                 240
   ggt tct att aag gcc tca atg ttc tct ata ttg cag ccg gag gac act     768
   Gly Ser Ile Lys Ala Ser Met Phe Ser Ile Leu Gln Pro Glu Asp Thr
                   245                 250                 255
   act cag ccc gtt aca ata gaa ttt gaa gga tat ggg tat aat cca cat      816
   Thr Gln Pro Val Thr Ile Glu Phe Glu Gly Tyr Gly Tyr Asn Pro His
               260                 265                 270
   cta ggt ggt gca aag ttt aca atg gat att ggc agt ttg ata gag aat      864
   Leu Gly Gly Ala Lys Phe Thr Met Asp Ile Gly Ser Leu Ile Glu Asn
           275                 280                 285
   aag ttt ttg gaa aca cac cca gcc ata aga act gat gaa ttg cac gct      912
   Lys Phe Leu Glu Thr His Pro Ala Ile Arg Thr Asp Glu Leu His Ala
       290                 295                 300
   aat ccc aag gcc tta gca aaa atc aac caa gca gca gag aag gca aag      960
   Asn Pro Lys Ala Leu Ala Lys Ile Asn Gln Ala Ala Glu Lys Ala Lys
   305                 310                 315                 320
   tta att tta agc gcc aat tct gag gca agt att aac ata gaa tca ctg     1008
   Leu Ile Leu Ser Ala Asn Ser Glu Ala Ser Ile Asn Ile Glu Ser Leu
                   325                 330                 335
   atc aac gat att gat ttc cgt act tct ata act aga cag gaa ttc gaa     1056
   Ile Asn Asp Ile Asp Phe Arg Thr Ser Ile Thr Arg Gln Glu Phe Glu
               340                 345                 350
   gaa ttt att gca gac tcg tta ttg gac att gtc aaa ccc ata aat gac     1104
   Glu Phe Ile Ala Asp Ser Leu Leu Asp Ile Val Lys Pro Ile Asn Asp
           355                 360                 365
   gct gtt aca aaa caa ttc ggt ggc tat gga aca aat tta cct gag ata     1152
   Ala Val Thr Lys Gln Phe Gly Gly Tyr Gly Thr Asn Leu Pro Glu Ile
       370                 375                 380
   aat ggg gtc att ttg gcg gga ggc tct tcc cga att ccc att gtg cag     1200
   Asn Gly Val Ile Leu Ala Gly Gly Ser Ser Arg Ile Pro Ile Val Gln
   385                 390                 395                 400
   gat caa tta atc aaa ctc gta tcc gaa gaa aaa gtg ttg aga aat gtc     1248
   Asp Gln Leu Ile Lys Leu Val Ser Glu Glu Lys Val Leu Arg Asn Val
                   405                 410                 415
   aat gct gat gaa tca gct gtg aat ggt gtt gtt atg aga ggg atc aag     1296
   Asn Ala Asp Glu Ser Ala Val Asn Gly Val Val Met Arg Gly Ile Lys
               420                 425                 430
   tta tct aat tcg ttt aag acc aag ccg tta aat gtt gtt gac cgt tct     1344
   Leu Ser Asn Ser Phe Lys Thr Lys Pro Leu Asn Val Val Asp Arg Ser
           435                 440                 445
   gta aat act tat tca ttc aaa tta tca aac gaa tct gaa ctg tat gat     1392
   Val Asn Thr Tyr Ser Phe Lys Leu Ser Asn Glu Ser Glu Leu Tyr Asp
       450                 455                 460
   gtg ttc acg cgc gga agt gct tat cca aac aaa aca tct att ttg aca     1440
   Val Phe Thr Arg Gly Ser Ala Tyr Pro Asn Lys Thr Ser Ile Leu Thr
   465                 470                 475                 480
   aac acg act gat tcg att cct aat aat ttt acc att gac tta ttt gag     1488
   Asn Thr Thr Asp Ser Ile Pro Asn Asn Phe Thr Ile Asp Leu Phe Glu
                   485                 490                 495
   aat ggt aaa ttg ttc gaa act atc aca gtt aat tca gga gct ata aag     1536
   Asn Gly Lys Leu Phe Glu Thr Ile Thr Val Asn Ser Gly Ala Ile Lys
               500                 505                 510
   aat tca tat tcc tct gat aag tgc tcg tca gga gtt gcg tat aac att     1584
   Asn Ser Tyr Ser Ser Asp Lys Cys Ser Ser Gly Val Ala Tyr Asn Ile
           515                 520                 525
   act ttc gac ttg tcc agt gat aga tta ttc tct att caa gag gtt aac     1632
   Thr Phe Asp Leu Ser Ser Asp Arg Leu Phe Ser Ile Gln Glu Val Asn
       530                 535                 540
   tgc att tgt cag agc gaa aat gac ata ggt aac tcc aag caa att aag     1680
   Cys Ile Cys Gln Ser Glu Asn Asp Ile Gly Asn Ser Lys Gln Ile Lys
   545                 550                 555                 560
   aac aaa ggc agc cgt ttg gct ttt act tct gag gat gtt gag atc aaa     1728
   Asn Lys Gly Ser Arg Leu Ala Phe Thr Ser Glu Asp Val Glu Ile Lys
                   565                 570                 575
   agg ctt tct cct tca gaa cgt tcg cgt ttg cat gag cat atc aag ttg     1776
   Arg Leu Ser Pro Ser Glu Arg Ser Arg Leu His Glu His Ile Lys Leu
               580                 585                 590
   ctc gat aaa cag gat aag gaa aga ttt caa ttc caa gaa aat tta aac     1824
   Leu Asp Lys Gln Asp Lys Glu Arg Phe Gln Phe Gln Glu Asn Leu Asn
           595                 600                 605
   gtt ctt gaa agt aac ttg tat gat gct aga aac ctg cta atg gat gat     1872
   Val Leu Glu Ser Asn Leu Tyr Asp Ala Arg Asn Leu Leu Met Asp Asp
       610                 615                 620
   gaa gtt atg caa aat gga cca aaa tcc caa gta gaa gag tta tcg gag     1920
   Glu Val Met Gln Asn Gly Pro Lys Ser Gln Val Glu Glu Leu Ser Glu
   625                 630                 635                 640
   atg gtt aaa gta tat ttg gat tgg ctc gaa gat gca tcc ttt gat act     1968
   Met Val Lys Val Tyr Leu Asp Trp Leu Glu Asp Ala Ser Phe Asp Thr
                   645                 650                 655
   gac cct gag gat ata gtt agc aga att aga gaa att gga ata tta aaa     2016
   Asp Pro Glu Asp Ile Val Ser Arg Ile Arg Glu Ile Gly Ile Leu Lys
               660                 665                 670
   aag aaa ata gaa ctt tac atg gat tct gca aag gaa cct ttg aac tct     2064
   Lys Lys Ile Glu Leu Tyr Met Asp Ser Ala Lys Glu Pro Leu Asn Ser
           675                 680                 685
   caa caa ttt aaa gga atg ctt gaa gaa ggc cat aag tta ctt cag gct     2112
   Gln Gln Phe Lys Gly Met Leu Glu Glu Gly His Lys Leu Leu Gln Ala
       690                 695                 700
   ata gaa acc cat aag aat acc gtt gaa gaa ttt ttg agt caa ttt gaa     2160
   Ile Glu Thr His Lys Asn Thr Val Glu Glu Phe Leu Ser Gln Phe Glu
   705                 710                 715                 720
   acc gag ttt gcg gat acc ata gat aat gtt aga gaa gaa ttt aaa aag     2208
   Thr Glu Phe Ala Asp Thr Ile Asp Asn Val Arg Glu Glu Phe Lys Lys
                   725                 730                 735
   att aag caa cca gcg tat gtg tcg aag gcg tta tct aca tgg gag gaa     2256
   Ile Lys Gln Pro Ala Tyr Val Ser Lys Ala Leu Ser Thr Trp Glu Glu
               740                 745                 750
   acc tta acc tct ttt aaa aat tcc att agc gaa ata gag aag ttc ctg     2304
   Thr Leu Thr Ser Phe Lys Asn Ser Ile Ser Glu Ile Glu Lys Phe Leu
           755                 760                 765
   gca aaa aac cta ttt ggc gaa gac ctt cgt gaa cat tta ttt gaa atc       2352
   Ala Lys Asn Leu Phe Gly Glu Asp Leu Arg Glu His Leu Phe Glu Ile
       770                 775                 780
   aaa tta caa ttt gat atg tat cgt acg aaa cta gag gaa aaa ctg cgt       2400
   Lys Leu Gln Phe Asp Met Tyr Arg Thr Lys Leu Glu Glu Lys Leu Arg
   785                 790                 795                 800
   tta ata aaa agc ggt gat gaa agt cgc tta aat gaa ata aag aag tta       2448
   Leu Ile Lys Ser Gly Asp Glu Ser Arg Leu Asn Glu Ile Lys Lys Leu
                   805                 810                 815
   cat tta aga aac ttc cgc cta caa aag aga aag gag gaa aag ttg aaa       2496
   His Leu Arg Asn Phe Arg Leu Gln Lys Arg Lys Glu Glu Lys Leu Lys
               820                 825                 830
   aga aag ctt gaa cag gaa aaa agc aga aac aac aat gaa aca gaa tcg       2544
   Arg Lys Leu Glu Gln Glu Lys Ser Arg Asn Asn Asn Glu Thr Glu Ser
           835                 840                 845
   aca gta atc aac tcg gct gac gat aaa act act att gtc aat gac aag       2592
   Thr Val Ile Asn Ser Ala Asp Asp Lys Thr Thr Ile Val Asn Asp Lys
       850                 855                 860
   acc acc gag tcg aat cca agt tct gag gaa gac att ttg cat gat gaa       2640
   Thr Thr Glu Ser Asn Pro Ser Ser Glu Glu Asp Ile Leu His Asp Glu
   865                 870                 875                 880
   tta tag                                                               2646
   Leu
<210> 62
   <211> 881
   <212> PRT
   <213> Saccharomyces cerevisiae
<400> 62
   Met Arg Asn Val Leu Arg Leu Leu Phe Leu Thr Ala Phe Val Ala Ile
   1               5                   10                  15
   Gly Ser Leu Ala Ala Val Leu Gly Val Asp Tyr Gly Gln Gln Asn Ile
               20                  25                  30
   Lys Ala Ile Val Val Ser Pro Gln Ala Pro Leu Glu Leu Val Leu Thr
           35                  40                  45
   Pro Glu Ala Lys Arg Lys Glu Ile Ser Gly Leu Ser Ile Lys Arg Leu
       50                  55                  60
   Pro Gly Tyr Gly Lys Asp Asp Pro Asn Gly Ile Glu Arg Ile Tyr Gly
   65                  70                  75                  80
   Ser Ala Val Gly Ser Leu Ala Thr Arg Phe Pro Gln Asn Thr Leu Leu
                   85                  90                  95
   His Leu Lys Pro Leu Leu Gly Lys Ser Leu Glu Asp Glu Thr Thr Val
               100                 105                 110
   Thr Leu Tyr Ser Lys Gln His Pro Gly Leu Glu Met Val Ser Thr Asn
           115                 120                 125
   Arg Ser Thr Ile Ala Phe Leu Val Asp Asn Val Glu Tyr Pro Leu Glu
       130                 135                 140
   Glu Leu Val Ala Met Asn Val Gln Glu Ile Ala Asn Arg Ala Asn Ser
   145                 150                 155                 160
   Leu Leu Lys Asp Arg Asp Ala Arg Thr Glu Asp Phe Val Asn Lys Met
                   165                 170                 175
   Ser Phe Thr Ile Pro Asp Phe Phe Asp Gln His Gln Arg Lys Ala Leu
               180                 185                 190
   Leu Asp Ala Ser Ser Ile Thr Thr Gly Ile Glu Glu Thr Tyr Leu Val
           195                 200                 205
   Ser Glu Gly Met Ser Val Ala Val Asn Phe Val Leu Lys Gln Arg Gln
       210                 215                 220
   Phe Pro Pro Gly Glu Gln Gln His Tyr Ile Val Tyr Asp Met Gly Ser
   225                 230                 235                 240
   Gly Ser Ile Lys Ala Ser Met Phe Ser Ile Leu Gln Pro Glu Asp Thr
                   245                 250                 255
   Thr Gln Pro Val Thr Ile Glu Phe Glu Gly Tyr Gly Tyr Asn Pro His
               260                 265                 270
   Leu Gly Gly Ala Lys Phe Thr Met Asp Ile Gly Ser Leu Ile Glu Asn
           275                 280                 285
   Lys Phe Leu Glu Thr His Pro Ala Ile Arg Thr Asp Glu Leu His Ala
       290                 295                 300
   Asn Pro Lys Ala Leu Ala Lys Ile Asn Gln Ala Ala Glu Lys Ala Lys
   305                 310                 315                 320
   Leu Ile Leu Ser Ala Asn Ser Glu Ala Ser Ile Asn Ile Glu Ser Leu
                   325                 330                 335
   Ile Asn Asp Ile Asp Phe Arg Thr Ser Ile Thr Arg Gln Glu Phe Glu
   340 345 350
   Glu Phe Ile Ala Asp Ser Leu Leu Asp Ile Val Lys Pro Ile Asn Asp
   355 360 365
   Ala Val Thr Lys Gln Phe Gly Gly Tyr Gly Thr Asn Leu Pro Glu Ile
   370 375 380
   Asn Gly Val Ile Leu Ala Gly Gly Ser Ser Arg Ile Pro Ile Val Gln
   385 390 395 400
   Asp Gln Leu Ile Lys Leu Val Ser Glu Glu Lys Val Leu Arg Asn Val
   405 410 415
   Asn Ala Asp Glu Ser Ala Val Asn Gly Val Val Met Arg Gly Ile Lys
   420 425 430
   Leu Ser Asn Ser Phe Lys Thr Lys Pro Leu Asn Val Val Asp Arg Ser
   435 440 445
   Val Asn Thr Tyr Ser Phe Lys Leu Ser Asn Glu Ser Glu Leu Tyr Asp
   450 455 460
   Val Phe Thr Arg Gly Ser Ala Tyr Pro Asn Lys Thr Ser Ile Leu Thr
   465 470 475 480
   Asn Thr Thr Asp Ser Ile Pro Asn Asn Phe Thr Ile Asp Leu Phe Glu
   485 490 495
   Asn Gly Lys Leu Phe Glu Thr Ile Thr Val Asn Ser Gly Ala Ile Lys
   500 505 510
   Asn Ser Tyr Ser Ser Asp Lys Cys Ser Ser Gly Val Ala Tyr Asn Ile
   515 520 525
   Thr Phe Asp Leu Ser Ser Asp Arg Leu Phe Ser Ile Gln Glu Val Asn
   530 535 540
   Cys Ile Cys Gln Ser Glu Asn Asp Ile Gly Asn Ser Lys Gln Ile Lys
   545 550 555 560
   Asn Lys Gly Ser Arg Leu Ala Phe Thr Ser Glu Asp Val Glu Ile Lys
   565 570 575
   Arg Leu Ser Pro Ser Glu Arg Ser Arg Leu His Glu His Ile Lys Leu
   580 585 590
   Leu Asp Lys Gln Asp Lys Glu Arg Phe Gln Phe Gln Glu Asn Leu Asn
   595 600 605
   Val Leu Glu Ser Asn Leu Tyr Asp Ala Arg Asn Leu Leu Met Asp Asp
   610 615 620
   Glu Val Met Gln Asn Gly Pro Lys Ser Gln Val Glu Glu Leu Ser Glu
   625 630 635 640
   Met Val Lys Val Tyr Leu Asp Trp Leu Glu Asp Ala Ser Phe Asp Thr
   645 650 655
   Asp Pro Glu Asp Ile Val Ser Arg Ile Arg Glu Ile Gly Ile Leu Lys
   660 665 670
   Lys Lys Ile Glu Leu Tyr Met Asp Ser Ala Lys Glu Pro Leu Asn Ser
   675 680 685
   Gln Gln Phe Lys Gly Met Leu Glu Glu Gly His Lys Leu Leu Gln Ala
   690 695 700
   Ile Glu Thr His Lys Asn Thr Val Glu Glu Phe Leu Ser Gln Phe Glu
   705 710 715 720
   Thr Glu Phe Ala Asp Thr Ile Asp Asn Val Arg Glu Glu Phe Lys Lys
   725 730 735
   Ile Lys Gln Pro Ala Tyr Val Ser Lys Ala Leu Ser Thr Trp Glu Glu
   740 745 750
   Thr Leu Thr Ser Phe Lys Asn Ser Ile Ser Glu Ile Glu Lys Phe Leu
   755 760 765
   Ala Lys Asn Leu Phe Gly Glu Asp Leu Arg Glu His Leu Phe Glu Ile
   770 775 780
   Lys Leu Gln Phe Asp Met Tyr Arg Thr Lys Leu Glu Glu Lys Leu Arg
   785 790 795 800
   Leu Ile Lys Ser Gly Asp Glu Ser Arg Leu Asn Glu Ile Lys Lys Leu
   805 810 815
   His Leu Arg Asn Phe Arg Leu Gln Lys Arg Lys Glu Glu Lys Leu Lys
   820 825 830
   Arg Lys Leu Glu Gln Glu Lys Ser Arg Asn Asn Asn Glu Thr Glu Ser
   835 840 845
   Thr Val Ile Asn Ser Ala Asp Asp Lys Thr Thr Ile Val Asn Asp Lys
       850                 855                 860
   Thr Thr Glu Ser Asn Pro Ser Ser Glu Glu Asp Ile Leu His Asp Glu
   865                 870                 875                 880
   Leu
<210> 63
   <211> 834
   <212> DNA
   <213> Saccharomyces cerevisiae
<220>
   <221> CDS
   <222> (1)..(834)
<400> 63
   atg aaa ttg cac ggc ttt tta ttt tcc gta tta tca aca tgc gtc gtc      48
   Met Lys Leu His Gly Phe Leu Phe Ser Val Leu Ser Thr Cys Val Val
   1               5                   10                  15
   att tta cca gcg ttg gcc tac agt gaa gct gtc acg atg gtc aag tcg      96
   Ile Leu Pro Ala Leu Ala Tyr Ser Glu Ala Val Thr Met Val Lys Ser
               20                  25                  30
   att gag cag tac ttc gat atc tgc aat agg aat gat tct tac aca atg     144
   Ile Glu Gln Tyr Phe Asp Ile Cys Asn Arg Asn Asp Ser Tyr Thr Met
           35                  40                  45
   ata aaa tac tac act tct tgg tgc caa cat tgt aaa act ctg gcc cca     192
   Ile Lys Tyr Tyr Thr Ser Trp Cys Gln His Cys Lys Thr Leu Ala Pro
       50                  55                  60
   gta tac gaa gag ctt ggt gag cta tac gcc aaa aaa gct aat aaa gat     240
   Val Tyr Glu Glu Leu Gly Glu Leu Tyr Ala Lys Lys Ala Asn Lys Asp
   65                  70                  75                  80
   gat acc cca att aac ttc ctt gaa gtt aac tgt gaa ttc ttc ggg cca     288
   Asp Thr Pro Ile Asn Phe Leu Glu Val Asn Cys Glu Phe Phe Gly Pro
                   85                  90                  95
   act tta tgt acc gac ttg cct gga ttt cca ata att gaa ctg gtc aaa     336
   Thr Leu Cys Thr Asp Leu Pro Gly Phe Pro Ile Ile Glu Leu Val Lys
               100                 105                 110
   cct cgt act aag ccc tta gtt ctt ccg aag ctc gat tgg tcg tct atg     384
   Pro Arg Thr Lys Pro Leu Val Leu Pro Lys Leu Asp Trp Ser Ser Met
           115                 120                 125
   aaa ttt cat gaa aga cta tgg caa aga atc aag acg tgg ttc aac aat     432
   Lys Phe His Glu Arg Leu Trp Gln Arg Ile Lys Thr Trp Phe Asn Asn
       130                 135                 140
   cct aag tac caa ctg gat acg tct agg gtt gtt cgt ttt gaa ggg agt     480
   Pro Lys Tyr Gln Leu Asp Thr Ser Arg Val Val Arg Phe Glu Gly Ser
   145                 150                 155                 160
   agg aac cta aag agt tta agc aac ttt atc gat act gta aga agt aaa      528
   Arg Asn Leu Lys Ser Leu Ser Asn Phe Ile Asp Thr Val Arg Ser Lys
                   165                 170                 175
   gat aca gaa gaa aga ttc ata gaa cat att ttc gat gat tct agg aat      576
   Asp Thr Glu Glu Arg Phe Ile Glu His Ile Phe Asp Asp Ser Arg Asn
               180                 185                 190
   tgc aat gaa gaa tta cgt tct caa cag ctt ctg tgt aaa gct ggt aaa      624
   Cys Asn Glu Glu Leu Arg Ser Gln Gln Leu Leu Cys Lys Ala Gly Lys
           195                 200                 205
   gaa tac tac tct gat act tta tct aaa tta tac ggt gac gtg aat ggg      672
   Glu Tyr Tyr Ser Asp Thr Leu Ser Lys Leu Tyr Gly Asp Val Asn Gly
       210                 215                 220
   ctg gaa aag gaa agg cga aga cta gaa gct tta att aag caa aat gga      720
   Leu Glu Lys Glu Arg Arg Arg Leu Glu Ala Leu Ile Lys Gln Asn Gly
   225                 230                 235                 240
   gat gac ttg agt aaa gaa gtt aaa gaa aaa ctg aaa atc att cgt cta      768
   Asp Asp Leu Ser Lys Glu Val Lys Glu Lys Leu Lys Ile Ile Arg Leu
                   245                 250                 255
   caa ttg agc cta tta tca cac ata gaa gac cag tta gaa gat acc agt      816
   Gln Leu Ser Leu Leu Ser His Ile Glu Asp Gln Leu Glu Asp Thr Ser
               260                 265                 270
   agt cat gac gag ctt tga                                              834
   Ser His Asp Glu Leu
           275
<210> 64
   <211> 277
   <212> PRT
   <213> Saccharomyces cerevisiae
<400> 64
   Met Lys Leu His Gly Phe Leu Phe Ser Val Leu Ser Thr Cys Val Val
   1               5                   10                  15
   Ile Leu Pro Ala Leu Ala Tyr Ser Glu Ala Val Thr Met Val Lys Ser
               20                  25                  30
   Ile Glu Gln Tyr Phe Asp Ile Cys Asn Arg Asn Asp Ser Tyr Thr Met
           35                  40                  45
   Ile Lys Tyr Tyr Thr Ser Trp Cys Gln His Cys Lys Thr Leu Ala Pro
       50                  55                  60
   Val Tyr Glu Glu Leu Gly Glu Leu Tyr Ala Lys Lys Ala Asn Lys Asp
   65                  70                  75                  80
   Asp Thr Pro Ile Asn Phe Leu Glu Val Asn Cys Glu Phe Phe Gly Pro
                   85                  90                  95
   Thr Leu Cys Thr Asp Leu Pro Gly Phe Pro Ile Ile Glu Leu Val Lys
               100                 105                 110
   Pro Arg Thr Lys Pro Leu Val Leu Pro Lys Leu Asp Trp Ser Ser Met
           115                 120                 125
   Lys Phe His Glu Arg Leu Trp Gln Arg Ile Lys Thr Trp Phe Asn Asn
       130                 135                 140
   Pro Lys Tyr Gln Leu Asp Thr Ser Arg Val Val Arg Phe Glu Gly Ser
   145                 150                 155                 160
   Arg Asn Leu Lys Ser Leu Ser Asn Phe Ile Asp Thr Val Arg Ser Lys
                   165                 170                 175
   Asp Thr Glu Glu Arg Phe Ile Glu His Ile Phe Asp Asp Ser Arg Asn
               180                 185                 190
   Cys Asn Glu Glu Leu Arg Ser Gln Gln Leu Leu Cys Lys Ala Gly Lys
           195                 200                 205
   Glu Tyr Tyr Ser Asp Thr Leu Ser Lys Leu Tyr Gly Asp Val Asn Gly
       210                 215                 220
   Leu Glu Lys Glu Arg Arg Arg Leu Glu Ala Leu Ile Lys Gln Asn Gly
   225                 230                 235                 240
   Asp Asp Leu Ser Lys Glu Val Lys Glu Lys Leu Lys Ile Ile Arg Leu
                   245                 250                 255
   Gln Leu Ser Leu Leu Ser His Ile Glu Asp Gln Leu Glu Asp Thr Ser
               260                 265                 270
   Ser His Asp Glu Leu
           275
<210> 65
   <211> 888
   <212> DNA
   <213> Saccharomyces cerevisiae
<220>
   <221> CDS
   <222> (1)..(888)
<400> 65
   atg aac ggt tac tgg aaa cct gcg ttg gtt gtc ctg gga ttg gta tct      48
   Met Asn Gly Tyr Trp Lys Pro Ala Leu Val Val Leu Gly Leu Val Ser
   1               5                   10                  15
   cta tca tat gct ttt acc acc att gaa aca gaa att ttc caa tta caa       96
   Leu Ser Tyr Ala Phe Thr Thr Ile Glu Thr Glu Ile Phe Gln Leu Gln
               20                  25                  30
   aat gaa ata agt acg aaa tat ggc cca gat atg aac ttc tac aag ttc      144
   Asn Glu Ile Ser Thr Lys Tyr Gly Pro Asp Met Asn Phe Tyr Lys Phe
           35                  40                  45
   ttg aag tta cct aaa ctg cag aat tct agt aca aag gag att aca aaa      192
   Leu Lys Leu Pro Lys Leu Gln Asn Ser Ser Thr Lys Glu Ile Thr Lys
       50                  55                  60
   aac tta aga aag cta tcc aag aag tac cat ccg gat aag aac cct aaa      240
   Asn Leu Arg Lys Leu Ser Lys Lys Tyr His Pro Asp Lys Asn Pro Lys
   65                  70                  75                  80
   tac cgt aaa ttg tat gaa agg tta aac ctc gct act caa att ctt tca      288
   Tyr Arg Lys Leu Tyr Glu Arg Leu Asn Leu Ala Thr Gln Ile Leu Ser
                   85                  90                  95
   aac agc tct aat cgt aag att tat gat tat tat cta cag aat ggc ttt      336
   Asn Ser Ser Asn Arg Lys Ile Tyr Asp Tyr Tyr Leu Gln Asn Gly Phe
               100                 105                 110
   cca aac tat gat ttc cat aag ggt ggt ttt tat ttt tcc aga atg aag      384
   Pro Asn Tyr Asp Phe His Lys Gly Gly Phe Tyr Phe Ser Arg Met Lys
           115                 120                 125
   cct aag act tgg ttc ctg ctg gcc ttt att tgg ata gtc gtt aat att      432
   Pro Lys Thr Trp Phe Leu Leu Ala Phe Ile Trp Ile Val Val Asn Ile
       130                 135                 140
   ggg cag tat atc att tct att att caa tat cgt tct caa aga tca aga      480
   Gly Gln Tyr Ile Ile Ser Ile Ile Gln Tyr Arg Ser Gln Arg Ser Arg
   145                 150                 155                 160
   att gaa aac ttc atc agt cag tgt aaa caa cag gat gat acc aat gga      528
   Ile Glu Asn Phe Ile Ser Gln Cys Lys Gln Gln Asp Asp Thr Asn Gly
                   165                 170                 175
   cta ggc gta aaa caa cta acg ttt aaa caa cat gaa aag gat gag ggt      576
   Leu Gly Val Lys Gln Leu Thr Phe Lys Gln His Glu Lys Asp Glu Gly
               180                 185                 190
   aaa agt ttg gtt gta agg ttt agc gat gtc tat gtt gta gag cct gat      624
   Lys Ser Leu Val Val Arg Phe Ser Asp Val Tyr Val Val Glu Pro Asp
           195                 200                 205
   gga agt gaa aca cta att tcg cca gat acc ttg gat aaa cct tca gta      672
   Gly Ser Glu Thr Leu Ile Ser Pro Asp Thr Leu Asp Lys Pro Ser Val
       210                 215                 220
   aag aac tgt ttg ttt tgg aga ata cct gct tcg gtt tgg aac atg acg      720
   Lys Asn Cys Leu Phe Trp Arg Ile Pro Ala Ser Val Trp Asn Met Thr
   225                 230                 235                 240
   ttt ggc aaa tct gtt ggt agc gca gga aaa gaa gaa ata ata acg gat      768
   Phe Gly Lys Ser Val Gly Ser Ala Gly Lys Glu Glu Ile Ile Thr Asp
                   245                 250                 255
   agt aaa aag tat gat ggt aac caa aca aaa aag ggg aac aaa gta aaa      816
   Ser Lys Lys Tyr Asp Gly Asn Gln Thr Lys Lys Gly Asn Lys Val Lys
               260                 265                 270
   aag ggt tct gca aag aaa ggc caa aag aaa atg gaa ttg cct aac ggt      864
   Lys Gly Ser Ala Lys Lys Gly Gln Lys Lys Met Glu Leu Pro Asn Gly
           275                 280                 285
   aaa gtg atc tat tca cgt aaa tga                                      888
   Lys Val Ile Tyr Ser Arg Lys
       290                 295
<210> 66
   <211> 295
   <212> PRT
   <213> Saccharomyces cerevisiae
<400> 66
   Met Asn Gly Tyr Trp Lys Pro Ala Leu Val Val Leu Gly Leu Val Ser
   1               5                   10                  15
   Leu Ser Tyr Ala Phe Thr Thr Ile Glu Thr Glu Ile Phe Gln Leu Gln
               20                  25                  30
   Asn Glu Ile Ser Thr Lys Tyr Gly Pro Asp Met Asn Phe Tyr Lys Phe
           35                  40                  45
   Leu Lys Leu Pro Lys Leu Gln Asn Ser Ser Thr Lys Glu Ile Thr Lys
       50                  55                  60
   Asn Leu Arg Lys Leu Ser Lys Lys Tyr His Pro Asp Lys Asn Pro Lys
   65                  70                  75                  80
   Tyr Arg Lys Leu Tyr Glu Arg Leu Asn Leu Ala Thr Gln Ile Leu Ser
                   85                  90                  95
   Asn Ser Ser Asn Arg Lys Ile Tyr Asp Tyr Tyr Leu Gln Asn Gly Phe
               100                 105                 110
   Pro Asn Tyr Asp Phe His Lys Gly Gly Phe Tyr Phe Ser Arg Met Lys
           115                 120                 125
   Pro Lys Thr Trp Phe Leu Leu Ala Phe Ile Trp Ile Val Val Asn Ile
       130                 135                 140
   Gly Gln Tyr Ile Ile Ser Ile Ile Gln Tyr Arg Ser Gln Arg Ser Arg
   145                 150                 155                 160
   Ile Glu Asn Phe Ile Ser Gln Cys Lys Gln Gln Asp Asp Thr Asn Gly
                   165                 170                 175
   Leu Gly Val Lys Gln Leu Thr Phe Lys Gln His Glu Lys Asp Glu Gly
               180                 185                 190
   Lys Ser Leu Val Val Arg Phe Ser Asp Val Tyr Val Val Glu Pro Asp
           195                 200                 205
   Gly Ser Glu Thr Leu Ile Ser Pro Asp Thr Leu Asp Lys Pro Ser Val
       210                 215                 220
   Lys Asn Cys Leu Phe Trp Arg Ile Pro Ala Ser Val Trp Asn Met Thr
   225                 230                 235                 240
   Phe Gly Lys Ser Val Gly Ser Ala Gly Lys Glu Glu Ile Ile Thr Asp
                   245                 250                 255
   Ser Lys Lys Tyr Asp Gly Asn Gln Thr Lys Lys Gly Asn Lys Val Lys
               260                 265                 270
   Lys Gly Ser Ala Lys Lys Gly Gln Lys Lys Met Glu Leu Pro Asn Gly
           275                 280                 285
   Lys Val Ile Tyr Ser Arg Lys
       290                 295
<210> 67
   <211> 1554
   <212> DNA
   <213> Saccharomyces cerevisiae
<220>
   <221> CDS
   <222> (1)..(1554)
<400> 67
   atg caa gtg acc aca aga ttt ata tct gcg ata gtc tcg ttt tgc ctg      48
   Met Gln Val Thr Thr Arg Phe Ile Ser Ala Ile Val Ser Phe Cys Leu
   1               5                   10                  15
   ttt gct tct ttc acg ttg gct gaa aac agc gca aga gct acg ccg gga      96
   Phe Ala Ser Phe Thr Leu Ala Glu Asn Ser Ala Arg Ala Thr Pro Gly
               20                  25                  30
   tca gat tta ctc gtt cta aca gag aag aaa ttt aaa tca ttc atc gaa     144
   Ser Asp Leu Leu Val Leu Thr Glu Lys Lys Phe Lys Ser Phe Ile Glu
           35                  40                  45
   tct cat ccg tta gtc ctc gtc gag ttt ttt gct cca tgg tgt ttg cat     192
   Ser His Pro Leu Val Leu Val Glu Phe Phe Ala Pro Trp Cys Leu His
       50                  55                  60
   tct cag atc tta cgc cct cac tta gaa gag gcc gcc tct att tta aag     240
   Ser Gln Ile Leu Arg Pro His Leu Glu Glu Ala Ala Ser Ile Leu Lys
   65                  70                  75                  80
   gag cat aac gtc cca gtt gtt caa att gat tgt gag gct aac agt atg      288
   Glu His Asn Val Pro Val Val Gln Ile Asp Cys Glu Ala Asn Ser Met
                   85                  90                  95
   gtt tgc ctg caa caa act ata aat acc tac cca acc ttg aaa atc ttt      336
   Val Cys Leu Gln Gln Thr Ile Asn Thr Tyr Pro Thr Leu Lys Ile Phe
               100                 105                 110
   aaa aat ggt cgt att ttt gat ggt caa gtc tat cgc ggt gtc aag atc      384
   Lys Asn Gly Arg Ile Phe Asp Gly Gln Val Tyr Arg Gly Val Lys Ile
           115                 120                 125
   acc gat gaa atc act cag tac atg att cag cta tac gag gct tct gtc      432
   Thr Asp Glu Ile Thr Gln Tyr Met Ile Gln Leu Tyr Glu Ala Ser Val
       130                 135                 140
   att tat tta aat tcc gaa gat gaa atc caa cca tac ttg gaa aat gca      480
   Ile Tyr Leu Asn Ser Glu Asp Glu Ile Gln Pro Tyr Leu Glu Asn Ala
   145                 150                 155                 160
   act tta cca gta gta ata aac aga ggc ttg aca ggc ttg aat gaa acg      528
   Thr Leu Pro Val Val Ile Asn Arg Gly Leu Thr Gly Leu Asn Glu Thr
                   165                 170                 175
   tat caa gaa gtc gca ctg gac ctt gct gag gat tac gtc ttt tta tcc      576
   Tyr Gln Glu Val Ala Leu Asp Leu Ala Glu Asp Tyr Val Phe Leu Ser
               180                 185                 190
   ctt cta gat tca gaa gat aag tca tta tca atc cac ttg cca aac act      624
   Leu Leu Asp Ser Glu Asp Lys Ser Leu Ser Ile His Leu Pro Asn Thr
           195                 200                 205
   aca gaa cca att ctg ttt gat gga aat gta gac tct ttg gtc gga aat      672
   Thr Glu Pro Ile Leu Phe Asp Gly Asn Val Asp Ser Leu Val Gly Asn
       210                 215                 220
   tcc gtt gct cta act cag tgg tta aaa gtg gta att tta cct tac ttt      720
   Ser Val Ala Leu Thr Gln Trp Leu Lys Val Val Ile Leu Pro Tyr Phe
   225                 230                 235                 240
   acc gac atc gaa cct gat ctc ttc ccc aag tac att tct agc aat ttg      768
   Thr Asp Ile Glu Pro Asp Leu Phe Pro Lys Tyr Ile Ser Ser Asn Leu
                   245                 250                 255
   ccg ttg gct tac ttc ttt tat act tct gag gaa gaa ttg gaa gat tac      816
   Pro Leu Ala Tyr Phe Phe Tyr Thr Ser Glu Glu Glu Leu Glu Asp Tyr
               260                 265                 270
   act gat ctt ttc acg cag tta ggt aag gaa aat cgt ggc caa ata aat      864
   Thr Asp Leu Phe Thr Gln Leu Gly Lys Glu Asn Arg Gly Gln Ile Asn
           275                 280                 285
   ttc att gca tta aac tct aca atg ttc cca cac cac gtt aga ttc cta      912
   Phe Ile Ala Leu Asn Ser Thr Met Phe Pro His His Val Arg Phe Leu
       290                 295                 300
   aat atg aga gaa cag ttc cca tta ttt gct atc cat aat atg atc aat      960
   Asn Met Arg Glu Gln Phe Pro Leu Phe Ala Ile His Asn Met Ile Asn
   305                 310                 315                 320
   aat ctg aaa tat ggt tta cca caa cta cca gaa gaa gag tac gcg aaa     1008
   Asn Leu Lys Tyr Gly Leu Pro Gln Leu Pro Glu Glu Glu Tyr Ala Lys
                   325                 330                 335
   tta gaa aaa cca caa cca cta gac aga gat atg atc gtt cag ttg gta     1056
   Leu Glu Lys Pro Gln Pro Leu Asp Arg Asp Met Ile Val Gln Leu Val
               340                 345                 350
   aaa gat tac cgt gaa ggt act gcc aag cca att gtt aag tca gaa gag     1104
   Lys Asp Tyr Arg Glu Gly Thr Ala Lys Pro Ile Val Lys Ser Glu Glu
           355                 360                 365
   att cca aaa gaa caa aag tcc aat gtt tat aaa ata gtt ggg aag aca     1152
   Ile Pro Lys Glu Gln Lys Ser Asn Val Tyr Lys Ile Val Gly Lys Thr
       370                 375                 380
   cat gac gac att gtt cat gat gat gac aag gat gtc ctt gtc aaa tat     1200
   His Asp Asp Ile Val His Asp Asp Asp Lys Asp Val Leu Val Lys Tyr
   385                 390                 395                 400
   tac gcg aca tgg tgt att cat agt aaa agg ttt gcg cct att tac gaa     1248
   Tyr Ala Thr Trp Cys Ile His Ser Lys Arg Phe Ala Pro Ile Tyr Glu
                   405                 410                 415
   gaa att gca aat gtc tta gca tct gat gaa tct gtt cgc gat aaa atc     1296
   Glu Ile Ala Asn Val Leu Ala Ser Asp Glu Ser Val Arg Asp Lys Ile
               420                 425                 430
   ttg atc gcc gaa gta gat tca ggg gca aat gat atc tta agt ttt cct     1344
   Leu Ile Ala Glu Val Asp Ser Gly Ala Asn Asp Ile Leu Ser Phe Pro
           435                 440                 445
   gtg aca gga tat cca acc att gct ttg tat cct gcc gga aat aac tct     1392
   Val Thr Gly Tyr Pro Thr Ile Ala Leu Tyr Pro Ala Gly Asn Asn Ser
       450                 455                 460
   aag cct att atc ttc aat aaa att aga aat ttg gaa gat gtt ttc gaa     1440
   Lys Pro Ile Ile Phe Asn Lys Ile Arg Asn Leu Glu Asp Val Phe Glu
   465                 470                 475                 480
   ttt atc aag gaa tca ggt aca cat cac att gac ggc cag gca att tat     1488
   Phe Ile Lys Glu Ser Gly Thr His His Ile Asp Gly Gln Ala Ile Tyr
                   485                 490                 495
   gat aaa ttg cac cag gcc aag gat tct gaa gtg tct act gaa gat acc     1536
   Asp Lys Leu His Gln Ala Lys Asp Ser Glu Val Ser Thr Glu Asp Thr
               500                 505                 510
   gta cat gat gaa tta taa                                             1554
   Val His Asp Glu Leu
           515
<210> 68
   <211> 517
   <212> PRT
   <213> Saccharomyces cerevisiae
<400> 68
   Met Gln Val Thr Thr Arg Phe Ile Ser Ala Ile Val Ser Phe Cys Leu
   1               5                   10                  15
   Phe Ala Ser Phe Thr Leu Ala Glu Asn Ser Ala Arg Ala Thr Pro Gly
               20                  25                  30
   Ser Asp Leu Leu Val Leu Thr Glu Lys Lys Phe Lys Ser Phe Ile Glu
           35                  40                  45
   Ser His Pro Leu Val Leu Val Glu Phe Phe Ala Pro Trp Cys Leu His
       50                  55                  60
   Ser Gln Ile Leu Arg Pro His Leu Glu Glu Ala Ala Ser Ile Leu Lys
   65                  70                  75                  80
   Glu His Asn Val Pro Val Val Gln Ile Asp Cys Glu Ala Asn Ser Met
                   85                  90                  95
   Val Cys Leu Gln Gln Thr Ile Asn Thr Tyr Pro Thr Leu Lys Ile Phe
               100                 105                 110
   Lys Asn Gly Arg Ile Phe Asp Gly Gln Val Tyr Arg Gly Val Lys Ile
           115                 120                 125
   Thr Asp Glu Ile Thr Gln Tyr Met Ile Gln Leu Tyr Glu Ala Ser Val
       130                 135                 140
   Ile Tyr Leu Asn Ser Glu Asp Glu Ile Gln Pro Tyr Leu Glu Asn Ala
   145                 150                 155                 160
   Thr Leu Pro Val Val Ile Asn Arg Gly Leu Thr Gly Leu Asn Glu Thr
                   165                 170                 175
   Tyr Gln Glu Val Ala Leu Asp Leu Ala Glu Asp Tyr Val Phe Leu Ser
               180                 185                 190
   Leu Leu Asp Ser Glu Asp Lys Ser Leu Ser Ile His Leu Pro Asn Thr
           195                 200                 205
   Thr Glu Pro Ile Leu Phe Asp Gly Asn Val Asp Ser Leu Val Gly Asn
       210                 215                 220
   Ser Val Ala Leu Thr Gln Trp Leu Lys Val Val Ile Leu Pro Tyr Phe
   225                 230                 235                 240
   Thr Asp Ile Glu Pro Asp Leu Phe Pro Lys Tyr Ile Ser Ser Asn Leu
                   245                 250                 255
   Pro Leu Ala Tyr Phe Phe Tyr Thr Ser Glu Glu Glu Leu Glu Asp Tyr
               260                 265                 270
   Thr Asp Leu Phe Thr Gln Leu Gly Lys Glu Asn Arg Gly Gln Ile Asn
               275                 280                 285
   Phe Ile Ala Leu Asn Ser Thr Met Phe Pro His His Val Arg Phe Leu
       290                 295                 300
   Asn Met Arg Glu Gln Phe Pro Leu Phe Ala Ile His Asn Met Ile Asn
   305                 310                 315                 320
   Asn Leu Lys Tyr Gly Leu Pro Gln Leu Pro Glu Glu Glu Tyr Ala Lys
                   325                 330                 335
   Leu Glu Lys Pro Gln Pro Leu Asp Arg Asp Met Ile Val Gln Leu Val
               340                 345                 350
   Lys Asp Tyr Arg Glu Gly Thr Ala Lys Pro Ile Val Lys Ser Glu Glu
           355                 360                 365
   Ile Pro Lys Glu Gln Lys Ser Asn Val Tyr Lys Ile Val Gly Lys Thr
       370                 375                 380
   His Asp Asp Ile Val His Asp Asp Asp Lys Asp Val Leu Val Lys Tyr
   385                 390                 395                 400
   Tyr Ala Thr Trp Cys Ile His Ser Lys Arg Phe Ala Pro Ile Tyr Glu
                   405                 410                 415
   Glu Ile Ala Asn Val Leu Ala Ser Asp Glu Ser Val Arg Asp Lys Ile
               420                 425                 430
   Leu Ile Ala Glu Val Asp Ser Gly Ala Asn Asp Ile Leu Ser Phe Pro
           435                 440                 445
   Val Thr Gly Tyr Pro Thr Ile Ala Leu Tyr Pro Ala Gly Asn Asn Ser
       450                 455                 460
   Lys Pro Ile Ile Phe Asn Lys Ile Arg Asn Leu Glu Asp Val Phe Glu
   465                 470                 475                 480
   Phe Ile Lys Glu Ser Gly Thr His His Ile Asp Gly Gln Ala Ile Tyr
                   485                 490                 495
   Asp Lys Leu His Gln Ala Lys Asp Ser Glu Val Ser Thr Glu Asp Thr
               500                 505                 510
   Val His Asp Glu Leu
           515
<210> 69
   <211> 1527
   <212> DNA
   <213> Homo sapiens
<220>
   <221> CDS
   <222> (1)..(1527)
<400> 69
atg ctg cgc cgc gct ctg ctg tgc ctg gcc gtg gcc gcc ctg gtg cgc      48
   gcc gac gcc ccc gag gag gag gac cac gtc ctg gtg ctg cgg aaa agc      96
   aac ttc gcg gag gcg ctg gcg gcc cac aag tac ctg ctg gtg gag ttc     144
   tat gcc cct tgg tgt ggc cac tgc aag gct ctg gcc cct gag tat gcc     192
   aaa gcc gct ggg aag ctg aag gca gaa ggt tcc gag atc agg ttg gcc     240
   aag gtg gac gcc acg gag gag tct gac ctg gcc cag cag tac ggc gtg     288
   cgc ggc tat ccc acc atc aag ttc ttc agg aat gga gac acg gct tcc     336
   ccc aag gaa tat aca gct ggc aga gag gct gat gac atc gtg aac tgg     384
   ctg aag aag cgc acg ggc ccg gct gcc acc acc ctg cct gac ggc gca     432
   gct gca gag tcc ttg gtg gag tcc agc gag gtg gct gtc atc ggc ttc     480
   ttc aag gac gtg gag tcg gac tct gcc aag cag ttt ttg cag gca gca     528
   gag gcc atc gat gac ata cca ttt ggg atc act tcc aac agt gac gtg     576
   ttc tcc aaa tac cag ctc gac aaa gat ggg gtt gtc ctc ttt aag aag     624
   ttt gat gaa ggc cgg aac aac ttt gaa ggg gag gtc acc aag gag aac      672
   ctg ctg gac ttt atc aaa cac aac cag ctg ccc ctt gtc atc gag ttc      720
   acc gag cag aca gcc ccg aag att ttt gga ggt gaa atc aag act cac      768
   atc ctg ctg ttc ttg ccc aag agt gtg tct gac tat gac ggc aaa ctg      816
   agc aac ttc aaa aca gca gcc gag agc ttc aag ggc aag atc ctg ttc      864
   atc ttc atc gac agc gac cac acc gac aac cag cgc atc ctc gag ttc      912
   ttt ggc ctg aag aag gaa gag tgc ccg gcc gtg cgc ctc atc acc ctg      960
   gag gag gag atg acc aag tac aag ccc gaa tcg gag gag ctg acg gca     1008
   gag agg atc aca gag ttc tgc cac cgc ttc ctg gag ggc aaa atc aag     1056
   ccc cac ctg atg agc cag gag ctg ccg gag gac tgg gac aag cag cct     1104
   gtc aag gtg ctt gtt ggg aag aac ttt gaa gac gtg gct ttt gat gag     1152
   aaa aaa aac gtc ttt gtg gag ttc tat gcc cca tgg tgt ggt cac tgc     1200
   aaa cag ttg gct ccc att tgg gat aaa ctg gga gag acg tac aag gac     1248
   cat gag aac atc gtc atc gcc aag atg gac tcg act gcc aac gag gtg     1296
   gag gcc gtc aaa gtg cac agc ttc ccc aca ctc aag ttc ttt cct gcc     1344
   agt gcc gac agg acg gtc att gat tac aac ggg gaa cgc acg ctg gat     1392
   ggt ttt aag aaa ttc ctg gag agc ggt ggc cag gat ggg gca ggg gat     1440
   gat gac gat ctc gag gac ctg gaa gaa gca gag gag cca gac atg gag     1488
   gaa gac gat gat cag aaa gct gtg aaa gat gaa ctg taa                 1527
<210> 70
   <211> 508
   <212> PRT
   <213> Homo sapiens
<400> 70
<210> 71
   <211> 1407
   <212> DNA
   <213> Homo sapiens
<220>
   <221> CDS
   <222> (1)..(1407)
<400> 71
   atg ggc cgc ggc tgg gga ttc ttg ttt ggc ctc ctg ggc gcc gtg tgg        48
   Met Gly Arg Gly Trp Gly Phe Leu Phe Gly Leu Leu Gly Ala Val Trp
   1               5                   10                  15
   ctg ctc agc tcg ggc cac gga gag gag cag ccc ccg gag aca gcg gca        96
   Leu Leu Ser Ser Gly His Gly Glu Glu Gln Pro Pro Glu Thr Ala Ala
               20                  25                  30
   cag agg tgc ttc tgc cag gtt agt ggt tac ttg gat gat tgt acc tgt       144
   Gln Arg Cys Phe Cys Gln Val Ser Gly Tyr Leu Asp Asp Cys Thr Cys
           35                  40                  45
   gat gtt gaa acc att gat aga ttt aat aac tac agg ctt ttc cca aga       192
   Asp Val Glu Thr Ile Asp Arg Phe Asn Asn Tyr Arg Leu Phe Pro Arg
       50                  55                  60
   cta caa aaa ctt ctt gaa agt gac tac ttt agg tat tac aag gta aac       240
   Leu Gln Lys Leu Leu Glu Ser Asp Tyr Phe Arg Tyr Tyr Lys Val Asn
   65                  70                  75                  80
   ctg aag agg ccg tgt cct ttc tgg aat gac atc agc cag tgt gga aga       288
   Leu Lys Arg Pro Cys Pro Phe Trp Asn Asp Ile Ser Gln Cys Gly Arg
                   85                  90                  95
   agg gac tgt gct gtc aaa cca tgt caa tct gat gaa gtt cct gat gga       336
   Arg Asp Cys Ala Val Lys Pro Cys Gln Ser Asp Glu Val Pro Asp Gly
               100                 105                 110
   att aaa tct gcg agc tac aag tat tct gaa gaa gcc aat aat ctc att       384
   Ile Lys Ser Ala Ser Tyr Lys Tyr Ser Glu Glu Ala Asn Asn Leu Ile
           115                 120                 125
   gaa gaa tgt gaa caa gct gaa cga ctt gga gca gtg gat gaa tct ctg      432
   Glu Glu Cys Glu Gln Ala Glu Arg Leu Gly Ala Val Asp Glu Ser Leu
       130                 135                 140
   agt gag gaa aca cag aag gct gtt ctt cag tgg acc aag cat gat gat      480
   Ser Glu Glu Thr Gln Lys Ala Val Leu Gln Trp Thr Lys His Asp Asp
   145                 150                 155                 160
   tct tca gat aac ttc tgt gaa gct gat gac att cag tcc cct gaa gct      528
   Ser Ser Asp Asn Phe Cys Glu Ala Asp Asp Ile Gln Ser Pro Glu Ala
                   165                 170                 175
   gaa tat gta gat ttg ctt ctt aat cct gag cgc tac act ggt tac aag      576
   Glu Tyr Val Asp Leu Leu Leu Asn Pro Glu Arg Tyr Thr Gly Tyr Lys
               180                 185                 190
   gga cca gat gct tgg aaa ata tgg aat gtc atc tac gaa gaa aac tgt      624
   Gly Pro Asp Ala Trp Lys Ile Trp Asn Val Ile Tyr Glu Glu Asn Cys
           195                 200                 205
   ttt aag cca cag aca att aaa aga cct tta aat cct ttg gct tct ggt      672
   Phe Lys Pro Gln Thr Ile Lys Arg Pro Leu Asn Pro Leu Ala Ser Gly
       210                 215                 220
   caa ggg aca agt gaa gag aac act ttt tac agt tgg cta gaa ggt ctc      720
   Gln Gly Thr Ser Glu Glu Asn Thr Phe Tyr Ser Trp Leu Glu Gly Leu
   225                 230                 235                 240
   tgt gta gaa aaa aga gca ttc tac aga ctt ata tct ggc cta cat gca      768
   Cys Val Glu Lys Arg Ala Phe Tyr Arg Leu Ile Ser Gly Leu His Ala
                   245                 250                 255
   agc att aat gtg cat ttg agt gca aga tat ctt tta caa gag acc tgg      816
   Ser Ile Asn Val His Leu Ser Ala Arg Tyr Leu Leu Gln Glu Thr Trp
               260                 265                 270
   tta gaa aag aaa tgg gga cac aac att aca gaa ttt caa cag cga ttt      864
   Leu Glu Lys Lys Trp Gly His Asn Ile Thr Glu Phe Gln Gln Arg Phe
           275                 280                 285
   gat gga att ttg act gaa gga gaa ggt cca aga agg ctt aag aac ttg      912
   Asp Gly Ile Leu Thr Glu Gly Glu Gly Pro Arg Arg Leu Lys Asn Leu
       290                 295                 300
   tat ttt ctc tac tta ata gaa cta agg gct tta tcc aaa gtg tta cca      960
   Tyr Phe Leu Tyr Leu Ile Glu Leu Arg Ala Leu Ser Lys Val Leu Pro
   305                 310                 315                 320
   ttc ttc gag cgc cca gat ttt caa ctc ttt act gga aat aaa att cag     1008
   Phe Phe Glu Arg Pro Asp Phe Gln Leu Phe Thr Gly Asn Lys Ile Gln
                   325                 330                 335
   gat gag gaa aac aaa atg tta ctt ctg gaa ata ctt cat gaa atc aag     1056
   Asp Glu Glu Asn Lys Met Leu Leu Leu Glu Ile Leu His Glu Ile Lys
               340                 345                 350
   tca ttt cct ttg cat ttt gat gag aat tca ttt ttt gct ggg gat aaa     1104
   Ser Phe Pro Leu His Phe Asp Glu Asn Ser Phe Phe Ala Gly Asp Lys
           355                 360                 365
   aaa gaa gca cac aaa cta aag gag gac ttt cga ctg cat ttt aga aat     1152
   Lys Glu Ala His Lys Leu Lys Glu Asp Phe Arg Leu His Phe Arg Asn
       370                 375                 380
   att tca aga att atg gat tgt gtt ggt tgt ttt aaa tgt cgt ctg tgg     1200
   Ile Ser Arg Ile Met Asp Cys Val Gly Cys Phe Lys Cys Arg Leu Trp
   385                 390                 395                 400
   gga aag ctt cag act cag ggt ttg ggc act gct ctg aag atc tta ttt     1248
   Gly Lys Leu Gln Thr Gln Gly Leu Gly Thr Ala Leu Lys Ile Leu Phe
                   405                 410                 415
   tct gag aaa ttg ata gca aat atg cca gaa agt gga cct agt tat gaa     1296
   Ser Glu Lys Leu Ile Ala Asn Met Pro Glu Ser Gly Pro Ser Tyr Glu
               420                 425                 430
   ttc cat cta acc aga caa gaa ata gta tca tta ttc aac gca ttt gga     1344
   Phe His Leu Thr Arg Gln Glu Ile Val Ser Leu Phe Asn Ala Phe Gly
           435                 440                 445
   aga att tct aca agt gtg aaa gaa tta gaa aac ttc agg aac ttg tta     1392
   Arg Ile Ser Thr Ser Val Lys Glu Leu Glu Asn Phe Arg Asn Leu Leu
       450                 455                 460
   cag aat att cat taa                                                 1407
   Gln Asn Ile His
   465
<210> 72
   <211> 468
   <212> PRT
   <213> Homo sapiens
<400> 72
   Met Gly Arg Gly Trp Gly Phe Leu Phe Gly Leu Leu Gly Ala Val Trp
   1               5                   10                  15
   Leu Leu Ser Ser Gly His Gly Glu Glu Gln Pro Pro Glu Thr Ala Ala
               20                  25                  30
   Gln Arg Cys Phe Cys Gln Val Ser Gly Tyr Leu Asp Asp Cys Thr Cys
           35                  40                  45
   Asp Val Glu Thr Ile Asp Arg Phe Asn Asn Tyr Arg Leu Phe Pro Arg
       50                  55                  60
   Leu Gln Lys Leu Leu Glu Ser Asp Tyr Phe Arg Tyr Tyr Lys Val Asn
   65                  70                  75                  80
   Leu Lys Arg Pro Cys Pro Phe Trp Asn Asp Ile Ser Gln Cys Gly Arg
                   85                  90                  95
   Arg Asp Cys Ala Val Lys Pro Cys Gln Ser Asp Glu Val Pro Asp Gly
               100                 105                 110
   Ile Lys Ser Ala Ser Tyr Lys Tyr Ser Glu Glu Ala Asn Asn Leu Ile
           115                 120                 125
   Glu Glu Cys Glu Gln Ala Glu Arg Leu Gly Ala Val Asp Glu Ser Leu
       130                 135                 140
   Ser Glu Glu Thr Gln Lys Ala Val Leu Gln Trp Thr Lys His Asp Asp
   145                 150                 155                 160
   Ser Ser Asp Asn Phe Cys Glu Ala Asp Asp Ile Gln Ser Pro Glu Ala
                   165                 170                 175
   Glu Tyr Val Asp Leu Leu Leu Asn Pro Glu Arg Tyr Thr Gly Tyr Lys
               180                 185                 190
   Gly Pro Asp Ala Trp Lys Ile Trp Asn Val Ile Tyr Glu Glu Asn Cys
           195                 200                 205
   Phe Lys Pro Gln Thr Ile Lys Arg Pro Leu Asn Pro Leu Ala Ser Gly
       210                 215                 220
   Gln Gly Thr Ser Glu Glu Asn Thr Phe Tyr Ser Trp Leu Glu Gly Leu
   225                 230                 235                 240
   Cys Val Glu Lys Arg Ala Phe Tyr Arg Leu Ile Ser Gly Leu His Ala
                   245                 250                 255
   Ser Ile Asn Val His Leu Ser Ala Arg Tyr Leu Leu Gln Glu Thr Trp
               260                 265                 270
   Leu Glu Lys Lys Trp Gly His Asn Ile Thr Glu Phe Gln Gln Arg Phe
           275                 280                 285
   Asp Gly Ile Leu Thr Glu Gly Glu Gly Pro Arg Arg Leu Lys Asn Leu
       290                 295                 300
   Tyr Phe Leu Tyr Leu Ile Glu Leu Arg Ala Leu Ser Lys Val Leu Pro
   305                 310                 315                 320
   Phe Phe Glu Arg Pro Asp Phe Gln Leu Phe Thr Gly Asn Lys Ile Gln
                   325                 330                 335
   Asp Glu Glu Asn Lys Met Leu Leu Leu Glu Ile Leu His Glu Ile Lys
               340                 345                 350
   Ser Phe Pro Leu His Phe Asp Glu Asn Ser Phe Phe Ala Gly Asp Lys
           355                 360                 365
   Lys Glu Ala His Lys Leu Lys Glu Asp Phe Arg Leu His Phe Arg Asn
       370                 375                 380
   Ile Ser Arg Ile Met Asp Cys Val Gly Cys Phe Lys Cys Arg Leu Trp
   385                 390                 395                 400
   Gly Lys Leu Gln Thr Gln Gly Leu Gly Thr Ala Leu Lys Ile Leu Phe
                   405                 410                 415
   Ser Glu Lys Leu Ile Ala Asn Met Pro Glu Ser Gly Pro Ser Tyr Glu
               420                 425                 430
   Phe His Leu Thr Arg Gln Glu Ile Val Ser Leu Phe Asn Ala Phe Gly
           435                 440                 445
   Arg Ile Ser Thr Ser Val Lys Glu Leu Glu Asn Phe Arg Asn Leu Leu
       450                 455                 460
   Gln Asn Ile His
   465
<210> 73
   <211> 1404
   <212> DNA
   <213> Homo sapiens
<220>
   <221> CDS
   <222> (1)..(1404)
<400> 73
   atg agc caa ggg gtc cgc cgg gca ggc gct ggg cag ggg gta gcg gcc       48
   Met Ser Gln Gly Val Arg Arg Ala Gly Ala Gly Gln Gly Val Ala Ala
   1               5                   10                  15
   gcg gtg cag ctg ctg gtc acc ctg agc ttc ctg cgg agc gtc gtc gag       96
   Ala Val Gln Leu Leu Val Thr Leu Ser Phe Leu Arg Ser Val Val Glu
               20                  25                  30
   gcg cag gtc act gga gtt ctg gat gat tgc ttg tgt gat att gac agc      144
   Ala Gln Val Thr Gly Val Leu Asp Asp Cys Leu Cys Asp Ile Asp Ser
           35                  40                  45
   atc gat aac ttc aat acc tac aaa atc ttc ccc aaa ata aaa aaa ttg      192
   Ile Asp Asn Phe Asn Thr Tyr Lys Ile Phe Pro Lys Ile Lys Lys Leu
       50                  55                  60
   caa gag aga gac tat ttt cgt tat tac aag gtt aat ctg aag cga cct      240
   Gln Glu Arg Asp Tyr Phe Arg Tyr Tyr Lys Val Asn Leu Lys Arg Pro
   65                  70                  75                  80
   tgt cct ttc tgg gca gaa gat ggc cac tgt tca ata aaa gac tgt cat      288
   Cys Pro Phe Trp Ala Glu Asp Gly His Cys Ser Ile Lys Asp Cys His
                   85                  90                  95
   gtg gag ccc tgt cca gag agt aaa att ccg gtt gga ata aaa gct ggg      336
   Val Glu Pro Cys Pro Glu Ser Lys Ile Pro Val Gly Ile Lys Ala Gly
               100                 105                 110
   cat tct aat aag tac ttg aaa atg gca aac aat acc aaa gaa tta gaa      384
   His Ser Asn Lys Tyr Leu Lys Met Ala Asn Asn Thr Lys Glu Leu Glu
           115                 120                 125
   gtt tgt gag caa gct aat aaa ctg gga gca att aac agc aca tta agt      432
   Val Cys Glu Gln Ala Asn Lys Leu Gly Ala Ile Asn Ser Thr Leu Ser
       130                 135                 140
   aat caa agc aaa gaa gct ttc att gac tgg gca aga tat gat gat tca      480
   Asn Gln Ser Lys Glu Ala Phe Ile Asp Trp Ala Arg Tyr Asp Asp Ser
   145                 150                 155                 160
   cgg gat cac ttt tgt gaa ctt gat gat gag aga tct cca gct gct cag      528
   Arg Asp His Phe Cys Glu Leu Asp Asp Glu Arg Ser Pro Ala Ala Gln
                   165                 170                 175
   tat gta gac cta ttg ctg aac cca gag cgt tac act ggc tat aaa ggg      576
   Tyr Val Asp Leu Leu Leu Asn Pro Glu Arg Tyr Thr Gly Tyr Lys Gly
               180                 185                 190
   acc tct gca tgg aga gtg tgg aac agc atc tat gaa gag aac tgt ttc      624
   Thr Ser Ala Trp Arg Val Trp Asn Ser Ile Tyr Glu Glu Asn Cys Phe
           195                 200                 205
   aag cct cga tct gtt tat cgt cct tta aat cct ctg gcg cct agc cga      672
   Lys Pro Arg Ser Val Tyr Arg Pro Leu Asn Pro Leu Ala Pro Ser Arg
       210                 215                 220
   ggc gaa gat gat gga gaa tca ttc tac aca tgg cta gaa ggt ttg tgt      720
   Gly Glu Asp Asp Gly Glu Ser Phe Tyr Thr Trp Leu Glu Gly Leu Cys
   225                 230                 235                 240
   ctg gag aaa aga gtc ttc tat aag ctt ata tcg gga ctt cat gct agc      768
   Leu Glu Lys Arg Val Phe Tyr Lys Leu Ile Ser Gly Leu His Ala Ser
                   245                 250                 255
   atc aat tta cat cta tgc gca aat tat ctt ttg gaa gaa acc tgg ggt      816
   Ile Asn Leu His Leu Cys Ala Asn Tyr Leu Leu Glu Glu Thr Trp Gly
               260                 265                 270
   aag ccc agt tgg gga cct aat att aaa gaa ttc aaa cac cgc ttt gac      864
   Lys Pro Ser Trp Gly Pro Asn Ile Lys Glu Phe Lys His Arg Phe Asp
           275                 280                 285
   cct gtg gaa acc aag gga gaa ggt cca aga agg ctc aag aat ctt tac      912
   Pro Val Glu Thr Lys Gly Glu Gly Pro Arg Arg Leu Lys Asn Leu Tyr
       290                 295                 300
   ttt tta tac ttg att gag ctt cga gct ttg tca aag gtg gct cca tat      960
   Phe Leu Tyr Leu Ile Glu Leu Arg Ala Leu Ser Lys Val Ala Pro Tyr
   305                 310                 315                 320
   ttt gag cgc tca att gtc gat ctt tac act gga aat gca gaa gaa gat     1008
   Phe Glu Arg Ser Ile Val Asp Leu Tyr Thr Gly Asn Ala Glu Glu Asp
                   325                 330                 335
   gct gac aca aaa act ctt cta ctg aat atc ttt caa gat aca aag tcc     1056
   Ala Asp Thr Lys Thr Leu Leu Leu Asn Ile Phe Gln Asp Thr Lys Ser
                   340                 345                 350
   ttt ccc atg cac ttt gat gag aaa tcc atg ttt gca ggt gac aaa aaa      1104
   Phe Pro Met His Phe Asp Glu Lys Ser Met Phe Ala Gly Asp Lys Lys
               355                 360                 365
   ggg gcc aag tca cta aag gag gaa ttc cga tta cat ttc aag aat atc      1152
   Gly Ala Lys Ser Leu Lys Glu Glu Phe Arg Leu His Phe Lys Asn Ile
       370                 375                 380
   tcc cgt ata atg gac tgt gtt gga tgt gac aaa tgc aga tta tgg gga      1200
   Ser Arg Ile Met Asp Cys Val Gly Cys Asp Lys Cys Arg Leu Trp Gly
   385                 390                 395                 400
   aaa tta cag act cag ggt tta gga act gcc ctg aag ata tta ttc tct      1248
   Lys Leu Gln Thr Gln Gly Leu Gly Thr Ala Leu Lys Ile Leu Phe Ser
                   405                 410                 415
   gaa aaa gaa atc caa aag ctt cca gag aat agt cca tct aaa ggc ttc      1296
   Glu Lys Glu Ile Gln Lys Leu Pro Glu Asn Ser Pro Ser Lys Gly Phe
               420                 425                 430
   caa ctc acc cga cag gaa ata gtt gct ctt tta aat gct ttt gga agg      1344
   Gln Leu Thr Arg Gln Glu Ile Val Ala Leu Leu Asn Ala Phe Gly Arg
           435                 440                 445
   ctt tct aca agt ata aga gac tta cag aat ttt aaa gtc tta tta caa      1392
   Leu Ser Thr Ser Ile Arg Asp Leu Gln Asn Phe Lys Val Leu Leu Gln
       450                 455                 460
   cac agt agg taa                                                      1404
   His Ser Arg
   465
<210> 74
   <211> 467
   <212> PRT
   <213> Homo sapiens
<400> 74
   Met Ser Gln Gly Val Arg Arg Ala Gly Ala Gly Gln Gly Val Ala Ala
   1               5                   10                  15
   Ala Val Gln Leu Leu Val Thr Leu Ser Phe Leu Arg Ser Val Val Glu
               20                  25                  30
   Ala Gln Val Thr Gly Val Leu Asp Asp Cys Leu Cys Asp Ile Asp Ser
           35                  40                  45
   Ile Asp Asn Phe Asn Thr Tyr Lys Ile Phe Pro Lys Ile Lys Lys Leu
       50                  55                  60
   Gln Glu Arg Asp Tyr Phe Arg Tyr Tyr Lys Val Asn Leu Lys Arg Pro
   65                  70                  75                  80
   Cys Pro Phe Trp Ala Glu Asp Gly His Cys Ser Ile Lys Asp Cys His
   85 90 95
   Val Glu Pro Cys Pro Glu Ser Lys Ile Pro Val Gly Ile Lys Ala Gly
   100 105 110
   His Ser Asn Lys Tyr Leu Lys Met Ala Asn Asn Thr Lys Glu Leu Glu
   115 120 125
   Val Cys Glu Gln Ala Asn Lys Leu Gly Ala Ile Asn Ser Thr Leu Ser
   130 135 140
   Asn Gln Ser Lys Glu Ala Phe Ile Asp Trp Ala Arg Tyr Asp Asp Ser
   145 150 155 160
   Arg Asp His Phe Cys Glu Leu Asp Asp Glu Arg Ser Pro Ala Ala Gln
   165 170 175
   Tyr Val Asp Leu Leu Leu Asn Pro Glu Arg Tyr Thr Gly Tyr Lys Gly
   180 185 190
   Thr Ser Ala Trp Arg Val Trp Asn Ser Ile Tyr Glu Glu Asn Cys Phe
   195 200 205
   Lys Pro Arg Ser Val Tyr Arg Pro Leu Asn Pro Leu Ala Pro Ser Arg
   210 215 220
   Gly Glu Asp Asp Gly Glu Ser Phe Tyr Thr Trp Leu Glu Gly Leu Cys
   225 230 235 240
   Leu Glu Lys Arg Val Phe Tyr Lys Leu Ile Ser Gly Leu His Ala Ser
   245 250 255
   Ile Asn Leu His Leu Cys Ala Asn Tyr Leu Leu Glu Glu Thr Trp Gly
   260 265 270
   Lys Pro Ser Trp Gly Pro Asn Ile Lys Glu Phe Lys His Arg Phe Asp
   275 280 285
   Pro Val Glu Thr Lys Gly Glu Gly Pro Arg Arg Leu Lys Asn Leu Tyr
   290 295 300
   Phe Leu Tyr Leu Ile Glu Leu Arg Ala Leu Ser Lys Val Ala Pro Tyr
   305 310 315 320
   Phe Glu Arg Ser Ile Val Asp Leu Tyr Thr Gly Asn Ala Glu Glu Asp
   325 330 335
   Ala Asp Thr Lys Thr Leu Leu Leu Asn Ile Phe Gln Asp Thr Lys Ser
               340                 345                 350
   Phe Pro Met His Phe Asp Glu Lys Ser Met Phe Ala Gly Asp Lys Lys
           355                 360                 365
   Gly Ala Lys Ser Leu Lys Glu Glu Phe Arg Leu His Phe Lys Asn Ile
       370                 375                 380
   Ser Arg Ile Met Asp Cys Val Gly Cys Asp Lys Cys Arg Leu Trp Gly
   385                 390                 395                 400
   Lys Leu Gln Thr Gln Gly Leu Gly Thr Ala Leu Lys Ile Leu Phe Ser
                   405                 410                 415
   Glu Lys Glu Ile Gln Lys Leu Pro Glu Asn Ser Pro Ser Lys Gly Phe
               420                 425                 430
   Gln Leu Thr Arg Gln Glu Ile Val Ala Leu Leu Asn Ala Phe Gly Arg
           435                 440                 445
   Leu Ser Thr Ser Ile Arg Asp Leu Gln Asn Phe Lys Val Leu Leu Gln
       450                 455                 460
   His Ser Arg
   465
<210> 75
   <211> 1965
   <212> DNA
   <213> Homo sapiens
<220>
   <221> CDS
   <222> (1)..(1965)
<400> 75
   atg aag ctc tcc ctg gtg gcc gcg atg ctg ctg ctg ctc agc gcg gcg      48
   Met Lys Leu Ser Leu Val Ala Ala Met Leu Leu Leu Leu Ser Ala Ala
   1               5                   10                  15
   cgg gcc gag gag gag gac aag aag gag gac gtg ggc acg gtg gtc ggc      96
   Arg Ala Glu Glu Glu Asp Lys Lys Glu Asp Val Gly Thr Val Val Gly
               20                  25                  30
   atc gac ctg ggg acc acc tac tcc tgc gtc ggc gtg ttc aag aac ggc     144
   Ile Asp Leu Gly Thr Thr Tyr Ser Cys Val Gly Val Phe Lys Asn Gly
           35                  40                  45
   cgc gtg gag atc atc gcc aac gat cag ggc aac cgc atc acg ccg tcc     192
   Arg Val Glu Ile Ile Ala Asn Asp Gln Gly Asn Arg Ile Thr Pro Ser
       50                  55                  60
   tat gtc gcc ttc act cct gaa ggg gaa cgt ctg att ggc gat gcc gcc      240
   Tyr Val Ala Phe Thr Pro Glu Gly Glu Arg Leu Ile Gly Asp Ala Ala
   65                  70                  75                  80
   aag aac cag ctc acc tcc aac ccc gag aac acg gtc ttt gac gcc aag      288
   Lys Asn Gln Leu Thr Ser Asn Pro Glu Asn Thr Val Phe Asp Ala Lys
                   85                  90                  95
   cgg ctc atc ggc cgc acg tgg aat gac ccg tct gtg cag cag gac atc      336
   Arg Leu Ile Gly Arg Thr Trp Asn Asp Pro Ser Val Gln Gln Asp Ile
               100                 105                 110
   aag ttc ttg ccg ttc aag gtg gtt gaa aag aaa act aaa cca tac att      384
   Lys Phe Leu Pro Phe Lys Val Val Glu Lys Lys Thr Lys Pro Tyr Ile
           115                 120                 125
   caa gtt gat att gga ggt ggg caa aca aag aca ttt gct cct gaa gaa      432
   Gln Val Asp Ile Gly Gly Gly Gln Thr Lys Thr Phe Ala Pro Glu Glu
       130                 135                 140
   att tct gcc atg gtt ctc act aaa atg aaa gaa acc gct gag gct tat      480
   Ile Ser Ala Met Val Leu Thr Lys Met Lys Glu Thr Ala Glu Ala Tyr
   145                 150                 155                 160
   ttg gga aag aag gtt acc cat gca gtt gtt act gta cca gcc tat ttt      528
   Leu Gly Lys Lys Val Thr His Ala Val Val Thr Val Pro Ala Tyr Phe
                   165                 170                 175
   aat gat gcc caa cgc caa gca acc aaa gac gct gga act att gct ggc      576
   Asn Asp Ala Gln Arg Gln Ala Thr Lys Asp Ala Gly Thr Ile Ala Gly
               180                 185                 190
   cta aat gtt atg agg atc atc aac gag cct acg gca gct gct att gct      624
   Leu Asn Val Met Arg Ile Ile Asn Glu Pro Thr Ala Ala Ala Ile Ala
           195                 200                 205
   tat ggc ctg gat aag agg gag ggg gag aag aac atc ctg gtg ttt gac      672
   Tyr Gly Leu Asp Lys Arg Glu Gly Glu Lys Asn Ile Leu Val Phe Asp
       210                 215                 220
   ctg ggt ggc gga acc ttc gat gtg tct ctt ctc acc att gac aat ggt      720
   Leu Gly Gly Gly Thr Phe Asp Val Ser Leu Leu Thr Ile Asp Asn Gly
   225                 230                 235                 240
   gtc ttc gaa gtt gtg gcc act aat gga gat act cat ctg ggt gga gaa      768
   Val Phe Glu Val Val Ala Thr Asn Gly Asp Thr His Leu Gly Gly Glu
                   245                 250                 255
   gac ttt gac cag cgt gtc atg gaa cac ttc atc aaa ctg tac aaa aag      816
   Asp Phe Asp Gln Arg Val Met Glu His Phe Ile Lys Leu Tyr Lys Lys
               260                 265                 270
   aag acg ggc aaa gat gtc agg aaa gac aat aga gct gtg cag aaa ctc      864
   Lys Thr Gly Lys Asp Val Arg Lys Asp Asn Arg Ala Val Gln Lys Leu
           275                 280                 285
   cgg cgc gag gta gaa aag gcc aaa cgg gcc ctg tct tct cag cat caa      912
   Arg Arg Glu Val Glu Lys Ala Lys Arg Ala Leu Ser Ser Gln His Gln
       290                 295                 300
   gca aga att gaa att gag tcc ttc tat gaa gga gaa gac ttt tct gag      960
   Ala Arg Ile Glu Ile Glu Ser Phe Tyr Glu Gly Glu Asp Phe Ser Glu
   305                 310                 315                 320
   acc ctg act cgg gcc aaa ttt gaa gag ctc aac atg gat ctg ttc cgg     1008
   Thr Leu Thr Arg Ala Lys Phe Glu Glu Leu Asn Met Asp Leu Phe Arg
                   325                 330                 335
   tct act atg aag ccc gtc cag aaa gtg ttg gaa gat tct gat ttg aag     1056
   Ser Thr Met Lys Pro Val Gln Lys Val Leu Glu Asp Ser Asp Leu Lys
               340                 345                 350
   aag tct gat att gat gaa att gtt ctt gtt ggt ggc tcg act cga att     1104
   Lys Ser Asp Ile Asp Glu Ile Val Leu Val Gly Gly Ser Thr Arg Ile
           355                 360                 365
   cca aag att cag caa ctg gtt aaa gag ttc ttc aat ggc aag gaa cca     1152
   Pro Lys Ile Gln Gln Leu Val Lys Glu Phe Phe Asn Gly Lys Glu Pro
       370                 375                 380
   tcc cgt ggc ata aac cca gat gaa gct gta gcg tat ggt gct gct gtc     1200
   Ser Arg Gly Ile Asn Pro Asp Glu Ala Val Ala Tyr Gly Ala Ala Val
   385                 390                 395                 400
   cag gct ggt gtg ctc tct ggt gat caa gat aca ggt gac ctg gta ctg     1248
   Gln Ala Gly Val Leu Ser Gly Asp Gln Asp Thr Gly Asp Leu Val Leu
                   405                 410                 415
   ctt gat gta tgt ccc ctt aca ctt ggt att gaa act gtg gga ggt gtc     1296
   Leu Asp Val Cys Pro Leu Thr Leu Gly Ile Glu Thr Val Gly Gly Val
               420                 425                 430
   atg acc aaa ctg att cca agg aac aca gtg gtg cct acc aag aag tct     1344
   Met Thr Lys Leu Ile Pro Arg Asn Thr Val Val Pro Thr Lys Lys Ser
           435                 440                 445
   cag atc ttt tct aca gct tct gat aat caa cca act gtt aca atc aag     1392
   Gln Ile Phe Ser Thr Ala Ser Asp Asn Gln Pro Thr Val Thr Ile Lys
       450                 455                 460
   gtc tat gaa ggt gaa aga ccc ctg aca aaa gac aat cat ctt ctg ggt     1440
   Val Tyr Glu Gly Glu Arg Pro Leu Thr Lys Asp Asn His Leu Leu Gly
   465                 470                 475                 480
   aca ttt gat ctg act gga att cct cct gct cct cgt ggg gtc cca cag     1488
   Thr Phe Asp Leu Thr Gly Ile Pro Pro Ala Pro Arg Gly Val Pro Gln
                   485                 490                 495
   att gaa gtc acc ttt gag ata gat gtg aat ggt att ctt cga gtg aca     1536
   Ile Glu Val Thr Phe Glu Ile Asp Val Asn Gly Ile Leu Arg Val Thr
               500                 505                 510
   gct gaa gac aag ggt aca ggg aac aaa aat aag atc aca atc acc aat     1584
   Ala Glu Asp Lys Gly Thr Gly Asn Lys Asn Lys Ile Thr Ile Thr Asn
           515                 520                 525
   gac cag aat cgc ctg aca cct gaa gaa atc gaa agg atg gtt aat gat     1632
   Asp Gln Asn Arg Leu Thr Pro Glu Glu Ile Glu Arg Met Val Asn Asp
       530                 535                 540
   gct gag aag ttt gct gag gaa gac aaa aag ctc aag gag cgc att gat     1680
   Ala Glu Lys Phe Ala Glu Glu Asp Lys Lys Leu Lys Glu Arg Ile Asp
   545                 550                 555                 560
   act aga aat gag ttg gaa agc tat gcc tat tct cta aag aat cag att     1728
   Thr Arg Asn Glu Leu Glu Ser Tyr Ala Tyr Ser Leu Lys Asn Gln Ile
                   565                 570                 575
   gga gat aaa gaa aag ctg gga ggt aaa ctt tcc tct gaa gat aag gag      1776
   Gly Asp Lys Glu Lys Leu Gly Gly Lys Leu Ser Ser Glu Asp Lys Glu
               580                 585                 590
   acc atg gaa aaa gct gta gaa gaa aag att gaa tgg ctg gaa agc cac      1824
   Thr Met Glu Lys Ala Val Glu Glu Lys Ile Glu Trp Leu Glu Ser His
           595                 600                 605
   caa gat gct gac att gaa gac ttc aaa gct aag aag aag gaa ctg gaa      1872
   Gln Asp Ala Asp Ile Glu Asp Phe Lys Ala Lys Lys Lys Glu Leu Glu
       610                 615                 620
   gaa att gtt caa cca att atc agc aaa ctc tat gga agt gca ggc cct      1920
   Glu Ile Val Gln Pro Ile Ile Ser Lys Leu Tyr Gly Ser Ala Gly Pro
   625                 630                 635                 640
   ccc cca act ggt gaa gag gat aca gca gaa aaa gat gag ttg tag          1965
   Pro Pro Thr Gly Glu Glu Asp Thr Ala Glu Lys Asp Glu Leu
                   645                 650
<210> 76
   <211> 654
   <212> PRT
   <213> Homo sapiens
<400> 76
   Met Lys Leu Ser Leu Val Ala Ala Met Leu Leu Leu Leu Ser Ala Ala
   1               5                   10                  15
   Arg Ala Glu Glu Glu Asp Lys Lys Glu Asp Val Gly Thr Val Val Gly
               20                  25                  30
   Ile Asp Leu Gly Thr Thr Tyr Ser Cys Val Gly Val Phe Lys Asn Gly
           35                  40                  45
   Arg Val Glu Ile Ile Ala Asn Asp Gln Gly Asn Arg Ile Thr Pro Ser
       50                  55                  60
   Tyr Val Ala Phe Thr Pro Glu Gly Glu Arg Leu Ile Gly Asp Ala Ala
   65                  70                  75                  80
   Lys Asn Gln Leu Thr Ser Asn Pro Glu Asn Thr Val Phe Asp Ala Lys
                   85                  90                  95
   Arg Leu Ile Gly Arg Thr Trp Asn Asp Pro Ser Val Gln Gln Asp Ile
               100                 105                 110
   Lys Phe Leu Pro Phe Lys Val Val Glu Lys Lys Thr Lys Pro Tyr Ile
           115                 120                 125
   Gln Val Asp Ile Gly Gly Gly Gln Thr Lys Thr Phe Ala Pro Glu Glu
   130 135 140
   Ile Ser Ala Met Val Leu Thr Lys Met Lys Glu Thr Ala Glu Ala Tyr
   145 150 155 160
   Leu Gly Lys Lys Val Thr His Ala Val Val Thr Val Pro Ala Tyr Phe
   165 170 175
   Asn Asp Ala Gln Arg Gln Ala Thr Lys Asp Ala Gly Thr Ile Ala Gly
   180 185 190
   Leu Asn Val Met Arg Ile Ile Asn Glu Pro Thr Ala Ala Ala Ile Ala
   195 200 205
   Tyr Gly Leu Asp Lys Arg Glu Gly Glu Lys Asn Ile Leu Val Phe Asp
   210 215 220
   Leu Gly Gly Gly Thr Phe Asp Val Ser Leu Leu Thr Ile Asp Asn Gly
   225 230 235 240
   Val Phe Glu Val Val Ala Thr Asn Gly Asp Thr His Leu Gly Gly Glu
   245 250 255
   Asp Phe Asp Gln Arg Val Met Glu His Phe Ile Lys Leu Tyr Lys Lys
   260 265 270
   Lys Thr Gly Lys Asp Val Arg Lys Asp Asn Arg Ala Val Gln Lys Leu
   275 280 285
   Arg Arg Glu Val Glu Lys Ala Lys Arg Ala Leu Ser Ser Gln His Gln
   290 295 300
   Ala Arg Ile Glu Ile Glu Ser Phe Tyr Glu Gly Glu Asp Phe Ser Glu
   305 310 315 320
   Thr Leu Thr Arg Ala Lys Phe Glu Glu Leu Asn Met Asp Leu Phe Arg
   325 330 335
   Ser Thr Met Lys Pro Val Gln Lys Val Leu Glu Asp Ser Asp Leu Lys
   340 345 350
   Lys Ser Asp Ile Asp Glu Ile Val Leu Val Gly Gly Ser Thr Arg Ile
   355 360 365
   Pro Lys Ile Gln Gln Leu Val Lys Glu Phe Phe Asn Gly Lys Glu Pro
   370 375 380
   Ser Arg Gly Ile Asn Pro Asp Glu Ala Val Ala Tyr Gly Ala Ala Val
   385                 390                 395                 400
   Gln Ala Gly Val Leu Ser Gly Asp Gln Asp Thr Gly Asp Leu Val Leu
                   405                 410                 415
   Leu Asp Val Cys Pro Leu Thr Leu Gly Ile Glu Thr Val Gly Gly Val
               420                 425                 430
   Met Thr Lys Leu Ile Pro Arg Asn Thr Val Val Pro Thr Lys Lys Ser
           435                 440                 445
   Gln Ile Phe Ser Thr Ala Ser Asp Asn Gln Pro Thr Val Thr Ile Lys
       450                 455                 460
   Val Tyr Glu Gly Glu Arg Pro Leu Thr Lys Asp Asn His Leu Leu Gly
   465                 470                 475                 480
   Thr Phe Asp Leu Thr Gly Ile Pro Pro Ala Pro Arg Gly Val Pro Gln
                   485                 490                 495
   Ile Glu Val Thr Phe Glu Ile Asp Val Asn Gly Ile Leu Arg Val Thr
               500                 505                 510
   Ala Glu Asp Lys Gly Thr Gly Asn Lys Asn Lys Ile Thr Ile Thr Asn
           515                 520                 525
   Asp Gln Asn Arg Leu Thr Pro Glu Glu Ile Glu Arg Met Val Asn Asp
       530                 535                 540
   Ala Glu Lys Phe Ala Glu Glu Asp Lys Lys Leu Lys Glu Arg Ile Asp
   545                 550                 555                 560
   Thr Arg Asn Glu Leu Glu Ser Tyr Ala Tyr Ser Leu Lys Asn Gln Ile
                   565                 570                 575
   Gly Asp Lys Glu Lys Leu Gly Gly Lys Leu Ser Ser Glu Asp Lys Glu
               580                 585                 590
   Thr Met Glu Lys Ala Val Glu Glu Lys Ile Glu Trp Leu Glu Ser His
           595                 600                 605
   Gln Asp Ala Asp Ile Glu Asp Phe Lys Ala Lys Lys Lys Glu Leu Glu
       610                 615                 620
   Glu Ile Val Gln Pro Ile Ile Ser Lys Leu Tyr Gly Ser Ala Gly Pro
   625                 630                 635                 640
   Pro Pro Thr Gly Glu Glu Asp Thr Ala Glu Lys Asp Glu Leu
                   645                 650